# EUROPEAN PATENT SPECIFICATION

(11) **EP 1 270 728 B1**
(45) Date of publication and mention of the grant of the patent: **18.04.2007**
(21) Application number: 01906278.5
(22) Date of filing: 23.02.2001
(51) Int. Cl.: C12P 19/62, C12N 9/00, C12N 15/52, C07C 327/30, C12R 1/465

(54) **PROCESS FOR PRODUCING AVERMECTIN DERIVATIVE**
VERFAHREN ZUR HERSTELLUNG VON AVERMECTINDERIVATEN
PROCEDE DE PRODUCTION D'UN DERIVE D'AVERMECTINE

(30) Priority: 24.02.2000 JP 2000047405
(43) Date of publication of application: 02.01.2003
(73) Proprietor: KYOWA HAKKO KOGYO CO., LTD., Chiyoda-ku, Tokyo 100-8185 (JP); THE KITASATO INSTITUTE, Tokyo 108-8641 (JP)
(72) Inventor: ENDO, Hirofumi, Kyowa Hakko Kogyo Co. Ltd., Tokyo 100-8185 (JP); YAMAGUCHI, Hiroyuki, Tokyo Res. Lab. Kyowa Hakko, Machida-shi, Tokyo 194-8533 (JP); KANDA, Yutaka, Pharm. Res. Inst. Kyowa Hakko, Sunto-gun, Shizuoka 411-8731 (JP); HASHIMOTO, Shinichi, Tokyo Res. Lab. Kyowa Hakko, Machida-shi, Tokyo 194-8533 (JP); OMURA, Satoshi, Setagaya-ku, Tokyo 157-0076 (JP); IKEDA, Haruo, Kawasaki-shi, Kanagawa 212-0007 (JP)
(74) Representative: Vossius & Partner
(86) International application number: PCT/JP2001/001381
(87) International publication number: WO 2001/062939

(56) References cited:
- EP-A1- 0 997 528
- EP-A2- 0 391 594
- WO-A-00/01827
- WO-A-99/03986
- WO-A1-00/50605
- JP-A- 6 189 774
- DATABASE EMBL 20 September 1999 (1999-09-20), "Streptomyces avermitilis polyketide synthase gene cluster (ave A1, aveA2, aveA3, aveA4) and aveC, aveE genes, complete cds" XP002294483 Database accession no. AB032367 -& IKEDA H ET AL: "ORGANIZATION OF THE BIOSYNTHETIC GENE CLUSTER FOR THE POLYKETIDE ANTHELMINTIC MACROLIDE AVERMECTIN IN STREPTOMYCES AVERMITILIS" PROCEEDINGS OF THE NATIONAL ACADEMY OF SCIENCES OF USA, NATIONAL ACADEMY OF SCIENCE. WASHINGTON, US, vol. 96, August 1999 (1999-08), pages 9509-9514, XP002928739 ISSN: 0027-8424
- DUTTON CHRISTOPHER J ET AL: "Avermectin biosynthesis: Intact incorporation of a diketide chain-assembly intermediate into the polyketide macrocyclic ring" TETRAHEDRON LETTERS, vol. 35, no. 2, 1994, pages 327-330, XP002294482 ISSN: 0040-4039
- A.F.A. MARSDEN ET AL.: 'Engineering broader specificity into an antibiotic-producing polyketide synthase' SCIENCE vol. 279, no. 5348, 1998, pages 199 - 202, XP002941701
- H. IKEDA ET AL.: 'Avermectin biosynthesis' CHEM. REV. vol. 97, no. 7, 1997, pages 2591 - 2609, XP002941702
- H. IKEDA ET AL.: 'Strategic strain improvement of antibiotic producer' ACTINOMYCETOLOGICA vol. 5, no. 2, 1991, pages 86 - 99, XP002941703
- Y.S. TSANTRIZOS ET AL.: 'Biosynthesis of the hypotensive metabolite oudenone by oudemansiella radicata. 1. Intact incorporation of a tetraketide chain elongation intermediate' J. ORG. CHEM. vol. 60, no. 21, 1995, pages 6922 - 6929, XP002941704
- Y. LIU ET AL.: 'Biosynthetic incorporation of advanced precursors into dehydrocurvularin, a polyketide phytotoxin from alternaria cinerariae' TETRAHEDRON vol. 54, no. 52, 1998, pages 15937 - 15958, XP002941705

## Description

### Technical Field

The present invention relates to a process for producing 22,23-dihydroavermectin B1a or a derivative thereof, which is useful as a medicine, a substrate compound and a modified avermectin aglycon synthase used in the production, and a gene encoding the enzyme.

### Background Art

A conventional process for producing 22,23-dihydroavermectin B1a involves a method comprising extracting an avermectin mixture with organic solvents from various microorganisms producing a plurality of avermectins, purifying avermectin B1 in the extract, and reducing the carbon bond between the 22nd and 23rd positions of avermectin B1 with hydrogen in the presence of a catalytic amount of compounds (Japanese Published Unexamined Patent Application No. 61198/79). A mixture of 22,23-dihydroavermectin B1a and 22,23-dihydroavermectin B1b obtained by the process, which is called 22,23-dihydroavermectin B1, is used as a medicine.

Avermectin is a polyketide compound which, as with other polyketide compounds, is biosynthesized through continuous condensation of lower fatty acids, reduction of a carbonyl group at β position of an elongated acyl group, dehydration, or enoyl reduction. These various repetitive synthetic processes of many polyketide compounds are carried out by a polymeric and multifunctional enzyme complexes, each of which has a specific active site (domain) required for each catalytic activity. A general reaction formula of polyketide biosynthesis is outlined, for example in Ann. Rev. Gen., 24, 37 (1990) and Ann. Rev. Microbiol., 47, 875 (1993).

DNA encoding a polyketide synthase usually encodes all the required activity sites for the synthesis of a polyketide backbone (aglycon), and contains modules, that is, repeating units involving condensation steps and modification steps following condensation. Depending on the genetic information existing in each module, the elongation or modification of an acyl group is determined. A polyketide synthase specifically acts on a specific carboxylic acid constitutional unit that is involved in each condensation step or acts on a site that defines the specific modifying function after condensation.

Regarding the biosynthetic mechanism of avermectin aglycon, it has been reported that, as with other polyketide compounds, avermectin aglycon contains lower fatty acids, such as acetic acid and propionic acid as its components [J. Antibiot., 39, 541-549 (1986)], and a polyketide synthase constituted by modules is present in avermectin-producing bacteria [Gene, 115, 119-125 (1992), Ann. New York Acad. of Sci., 721, 123-132 (1994)]. DNA fragments involved in the biosynthesis of avermectin (Japanese Published Unexamined Patent Application No. 15391/91) or domain structures of some modules [Ann. New York Acad. Sci., 721, 123-132 (1994)] have been reported although the nucleotide sequence, which is the basis thereof, is not disclosed. That is, the existence of some modules in the avermectin aglycon synthase is merely presumed while the structure of the entire synthase has not been elucidated. The present inventors made an intensive investigation into avermectin aglycon biosynthase genes, thereby precisely deducing the domain structure of each module involved in the biosynthesis of avermectin aglycon.

Among 22,23-dihydroavermectin B1 components, 22,23-dihydroavermectin B1a is known as a highly effective medicine [Antimicrobial Agent and Chemotherapy, 15, 372-378 (1979) and Japanese Published Examined Publication No. 54113/87]. Avermectin B1a, which is a raw material for synthesizing 22,23-dihydroavermectin B1a, is obtained by culturing avermectin B1a producing microorganisms and purifying it from the culture.

Document WO 00/01827 discloses nucleic acid molecules encoding at least part of a Type I polketide synthase, as well as nucleic acid molecules having a polylinker with multiple restriction enzyme sites in place of one or more PKS genes encoding enzymes associated with reduction.

Document WO 99/03986 describes a method to produce novel polyketides. In particular, modified PKS gene clusters which produce novel polyketides in an efficient system in a host cell or in a cell free extract are described. The novel polyketides result from the incorporation of diketides of formula 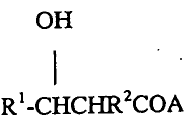 or 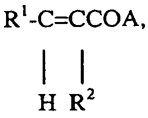 wherein A is a moiety that activates the diketide, and at least one of R¹and R² is a substituent other than that natively occurring in the diketide normally processed by the modified PKS cluster.

*Streptomyces avermitilis*, which produces avermectin, produces 8 components of avermectins having analogous structures (Japanese published Examined Publication No. 17558/90). Among strains selectively producing avermectin component which were mutated and bred from *Streptomyces avermitilis*, any strains which produce only avermectin B1a are not obtained. Accordingly, avermectin B1a should be isolated from avermectins having analogous structures for the purpose of producing 22,23-dihydroavermectin B1a. However, since there are extraordinary similarities between avermectin structures, it is very difficult to industrially isolate only avermectin B1a. For this reason, it is considered that a currently used 22,23-dihydroavermectin preparation consists of dihydroavermectin B1a and dihydroavermectin B1b.

According to Ikeda et al., PNAS, 96, 1999, p. 9509-9514, analysis of the gene cluster from *Streptomyces avermitilis* that forms the biosynthesis of the polyketide anthelmintic avermectin revealed that it contains four larger ORFs encoding giant multifunctional polypeptides of the avermectin polyketide synthese (AVES1, AVES2, AVES3 and AVES4). These clustered polyketides synthese genes, responsible for avermectin biosynthesis together encode 12 homologous sets of enzyme activities (modules), each catalyzing a specific round of polyketide chain elongation.

The necessity of hydrogenation with a special catalyst after purification complicates the process for producing 22,23-dihydroavermectin B1 and results in increased cost.

Accordingly, if only 22,23-dihydroavermectin B1a can be directly produced, all the problems involved in conventional industrial production can be solved and medicines containing only 22,23-dihydroavermectin B1a, which has the highest antiparasitic activity in its component, can be produced. A process for selectively and directly producing 22,23-dihydroavermectin B1a, however, is not known yet.

### DISCLOSURE OF THE INVENTION

The object of the present invention is to provide a process for selectively and directly producing only 22,23-dihydroavermectin B1a.

The present inventors have made an intensive investigation into studies in order to attain the above object and, have found that 22,23-dihydroavermectin B1a or a derivative thereof can be directly produced by modifying a gene encoding an avermectin aglycon synthase to obtain a modified enzyme and allowing a compound, which is a substrate of the modified enzyme, to act on a cell in which the modified genes have been expressed. The present invention has been completed on the basis of this result. The present invention relates to the following (1) to (10).
(1) An N-acetylcysteamine thioester compound represented by formula (I): 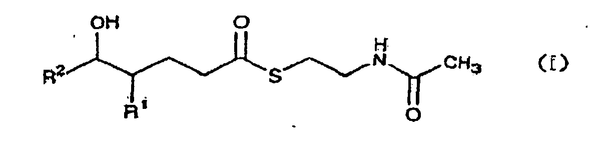 wherein R¹ is methyl and R² is sec-butyl.
(2) A process for producing an N-acetylcysteamine thioester compound of (1), which is characterized by employing, as a starting material, a compound represented by formula (II): 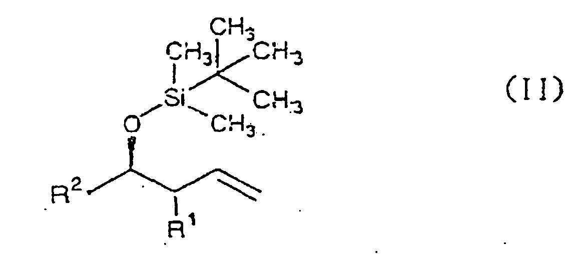 wherein R¹ is methyl and R² is sec-butyl, and comprising the steps of:
   (a) ozone-oxidating the compound, and thereafter adding carbon chains by the Wittig reaction;
   (b) deprotecting t-butyldimethylsilyl group of the compound obtained in step (a) and reintroducing another protecting group using chlorotriethylsilane;
   (c) reducing α-β unsaturated carbon bond of the resultant compound in the presence of a palladium-carbon catalyst, hydrolyzing an ester with potassium hydroxide, neutralizing the reaction mixture, and adding N-acetylcysteamine in the presence of a condensing agent to obtain thioester compound; and
   (d) removing the protecting group by adding acetic acid to the thioester compound.
(3) A process for producing 22,23-dihydroavermectin B1a, comprising the steps of:
   (a) contacting a culture of a non-human transformant which contains a DNA encoding a modified avermectin aglycone synthase comprising at least one domain with an eliminated activity, wherein the domain is selected from the group consisting of ATs [amino acid 29 to 344 of SEQ ID NO: 4], ACPs [amino acid 366 to 451 of SEQ ID NO: 4], KS1 [amino acid 481 to 914 of SEQ ID NO: 4], AT1 [amino acid 1051 to 1356 of SEQ ID NO: 4], ACP1 [amino acid 1979 to 2060 of SEQ ID NO: 4] and KS2 [amino acid 2086 to 2515 of SEQ m NO: 4], or a treated product thereof or the synthase encoded by the DNA with the N-acetylcysteamine thioester compound according to (1)in a medium; and
   (b) collecting 22,23-dihydroavermectin B1a produced and accumulated in the medium.
(4) The process for producing 22,23-dihydroavermectin B1a of (3), characterized in that an N-acetylcysteamine thioester compound of (1) is employed as a substrate compound for a modified avermectin aglycon synthase comprising at least one domain with an eliminated activity, wherein the domain is selected from the group consisting of ATs [amino acid 29 to 344 of SEQ ID NO: 4], ACPs[amino acid 366 to 451 of SEQ ID NO: 4], KS1 [amino acid 481 to 914 of SEQ ID NO: 4], AT1 [amino acid 1051 to 1356 of SEQ ID NO: 4], ACP1 [amino acid 1979 to 2060 of SEQ ID NO: 4] and KS2 [amino acid 2086 to 2515 of SEQ ID NO: 4].
(5) The process of (3) or (4),-wherein the modified avermectin aglycon synthase comprises mutated ATs [amino acid 29 to 344 of SEQ ID NO: 4], ACPs [amino acid 366 to 451 of SEQ ID NO: 4], KS1 [amino acid 481 to 914 of SEQ ID NO: 4], AT1 [amino acid 1051 to 1356 of SEQ ID NO: 4], ACP1 [amino acid 1979 to 2060 of SEQ ID NO: 4] and KS2 [amino acid 2086 to 2515 of SEQ ID NO: 4] in which a specific amino acid in an active center is substituted with another amino acid.
(6) The process of any one of (3) to (5), wherein the modified avermectin aglycon synthase comprises a polypeptide consisting of the amino acid sequence shown in SEQ ID NO: 8.
(7) The process of (3), wherein the DNA comprises a DNA consisting of the nucleotide sequence shown in SEQ ID NO: 3.
(8) The process of (3), wherein the non-human transformant is a microorganism.
(9) The process of (8), wherein the microorganism belongs to the genus *Streptomyces.*
(10) The process of (9), wherein the microorganism belonging to the genus *Streptomyces* is *Streptomyces avermitilis.*

The present invention will be described in detail below.

### [1] Structural analysis of avermectin aglycon synthase

### (1) Isolation of avermectin aglycon synthase gene and determination of nucleotide sequence

Methods for isolating avermectin aglycon synthase genes include a method described in Japanese Published Unexamined Patent Application No. 15391/91 and colony hybridization described in Molecular Cloning, 2nd Edition.

More specifically, chromosomal DNA of *Streptomyces avermitilis* is partially digested with a suitable restriction enzyme, for example, *Sau*3AI. Examples include the following method. A cosmid vector, which can replicate in *Escherichia coli,* is cleaved at a unique restriction enzyme site, such as the *Bam*HI site. The cleaved cosmid vector is linked to the digested chromosomal DNA, and *Escherichia coli* is then transformed with this recombinant DNA, and a transformant carrying avermectin aglycon synthase genes is selected from the obtained transformants by colony hybridization.

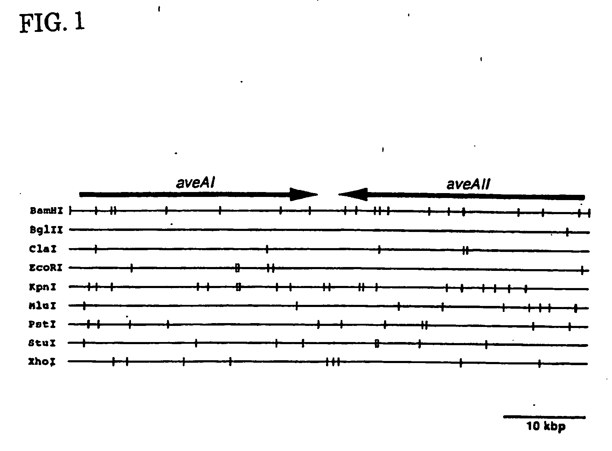

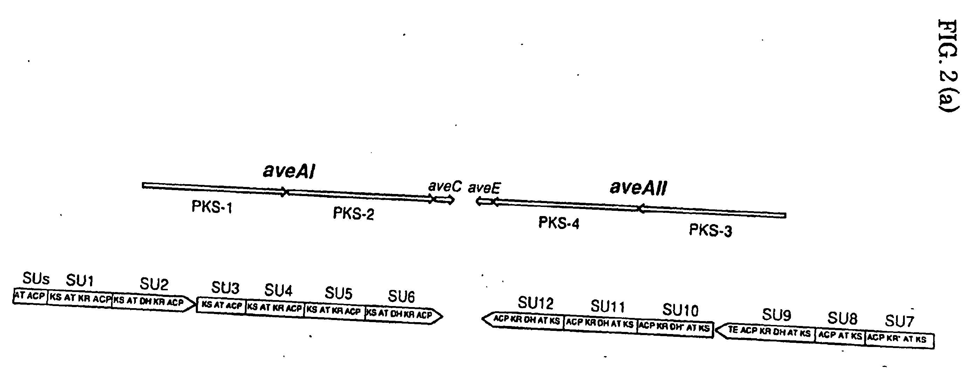

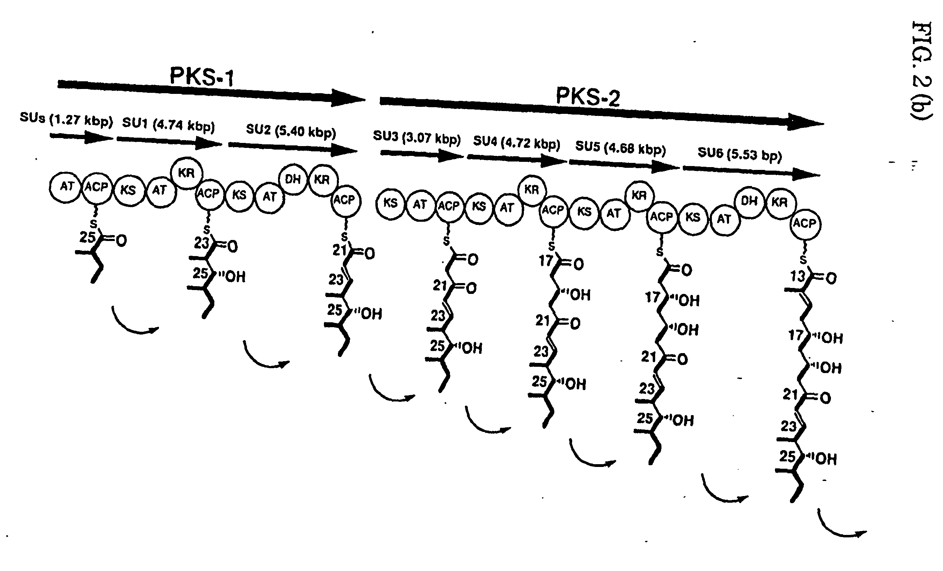

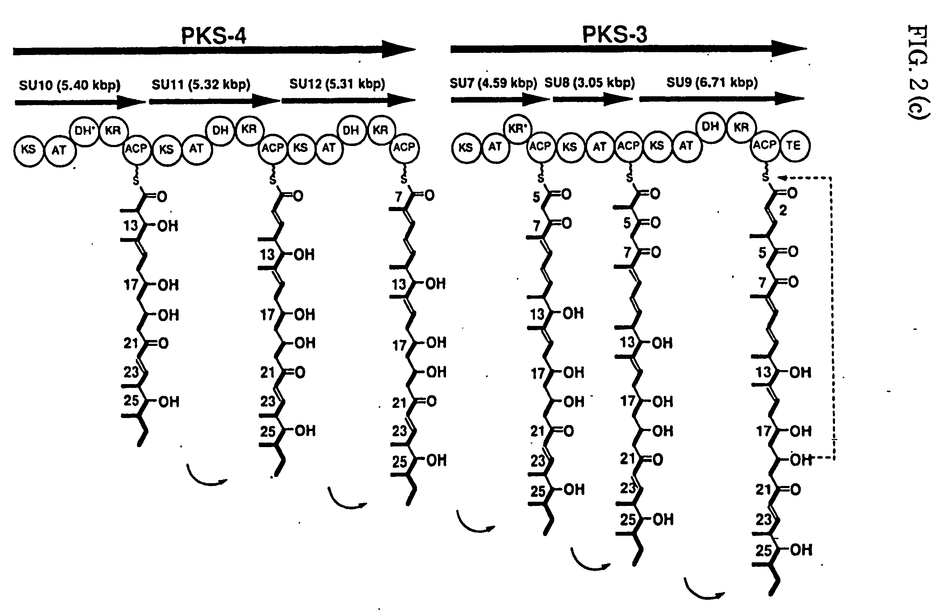

Specific examples of DNA obtained by the method can include DNA having the nucleotide sequence shown in SEQ ID NO: 1 or 2. The open reading frames (ORF) contained in these sequences are ORF1 (nucleotide nos. 1 to 11916 of SEQ ID NO: 1), ORF2 (nucleotide nos. 11971 to 30688 of SEQ ID NO: 1), ORF3 (nucleotide nos. 1 to 14643 of SEQ ID NO: 2), and ORF4 (nucleotide nos. 14824 to 31419 of SEQ ID NO: 2). Examples of the amino acid sequence of the polypeptide encoded by these sequences include sequences respectively shown in SEQ ID NOs: 4, 5, 6 and 7. Fig. 1 shows a restriction map of avermectin aglycon synthase gene regions (aveAI and aveAII) in genome DNA of *Streptomyces avermitilis* together with the deduced transcription unit (arrow).

### (2) Deduction of module and domain of avermectin aglycon synthase

Modules, domains and ORFs, which are relevant to the avermectin aglycon synthase genes, can be determined by comparing similarity with the sequences of 3 types of polyketide synthase domains of erythromycin [Nature, 348, 176-178 (1990), Science, 252, 675-679 (1991), Eur. J. Biochem., 204, 39-49 (1992)].

The condensation reaction, which is a basic reaction in the synthesis of polyketide, requires various catalytic activities including an acyl carrier protein (ACP), a β-ketoacyl ACP synthase (KS) and an acyltransferase (AT).

In many cases, β-carbonyl groups generated by the condensation reaction are modified. However, depending on a module, some β-carbonyl groups may not be modified and may be used for the next condensation reaction.

Catalytic activities associated with the modification of a β-carbonyl group after the condensation reaction include a β-ketoacyl ACP reductase (KR), a dehydratase (DH) and an enoyl reductase (ER). The biosynthesis of a polyketide chain is terminated by separating from a polyketide synthase by the thioesterase (TE) activity. All or several of these modification activities act in each condensation process, thereby determining the structure of a final product.

The avermectin aglycon synthase genes (aveAI and aveAII) of *Streptomyces avermitilis* are characterized by genes that have several open reading frames each of which comprises one or more repeating units called a module, just as the other known polyketide biosynthetic genes have. The module is defined as a gene fragment which encodes activities for a one-time synthesis, that is, a one-time condensation reaction and other various subsequent modification reactions of the β-carbonyl group. Each module encodes all or several of ACP, KS and AT associated with the condensation reaction in polyketide synthesis, and KR, DH and ER associated with the modification reaction of the β-carbonyl group. Furthermore, there is also a module which does not have any domain for a modification reaction. A polypeptide encoded by such a module is referred to as a synthase unit (SU).

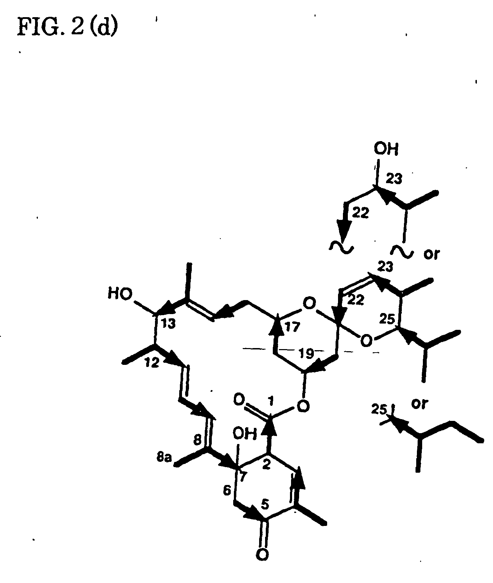

Fig. 2 (b) and (c) show a biosynthetic pathway of 6,8a-seco-6,8a-deoxy-5-oxo-avermectin aglycon synthesized with avermectin aglycon synthases of *Streptomyces avermitilis*.

PKS-1 is obviously associated with initiation reaction, since the initiation module (SUs), differing from other modules, has acyltransferase (AT) activity on the N-terminal side. PKS-3 is also obviously associated with the final reaction of polyketide, since module 9 (SU9) has a thioesterase (TE) domain.

Examples of deduced modules of avermectin synthase genes, a synthesis unit encoded by the modules, the domain constituting each synthesis unit and a subdomain which is a DNA encoding the domain, include the following sequences.

The terms used in the present invention are defined as follows.

Module represents a gene fragment encoding the activities of the one-time condensation reaction and various subsequent modification reaction of the β-carbonyl group.

Synthase unit (SU) represents a polypeptide encoded by a module.

Domain represents polypeptide having each catalytic activity constituting a synthase unit.

Subdomain represents a gene fragment encoding a domain.

These modules are represented as the following nucleotide numbers in SEQ ID NOs: 1 and 2. That is to say, the modules are shown in SEQ ID NO: 1 as,
Initiation Module: 85 to 1353,
Module 1: 1441 to 6180,
Module 2: 6256 to 11658,
Module 3: 12076 to 15147,
Module 4: 15217 to 19938,
Module 5: 20008 to 24690,
Module 6: 24781 to 30309, and,
   are represented in SEQ ID NO: 2 as,
Module 7: 100 to 4692,
Module 8: 4771 to 7818,
Module 9: 7906 to 14619,
Module 10: 14935 to 20334,
Module 11: 20413 to 25734,
Module 12: 25810 to 31125.

The amino acid sequences of various synthase units (SU) encoded by these modules are represented as the following amino acids. That is to say, the sequences are represented in SEQ ID NO: 4 as,
Initiation SU: 29 to 451,
SU1: 481 to 2060,
SU2: 2086 to 3886;
in SEQ ID NO: 5 as,
SU3: 36 to 1059,
SU4: 1083 to 2656,
SU5: 2680 to 4240,
SU6: 4271 to 6113;
in SEQ ID NO: 6 as,
SU7: 34 to 1564,
SU8: 1591 to 2606,
SU9: 2636 to 4873; and,
in SEQ ID NO: 7 as,
SU10: 38 to 1837,
SU11: 1864 to 3637,
SU12: 3663 to 5434.

DNAs encoding avermectin aglycon synthase domains (subdomains) are represented as the following nucleotide numbers. That is to say, the DNAs are represented in SEQ ID NO: 1 as,
in Initiation Module,
ATs: 85 to 1032,
ACPs: 1096 to 1353;
in Module 1,
KS1: 1441 to 2742,
AT1: 3148 to 4068,
KR1: 5143 to 5676,
ACP1: 5935 to 6180;
in Module 2,
KS2: 6256 to 7545,
AT2: 7906 to 8829,
DH2: 8947 to 9384,
KR2: 10609 to 11142,
ACP2: 11413 to 11658;
in Module 3,
KS3: 12076 to 13368,
AT3: 13756 to 14694,
ACP3: 14902 to 15147;
in Module 4,
KS4: 15217 to 16506,
AT4: 16917 to 17862,
KR4: 18886 to 19419,
ACP4: 19693 to 19938;
in Module 5,
KS5: 20008 to 21297,
AT5: 21658 to 22584,
KR5: 23602 to 24138,
ACP5: 24445 to 24690;
in Module 6,
KS6: 24781 to 26079,
AT6: 26413 to 27336,
DH6: 27475 to 27894,
KR6: 29227 to 29760,
ACP6: 30064 to 30309; and,
are also represented in SEQ ID NO: 2 as,
in Module 7,
KS7: 100 to 1383,
AT7: 1648 to 2673,
KR7: 3634 to 4188,
ACP7: 4447 to 4692;
in Module 8,
KS8: 4771 to 6060,
AT8: 6322 to 7344,
ACP8: 7573 to 7818;
in Module 9,
KS9: 7906 to 9258,
AT9: 9676 to 10773,
DH9: 10885 to 11289,
KR9: 12547 to 13104,
ACP9: 13378 to 13659,
TE9: 13879 to 14619;
in Module 10,
KS10: 14935 to 16224,
AT10: 16543 to 17565,
DH10: 17689 to 18066,
KR10: 19285 to 19842,
ACP10: 20089 to 20334;
in Module 11,
KS11: 20413 to 21705,
AT11: 21991 to 23019,
DH11: 23149 to 23529,
KR11: 24685 to 25242,
ACP11: 25489 to 25734;
in Module 12,
KS12: 25810 to 27102,
AT12: 27367 to 28392,
DH12: 28516 to 28878,
KR12: 30076 to 30633,
ACP12: 30880 to 31125.

The deduced amino acid sequences of various domains encoded by these subdomains are represented as:
in SEQ ID NO: 4,
   ATs: 29 to 344,
   ACPs: 366 to 451,
   KS1: 481 to 914,
   AT1: 1050 to 1356,
   KR1: 1715 to 1892,
   ACP1: 1979 to 2060,
   KS2: 2086 to 2515,
   AT2: 2636 to 2943,
   DH2: 2983 to 3128,
   KR2: 3537 to 3714,
   ACP2: 3805 to 3886;
in SEQ ID NO: 5,
   KS3: 36 to 466,
   AT3: 596 to 908,
   ACP3: 978 to 1059,
   KS4: 1083 to 1512,
   AT4: 1653 to 1964,
   KR4: 2306 to 2483,
   ACP4: 2575 to 2656,
   KS5: 2680 to 3109,
   AT5: 3230 to 3538,
   KR5: 3878 to 4056,
   ACP5: 4159 to 4240,
   KS6: 4271 to 4703,
   AT6: 4741 to 5048,
   DH6: 5095 to 5234,
   KR6: 5679 to 5856,
   ACP6: 5955 to 6036;
in SEQ ID NO: 6,
   KS7: 34 to 461,
   AT7: 550 to 891,
   KR7: 1212 to 1396,
   ACP7: 1483 to 1564,
   KS8: 1591 to 2020,
   AT8: 2108 to 2448,
   ACP8: 2525 to 2606,
   KS9: 2636 to 3086,
   AT9: 3226 to 3591,
   DH9: 3629 to 3763,
   KR9: 4183 to 4363,
   ACP9: 4460 to 4553,
   TE9: 4627 to 4873; and,
in SEQ ID NO: 7,
   KS10: 38 to 467,
   AT10: 574 to 914,
   DH10: 956 to 1081,
   KR10: 1488 to 1673,
   ACP10: 1756 to 1837,
   KS11: 1864 to 2294,
   AT11: 2390 to 2732,
   DH11: 2776 to 2902,
   KR11: 3288 to 3473,
   ACP11: 3556 to 3637,
   KS12: 3663 to 4093,
   AT12: 4182 to 4523,
   DH12: 4565 to 4685,
   KR12: 5085 to 5270,
   ACP12: 5353 to 5434.

### [2] Preparation of modified avermectin aglycon synthase

### (1) Introduction of site-specific mutation

DNA which encodes a modified avermectin aglycon synthase having a mutation so as to eliminate or significantly lower the activity in at least one domain is prepared based on the above information. The domain in which the activity is eliminated or significantly lowered may be any of the above-described domains and are preferably ATs, ACPs, KS1, AT1, KR1, ACP1, KS2, DH2 and KR2.

Mutations for eliminating or significantly lowering the activity in these domains are not particularly limited. Examples thereof include the deletion or substitution of an amino acid residue in the active center. It is important that an avermectin aglycon synthase protein is produced by being translated from two large transcription units. Thus, when a termination codon or a frameshift mutation is introduced into the gene existing in the upstream domain of the transcription unit, the transcription is terminated in mid course and, in some cases, the activity of the downstream domain is not expressed. In such a case, even thought there is no mutation existing in the downstream domain of the gene, per se, the entire mutated transcription unit is considered as having been deactivated. In order to minimize the influence on the entire transcription unit, the mutation to be introduced is preferably carried out by preventing the introduction of frameshift or termination codon. More preferably, mutation is carried out by substituting a specific amino acid in an active center with another amino acid. Examples of such mutation include mutation in which serine as an active center of AT, serine as an active center of ACP, or cysteine as an active center of KS [Eur. J. Biochem., 204, 39-49 (1992)] is substituted with another amino acid. More specific examples include a mutation in which "T" represented as the nucleotide 1969 in the nucleotide sequence shown in SEQ ID NO: 1 encoding KS1 is substituted with "G." As a result of this mutation, a cysteine residue, which is represented as the amino acid 657 in the amino acid sequence shown in SEQ ID NO: 4, is replaced with a glycine residue. The cysteine residue, which is represented as the amino acid 657 in the amino acid sequence shown in SEQ ID NO: 4 is also conserved in other ketosynthase [Eur. J. Biochem., 204, 39-49 (1992)] and is concluded to be essential in expressing the activity in this domain.

Methods for introducing mutation are not particularly limited and include: a method in which cells having DNA encoding avermectin aglycon synthase without mutation are subjected to mutation by NTG treatment or UV irradiation; a method in which DNA per se encoding avermectin aglycon synthase without mutation is processed with a mutagen such as hydroxyurea; and a method in which a site-specific mutation is introduced based on the nucleotide sequence information of the avermectin aglycon synthase gene. Among these, the method for introducing site-specific mutation based on the nucleotide sequence information is suitable because a specific mutation can be introduced into enormous genes such as avermectin aglycon synthase gene without causing any unintended mutation. For example, mutation can be introduced in accordance with methods described in Molecular Cloning, 2nd Edition, Current Protocols in Molecular Biology, Nucleic Acids Research, 10, 6487 (1982), Proc. Natl. Acad. Sci. USA, 79, 6409 (1982), Gene, 34, 315 (1985), Nucleic Acids Research, 13, 4431 (1985), and Proc. Natl. Acad. Sci. USA, 82, 488 (1985).

### (2) Preparation of cells transformed with recombinant DNA and preparation of modified avermectin aglycon synthase

Methods for obtaining a modified avermectin synthase include a method using strains having the modified avermectin aglycon synthase described in (1) and a method using a transformant, which is prepared by ligating the mutagen-treated DNA or the site-specific mutation-introduced DNA described in (1) and vector DNA to prepare a recombinant DNA, and the recombinant DNA is introduced into a host cell, thereby preparing a transformant. The host cell used in the latter method includes bacteria, yeast, filamentous fungus, animal cells, plant cells, and insect cells as long as the introduced modified genes are expressible in the cell. As an expression vector, it is possible to use any vector that can autonomously replicate in the above host cells or can be integrated into chromosomes thereof and that contains a promoter at a site which permits transcription of the introduced modified genes (hereinafter referred to as DNA encoding the polypeptide of the present invention).

When a prokaryote (e.g., bacteria) is used as a host cell, a preferred recombinant vector comprising DNA which encodes the polypeptide of the present invention may be autonomously replicative in prokaryotes and comprises a promoter, a ribosome-binding sequence, the DNA of the present invention and a terminator. The vector may further comprise a gene that regulates the promoter.

Examples of expression vectors include pBTrp2, pBTac1, pBTac2 (each of which is commercially available from Boehringer Mannheim), pKK233-2 (manufactured by Pharmacia), pSE280 (manufactured by Invitrogen), pGEMEX-1 (manufactured by Promega), pQE-8, pQE-9, pQE-60, pQE-70 (each of which is manufactured by QIAGEN), pKYP10 (Japanese Published Unexamined Patent Application No. 110600/83), pKYP200 [Agric. Biol. Chem., 48, 669 (1984)], pLSA1 [Agric. Biol. Chem., 53, 277 (1989)], pGEL1 [Prqc. Natl. Acad. Sci. USA, 82, 4306 (1985)], pBluescript II SK(-) (manufactured by Stratagene), pTrS30 [prepared from *Escherichia coli* JM109/pTrS30 (FERM BP-5407)], pTrS32 [prepared from *Escherichia coli* JM109/pTrS32 (FERM BP-5408)], pGHA2 [prepared from *Escherichia coli* IGHA2 (FERM BP-400), Japanese Published Unexamined Patent Application No. 221091/85], pGKA2 [prepared from *Escherichia coli* IGKA2 (FERM BP-6798), Japanese Published Unexamined Patent Application No. 221091/85], pTerm2 (US4686191, US4939094, US5160735), pSupex, pUB110, pTP5, pC194, pEG400 [J. Bacteriol., 172, 2392 (1990)], pGEX (manufactured by Pharmacia), pUC19 [Gene, 33, 103 (1985)], pUC118 (manufactured by Pharmacia), pET system (manufactured by Novagen), pIJ702, and pIJ922, etc.

Examples of chromosomal integration vectors include a vector derived from actinophage R4 [J. Bacteriol., 173, 4237 (1991)].

Examples of homologous recombination vectors include pKC7 (Japanese Published Unexamined Patent Application No. 189774/94).

Any promoter capable of functioning in host cells may be used, including promoters derived from *Escherichia coli* or a phage such as *trp* promoter (P*trp*), *lac* promoter (P*lac*), P_{L} promoter, P_{R} promoter and T7 promoter. An artificially designed, modified promoter may also be used, including a promoter obtained by binding two P*trp* promoters in tandem (P*trp* × 2), *tac* promoter, lac T7 promoter and let I promoter.

It is preferable to use a plasmid having an appropriate distance (e.g., 6-18 nucleotides) between Shine-Dalgarno sequence (i.e., ribosome-binding sequence) and an initiation codon. In the recombinant vector of the present invention, a terminator is not necessarily required for the expression of the DNA of the present invention, but it is desirably located immediately downstream of a structural gene.

Host cells include a microorganism belonging to *Escherichia, Serratia, Bacillus, Brevibacterium, Corynebacterium, Microbacterium, Pseudomonas, Streptomyces* and the like. Specific examples include *Escherichia coli* XL1-Blue, *Escherichia coli* XL2-Blue, *Escherichia coli* DH1, *Escherichia coli* MC1000, *Escherichia coli* KY3276, *Escherichia coli* W1485, *Escherichia coli* JM109, *Escherichia coli* HB101, *Escherichia coli* No.49, *Escherichia coli* W3110, *Escherichia coli* NY49, *Escherichia coli* GI698, *Escherichia coli* TB1, *Serratia ficaria, Serratia fonticola, Serratia liquefaciens, Serratia marcescens, Bacillus subtilis*, *Bacillus amyloliquefacines, Brevibacterium ammoniagenes, Brevibacterium immariophilum* ATCC14068, *Brevibacterium saccharolyticum* ATCC14066, *Brevibacterium flavum* ATCC14067, *Brevibacterium lactofermentum* ATCC13869, *Corynebacterium glutamicum* ATCC13032, *Corynebacterium glutamicum* ATCC13869, *Corynebacterium acetoacidophilum* ATCC13870, *Microbacterium ammoniaphilum* ATCC15354, *Pseudomonas putida, Pseudomonas* sp. D-0110, *Streptomyces lividans* TK23, *Streptomyces lividans* ATCC69411, *Streptomyces coelicolor* ATCC13405, *Streptomyces griseus* ATCC23915, *Streptomyces avermitilis* ATCC31267, *Streptomyces avermitilis* FERM BP-2773, and *Streptomyces avermitilis* FERM BP-2775, etc.

The recombinant vector may be introduced by any of the method for introducing DNA into the above host cells: for example, the method using calcium ion [Proc. Natl. Acad. Sci. USA, 69, 2110 (1972)], the protoplast method (Japanese Published Unexamined Patent Application No. 248394/88) and the method described in Gene, 17, 107 (1982) and Molecular & General Genetics, 168, 111 (1979).

When yeast is used as a host cell, examples of usable expression vector include YEP13 (ATCC37115), YEp24 (ATCC37051), YCp50 (ATCC37419), pHS19 and pHS15, etc.

Any promoter capable of functioning in yeast cells may be used, including glycolytic gene promoters such as hexose kinase, PHO5 promoter, PGK promoter, GAP promoter, ADH promoter, gal 1 promoter, gal 10 promoter, heat shock polypeptide promoter, MF α1 promoter and CUP 1 promoter.

Host cells include microorganisms belonging to *Saccharomyces, Schizosaccharomyces, Kluyveromyces, Trichosporon, Schwanniomyces, Pichia* and the *Candida.* Specific examples include *Saccharomyces cerevisiae, Schizosaccharomyces pombe, Kluyveromyces lactis, Trichosporon pullulans, Schwanniomyces alluvius,* or *Candida utilis,* etc.

The recombinant vector may be introduced by any of the method for introducing DNA into yeast: for example, electroporation [Methods Enzymol., 194, 182 (1990)], the spheroplast method [Proc. Natl. Acad. Sci. USA, 75, 1929 (1978)], the lithium acetate method [J. Bacteriology, 153, 163 (1983)] and the method described in Proc. Natl. Acad. Sci. USA, 75, 1929 (1978).

When an animal cell is used as a host cell, examples of usable expression vectors include pcDNAI, pcDM8 (manufactured by Funakoshi), pAGE107 [Japanese Published Unexamined Patent Application No. 22979/91, Cytotechnology, 3, 133 (1990)], pAS3-3 (Japanese Published Unexamined Patent Application No. 227075/90), pCDM8 [Nature, 329, 840 (1987)], pcDNAI/Amp (manufactured by Invitrogen), pREP4 (manufactured by Invitrogen), pAGE103 [J. Biochem., 101, 1307 (1987)], and pAGE210, etc.

Any promoter capable of functioning in animal cells may be used, including a promoter for immediate early (IE) gene of Cytomegalovirus (CMV), SV40 early promoter, retroviral promoter, metallothionein promoter, heat shock promoter, and SRa promoter. An enhancer for IE gene of Human CMV may also be used together with such a promoter.

Host cells include human Namalwa cells, monkey COS cells, chinese hamster CHO cells, or HBT5637 (Japanese Published Unexamined Patent Application No. 299/88).

The recombinant vector may be introduced into animal cells by any of the method for introducing DNA into animal cells: for example, electroporation [Cytotechnology, 3, (1990)], calcium , phosphate method (Japanese Published Unexamined Patent Application No. 227075/90), lipofection method [Proc. Natl. Acad. Sci. USA, 84, 7413 (1987)] and the method described in Virology, 52, 456 (1973), etc.

When an insect cell is used as a host cell, a polypeptide may be expressed by a method described in Current Protocols in Molecular Biology; Baculovirus Expression Vectors, A Laboratory Manual, W. H. Freeman and Company, New York (1992); or Bio/Technology, 6, 47 (1988).

More specifically, a recombinant gene-transfer vector and a baculovirus may be co-introduced into insect cells to obtain a recombinant virus in the supernatant from the culture of insect cells. Thereafter, insect cells may be further infected with the resulting recombinant virus to express the polypeptide.

A gene-transfer vector to be used in the above procedure includes pVL1392, pVL1393 and pBlueBacIII (manufactured by Invitrogen, respectively). As a baculovirus, for example, Autographa californica nuclear polyhedrosis virus, which infects *Noctuidae* insects, may be used.

Insect cells include *Spodoptera frugiperda* ovarian cells, Sf9 and Sf21, [Baculovirus Expression Vectors, A Laboratory Manual, W. H. Freeman and Company, New York (1992)], and *Trichoplusia ni* ovarian cells, High 5, (manufactured by Invitrogen), etc.

Co-introduction of the recombinant gene-transfer vector and the baculovirus into insect cells for recombinant virus production may be accomplished by the calcium phosphate method (Japanese Published Unexamined Patent Application No. 227075/90) or the lipofection method [Proc. Natl. Acad. Sci. USA, 84, 7413 (1987)].

When a plant cell is used as a host cell, examples of an expression vector include Ti plasmid and tobacco mosaic virus vector, etc.

Any promoter capable of functioning in plant cells may be used, including cauliflower mosaic virus (CaMV) 35S promoter and rice actin 1 promoter.

Host cells include plant cells such as tobacco, potato, tomato, carrot, soy bean, Brassica, alfalfa, rice, wheat and barley.

The recombinant vector may be introduced by any method for introducing DNA into plant cells: for example, *Agrobacterium* method (Japanese Published Unexamined Patent Application No. 140885/84, Japanese Published Unexamined Patent Application No. 70080/85, WO94/00977), electroporation method (Japanese Published Unexamined Patent Application No. 251887/85), and particle gun method (Japanese Patent No. 2606856, Japanese Patent No. 2517813).

The polypeptide of the present invention may be obtained by culturing a transformant of the present invention prepared as stated above in a medium until the polypeptide of the present invention is produced and accumulated in the culture, and collecting the polypeptide from the culture.

The transformant of the present invention may be cultured in a medium according to a conventional method used for culturing host cells.

When the transformant of the present invention is derived from a prokaryotic host such as *Escherichia coli* or a eukaryotic host such as yeast, the medium for culturing the transformant may be a natural or synthetic medium insofar as the medium contains a carbon source, a nitrogen source, inorganic salts etc., which can be assimilated by the transformant, and enables efficient culturing of the transformant.

Any carbon source assimilated by the transformant can be used. Examples include carbohydrates such as glucose, fructose, sucrose, molasses containing the same, starch and starch hydrolysates; organic acids such as acetic acid and propionic acid alcohols such as ethanol and propanol.

Examples of usable nitrogen source include ammonia, ammonium salts of inorganic or organic acids, such as ammonium chloride, ammonium sulfate, ammonium acetate, and ammonium phosphate; other nitrogen-containing compounds; and peptones, meat extracts, yeast extracts, corn steep liquor, casein hydrolysates, soy bean meal, soy bean meal hydrolysates, various fermented microorganism cells and hydrolysates thereof.

Inorganic salts usable herein include potassium dihydrogen phosphate, dipotassium hydrogen phosphate, magnesium phosphate, magnesium sulfate, sodium chloride, ferrous sulfate, manganese sulfate, copper sulfate, calcium carbonate, and the like.

Culturing is carried out under aerobic conditions as used for shaking culture or submerged aeration stirring culture. Culture temperature is preferably 15 to 40°C, and culture duration is usually for 16 hours to 7 days. During the culture, pH is preferably maintained at 3.0 to 9.0. pH is adjusted by using an inorganic or organic acid, an alkaline solution, urea, calcium carbonate, ammonia and the like.

If necessary, antibiotics such as ampicillin and tetracycline may be added to a medium during the culture.

Where a microorganism is transformed with a recombinant vector that contains inducible promoter, the transformant may be cultured in a medium supplemented with an inducer, if necessary. For example, in the case of a microorganism transformed with a recombinant vector comprising *lac* promotor, isopropyl-β-D-thiogalactopyranoside or the like may be add to the medium, and in the case of a microorganism transformed with a recombinant vector comprising *trp* promoter, indole acrylic acid or the like may be added.

A medium for culturing a transformant derived from an animal host cell may be a generally used medium such as RPMI 1640 medium [The Journal of the American Medical Association, 199, 519 (1967)], Eagle's MEM medium [Science, 122, 501 (1952)], Dulbecco's modified MEM medium [Virology, 8, 396 (1959)], 199 medium [Proceeding of the Society for the Biological Medicine, 73, 1 (1950)] or any one of these media further supplemented with fetal calf serum.

Culturing is usually carried out at pH 6 to 8, at a temperature of 30 to 40°C for a period of 1 to 7 days in the presence of 5% CO₂

If necessary, antibiotics such as kanamycin and penicillin may be added to the medium during the culture.

The medium for culturing a transformant derived from an insect host cell may be a generally used medium such as TNM-FH medium (manufactured by Pharmingen), Sf-900 II SFM medium (manufactured by Life Technologies), ExCell 400 and ExCell 405 [both manufactured by JRH Biosciences], Grace's Insect Medium [Nature, 195, 788 (1962)] or the like.

Culturing is carried out at pH 6 to 7, at a temperature of 25 to 30°C for a period of 1 to 5 days.

If necessary, antibiotics such as gentamycin may be added to the medium during the culture.

The transformant derived from a plant host cell may be cultured as a cell or may be allowed to differentiate into plant cells or organs. The medium for culturing such a transformant may be a generally used medium such as Murashige and Skoog (MS) medium, White medium, or any one of these media further supplemented with a plant hormone such as auxin or cytokinin.

Culturing is usually carried out at pH 5 to 9, at a temperature of 20 to 40°C for a period of 3 to 60 days.

If necessary, antibiotics such as kanamycin and hygromycin may be added to a medium during the culture.

As stated above, the polypeptide of the present invention may be obtained by culturing a microorganism-, animal cell-, or plant cell-derived transformant carrying a recombinant vector comprising a DNA that encodes the polypeptide in a general manner to produce and accumulate the polypeptide, and then recovering the polypeptide from the culture.

A gene of interest may be either expressed directly, or as a secretory protein or fusion polypeptide according to the method as described in Molecular Cloning, 2nd Edition.

Expression in yeast, animal, insect or plant cells can provide a polypeptide with sugar or sugar chain added thereto.

The protein of the present invention may be produced by intracellular production by host cells, extracellular secretion by host cells or production on outer membranes by host cells. Such production method can be selected depending on the kind of the host cells used or on alteration of the structure of the portein.

If the polypeptide of the present invention is produced in host cells or on the outer membranes of host cells, the polypeptide can be efficiently secreted extracellularly from the host cells by using the method of Paulson et al. [J. Biol. Chem., 264, 17619 (1989)], the method of Lowe et al. [Proc. Natl. Acad. Sci. USA, 86, 8227 (1989), Genes Develop., 4, 1288 (1990)] or methods as described in Japanese Published Unexamined Patent Application Nos. 336963/93 and 823021/94.

More specifically, the polypeptide of the present invention can be efficiently secreted from host cells by expressing it with a signal peptide, then using genetic recombination techniques, adding the signal peptide upstream of a polypeptide containing the active site of the polypeptide of the present invention.

Polypeptide production can be enhanced by utilizing a gene amplification system that uses a dihydrofolate reductase gene or the like according to the method described in Japanese Published Unexamined Patent Application No. 227075/90.

Further, animal or plant cells carrying a transgene may be re-differentiated to create an animal individual carrying a transgene (transgenic non-human animal) or a plant individual carrying a transgene (transgenic plant), which may be used for producing the polypeptide of the present invention.

When the transformant is an animal or plant individual, the polypeptide may be obtained by feeding or cultivating the individual in a general manner to produce and accumulate the polypeptide, and then recovering the polypeptide from the animal or plant individual

In order to produce the polypeptide of the present invention using an animal individual, for example, an animal carrying a transgene may be allowed to produce therein the polypeptide of the present invention in a known manner as described in American Journal of Clinical Nutrition, 63, 639S (1996); American Journal of Clinical Nutrition, 63, 627S (1996); and Bio/Technotogy, 9, 830 (1991).

In the case of an animal individual, for example, the polypeptide of the present invention may be obtained by feeding a transgenic non-human animal carrying a DNA insert that encodes the polypeptide of the present invention to produce and accumulate therein the polypeptide, and then collecting the polypeptide from the animal. The polypeptide may be produced and accumulated in the animal's milk (Japanese Published Unexamined Patent Application No. 309192/88), egg and the like. Any promoter capable of functioning in an animal may be used, for example, mammary gland cell-specific promoters such as α-casein promoter, β-casein promoter, β-lactoglobulin promoter and whey acidic protein promoter being preferred.

In order to produce the polypeptide of the present invention using a plant individual, for example, a transgenic plant carrying a DNA insert encoding the polypeptide of the present invention may be cultivated to produce and accumulate therein the polypeptide in a known manner as described in Tissue Culture (Soshiki Baiyo), 20 (1994); Tissue Culture, 21 (1995); and Trends in Biotechnology, 15, 45 (1997), and then the polypeptide may be recovering from the plant.

For isolation and purification of the polypeptide produced from the transformant of the present invention, conventional methods for the isolation and purification of enzymes can be used.

For example, if the polypeptide of the present invention is expressed in a soluble form in cells, after completion of culturing, the cells are collected by centrifugation, suspended in an aqueous buffer and then disrupted with ultrasonic disrupter, French Press, Manton-Gaulin homogenizer, Dynomill or the like, thereby obtaining a cell-free extract. A purified preparation can be obtained by centrifuging the cell-free extract. The obtained supernatant is then subjected to conventional isolation and purification methods for enzymes, i.e., solvent extraction, salting-out or desalting with sulfate ammonium etc., precipitation with organic solvent, anion-exchange chromatography on resin such as diethylaminoethyl (DEAE)-Sepharose or DIAION HPA-75 (manufactured by Mitsubishi Chemical Industries Ltd.), cation-exchange chromatography on resin such as S-Sepharose FF (manufactured by Pharmacia), hydrophobic chromatography on resin such as butyl Sepharose or phenyl Sepharose, gel filtration using molecular sieve, affinity chromatography, chromatofocusing, or electrophoresis such as isoelectric focusing, or combinations thereof.

If the polypeptide is expressed as inclusion body in cells, the cells are similarly collected, disrupted and centrifuged to give an insoluble matter of the polypeptide as a precipitated fraction. The resulting insoluble polypeptide is then solubilized with a protein-denaturing agent. The solubilized solution is then diluted or dialyzed to reduce the agent to a lower concentration, thereby allowing the polypeptide to be renatured to its normal conformation. The purified preparation of the polypeptide can be then obtained by use of the same isolation and purification methods as described above.

If the polypeptide of the present invention or a derivative thereof having a sugar chain added thereto is extracellularly secreted, the polypeptide or its derivatives may be recovered in the culture supernatant. Namely, the culture is subjected to the same process, such as centrifugation, as described above to give a culture supernatant. From the culture supernatant, a purified preparation can be obtained in the same manner for isolation and purification as described above.

The polypeptide thus obtained may be, for example, a polypeptide having the amino acid sequence shown in SEQ ID NO: 8.

The polypeptide of the present invention may be produced by chemical synthesis methods including Fmoc method (fluorenyl methyloxycarbonyl method), t-Boc method (t-butyloxycarbonyl method), and so on. Also, it may be chemically synthesized using a peptide synthesizer available from Advanced ChemTech, Perkin Elmer, Pharmacia, Protein Technology Instrument, Synthecell-Vega, PerSeptive or Shimadzu Corporation, etc.

In contrast, a method for inserting DNA having mutation which has been introduced *in vitro* into the chromosomal DNA of the host cell can be carried out by any method utilizing the homologous recombination of DNA. Examples of such methods include a method described in Japanese Published Unexamined Patent Application No. 189774/94.

Cells having a modified avermectin aglycon synthase gene having mutation introduced as described above are not particularly limited insofar as cells can carry the gene and may be any prokaryotic cells such as *Escherichia coli, Bacillus subtilis,* and *Actinomyces.* Examples thereof include microorganisms belonging to *Streptomyces avermitilis.*

### [3] Preparation of substrate compound for producing 22,23-dihydroavermectin B1a or derivative thereof

In the present invention, the substrate compound for producing 22,23-dihydroavermectin B1a or a derivative thereof may be any substance insofar as the substance can be used as a substrate for the modified avermectin aglycon synthase as described above. More specifically, in the process for synthesizing avermectin aglycon, the substance can be a substrate for the domain responsible for the later reaction step in the modified domain and an N-acetylcysteamine compound is preferably used. For example, when the KS domain of SU1 shown in Fig. 2 is modified, the N-acetylcysteamine compound preferably has a structure as represented by formula (I): 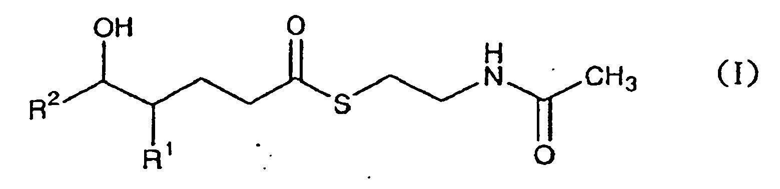 wherein R¹ is methyl and R² is sec-butyl.

Specific examples of such compounds include a compound (Compound 4 shown in the table below) represented by the above formula, wherein R¹ is methyl and R² is sec-butyl. The compound employs, for example, Compound A shown in the table below as a starting material and can be chemically synthesized in the following manner through Compounds 1 to 3 similarly shown in the table.

At the outset, Compound 1 is prepared using Compound A as a starting material and performing ozone oxidation, followed by the Wittig reaction to add carbon chains. After t-butyldimethylsilyl of Compound 1 is deprotected, a protective group is reintroduced using chloroethyl-tri-silane to obtain Compound 2. Subsequently, α-β unsaturated carbon bond in compound 2 is reduced in the presence of a palladium-carbon catalyst, ester is hydrolyzed with potassium hydroxide and neutralized, followed by the addition of N-acetylcysteamine in the presence of a condensing agent. Thus, a thioester compound, Compound 3, is obtained. Finally, acetic acid is added to Compound 3 to remove the protective group. Thus, Compound 4 is prepared.

The intermediates and the compounds of interest in the above production method are subjected to separation purification methods, which are commonly used in organic synthetic chemistry, for example, filtration, extraction, washing, drying, concentration, recrystallization, or various chromatographies and, thus, they can be isolated and purified. The intermediate can be applied to the subsequent reaction without purification.

**Table 1**

| Compounds | |
|---|---|
| A | 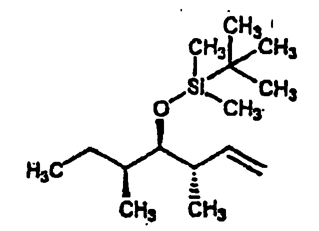 |
| 1 | 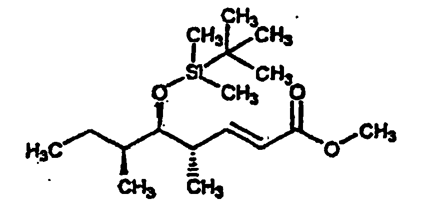 |
| 2 | 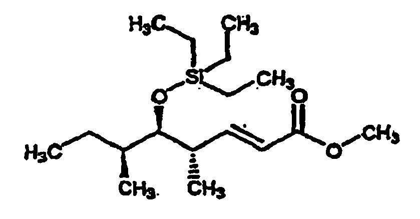 |
| 3 | 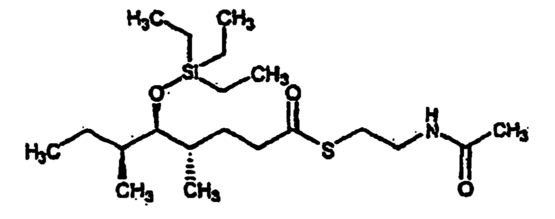 |
| 4 | 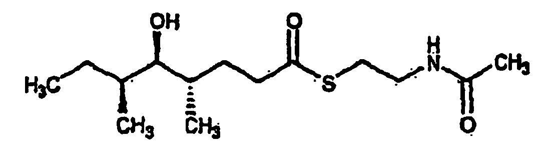 |

### [4] Production of 22,23-dihydroavermectin B1a or derivative thereof

Any of the culture, cells or treated cells of the cells obtained by transforming the host cell in [2]-2 can be used in the reaction with the substrate compounds so far as the modified avermectin aglycon expressed in the transformed cell are functioned.

Treated cells include dried cells, freeze-dried products, surfactant- or organic solvent-processed products, enzyme-processed products, ultrasonicated products, mechanically ground products, protein fractions of cells, and immobilized cells of treated cells.

Any method of making the substrate compound acting upon the transformed host cell can be used so far as the synthesis of avermectin aglycon is disturbed. Specific examples thereof include a method in which the culture of cells or treated products thereof are reacted with the substrate in a suitable medium and a method in which the cells are cultured by adding the substrate in initially or mid course of the culturing.

Media used in the reaction include water, buffers such as phosphate, carbonate, acetate, borate, citrate and Tris, aqueous solutions containing organic solvents, for example, alcohols such as methanol and ethanol, esters such as ethyl acetate, ketones such as acetone, and amides such as acetamide. If necessary, surfactants such as Triton X-100 (manufactured by Nacalai Tesque, Inc.) or Nonion HS204 (manufactured by NOF Corp.) or organic solvents such as toluene and xylene may be added in an amount of about 0.1 to 20 g/l.

Reaction is carried out in the above aqueous solution at pH 5 to 10, preferably pH 6 to 8, at 20 to 50°C for 1 to 96 hours.

When the host cell is cultured in a medium, culture can be carried out in the same manner as for obtaining the polypeptide.

22,23-dihydroavermectin B1a or a derivative thereof can be isolated from the reaction product or the culture obtained by any of the above methods in accordance with conventional isolation methods. For example, the cultured cell is treated with acetone or methanol to extract 22,23-dihydroavermectin B1a or a derivative thereof and, after the removal of the residue, concentrated. The concentrate is processed with methylene chloride, the methylene chloride layer is fractionated and further concentrated under reduced pressure. Thus, the subject compound can be obtained.

### BRIEF DESCRIPTION OF DRAWINGS

Fig. 1 is a diagram showing a restriction map of *Bam*HI, *Bgl*II*, Cla*I*, Eco*RI*, Kpn*I*, Mlu*I*, Pst*I*, StuI,* and *Xho*I sites of avermectin aglycon synthase genes aveAl and aveAII of *Streptomyces avermitilis.* The arrows indicate the deduced transcription direction of each gene.
Fig. 2(a) shows the location of avermectin aglycon synthase genes on the chromosome and the domain sequence of synthase units, Figs. 2(b) and 2(c) show the deduced steps of avermectin aglycon synthesis, and Fig. 2(d) shows the structure of 6,8-sec-6,8a-deoxy-5-oxoavermectin aglycon and the location of integrated lower fatty acids in its skeleton which had been synthesized by a polyketide synthase, which is a gene product of avermectin aglycon synthase genes aveAI and aveAII.

### (Description of reference characters)

ACP: acyl carrier protein
KS: β-ketoacyl ACP synthase
AT: acyltransferase
KR: β-ketoacyl ACP reductase
DH: dehydratase
ER: enoyl reductase
TE: thioesterase

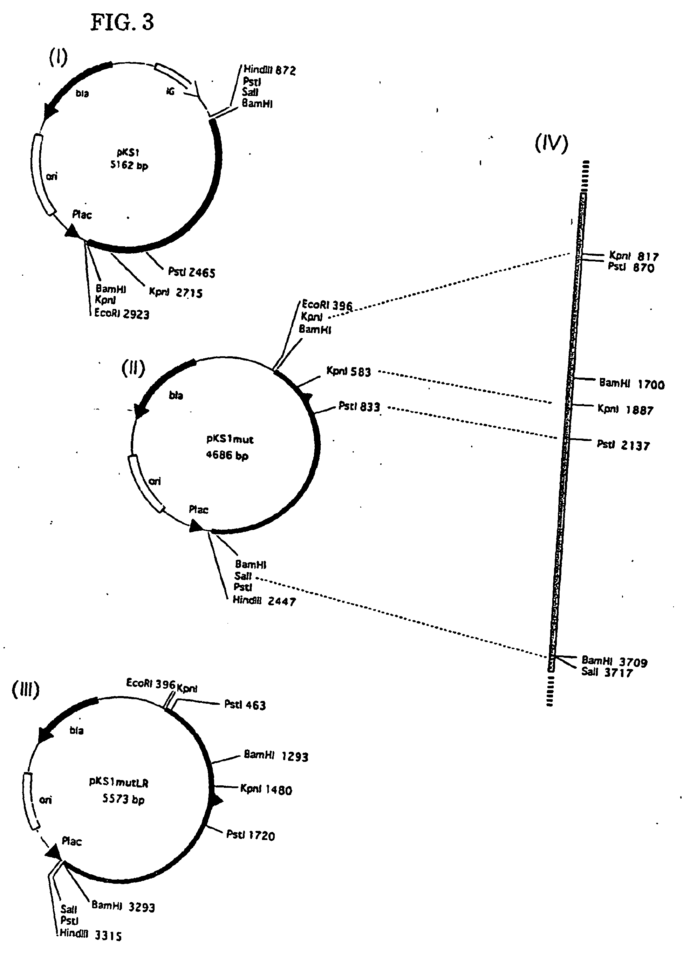

Fig. 3 is a diagram showing a procces for constructing a plasmid to be used in the transformation of *Streptomyces avermitilis* wherein (I) shows plasmid pKS1 prepared by cloning KS1 containing DNA encoding an amino acid residue in an active center, (II) shows plasmid pKSmut prepared by cloning DNA encoding KS1 prepared by substituting an amino acid residue in an active center, (III) shows plasmid pKSmutRL prepared by applying addition and substitution of a DNA fragment shown in (IV) to pKSmut, and (IV) is the restriction map of DNA encoding KS1 used in the construction of pKSmutRL.

In the drawing, "^" indicates the location of the nucleotides which have been substituted, and *Hind*III*, Pst*I*, Bam*HI*, Kpn*I*,* and *Eco*RI indicate the DNA cleavage sites of each restriction enzyme. Numerical values in (I), (II), and (III) indicate, when a desired nucleotide of each plasmid is determined as No. 1, the distance (bp) from the nucleotide and numerical values with in the circle indicate the total plasmid length (bp). Numerical values in (IV) are in accordance with the nucleotides shown in SEQ ID NO: 1. Abbreviations in the drawings are as follows.

### (Description of reference characters)

bla: β-lactamase (arrow indicates the direction of transcription)
ori: replication origin (origin)
Plac: β-lactamase promoter (arrow indicates the direction of the promoter)
IG: M13 phage intergenic region (M13 Intergenic region)

### Best Modes for Carrying Out the Invention

The present invention will be described in more detail with reference to examples; however, these examples are not intended to limit the scope of the present invention.

### [Example 1] Determination of nucleotide sequence and structure of avermectin aglycon synthase gene

A nucleotide sequence of DNA encoding avermectin aglycon synthase derived from *Streptomyces avermitilis* K2033 (US Patent No. 5206155, FERM BP-2773) was determined as follows.

A continuous or overlapping DNA fragment within the avermectin aglycon synthase gene was subcloned from a cosmid containing fragments of the avermectin aglycon synthase genes (aveAl and aveAII) co-isolated with a gene encoding avermectin B5-O-transmethylase [aveD; Gene, 206, 175-180 (1998)]. Nucleotide sequences of the inserted DNA fragments in these subclones were then determined.

More specifically, the entire nucleotide sequences of aveAI and aveAII were determined by subcloning *Bam*HI-digested fragments of 3.4 kbp, 2.0 kbp, 0.5 kbp, 6.8 kbp, 7.0 kbp, 7.8 kbp, 3.7 kbp, 4.8 kbp, 1.3 kbp, 2.4 kbp, 0.7 kbp, 1.0 kbp, 5.4 kbp, 2.5 kbp, 1.9 kbp, 0.1 kbp, 7.0 kbp, 3.1 kbp, 4.7 kbp and 1.3 kbp found in the *Bam*HI-restriction map of aveAI and aveAII shown in Figure 1; digesting the inserted DNA fragments in these subclones with exonuclease III and S1 nuclease to prepare a series of deletion fragments; and then performing a cycle-sequencing reaction using fluorescently-labeled primers to determine a nucleotide sequence of each deleted fragment. aveAI and aveAII had the nucleotide sequences shown in SEQ ID NO: 1 and SEQ ID NO: 2, respectively.

### [Example 2] Preparation of strain applied for the direct production of 22,23-dihydroavermectin B1a

The plasmid shown in Fig. 3 was produced in accordance with the following method and used in the transformation of *Streptomyces avermitilis.*

### (1) Subcloning of a DNA fragment containing KS1

The cosmid DNA containing KS1, from among cosmid DNAs containing avermectin aglycon synthase genes, was digested with the restriction enzyme *Bam*HI (manufactured by Takara Shuzo Co., Ltd.) followed by agarose gel electrophoresis (described in Molecular Cloning, 2nd Edition), and 2.0 kb DNA fragment (see Fig. 1, 1701 to 3716 shown in SEQ ID NO: 1) containing a cysteine residue (amino acid 657 shown in SEQ ID NO: 4), which is an active center of KS1, was separated and purified in accordance with the method described in Molecular Cloning, 2nd Edition. Plasmid pUC118 (manufactured by Takara Shuzo Co., Ltd.) was digested with *Bam*HI and dephosphorylated with alkaline phosphatase from calf intestine (manufactured by Takara Shuzo Co., Ltd.). About 0.1 µg each of 2.0 kb DNA fragment containing KS 1 and a *Bam*HI digested pUC118 were ligated 16°C for 16 hours using Ligation High (manufactured by Toyobo Co., Ltd.). 10 µl of this DNA ligation reactant was brought into contact with a competent cell of *Escherichia coli* DH5α (manufactured by Nippon Gene Co., Ltd.) and transformed in accordance with the method described in Molecular Cloning, 2nd Edition. In selecting the transformant, an LB agar medium containing 50 µg/ml ampicillin (manufactured by Wako Pure Chemical Industries, Ltd.) was used. 50 µl of aqueous solution of 0.1 mol/l isopropyl-β-D-thiogalactopyranoside (IPTG, manufactured by Wako Pure Chemical Industries, Ltd.) and 50 µl of 2% solution of 5-bromo-4-chloro-3-indolyl-β-D-galactoside (X-gal, manufactured by Nacalai Tesque, Inc.) in dimethylformamide (manufactured by Nacalai Tesque, Inc.) were previously spread on the 20 ml of LB agar medium. The colony of the transformant carrying the recombinant plasmid has lost its β-galactosidase activity, and thus, cannot decompose 5-bromo-4-chloro-3-indolyl-β-D-galactoside while developing white color. This white colony was collected with the aid of ase, inoculated on 10 ml of LB medium, and subjected to shaking culture at 37°C for 16 hours. The plasmid was then extracted from the cells and purified in accordance with the alkaline method described in Molecular Cloning, 2nd Edition. A part of the resulting recombinant plasmid was digested with a restriction enzymes *Pst*I and it was confirmed that plasmid pKS1, into which a DNA fragment containing KS1 genes was inserted in the same direction with lacZ encoded by pUC118, was obtained.

### (2) Introduction of nucleotide substitution into the active center of KS 1

Nucleotide was substituted using Takara LA PCR in vitro Mutagenesis Kit (manufactured by Takara Shuzo Co., Ltd.). Nucleotide was hereinafter substituted in accordance with the protocol attached to the kit. The recombinant plasmid containing KS1 genes prepared in (1) above was used as template DNA for the 1st PCR. As a primer for the 1st PCR-(a), 5'-ACCGTGGACACGGGGGGCTCGGCATCGCTCGT-3' shown in SEQ ID NO: 9 (corresponding to 1954 to 1985 shown in SEQ ID NO: 1, "T" at the 1969 position was substituted with "G") and M13M4 primer (attached to the kit) were used as a primer for introducing mutation. M13RV primer and MUT4 primer (attached to the kit) were used as primers for the 1st PCR-(b). In the 1st PCR, incubation at 98°C for 5 minutes, and then 30 cycles of reaction constituted by 30 seconds at 94°C, 2 minutes at 55°C and 3 minutes at 72°C as one cycle were carried out. TaKaRa PCR Thermal Cycler 480 (manufactured by Takara Shuzo Co., Ltd.) was used in PCR. Each reaction solution was subjected to agarose gel electrophoresis and about 1.8 kb amplified fragment in the 1st PCR-(a) and about 2.0 kb amplified fragment in the 1st PCR-(b) were respectively separated and purified for use in the subsequent step. Heteroduplex DNA between amplified fragments obtained in the 1st PCR was formed by incubating at 98°C for 15 minutes, lowering the temperature to 37°C over the course of 1 hour, and then incubating at 37°C for 15 minutes. After LA Taq polymerase was added to the reaction solution, the mixture was incubated at 72°C for 3 minutes to convert the terminus of the heteroduplex DNA into a blunt-ended terminus. In the subsequent 2nd PCR, 30 cycles of reaction constituted by 20 seconds at 94°C, 30 seconds at 60°C and 3 minutes at 72°C as one cycle were carried out. A part of the 2nd PCR product was subjected to agarose gel electrophoresis and the amplification of about 2.0 kb fragment was confirmed. The remaining solution of the 2nd PCR was thoroughly mixed with a phenol : chloroform = 1 : 1 solution saturated with water and then centrifuged. The supernatant was subjected to ethanol precipitation in accordance with the method described in Molecular Cloning, 2nd Edition, dried, and then redissolved in water. Restriction enzymes *Hind*III and *Eco*RI (manufactured by Takara Shuzo Co., Ltd.) were added to the DNA solution and the DNA was digested. Agarose gel electrophoresis was subsequently performed, thereby separating and purifying 2.0 kb DNA fragment. Plasmid vector pUC19 (manufactured by Takara Shuzo Co., Ltd.) was also digested with *Hind*III and *Eco*RI*.* 2.7 kb fragment was then separated and purified by agarose gel electrophoresis. 2.0 kb DNA fragment digested with *HindIII* and *Eco*RI was ligated to pUC19 using Ligation High and used to the transformation of *Escherichia coli* DH5α. As with (1) above, IPTG and X-gal were spread on the LB agar medium containing 50 µg/ml ampicillin for the selection of the transformant. Several strains were selected among from the transformants obtained as white colonies and inoculated on 10 ml of LB medium containing 50 µg/ml ampicillin and subjected to shaking culture at 37°C for 16 hours. Thereafter, strains were harvested and plasmid DNA carried by each strain was extracted and purified by an alkaline method.

### (3) Confirmation of introduction of nucleotide substitution by nucleotide sequencing

In nucleotide sequencing, ABI PRISM DNA Sequencing Kits-Dye primer Cycle Sequencing Ready Reaction Kits with AmpliTaqR DNA Polymerase, FS -21M13- (manufactured by PE Applied Biosystems), and ABI373A were used. Each recombinant plasmid DNA, which is considered to have nucleotide substitution introduced KS1 obtained in (2) above, was used as templates and sequencing samples were produced by PCR in accordance with the protocol attached to the Sequencing Kits. Each sample was subjected to electrophoresis using ABI373A and the resultant data was analyzed using a software for gene analysis, Genetyx (manufactured by Software Development Co., Ltd.). As a result, it was confirmed that plasmid DNA (pKS1mut) containing about 2.0 kb *BamHI* fragment corresponding to 1701 to 3716 in SEQ ID NO: 3 was obtained. SEQ ID NO: 3 comprises a nucleotide sequence in which thymine at the 1969 position is substituted with guanine in the 1^{st} to 11916^{th} nucleotide sequences shown in SEQ ID NO: 1.

### (4) Introduction of nucleotide substitution into chromosomal DNA of Streptomyces avermitilis

In order to introduce the plasmid mutation into chromosomal DNA through homologous recombination, a reasonably long homologous region is necessary. Since mutation is introduced into the DNA by PCR, mutation may be introduced in the region other than the targeted site. Thus, the broadest possible region other than the mutation site should be substituted with DNA derived from chromosomal DNA of *Streptomyces avermitilis* to eliminate unnecessary mutation. Plasmid DNA used in the homologous recombination was constructed in the following manner and applied to the transformation of *Streptomyces avermitilis.*

pKS1mut produced in (3) above was digested with restriction enzymes *PstI* and *Sal*I (manufactured by Takara Shuzo Co., Ltd.) and then subjected to agarose gel electrophoresis to separate and purify 4.1 kb DNA fragment. Subsequently, pKS1 was digested with *Pst*I and *Sal*I*,* followed by electrophoresis and 1.57 kb *Pst*I and *Sal*I digested fragments were separated and purified. Each collected DNA fragment was ligated using Ligation High and then brought into contact with a competent cell of *Escherichia coli* DH5α for transformation. The transformant was selected using LB agar medium containing 50 µg/ml ampicillin. Transformants were cultured at 37°C for 16 hours and ten-odd colonies were collected with the aid of ase, inoculated on 10 ml of LB medium containing 50 µg/ml ampicillin, subjected to shaking culture at 37°C for 16 hours, harvested, and plasmid carried by each strain was purified by the alkaline method. Each plasmid was digested with restriction enzymes *Pst*I and *Sal*I*,* subjected to agarose gel electrophoresis, and it was confirmed that plasmid pKS1mutR containing 4.1 kb and 1.57 kb DNA fragments was obtained.

Subsequently, pKS1mutR was digested with restriction enzyme *Kpn*I (manufactured by Takara Shuzo Co., Ltd.) and treated with alkaline phosphatase. Then, a cosmid, which contains a *Kpn*I region represented by nucleotide 817 to 1887 shown in SEQ ID NO: 1, was digested with *Kpn*I*,* followed by electrophoresis, and about 1.1 kb *Kpn*I fragment was separated and purified. Each purified DNA fragment was ligated using Ligation High and then brought into contact with a competent cell of *Escherichia coli* DH5α for transformation. The transformant was selected using the LB agar medium containing 50 µg/ml ampicillin. Transformants were cultured at 37°C for 16 hours and ten-odd colonies were collected with the aid of ase, inoculated on 10 ml of LB medium containing 50 µg/ml ampicillin, subjected to shaking culture at 37°C for 16 hours, harvested, and plasmid carried by each strain was purified by the alkaline method. Each plasmid was digested with restriction enzyme *Pst*I*,* subjected to agarose gel electrophoresis, and it was confirmed that plasmid pKS1mutRL containing 1.27 kb, 1.57 kb, and 2.7 kb DNA fragments was obtained.

Subsequently, pKS1mutRL was digested with restriction enzymes *Hind*III and *Eco*RI and 2.9 kb *Hind*III and *Eco*RI DNA fragments were separated and purified by agarose gel electrophoresis. Plasmid vector pKC7 (Japanese Published Unexamined Patent Application No. 189774/94) was also digested with *Hind*III and *Eco*RI and then purified by agarose gel electrophoresis. These two DNA fragments were ligated at 16°C for 16 hours using Ligation High and then brought into contact with a competent cell of *Escherichia coli* DH5α for transformation. Transformants were selected using the LB agar medium containing 50 µg/ml ampicillin. Those transformants were cultured at 37°C for 16 hours and ten-odd colonies were collected with the aid of ase, and inoculated on 10 ml of LB medium containing 50 µg/ml ampicillin. Those transformants were cultured at 37°C for 16 hours, and then cells were harvested and plasmid carried by each strain was purified by the alkaline method. Each plasmid was digested with restriction enzymes *Hind*III and *Eco*RI and then subjected to agarose gel electrophoresis. Thus, it was confirmed that plasmid pKC-KS1mut carrying 2.9 kb fragment was obtained.

KS1mut fragment was integrated into the KS1 region of the chromosome of *Streptomyces avermitilis* K2038 (FERM BP-2775) by homologous recombination using pKC-KS1mut in accordance with the method described in Japanese Published Unexamined Patent Application No. 189774/94. In order to confirm that KS1mut was replaced on the chromosomal DNA, the chromosomal DNA of the thus obtained recombinant strain was prepared by the method described in Japanese Published Unexamined Patent Application No. 189774/94, and PCR was carried out using the chromosomal DNA as a template and using the synthetic DNA shown in SEQ ID NO: 10 (5'-ATAAGCTTAATCGATCCGCTGTCCGGTA-3', containing a sequence corresponding to nucleotides 1758 to 1776 in SEQ ID NO: 1) and the synthetic DNA shown in SEQ ID NO: 11 (5'-ATGAATTCCCTCCAAAATCACATGCGCATT-3', containing a sequence corresponding to nucleotides 2710 to 2729 in SEQ ID NO: 1) as a primer set. The about 1.0 kb amplified DNA fragment was digested with restriction enzymes *Hind*III and *Eco*RI and about 1.0 kb amplified fragment was then separated and purified by agarose gel electrophoresis. Plasmid vector pUC19 was also digested with restriction enzymes *Hind*III and *Eco*RI and then separated and purified by agarose gel electrophoresis. The two DNA fragments thus obtained were ligated at 16°C for 16 hours using Ligation High and then used to the transformation of *Escherichia coli* DH5α. IPTG and X-gal were spread on the LB agar medium containing 50 µg/ml ampicillin for selecting the transformant. Several strains were selected among from the transformants, obtained as white colonies, and inoculated on 10 ml of LB medium containing 50 µg/ml ampicillin. After the transformants were cultured by shaking, cells were harvested and plasmid carried by each strain was extracted and purified by the alkaline method. The thus obtained plasmid was used to determine the nucleotide sequence in the manner as described in (3) above. Thus, it was confirmed that the subject recombinant *Streptomtces avermitilis* KS 1 mut strain was obtained.

### [Example 3] Synthesis of substrate compound

Physicochemical data of the following compounds were measured using the following instruments.

| | | |
|---|---|---|
| MS | JEOL. Ltd | HX/HX110A |
| ¹H NMR | JEOL. Ltd | Lambda 300 (300 MHz) |

In the physical data of the compounds, "FABMS" indicates the mass spectrum obtained by the "FAB" method. The term "conventional post-processing" refers to processing after the reaction.

After the completion of the reaction in each step, water, acids, buffers or the like is optionally added to the reaction solution to extract with a non-aqueous solvent such as ethyl acetate, ether, chloroform, and dichloromethane. The extract is washed with water, a saline solution, etc. and then dried over anhydrous sodium sulfate, thereby removing the solvent by distillation under reduced pressure.

### (1) Synthesis of Compound 1

Compound A (16 g, 0.060 mol; Table 1) was dissolved in methanol (620 mL) and ozone-air current was blown at -78°C while stirring for 4 hours. After air was blown into the reaction solution for 15 minutes, dimethylsulfide (44 mL, 0.60 mol) was added thereto, and the mixture was stirred at 25°C for 15 hours. After the conventional post-processing, the residue was dissolved in toluene (290 mL), methyl (triphenylphosphoranylidene) acetate (33.7 g, 0.10 mol) was added, and the mixture was stirred at 65°C for 17 hours. After conventional post-processing, purification was carried out by chromatography on silica gel (eluted at hexane/ethyl acetate = 100/0 to 10/1) to give Compound 1 (9.4 g, yield 53%; Table 1).

¹H NMR (CDCl₃) δ ppm; 7.04 (dd, J = 8.3, 15.8 Hz, 1H), 5.78 (dd, J = 1.1, 15.7 Hz, 1H), 3.72 (s, 3H), 3.48 (t, J = 3.5 Hz, 1H), 2.52 (m, 1H), 1.35-1.54 (m, 2H), 1.10 (m, 1H), 1.04 (d, J = 7.0 Hz, 3H), 0.40 (s, 9H), 0.37 (d, J = 7.4 Hz, 3H), 0.35 (d, J = 6.8 Hz, 3H), 0.03(s, 3H), 0.02 (s, 3H)
FABMS: M/Z 315 (M+H)⁺
Molecular formula-based theoretical value: C₁₇H₃₄N₃Si = 314

### (2) Synthesis of Compound 2

Compound 1 (0.20 g, 0.63 mmol) was dissolved in methanol (8.9 mL) and 10% hydrogen chloride/methanol solution (0.99 mL) was added thereto, and the mixture was stirred at 50°C for 1 hour. After conventional post-processing, the residue was dissolved in N,N-dimethylformamide (6.2 mL), chlorotritylsilane (0.31 mL, 1.8 mmol) and imidazole (0.21 g, 3.1 mmol) was added thereto, and the mixture was stirred at 25°C for 1.5 hours. After conventional post-processing, purification was carried out by chromatography on silica gel (eluted at hexane/ethyl acetate = 25/1) to give Compound 2 (0.18 g, yield 93%; Table 1).
¹H NMR (CDCl₃) δ ppm; 7.04 (dd, J = 8.4, 15.7 Hz, 1H), 5.79 (dd, J = 1.1, 15.7 Hz, 1H), 3.73 (s, 3H), 3.48 (dd, J = 4.1, 5.4 Hz, 1H), 2.51 (m, 1H), 1.35-1.51 (m, 2H), 1.12 (m, 1H), 0.81-1.08 (m, 18H), 0.47-0.66 (m, 6H)
FABMS: m/z 315 (M+H)⁺
Molecular formula-based theoretical value: C₁₇H₃₄N₃Si = 314

### (3) Synthesis of Compound 3

Compound 2 (4.1 g, 0.013 mol) was dissolved in ethanol (200 mL), 10% palladium-carbon (0.41 g) was added thereto, and the mixture was stirred under hydrogen atmosphere at 25°C for 4.5 hours. After the reaction solution was passed through Celite R545, the solvent was removed by distillation under reduced pressure. The residue was dissolved in 1,4-dioxane (100 mL) and water (100 mL), an aqueous solution of 4 mol/l potassium hydroxide (6.4 mL, 0.026 mol) was added thereto, and the mixture was stirred at 60°C for 3.5 hours. DOWEX 50W was added to the reaction solution for neutralization and the solvent was then removed by distillation under reduced pressure. The residue was dissolved in dichloromethane (200 mL), N-acetylcysteamine (1.8 mL, 0.017 mol), hydrochloric acid/1-ethyl-3-(3'-dimethylaminopropyl)carbodiimide (3.2 g, 0.017 mol), and 4-dimethylaminopyridine (0.32 g, 0.0026 mol) were added thereto, and the mixture was stirred at 25°C for 11 hours. After conventional post-processing, purification was carried out by chromatography on silica gel (eluted at hexane/ethyl acetate = 1/1) to give Compound 3 (3.8 g, yield 74%; Table 1).
¹H NMR (CDCl₃) δ ppm; 5.80 (br s, 1H), 3.43 (dd, J= 6.1, 12.5 Hz, 2H), 3.32 (dd, J= 3.7, 5.3 Hz, 1H), 3.02 (t, J= 6.6 Hz, 2H), 2.63 (dd, J= 5.3, 9.9 Hz, 1H), 2.54 (dd, J= 6.3, 9.4 Hz, 1H), 1.97 (s, 3H), 1.94 (m, 1H), 1.58 (m, 1H), 1.31-1.54 (m, 3H), 1.16 (m, 1H), 0.81-1.00 (m, 18H), 0.61 (q, J =7.6Hz, 6H)
FABMS: m/z 404 (M+H)⁺
Molecular formula-based theoretical value: C₂₀H₄₁NO₃SiS= 403

### (4) Synthesis of Compound 4

Compound 3 (15 mg, 0.038 mmol) was dissolved in tetrahydrofuran (0.46 mL) and water (0.46 mL), acetic acid (0.45 mL) was added thereto, and the mixture was stirred at 0°C for 2 hours. After conventional post-processing, purification was carried out by thin-layer chromatography (eluted at chloroform/methanol = 10/1) to give Compound 4 (7.7 mg, yield 71%, purity 63%; Table 1).
¹H NMR (CDCl₃) δ ppm; 5.88 (br s, 1H), 3.64 (dd, J= 6.0, 12.3 Hz, 2H), 3.20 (m, 1H), 3.02 (dt, J= 1.8, 6.4 Hz, 2H), 2.58-2.72 (m, 2H), 2.06 (m, 1H), 1.97 (s, 3H), 1.43-1.70 (m, 3H), 1.33 (m, 1H), 1.28 (m, 1H), 0.82-0.95 (m, 9H)
FABMS: m/z 290 (M+H)⁺
Molecular formula-based theoretical value: C₁₄H₂₇NO₃S = 289

### [Example 4] Direct production of 22,23-dihydroavermectin B1a

10 µl of spore suspension of *Streptomyces avermitilis* KS1mut obtained in Example 2 was inoculated in a test tube containing 10 ml of seed culture medium [a medium prepared by adjusting a solution containing 20 g of lactose, 15 g of Distillers solubles, 2.5 g of autolysed yeast (Difco), and 1,000 ml of distilled water at pH 7.2 with 2 mol/l potassium hydroxide, followed by high pressure steam sterilization at 121°C for 15 minutes] and was cultured by shaking at 28°C for 20 hours to obtain a seed culture. 0.4 ml of this seed culture was transferred to a conical flask (volume 100 ml) containing 20 ml of production medium [a medium prepared by subjecting 46 g of glucose, 24 g of peptonized milk (Oxoid), 2.5 g of autolysed yeast (Difco), 2.5 ml of polypropylene glycol #2000, and 1,000 ml of distilled water to high pressure steam sterilization at 121°C for 15 minutes] and was cultured using a rotary shaker at 28°C for 3 days at 220 rpm, then 50 µl of 1 mg/ml methanol solution of Compound 4 synthesized in Example 3 (containing 50% Compound 4) was added to the culture, and culturing by shaking was carried out again at 28°C for 2 days. After the completion of culture, a double amount of methanol was added to the culture and the mixture was thoroughly stirred. Thereafter, the stirred product was centrifuged at room temperature at 3,000 rpm for 5 minutes to precipitate cells. The supernatant was then subjected to high-performance liquid chromatography (HPLC) analysis.
HPLC analysis
Chromatography condition
Column: Inertsil ODS-2 (4.6 x 150 mm, manufactured by GL Sciences Inc.)
Guard column: Guard column E cartridge (4 x 10 mm, manufactured by GL Sciences Inc.)
Mobile phase: acetonitrile : methanol : water = 70 : 10 : 20
Flow rate: 0.6 ml/min
Detection: 246 nm
Temperature: 55°C

The methanol extract of the culture was analyzed under the above conditions for analysis and, as a result, a peak was observed at a retention time of 21. 7 minutes only in the culture extract to which Compound 4 was added. As a result of the analysis of 22,23-dihydroavermectin B1a under the equivalent condition, the retention time was the same, i.e., 21.7 minutes. When 22,23-dihydroavermectin B1a was determined as the standard, the yield of the substance exhibiting the retention time of 21.7 minutes, which was obtained from the culture extract, was 23.3 mg/L.

Three-dimensional HPLC analysis was carried out using a multi-wavelength detector MD-915 (manufactured by Jasco) and, as a result, the maximal absorption wavelength of the peak at the retention time of 21.7 minutes was 248 nm and the spectrum thereof coincided with that of 22,23-dihydroavermectin B1a.

The peak at the retention time of 21.7 minutes was fractionated by HPLC and 5 mg of white powder was obtained and subjected to mass spectometry. The results were as follows.

m/z 873.5 (M+) C₄₈H₇₃O₁₄

This coincided with data of 22,23-dihydroavermectin B1a described in Ivermectin and Abamectin, William C. Campbell (1989).

As is apparent from the foregoing description, the substance, which was obtained by adding Compound 4 to *Streptomyces avermitilis* KS1mut and culturing the strain, was 22,23-dihydrbavermectin B1a. In the above culturing with addition of compound 4, avermectin analog other than 22,23-dihydroavermectin B1a was not produced at all. Since the single production of 22,23-dihydroavermectin B1a was realized, the production of 22,23-dihydroavermectin B1a was shown to have been significantly facilitated.

### INDUSTRIAL APPLICABILITY

According to the present invention, 22,23-dihydroavermectin B1a, which is useful as a medicine, a veterinary drug, and a pesticide, can be directly produced. Therefore, the conventional processes for purifying avermectin B1a at an industrial level and for chemically modifying avermectin B1a, which are complicated and difficult, can be omitted. This can significantly decrease the cost and the time for the industrial production of 22,23-dihydroavermectin B1a. This also realizes the production of the formulation containing only 22,23-dihydroavermectin B1a, which is highly effective as medicines.

### [Sequence Listing Free Text]

SEQ ID NO: 9 represents synthetic DNA based on the sequence between nucleotides 1954 and 1985 shown in SEQ ID NO: 1

SEQ ID NO: 10 represents synthetic DNA based on the sequence between nucleotides 1758 and 1776 shown in SEQ ID NO: 1

SEQ ID NO: 11 represents synthetic DNA based on the sequence between nucleotides 2710 and 2729 in SEQ ID NO: 1

## Claims

1. An N-acetylcysteamine thioester compound represented by formula (I): 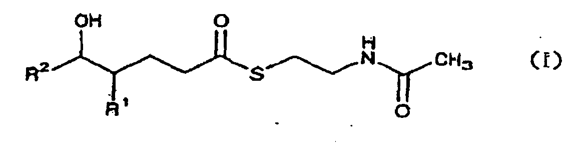 wherein R¹ is methyl and R² is sec-butyl.

2. A process for producing an N-acetylcysteamine thioester compound of claim 1, which is **characterized by** employing, as a starting material, a compound represented by formula (II): 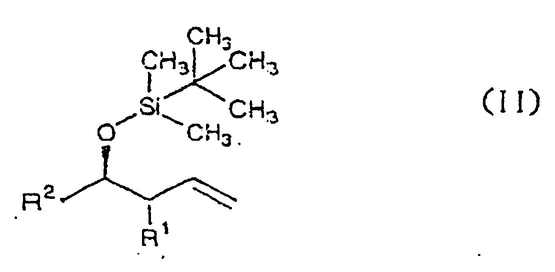 wherein R¹ is methyl and R² is sec-butyl, and comprising the steps of:
(a) ozone-oxidating the compound, and thereafter adding carbon chains by the Wittig reaction;
(b) deprotecting t-butyldimethylsilyl group of the compound obtained in step (a) and reintroducing another protecting group using chlorotriethylsilane;
(c) reducing α-β unsaturated carbon bond of the resultant compound in the presence of a palladium-carbon catalyst, hydrolyzing an ester with potassium hydroxide, neutralizing the reaction mixture, and adding N-acetylcysteamine in the presence of a condensing agent to obtain a thioester compound; and
(d) removing the protecting group by adding acetic acid to the thioester compound.

3. A process for producing 22,23-dihydroavermectin B1a, comprising the steps of:
(a) contacting a culture of a non-human transformant which contains a DNA encoding a modified avermectin aglycon synthase comprising at least one domain with an eliminated activity, wherein the domain is selected from the group consisting of ATs [amino acid 29 to 344 of SEQ ID NO: 4], ACPs [amino acid 366 to 451 of SEQ ID NO: 4], KS₁ [amino acid 481 to 914 of SEQ ID NO: 4], AT1 [amino acid 1051 to 1356 of SEQ ID NO: 4], ACP1 [amino acid 1979 to 2060 of SEQ ID NO: 4] and KS2 [amino acid 2086 to 2515 of SEQ ID NO: 4], or a treated product thereof or the synthase encoded by the DNA with the N-acetylcysteamine thioester compound according to claim 1 in a medium; and
(b) collecting 22,23-dihydroavermectin B1a produced and accumulated in the medium.

4. The process for producing 22,23-dihydroavermectin B1a of claim 3, **characterized in that** an N-acetylcysteamine thioester compound of claim 1 is employed as a substrate compound for a modified avermectin aglycon synthase comprising at least one domain with an eliminated activity, wherein the domain is selected from the group consisting of ATs [amino acid 29 to 344 of SEQ ID NO: 4], ACPs [amino acid 366 to 451 of SEQ ID NO: 4], KS1 [amino acid 481 to 914 of SEQ ID NO: 4], AT1 [amino acid 1051 to 1356 of SEQ ID NO: 4], ACP1 [amino acid 1979 to 2060 of SEQ ID NO: 4] and KS2 [amino acid 2086 to 2515 of SEQ ID NO: 4].

5. The process of claim 3 or 4, wherein the modified avermectin aglycon synthase comprises mutated ATs [amino acid 29 to 344 of SEQ ID NO: 4], ACPs [amino acid 366 to 451 of SEQ ID NO: 4], KS1 [amino acid 481 to 914 of SEQ ID NO: 4], AT1 [amino acid 1051 to 1356 of SEQ ID NO: 4], ACP1 [amino acid 1979 to 2060 of SEQ ID NO: 4] and KS2 [amino acid 2086 to 2515 of SEQ ID NO: 4] in which a specific amino acid in an active center is substituted with another amino acid.

6. The process of any one of claims 3 to 5, wherein the modified avermectin aglycon synthase comprises a polypeptide consisting of the amino acid sequence shown in SEQ ID NO: 8.

7. The process of claim 3, wherein the DNA comprises a DNA consisting of the nucleotide sequence shown in SEQ ID NO: 3.

8. The process of claim 3, wherein the non-human transformant is a microorganism.

9. The process of claim 8, wherein the microorganism belongs to the genus *Streptomyces.*

10. The process of claim 9, wherein the microorganism belonging to the genus *Streptomyces* is *Streptomyces avermitilis.*

## Patentansprüche

1. N-Acetylcysteanünthioester-Verbindung dargestellt durch die Formel (I): 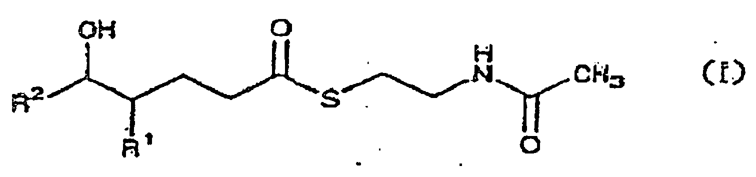 wobei R¹ Methyl ist und R² sec-Butyl ist.

2. Verfahren zur Herstellung der N-Acetylcysteaminthioester-Verbindung gemäß Anspruch 1, **dadurch gekennzeichnet, dass** eine Verbindung, dargestellt durch Formel (11) 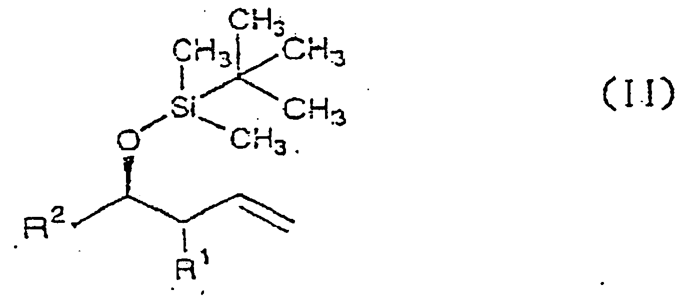 als Ausgangsmaterial verwendet wird,
wobei R¹ Methyl ist und R² sec-Butyl ist und das Verfahren die Schritte umfaßt:
(a) Ozonoxidation der Verbindung und nachfolgende Zugabe von Kohlenstoffketten mittels der Wittig-Reaktion;
(b) Entfernen der Schutzgruppe t-Butyldimethylsilyl von der in Schritt (a) erhaltenen Verbindung und Wiedereinführen einer anderen Schutzgruppe unter Verwendung von Chlortriethylsilan;
(c) Reduzieren der ungesättigten α-β-Kohlenstoffbindung der resultierenden Verbindung in Gegenwart eines Palladium-Kohlenstoff-Katalysators, Hydrolysieren eines Esters mit Kaliumhydroxid, Neutralisieren des Reaktionsgemisches und Zugabe von N-Acetylcysteamin in Gegenwart eines Kondensierungsmittels, um eine Thioesterverbindung zu erhalten; und
(d) Entfernen der Schutzgruppe durch Zugabe von Essigsäure zu der Thioesterverbindung.

3. Verfahren zur Herstellung von 22,23-Dihydroavermectin B1a, das die Schritte umfasst:
(a) das Inkontaktbringen einer Kultur einer nicht-menschlichen Transformanten, die eine DNA enthält, die eine modifizierte Avermectin-Aglyconsynthase codiert, die mindestens eine Domäne mit einer beseitigten Aktivität umfasst, wobei die Domäne ausgewählt ist aus der Gruppe bestehend aus ATs [Aminosäure 29 bis 344 von SEQ ID NO. 4], ACPs [Aminosäure 366 bis 451 von SEQ ID NO. 4], KS1 [Aminosäure 481 bis 914 von SEQ ID NO. 4], AT1 [Aminosäure 1051 bis 1356, von SEQ ID NO. 4], ACP1 [Aminosäure 1979 bis 2060 von SEQ ID NO. 4] und KS2 [Aminosäure 2086 bis 2515 von SEQ ID NO. 4], oder ein behandeltes Produkt davon, oder die Synthase, die durch die DNA codiert wird, mit der N-Acetylcysteaminthioester-Verbindung gemäß Anspruch 1 in einem Medium;
(b) das Sammeln des produzierten und in dem Medium angereicherten 22, 23-Dihydroavermectins B1a.

4. Verfahren zur Herstellung von 22,23-Dihydroavermectin B1a gemäß Anspruch 3, **dadurch gekennzeichnet, dass** eine N-Acetylcysteaminthioester-Verbindung gemäß Anspruch 1 als Substratverbindung für eine modifizierte Avermectin-Aglyconsynthase benutzt wird, die mindestens eine Domäne mit einer beseitigten Aktivität umfasst, wobei die Domäne ausgewählt ist aus der Gruppe bestehend aus ATs [Aminosäure 29 bis 344 von SEQ ID NO. 4], ACPs [Aminosäure 366 bis 451 von SEQ ID NO. 4], KS1 [Aminosäure 481 bis 914 von SEQ ID NO. 4], AT1 [Aminosäure 1051 bis 1356, von SEQ ID NO. 4], ACP1 [Aminosäure 1979 bis 2060 von SEQ ID NO. 4] und KS2 [Aminosäure 2086 bis 2515 von SEQ ID NO. 4]

5. Verfahren gemäß Anspruch 3 oder 4, wobei die modifizierte Avermectin-Aglyconsynthase mutierte ATs [Aminosäure 29 bis 344 von SEQ ID NO. 4], ACPs [Aminosäure 366 bis 451 von SEQ ID NO. 4], KS1 [Aminosäure 481 bis 914 von SEQ ID NO. 4], AT1 [Aminosäure 1051 bis 1356, von SEQ ID NO. 4], ACP1 [Aminosäure 1979 bis 2060 von SEQ ID NO. 4] und KS2 [Aminosäure 2086 bis 2515 von SEQ ID NO. 4] umfasst, in denen eine spezifische Aminosäure in einem aktiven Zentrum mit einer anderen Aminosäure ausgetauscht ist.

6. Verfahren gemäß einem der Ansprüche 3 bis 5, wobei die modifizierte Avermectin-Aglyconsynthase ein Polypeptid umfasst, das aus der Aminosäuresequenz wie dargestellt in SEQ ID NO. 8 besteht.

7. Verfahren gemäß Anspruch 3, wobei die DNA eine DNA umfasst, die aus der Nucleotidsequenz wie dargestellt in SEQ ID NO. 3 besteht.

8. Verfahren gemäß Anspruch 3, wobei die nicht-menschliche Transformante ein Mikroorganismus ist.

9. Verfahren gemäß Anspruch 8, wobei der Mikroorganismus der Gattung *Streptomyces* angehört.

10. Verfahren nach Anspruch 9, wobei der Mikroorganismus, der der Gattung Streptomyces angehört, *Streptomyces avermitilis* ist.

## Revendications

1. Composé thioester de la N-acétylcystéamine représenté par la formule (I) : 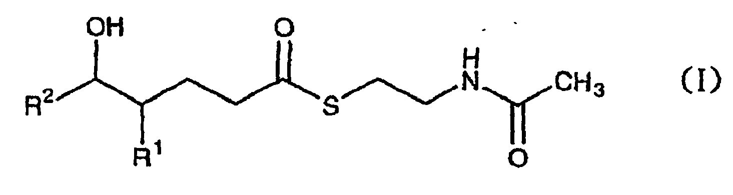 dans laquelle R¹ est un groupe méthyle et R² est un groupe sec-butyle.

2. Procédé de production d'un composé thioester de la N-acétylcystéamine selon la revendication 1, lequel est **caractérisé par** l'utilisation, comme substance de départ, d'un composé représenté par la formule (II) : 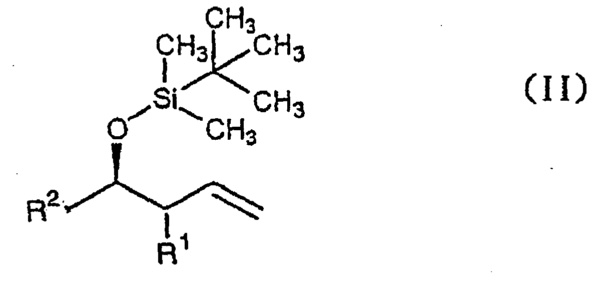 dans laquelle R¹ est un groupe méthyle et R² est un groupe sec-butyle, et comprenant les étapes consistant :
(a) à oxyder par l'ozone le composé, et après cela à ajouter des chaînes carbonées par la réaction de Wittig ;
(b) à déprotéger le groupe ter-butyldiméthylsilyle du composé obtenu dans l'étape (a) et à réintroduire un autre groupe protecteur à l'aide de chlorotriéthylsilane ;
(c) à réduire la liaison carbonée α-β-insaturée du composé résultant en présence d'un catalyseur au palladium-carbone, à hydrolyser un ester avec de l'hydroxyde de potassium, à neutraliser le mélange réactionnel, et à ajouter de la N-acétylcystéamine en présence d'un agent de condensation pour obtenir un composé thioester ; et
(d) à éliminer le groupe protecteur en ajoutant de l'acide acétique au composé thioester.

3. Procédé de production de 22,23-dihydroavermectine B1a, comprenant les étapes consistant :
(a) à mettre en contact une culture d'une bactérie transformée non humaine qui contient un ADN codant pour une synthase d'avermectine aglycone modifiée comprenant au moins un domaine ayant une activité éliminée, dans lequel le domaine est choisi dans le groupe constitué par les domaines AT [acides aminés 29 à 344 de la SEQ ID n° 4], ACP [acides aminés 366 à 451 de la SEQ ID n° 4], KS1 [acides aminés 481 à 914 de la SEQ ID n° 4], AT1 [acides aminés 1051 à 1356 de la SEQ ID n° 4], ACP1 [acides aminés 1979 à 2060 de la SEQ ID n° 4] et KS2 [acides aminés 2086 à 2515 de la SEQ ID n° 4], ou un produit traité de celui-ci ou la synthase codée par l'ADN avec le composé thioester de la N-acétylcystéamine selon la revendication 1 dans un milieu ; et
(b) à recueillir la 22,23-dihydroavermectine B1a produite et accumulée dans le milieu.

4. Procédé de production de 22,23-dihydroavermectine B1a selon la revendication 3, **caractérisé en ce que** le composé thioester de la N-acétylcystéamine selon la revendication 1 est utilisé comme composé substrat pour une synthase d'avermectine aglycone modifiée comprenant au moins un domaine ayant une activité éliminée, dans lequel le domaine est choisi dans le groupe constitué par les domaines AT [acides aminés 29 à 344 de la SEQ ID n° 4], ACP [acides aminés 366 à 451 de la SEQ ID n° 4], KS1 [acides aminés 481 à 914 de la SEQ ID n° 4], AT1 [acides aminés 1051 à 1356 de la SEQ ID n° 4], ACP1 [acides aminés 1979 à 2060 de la SEQ ID n° 4] et KS2 [acides aminés 2086 à 2515 de la SEQ ID n° 4].

5. Procédé selon la revendication 3 ou 4, dans lequel la synthase d'avermectine aglycone modifiée comprend des domaines AT [acides aminés 29 à 344 de la SEQ ID n° 4], ACP [acides aminés 366 à 451 de la SEQ ID n° 4], KS1 [acides aminés 481 à 914 de la SEQ ID n° 4], AT1 [acides aminés 1051 à 1356 de la SEQ ID n° 4], ACP1 [acides aminés 1979 à 2060 de la SEQ ID n° 4] et KS2 [acides aminés 2086 à 2515 de la SEQ ID n° 4] mutés dans lesquels un acide aminé spécifique dans un centre actif est remplacé par un autre acide aminé.

6. Procédé selon l'une quelconque des revendications 3 à 5, dans lequel la synthase d'avermectine aglycone modifiée comprend un polypeptide constitué par la séquence d'acides aminés montrée dans la SEQ ID n° 8.

7. Procédé selon la revendication 3, dans lequel l'ADN comprend un ADN constitué par la séquence nucléotidique montrée dans la SEQ ID n° 3.

8. Procédé selon la revendication 3, dans lequel la bactérie transformée non humaine est un microorganisme.

9. Procédé selon la revendication 8, dans lequel le microorganisme appartient au genre *Streptomyces.*

10. Procédé selon la revendication 9, dans lequel le microorganisme appartenant au genre *Streptomyces* est *Streptomyces avermitilis.*


### SEQUENCE LISTING

<110> KYOWA HAKKO KOGYO CO., LTD. ; THE KITASATO INSTITUTE
<120> A METHOD FOR PROCUDING AVERMECTIN DERIVATIVES
<130> 11278W01
<150> JP 00/047405
   <151> 2000-02-24
<160> 11
<170> Patentln Ver. 2.0
<210> 1
   < 211 > 30690
   <212> DNA
   <213> Streptomyces avermitilis
<220>
   <221> CDS
   <222> (1)..(11916)
<220>
   <221> CDS
   <222> (11971).. (30687)
<400> 1
   gtg cag agg atg gac ggc ggg gaa gaa ccc cgc cct gcg gca ggg gag      48
   Val Gln Arg Met Asp Gly Gly Glu Glu Pro Arg Pro Ala Ala Gly Glu
    1               5                  10                  15
   gtc ctc gga gtg gcc gac gag gcg gac ggc ggc gtc gtc ttc gtt ttt      96
   Val Leu Gly Val Ala Asp Glu Ala Asp Gly Gly Val Val Phe Val Phe
               20                  25                  30
   ccc ggg cag ggc ccg caa tgg ccg ggc atg gga agg gaa ctt ctc gac     144
   Pro Gly Gln Gly Pro Gln Trp Pro Gly Met Gly Arg Glu Leu Leu Asp
           35                  40                  45
   gct tcc gac gtc ttc cgg gag agc gtc cgc gcc tgc gaa gcc gcg ttc     192
   Ala Ser Asp Val Phe Arg Glu Ser Val Arg Ala Cys Glu Ala Ala Phe
       50                  55                  60
   gcg ccc tac gtc gac tgg tcg gtg gag cag gtg ttg cgg gac tcg ccg     240
   Ala Pro Tyr Val Asp Trp Ser Val Glu Gln Val Leu Arg Asp Ser Pro
   65                  70                  75                  80
   gac gct ccc ggg ctg gac cgg gtg gac gtc gtc cag ccg acc ctg ttc     288
   Asp Ala Pro Gly Leu Asp Arg Val Asp Val Val Gln Pro Thr Leu Phe
                   85                  90                  95
   gcc gtc atg atc tcc ctg gcc gcc ctc tgg cgc tcg caa ggg gtc gag     336
   Ala Val Met Ile Ser Leu Ala Ala Leu Trp Arg Ser Gln Gly Val Glu
               100                 105                 110
   ccg tgc gcg gtg ctg gga cac agc ctg ggc gag atc gcg gca gcc cac      384
   Pro Cys Ala Val Leu Gly His Ser Leu Gly Glu Ile Ala Ala Ala His
           115                 120                 125
   gtc tcg gga ggc ctg tcc ctg gcc gac gcc gca cgc gtg gtg acg ctt      432
   Val Ser Gly Gly Leu Ser Leu Ala Asp Ala Ala Arg Val Val Thr Leu
       130                 135                 140
   tgg agc cag gca cag acc acc ctt gcc ggg acc ggc gcg ctc gtc tcc      480
   Trp Ser Gln Ala Gln Thr Thr Leu Ala Gly Thr Gly Ala Leu Val Ser
   145                 150                 155                 160
   gtc gcc gcc acg ccg gat gag ctc ctg ccc cga atc gct ccg tgg acc      528
   Val Ala Ala Thr Pro Asp Glu Leu Leu Pro Arg Ile Ala Pro Trp Thr
                   165                 170                 175
   gag gac aac ccg gcg cgg ctc gcc gtc gca gcc gtc aac gga ccc cgg      576
   Glu Asp Asn Pro Ala Arg Leu Ala Val Ala Ala Val Asn Gly Pro Arg
               180                 185                 190
   agc aca gtc gtt tcc ggt gcc cgc gag gcc gtc gcg gac ctg gtg gcc      624
   Ser Thr Val Val Ser Gly Ala Arg Glu Ala Val Ala Asp Leu Val Ala
           195                 200                 205
   gac ctc acc gcc gcg cag gtg cgc acg cgc atg atc ccg gtg gac gtt      672
   Asp Leu Thr Ala Ala Gln Val Arg Thr Arg Met Ile Pro Val Asp Val
       210                 215                 220
   ccc gcc cac tcc ccc ctg atg tac gcc atc gag gaa cgg gtc gtc agc      720
   Pro Ala His Ser Pro Leu Met Tyr Ala Ile Glu Glu Arg Val Val Ser
   225                 230                 235                 240
   ggc ctg ctg ccc atc acc cca cgc ccc tcc cgc atc ccc ttc cac tcc      768
   Gly Leu Leu Pro Ile Thr Pro Arg Pro Ser Arg Ile Pro Phe His Ser
                   245                 250                 255
   tcg gtg acc ggc ggc cgc ctc gac acc cgc gag cta gac gcg gcg tac      816
   Ser Val Thr Gly Gly Arg Leu Asp Thr Arg Glu Leu Asp Ala Ala Tyr
               260                 265                 270
   tgg tac cgc aac atg tcg agc acg gtc cgg ttc gag ccc gcc gcc cgg      864
   Trp Tyr Arg Asn Met Ser Ser Thr Val Arg Phe Glu Pro Ala Ala Arg
           275                 280                 285
   ctg ctt ctg cag cag ggg ccc aag acg ttc gtc gag atg agc ccg cac      912
   Leu Leu Leu Gln Gln Gly Pro Lys Thr Phe Val Glu Met Ser Pro His
       290                 295                 300
   ccg gtg ctg acc atg ggc ctc cag gag ctc gcc ccg gac ctg ggc gac      960
   Pro Val Leu Thr Met Gly Leu Gln Glu Leu Ala Pro Asp Leu Gly Asp
   305                 310                 315                 320
   acc acc ggc acc gcc gac acc gtg atc atg ggc acg ctg cgc cgc ggc     1008
   Thr Thr Gly Thr Ala Asp Thr Val Ile Met Gly Thr Leu Arg Arg Gly
                   325                 330                 335
   cag ggc acc ctg gac cac ttc ctg acg tct ctc gcc caa cta cgg ggg     1056
   Gln Gly Thr Leu Asp His Phe Leu Thr Ser Leu Ala Gln Leu Arg Gly
               340                 345                 350
   cat ggt gag acg tcg gcg acc acc gtc ctc tcg gca cgc ctg acc gcg     1104
   His Gly Glu Thr Ser Ala Thr Thr Val Leu Ser Ala Arg Leu Thr Ala
           355                 360                 365
   ctg tcc ccc acg cag cag cag tcg ctg ctc ctg gac ctg gtg cgc gcc     1152
   Leu Ser Pro Thr Gln Gln Gln Ser Leu Leu Leu Asp Leu Val Arg Ala
       370                 375                 380
   cac acc atg gcg gtg ctg aac gac gac gga aac gag cgc acc gcg tcg     1200
   His Thr Met Ala Val Leu Asn Asp Asp Gly Asn Glu Arg Thr Ala Ser
   385                 390                 395                 400
   gat gcc ggc cca tcg gcg agt ttc gcc cac ctc ggc ttc gac tcc gtc     1248
   Asp Ala Gly Pro Ser Ala Ser Phe Ala His Leu Gly Phe Asp Ser Val
                   405                 410                 415
   atg ggt gtc gaa ctg cgc aac cgc ctc agc aag gcc acg ggc ctg cgg     1296
   Met Gly Val Glu Leu Arg Asn Arg Leu Ser Lys Ala Thr Gly Leu Arg
               420                 425                 430
   ttg ccc gtg acg ctc atc ttc gac cac acc acg ccg gcc gcg gtc gcc     1344
   Leu Pro Val Thr Leu Ile Phe Asp His Thr Thr Pro Ala Ala Val Ala
           435                 440                 445
   gcg cgc ctt cgg acc gcg gcg ctc ggc cac ctc gac gag gac acc gcg     1392
   Ala Arg Leu Arg Thr Ala Ala Leu Gly His Leu Asp Glu Asp Thr Ala
       450                 455                 460
   ccc gta ccg gac tca ccc agc ggc cac gga ggc acg gca gcg gcg gac     1440
   Pro Val Pro Asp Ser Pro Ser Gly His Gly Gly Thr Ala Ala Ala Asp
   465                 470                 475                 480
   gac ccg atc gcc atc atc ggc atg gca tgc cgt ttc ccg ggc gga gtc     1488
   Asp Pro Ile Ala Ile Ile Gly Met Ala Cys Arg Phe Pro Gly Gly Val
                   485                 490                 495
   cgg tcc ccg aag gac ctg tgg gag ctg gcc gcc tcg ggc gga gac gcc     1536
   Arg Ser Pro Lys Asp Leu Trp Glu Leu Ala Ala Ser Gly Gly Asp Ala
               500                 505                 510
   atc ggg ccg ttc ccc acc gac cgc gga tgg ccc acg gaa cag cgt cac     1584
   Ile Gly Pro Phe Pro Thr Asp Arg Gly Trp Pro Thr Glu Gln Arg His
           515                 520                 525
   gcc cag gac ccc acg cag ccc ggc acg ttc tat ccg cag gga ggc ggg     1632
   Ala Gln Asp Pro Thr Gln Pro Gly Thr Phe Tyr Pro Gln Gly Gly Gly
       530                 535                 540
   ttc ctt cac gac gcg gcg cac ttc gac gcc ggc ttc ttc gga atc agt     1680
   Phe Leu His Asp Ala Ala His Phe Asp Ala Gly Phe Phe Gly Ile Ser
   545                 550                 555                 560
   cca cgt gag gca ctg gcg atg gat ccg cag cag cgg ctg ctg ctg gag     1728
   Pro Arg Glu Ala Leu Ala Met Asp Pro Gln Gln Arg Leu Leu Leu Glu
                   565                     570                     575
   acg tcc tgg gag gcg ttc gag cgg gcg gga atc gat ccg ctg tcg gta      1776
   Thr Ser Trp Glu Ala Phe Glu Arg Ala Gly Ile Asp Pro Leu Ser Val
               580                     585                     590
   cgc ggg tcc cgt acg ggc gtc ttc gcg ggc gcc ctc tcc ttc gac tac      1824
   Arg Gly Ser Arg Thr Gly Val Phe Ala Gly Ala Leu Ser Phe Asp Tyr
           595                     600                     605
   ggc ccg cgt atg gac acc gcg tcg tcg gag ggc gcc gcg gac gtg gag      1872
   Gly Pro Arg Met Asp Thr Ala Ser Ser Glu Gly Ala Ala Asp Val Glu
       610                     615                     620
   ggc cac atc ctc acc ggt acc acg ggc agc gtc ctg tcg ggc cgt atc      1920
   Gly His Ile Leu Thr Gly Thr Thr Gly Ser Val Leu Ser Gly Arg Ile
   625                     630                     635                     640
   gcc tac agc ttc ggg ctg gaa ggg ccg gcg atc acc gtg gac acg ggg      1968
   Ala Tyr Ser Phe Gly Leu Glu Gly Pro Ala Ile Thr Val Asp Thr Gly
                   645                     650                     655
   tgc tcg gca tcg ctc gtg acg ctg cat ctg gcg tgc cag tcg ctg cgg      2016
   Cys Ser Ala Ser Leu Val Thr Leu His Leu Ala Cys Gln Ser Leu Arg
               660                     665                     670
   tcg ggt gag tgc acg ctc gcg ctg gcc ggc ggc gtc tcg gtc atg tcc      2064
   Ser Gly Glu Cys Thr Leu Ala Leu Ala Gly Gly Val Ser Val Met Ser
           675                     680                     685
   acc ctc ggc atg ttc atc gag ttc tcc cgg cag cgc ggg ctg tcg gtg      2112
   Thr Leu Gly Met Phe Ile Glu Phe Ser Arg Gln Arg Gly Leu Ser Val
       690                     695                     700
   gac ggc agg tgc aag gcg tac tcg gct gca gcc gac ggc acc ggc tgg      2160
   Asp Gly Arg Cys Lys Ala Tyr Ser Ala Ala Ala Asp Gly Thr Gly Trp
   705                     710                     715                     720
   ggc gag ggc gtc ggg atg ctg ttg gtg gag cgg ttg tcg gat gcg gtg      2208
   Gly Glu Gly Val Gly Met Leu Leu Val Glu Arg Leu Ser Asp Ala Val
                   725                     730                     735
   cgg ctg ggg cat cgg gtg ctg gcg gtg gta cgc ggc agt gcg gtc aac      2256
   Arg Leu Gly His Arg Val Leu Ala Val Val Arg Gly Ser Ala Val Asn
               740                     745                     750
   cag gac ggt gcg tcg aat ggg ctg acg gcg ccg aac ggt ccg gct cag      2304
   Gln Asp Gly Ala Ser Asn Gly Leu Thr Ala Pro Asn Gly Pro Ala Gln
           755                     760                     765
   gag cgg gtg atc cgg cag gcg ttg gcg aac gcg ggg ttg tcc gtg gcg      2352
   Glu Arg Val Ile Arg Gln Ala Leu Ala Asn Ala Gly Leu Ser Val Ala
       770                     775                     780
   gat gtg gat gtg gtg gag ggg cac ggg acg ggc acg acg ctg ggt gat      2400
   Asp Val Asp Val Val Glu Gly His Gly Thr Gly Thr Thr Leu Gly Asp
   785                     790                     795                     800
   ccg atc gag gca cag gcg ttg ctc gcc acg tac ggg cag cgg gcc ggt    2448
   Pro Ile Glu Ala Gln Ala Leu Leu Ala Thr Tyr Gly Gln Arg Ala Gly
                   805                 810                 815
   gac agg ccg ctg tgg ctg ggg tct ctg aag tcc aac atc ggg cac acc    2496
   Asp Arg Pro Leu Trp Leu Gly Ser Leu Lys Ser Asn Ile Gly His Thr
               820                 825                 830
   atg gct gcc gcg ggt gtg ggt ggg gtc atc aag atg gtg atg gcg ttg    2544
   Met Ala Ala Ala Gly Val Gly Gly Val Ile Lys Met Val Met Ala Leu
           835                 840                 845
   cgg gag ggg gtg ttg ccg cgg acg ttg cat gtg gat aag ccg tcg ccg    2592
   Arg Glu Gly Val Leu Pro Arg Thr Leu His Val Asp Lys Pro Ser Pro
       850                 855                 860
   cag gtg gac tgg tcc gcg ggg gcg gtg cgg ctg ctg acg gag gcg gtg    2640
   Gln Val Asp Trp Ser Ala Gly Ala Val Arg Leu Leu Thr Glu Ala Val
   865                 870                 875                 880
   ccg tgg ccg ggg gac gcg gca ggg cgg ttg cgg cgg gcg gga gtg tcg    2688
   Pro Trp Pro Gly Asp Ala Ala Gly Arg Leu Arg Arg Ala Gly Val Ser
                   885                 890                 895
   tcg ttc ggg atc ggc ggc acg aat gcg cat gtg att ttg gag gag gcg    2736
   Ser Phe Gly Ile Gly Gly Thr Asn Ala His Val Ile Leu Glu Glu Ala
               900                 905                 910
   ccg gcg gcg ggg ggc tgt gtt gcc ggg ggt ggg gtg ttg gag ggt gct    2784
   Pro Ala Ala Gly Gly Cys Val Ala Gly Gly Gly Val Leu Glu Gly Ala
           915                 920                 925
   ccg ggt ctt gcc att tcg gtg gct gag tcg gtg gcc gct cca gtg gct    2832
   Pro Gly Leu Ala Ile Ser Val Ala Glu Ser Val Ala Ala Pro Val Ala
       930                 935                 940
   gtg tct gcg ccg gtg gct gag tcg gtg ccg gtg ccg gtg ccg gtg ccg    2880
   Val Ser Ala Pro Val Ala Glu Ser Val Pro Val Pro Val Pro Val Pro
   945                 950                 955                 960
   gtt cct gtg ccg gtg tcg gct agg tct gag gct ggg ttg cgg gcg cag    2928
   Val Pro Val Pro Val Ser Ala Arg Ser Glu Ala Gly Leu Arg Ala Gln
                   965                 970                 975
   gcg gag gcg ttg cgt cag tac gtg gca gtc cgg ccg gac gtt tcg ctt    2976
   Ala Glu Ala Leu Arg Gln Tyr Val Ala Val Arg Pro Asp Val Ser Leu
               980                 985                 990
   gcc gat gtg ggt gcg ggt ctg gcc tgt ggg cgg gct gtg ctg gag cat    3024
   Ala Asp Val Gly Ala Gly Leu Ala Cys Gly Arg Ala Val Leu Glu His
           995                1000                1005
   cgt gcg gtc gtc ctg gcc gcg gac cgt gag gag ctg gtg caa ggg ttg    3072
   Arg Ala Val Val Leu Ala Ala Asp Arg Glu Glu Leu Val Gln Gly Leu
      1010                1015                1020
   ggg gcg ctg gcg gcg ggt gag ccg gat cgg cgg gtg acc acg ggt cat    3120
   Gly Ala Leu Ala Ala Gly Glu Pro Asp Arg Arg Val Thr Thr Gly His
   1025                1030                1035                1040
   gcg ccg ggt ggt gac cgg ggc ggt gtc gtc ttc gtg ttt ccc gga cag    3168
   Ala Pro Gly Gly Asp Arg Gly Gly Val Val Phe Val Phe Pro Gly Gln
                   1045                1050                1055
   ggt ggg cag tgg gcc ggg atg ggt gtg cgt ctg ctc gcc tcc tct ccg    3216
   Gly Gly Gln Trp Ala Gly Met Gly Val Arg Leu Leu Ala Ser Ser Pro
               1060                1065                1070
   gtg ttc gcc cgg cgg atg cag gcg tgc gag gag gct ctg gcg ccg tgg    3264
   Val Phe Ala Arg Arg Met Gln Ala Cys Glu Glu Ala Leu Ala Pro Trp
           1075                1080                1085
   gtg gac tgg tct gtg gtg gac atc ctg cgc cgg gac gcg ggg gat gcg    3312
   Val Asp Trp Ser Val Val Asp Ile Leu Arg Arg Asp Ala Gly Asp Ala
       1090                1095                1100
   gtg tgg gag cgg gcc gat gtg gtc cag cct gtg ctg ttc agc gtc atg    3360
   Val Trp Glu Arg Ala Asp Val Val Gln Pro Val Leu Phe Ser Val Met
   1105                1110                1115                1120
   gtg tct ttg gct gct ctg tgg cgt tcc tac ggt atc gaa ccc gac gcg    3408
   Val Ser Leu Ala Ala Leu Trp Arg Ser Tyr Gly Ile Glu Pro Asp Ala
                   1125                1130                1135
   gtc ctt ggc cat tcc cag ggc gag atc gcg gcc gcg cat gtg tgt ggg    3456
   Val Leu Gly His Ser Gln Gly Glu Ile Ala Ala Ala His Val Cys Gly
               1140                1145                1150
   gcg ctg agc ctg aag gac gcg gcg aag act gtt gcg ctg cgc agc cgg    3504
   Ala Leu Ser Leu Lys Asp Ala Ala Lys Thr Val Ala Leu Arg Ser Arg
           1155                1160                1165
   gcg ctg gcc gct gtg cgg ggc cgg ggc ggc atg gcc tca gtg ccg ctg    3552
   Ala Leu Ala Ala Val Arg Gly Arg Gly Gly Met Ala Ser Val Pro Leu
       1170                1175                1180
   cct gcc cag gag gtg gag cag ctc att ggt gag cgg tgg gcg ggg cgg    3600
   Pro Ala Gln Glu Val Glu Gln Leu Ile Gly Glu Arg Trp Ala Gly Arg
   1185                1190                1195                1200
   ttg tgg gtg gcg gcg gtc aac ggc ccc cgc tcc acc gcc gtc tcg ggg    3648
   Leu Trp Val Ala Ala Val Asn Gly Pro Arg Ser Thr Ala Val Ser Gly
                   1205                1210                1215
   gat gcc gag gcg gtg gac gag gtg ctg gcg tac tgt gcc ggc acc ggg    3696
   Asp Ala Glu Ala Val Asp Glu Val Leu Ala Tyr Cys Ala Gly Thr Gly
               1220                1225                1230
   gtg cgg gcc cgg cgg atc ccg gtc gac tat gcc tcg cac tgc ccc cat    3744
   Val Arg Ala Arg Arg Ile Pro Val Asp Tyr Ala Ser His Cys Pro His
           1235                1240                1245
   gtg cag ccc ctg cgg gag gag ttg ctg gag ctg ctg ggg gac atc agc    3792
   Val Gln Pro Leu Arg Glu Glu Leu Leu Glu Leu Leu Gly Asp Ile Ser
   1250 1255 1260
   ccg cag ccg tcc ggc gtg ccg ttc ttc tcc acg gtg gag ggc acc tgg 3840
   Pro Gln Pro Ser Gly Val Pro Phe Phe Ser Thr Val Glu Gly Thr Trp
   1265 1270 1275 1280
   ctg gac acc aca acc ctg gac gcc gcc tac tgg tac cgc aac ctg cac 3888
   Leu Asp Thr Thr Thr Leu Asp Ala Ala Tyr Trp Tyr Arg Asn Leu His
   1285 1290 1295
   cag ccg gtc cgt ttc agc gat gcc gtc cag gcc ctg gcg gat gac gga 3936
   Gln Pro Val Arg Phe Ser Asp Ala Val Gln Ala Leu Ala Asp Asp Gly
   1300 1305 1310
   cac cgc gtc ttc gtc gaa gtc agc ccc cac ccc acc ctc gtc ccc gcc 3984
   His Arg Val Phe Val Glu Val Ser Pro His Pro Thr Leu Val Pro Ala
   1315 1320 1325
   atc gaa gac acc acc gaa gac acc gcc gaa gac gtc acc gcg atc ggc 4032
   Ile Glu Asp Thr Thr Glu Asp Thr Ala Glu Asp Val Thr Ala Ile Gly
   1330 1335 1340
   agc ctc cgc cgc ggc gac aac gac acc cgc cgc ttc ctc acc gcc ctc 4080
   Ser Leu Arg Arg Gly Asp Asn Asp Thr Arg Arg Phe Leu Thr Ala Leu
   1345 1350 1355 1360
   gcc cac acc cat acc acc ggc atc ggc aca ccc acc acc tgg cac cac 4128
   Ala His Thr His Thr Thr Gly Ile Gly Thr Pro Thr Thr Trp His His
   1365 1370 1375
   cac tac acc cac cac cac acc cac ccc cac ccc cac acg cac ctc gac 4176
   His Tyr Thr His His His Thr His Pro His Pro His Thr His Leu Asp
   1380 1385 1390
   ctg ccc acc tac ccc ttc caa cac cag cac tac tgg ctc gag agc tca 4224
   Leu Pro Thr Tyr Pro Phe Gln His Gln His Tyr Trp Leu Glu Ser Ser
   1395 1400 1405
   cag ccg ggt gcc gga tcc ggt tcg ggt gcc ggt gcc ggt tcg ggt gcc 4272
   Gln Pro Gly Ala Gly Ser Gly Ser Gly Ala Gly Ala Gly Ser Gly Ala
   1410 1415 1420
   ggt tcc ggg cgg gca ggg act gcg ggc ggg acg gca gag gtg gag tcg 4320
   Gly Ser Gly Arg Ala Gly Thr Ala Gly Gly Thr Ala Glu Val Glu Ser
   1425 1430 1435 1440
   cgg ttc tgg gac gcg gtg gcc cgc cag gac ctg gaa acg gtc gcg acc 4368
   Arg Phe Trp Asp Ala Val Ala Arg Gln Asp Leu Glu Thr Val Ala Thr
   1445 1450 1455
   aca ctc gcc gtg ccc ccc tcc gcc ggc ctg gac acg gtg gtg ccc gca 4416
   Thr Leu Ala Val Pro Pro Ser Ala Gly Leu Asp Thr Val Val Pro Ala
   1460 1465 1470
   ctc tcc gcc tgg cac cgc cac caa cac gac caa gcc cgc atc aac acc 4464
   Leu Ser Ala Trp His Arg His Gln His Asp Gln Ala Arg Ile Asn Thr
           1475                1480                1485
   tgg acc tac cag gaa acc tgg aaa ccc ctc acc ctc ccc acc acc cac    4512
   Trp Thr Tyr Gln Glu Thr Trp Lys Pro Leu Thr Leu Pro Thr Thr His
       1490                1495                1500
   caa ccc cac caa acc tgg ctc atc gcc atc ccc gaa acc cag acc cac    4560
   Gln Pro His Gln Thr Trp Leu Ile Ala Ile Pro Glu Thr Gln Thr His
   1505                1510                1515                1520
   cac ccc cac atc acc aac atc ctc acc aac ctc cac cac cac ggc atc    4608
   His Pro His Ile Thr Asn Ile Leu Thr Asn Leu His His His Gly Ile
                   1525                1530                1535
   acc ccc atc ccc ctc acc ctc aac cac acc cac acc aac ccc caa cac    4656
   Thr Pro Ile Pro Leu Thr Leu Asn His Thr His Thr Asn Pro Gln His
               1540                1545                1550
   ctc cac cac acc ctc cac cac acc cga caa caa gcc caa aac cac acc    4704
   Leu His His Thr Leu His His Thr Arg Gln Gln Ala Gln Asn His Thr
           1555                1560                1565
   acc gga gcc atc acc ggc ctg ctc tcc ctc ctc gcc ctc gac gaa aca    4752
   Thr Gly Ala Ile Thr Gly Leu Leu Ser Leu Leu Ala Leu Asp Glu Thr
       1570                1575                1580
   ccc cac ccc cac cac ccc cac aca ccc acc ggc acc ctc ctc aac ctc    4800
   Pro His Pro His His Pro His Thr Pro Thr Gly Thr Leu Leu Asn Leu
   1585                1590                1595                1600
   acc ctc acc caa acc cac acc caa acc cac cca cca acc ccc ctc tgg    4848
   Thr Leu Thr Gln Thr His Thr Gln Thr His Pro Pro Thr Pro Leu Trp
                   1605                1610                1615
   tac gcc acc acc aac gcc acc acc acc cac ccc aac gac ccc ctc aca    4896
   Tyr Ala Thr Thr Asn Ala Thr Thr Thr His Pro Asn Asp Pro Leu Thr
               1620                1625                1630
   cac ccc acc caa gcc caa acc tgg gga ctc gcc cgc acc acc ctc ctc    4944
   His Pro Thr Gln Ala Gln Thr Trp Gly Leu Ala Arg Thr Thr Leu Leu
           1635                1640                1645
   gaa cac ccc acc cac acc gcc gga atc atc gac ctc ccc acc acc ccc    4992
   Glu His Pro Thr His Thr Ala Gly Ile Ile Asp Leu Pro Thr Thr Pro
       1650                1655                1660
   acc ccc cac acc ctc cag cac ctc acc caa acc ctc acc caa ccc cac    5040
   Thr Pro His Thr Leu Gln His Leu Thr Gln Thr Leu Thr Gln Pro His
   1665                1670                1675                1680
   cac caa acc caa ctc gcc atc cgc acc acc ggc acc cac acc cgc cgc    5088
   His Gln Thr Gln Leu Ala Ile Arg Thr Thr Gly Thr His Thr Arg Arg
                   1685                1690                1695
   ctc acc ccc acc acc ctc acc ccc aca cac caa cca ccc acc ccc acc    5136
   Leu Thr Pro Thr Thr Leu Thr Pro Thr His Gln Pro Pro Thr Pro Thr
               1700                1705                1710
   ccc cac gga acc acc ctc atc acc ggc gga acc ggc gcc ctc gcc acc     5184
   Pro His Gly Thr Thr Leu Ile Thr Gly Gly Thr Gly Ala Leu Ala Thr
           1715                1720                1725
   cac ctc acc cac cac ctc acc acc cac caa ccc acc caa cac ctc ctc     5232
   His Leu Thr His His Leu Thr Thr His Gln Pro Thr Gln His Leu Leu
       1730                1735                1740
   ctc acc agc cga acc ggc ccc cac acc ccc cac gca caa cac ctc acc     5280
   Leu Thr Ser Arg Thr Gly Pro His Thr Pro His Ala Gln His Leu Thr
   1745                1750                1755                1760
   acc caa ctc caa caa aaa ggc atc cac ctc acc atc acc acc tgc gac     5328
   Thr Gln Leu Gln Gln Lys Gly Ile His Leu Thr Ile Thr Thr Cys Asp
                   1765                1770                1775
   acc agc aac cca gac caa ctc caa caa ctc ctc aac acc atc ccc cca     5376
   Thr Ser Asn Pro Asp Gln Leu Gln Gln Leu Leu Asn Thr Ile Pro Pro
               1780                1785                1790
   caa cac ccc ctc acc acc gtc atc cac acc gca ggc atc ctc gac gac     5424
   Gln His Pro Leu Thr Thr Val Ile His Thr Ala Gly Ile Leu Asp Asp
           1795                1800                1805
   gcc acc ctc acc aac ctc acc ccc acc caa ctc aac aac gtc ctc cgc     5472
   Ala Thr Leu Thr Asn Leu Thr Pro Thr Gln Leu Asn Asn Val Leu Arg
       1810                1815                1820
   gcc aaa gcc cac agc gcc cac ctc ctc cac caa ctc acc caa cac acc     5520
   Ala Lys Ala His Ser Ala His Leu Leu His Gln Leu Thr Gln His Thr
   1825                1830                1835                1840
   ccc ctc acc gcc ttc gtc ctc tac tcc tcc gcc gcc gcc acc ttc ggc     5568
   Pro Leu Thr Ala Phe Val Leu Tyr Ser Ser Ala Ala Ala Thr Phe Gly
                   1845                1850                1855
   gca ccc ggc caa gcc aac tac gcc gca gcc aac gcc tac ctc gac gcc     5616
   Ala Pro Gly Gln Ala Asn Tyr Ala Ala Ala Asn Ala Tyr Leu Asp Ala
               1860                1865                1870
   ctc gcc cac cac cgc cac acc cac cac ctc ccc gcc acc agc atc gcc     5664
   Leu Ala His His Arg His Thr His His Leu Pro Ala Thr Ser Ile Ala
           1875                1880                1885
   tgg ggc acc tgg caa gga aac gga ctc gct gat tcg gac aag gcc cgc     5712
   Trp Gly Thr Trp Gln Gly Asn Gly Leu Ala Asp Ser Asp Lys Ala Arg
       1890                1895                1900
   gca tat ctc gac cgc cgc ggg ttt cga ccc atg tca ccc gag ttg gcc     5760
   Ala Tyr Leu Asp Arg Arg Gly Phe Arg Pro Met Ser Pro Glu Leu Ala
   1905                1910                1915                1920
   acg gca gcg gtc acg cag gcg atc gcg gac acc gaa cgg ccg tat gtc     5808
   Thr Ala Ala Val Thr Gln Ala Ile Ala Asp Thr Glu Arg Pro Tyr Val
                   1925                1930                1935
   gtc atc gcc gac atc gac tgg agc aag atc gaa cac acc tct cag acc    5856
   Val Ile Ala Asp Ile Asp Trp Ser Lys Ile Glu His Thr Ser Gln Thr
               1940                1945                1950
   agc gac ctg gtg agc gcg gcc cgg gaa agg gag cca gct gtc cag cgc    5904
   Ser Asp Leu Val Ser Ala Ala Arg Glu Arg Glu Pro Ala Val Gln Arg
           1955                1960                1965
   ccc act cca ccg gcg gag ttg cac aaa acg ctg gcc cat cag acg tcg    5952
   Pro Thr Pro Pro Ala Glu Leu His Lys Thr Leu Ala His Gln Thr Ser
       1970                1975                1980
   gcc gac caa cgg gcc gca ttg ctc gag ctc gta cga gac cat gtg gcg    6000
   Ala Asp Gln Arg Ala Ala Leu Leu Glu Leu Val Arg Asp His Val Ala
   1985                1990                1995                2000
   gca gtg ctc cgg cac gcg gac ccg aaa gcc atc gcg ccc gac cag tcg    6048
   Ala Val Leu Arg His Ala Asp Pro Lys Ala Ile Ala Pro Asp Gln Ser
                   2005                2010                2015
   ttc cgt gca ctc ggc ttc gat tca ctc acg gcc gtc gag ttc cga aac    6096
   Phe Arg Ala Leu Gly Phe Asp Ser Leu Thr Ala Val Glu Phe Arg Asn
               2020                2025                2030
   ctg ctg atc aag gca aca gga ctc cgc ctt cct gtc tcg ctg gtc ttc    6144
   Leu Leu Ile Lys Ala Thr Gly Leu Arg Leu Pro Val Ser Leu Val Phe
           2035                2040                2045
   gac cac ccg acc cct gcc aaa ctc gcc gta cac ctg cag aac caa ctg    6192
   Asp His Pro Thr Pro Ala Lys Leu Ala Val His Leu Gln Asn Gln Leu
       2050                2055                2060
   cgg ggc aca gca gcg gag tcg gct cct tca gcg gca gcc gtt acc gcc    6240
   Arg Gly Thr Ala Ala Glu Ser Ala Pro Ser Ala Ala Ala Val Thr Ala
   2065                2070                2075                2080
   gag gct tct gtc acc gag ccg atc gcc atc gtt ggc atg gcc tgt cgt    6288
   Glu Ala Ser Val Thr Glu Pro Ile Ala Ile Val Gly Met Ala Cys Arg
                   2085                2090                2095
   ttc ccc ggc gga gtg acc tcg gcg gac gac ttc tgg gat ctg atc tcc    6336
   Phe Pro Gly Gly Val Thr Ser Ala Asp Asp Phe Trp Asp Leu Ile Ser
               2100                2105                2110
   tcc gag cag gac gcg atc ggc gga ttc ccc acc gac cgc ggc tgg gac    6384
   Ser Glu Gln Asp Ala Ile Gly Gly Phe Pro Thr Asp Arg Gly Trp Asp
           2115                2120                2125
   ctg gac acg ctc tac gac ccc gac ccc gac cac ccc ggc acc tgc tac    6432
   Leu Asp Thr Leu Tyr Asp Pro Asp Pro Asp His Pro Gly Thr Cys Tyr
       2130                2135                2140
   acc cga aac ggc gga ttc ctc tac gac gca ggc cac ttc gac gcc gaa    6480
   Thr Arg Asn Gly Gly Phe Leu Tyr Asp Ala Gly His Phe Asp Ala Glu
   2145                2150                2155                2160
   ttc ttc ggc atc agc ccc cgc gaa gcc ctc gcc atg gac ccc cag caa    6528
   Phe Phe Gly Ile Ser Pro Arg Glu Ala Leu Ala Met Asp Pro Gln Gln
                   2165                    2170                    2175
   cga ctc ctc ctc gaa acc gcc tgg gaa acc atc gaa cac gcc ggc atc     6576
   Arg Leu Leu Leu Glu Thr Ala Trp Glu Thr Ile Glu His Ala Gly Ile
               2180                    2185                    2190
   aac ccc cac acc ctc cac ggc acc ccc acc gga gtc ttc acc ggc acc     6624
   Asn Pro His Thr Leu His Gly Thr Pro Thr Gly Val Phe Thr Gly Thr
           2195                    2200                    2205
   aac gga cag gac tac gca ctt cgc gtg cac aac gcg ggc cag tca acc     6672
   Asn Gly Gln Asp Tyr Ala Leu Arg Val His Asn Ala Gly Gln Ser Thr
       2210                    2215                    2220
   gat ggt ttc gca ctg acc gga acc gcc ggc agc gtc atc tcc ggt cgt     6720
   Asp Gly Phe Ala Leu Thr Gly Thr Ala Gly Ser Val Ile Ser Gly Arg
   2225                    2230                    2235                    2240
   atc tcg tac acg ttt ggt ttt gag ggt cct gcg gtg tcg gtg gac acg     6768
   Ile Ser Tyr Thr Phe Gly Phe Glu Gly Pro Ala Val Ser Val Asp Thr
                   2245                    2250                    2255
   gct tgt tcc tcg tcg ttg gtg gct ttg cat ctg gcc tgt cag gcg ttg     6816
   Ala Cys Ser Ser Ser Leu Val Ala Leu His Leu Ala Cys Gln Ala Leu
               2260                    2265                    2270
   cgt gcg ggt gag tgc tcg atg gcg ctt gcc ggg ggt gtg acg gtg atg     6864
   Arg Ala Gly Glu Cys Ser Met Ala Leu Ala Gly Gly Val Thr Val Met
           2275                    2280                    2285
   tcg tct ccg ggt gcc ttc gtg gag ttt tcg cgg cag cgg ggt ctg gcc     6912
   Ser Ser Pro Gly Ala Phe Val Glu Phe Ser Arg Gln Arg Gly Leu Ala
       2290                    2295                    2300
   gcg gac ggg cat tgc aag gcg ttc tcg gcg gcg gcg gac ggg acc ggc     6960
   Ala Asp Gly His Cys Lys Ala Phe Ser Ala Ala Ala Asp Gly Thr Gly
   2305                    2310                    2315                    2320
   tgg ggt gag ggt gtg ggg atg ctg ctg gtg gag cgg ctc tcc gac gcc     7008
   Trp Gly Glu Gly Val Gly Met Leu Leu Val Glu Arg Leu Ser Asp Ala
                   2325                    2330                    2335
   cat cgc aac ggt cac cgt gtc ctg gcc gtg gtg cgt ggc agt gcg gtc     7056
   His Arg Asn Gly His Arg Val Leu Ala Val Val Arg Gly Ser Ala Val
               2340                    2345                    2350
   aac cag gac ggt gcg agc aac ggt ctg acc gcg ccc aac ggg ccg tcc     7104
   Asn Gln Asp Gly Ala Ser Asn Gly Leu Thr Ala Pro Asn Gly Pro Ser
           2355                    2360                    2365
   cag cag cgt gtc atc cgc cag gcc ctc gcc aac gcc ggc ttg tcg gcc     7152
   Gln Gln Arg Val Ile Arg Gln Ala Leu Ala Asn Ala Gly Leu Ser Ala
       2370                    2375                    2380
   ggt gat gtc gac gcg gtg gag gcc cac ggc acc ggc acc act ttg ggc     7200
   Gly Asp Val Asp Ala Val Glu Ala His Gly Thr Gly Thr Thr Leu Gly
   2385                2390                2395                2400
   gac ccg atc gag gcc cag gcc ctc ctc gcg acc tac gga cag gac cgt    7248
   Asp Pro Ile Glu Ala Gln Ala Leu Leu Ala Thr Tyr Gly Gln Asp Arg
                   2405                2410                2415
   gcc ggc gag ggg ccg ctg tgg ctg ggc tcg gtc aag tcc aat gtc ggt    7296
   Ala Gly Glu Gly Pro Leu Trp Leu Gly Ser Val Lys Ser Asn Val Gly
               2420                2425                2430
   cac aca cag gct gcc gcg ggc gtc gcc ggg gtg atc aag atg gtg atg    7344
   His Thr Gln Ala Ala Ala Gly Val Ala Gly Val Ile Lys Met Val Met
           2435                2440                2445
   gcg ctg cgg cat ggt ctg ctg ccg cgg acg ttg cat gtg gat gag ccg    7392
   Ala Leu Arg His Gly Leu Leu Pro Arg Thr Leu His Val Asp Glu Pro
       2450                2455                2460
   tcg ccg cat gtg gac tgg tcc gcg ggt gcg gtg cag ctg ctg acg gag    7440
   Ser Pro His Val Asp Trp Ser Ala Gly Ala Val Gln Leu Leu Thr Glu
   2465                2470                2475                2480
   acg gtg ccc tgg ccc ggc ggg gag ggg cgg cta cgg cgg gca gga gtg    7488
   Thr Val Pro Trp Pro Gly Gly Glu Gly Arg Leu Arg Arg Ala Gly Val
                   2485                2490                2495
   tca tca ttc ggc gtc agc ggc acc aac gcc cac gtc atc ctc gaa gaa    7536
   Ser Ser Phe Gly Val Ser Gly Thr Asn Ala His Val Ile Leu Glu Glu
               2500                2505                2510
   gca ccc gcc gac gac gtt ccg ggg gga cca ccc gcc ggc gag ggt gac    7584
   Ala Pro Ala Asp Asp Val Pro Gly Gly Pro Pro Ala Gly Glu Gly Asp
           2515                2520                2525
   gcg ggc agc gac gat gag gct gct gcc ggc agt cct ggg gtg tgg ccg    7632
   Ala Gly Ser Asp Asp Glu Ala Ala Ala Gly Ser Pro Gly Val Trp Pro
       2530                2535                2540
   tgg ctg gtg tcg gcc aag tcg cag ccg gcc ctg cgc gcc cag gcc cag    7680
   Trp Leu Val Ser Ala Lys Ser Gln Pro Ala Leu Arg Ala Gln Ala Gln
   2545                2550                2555                2560
   gcc ctg cac gcc cac ctc acc gac cac ccc ggc ctc gac ctc gcg gat    7728
   Ala Leu His Ala His Leu Thr Asp His Pro Gly Leu Asp Leu Ala Asp
                   2565                2570                2575
   gtc gga tac acc ctc gcc cac gcc cgc gcc gtg ttc gac cac cgc gcc    7776
   Val Gly Tyr Thr Leu Ala His Ala Arg Ala Val Phe Asp His Arg Ala
               2580                2585                2590
   acc ctc atc gcc gcg gac cgc gac acg ttc ctg caa gca ctc cag gca    7824
   Thr Leu Ile Ala Ala Asp Arg Asp Thr Phe Leu Gln Ala Leu Gln Ala
           2595                2600                2605
   ctc gcc gca ggc gag ccc cac ccc gcc gtc atc cac agc agc gcc ccg    7872
   Leu Ala Ala Gly Glu Pro His Pro Ala Val Ile His Ser Ser Ala Pro
       2610                2615                2620
   ggc ggg acc ggg acc ggg gag gcc gca gga aag acc gca ttc atc tgc     7920
   Gly Gly Thr Gly Thr Gly Glu Ala Ala Gly Lys Thr Ala Phe Ile Cys
   2625                2630                2635                2640
   tcc gga cag ggc acc caa cgc ccc ggc atg gcc cac ggc ctc tac cac     7968
   Ser Gly Gln Gly Thr Gln Arg Pro Gly Met Ala His Gly Leu Tyr His
                   2645                2650                2655
   acc cac ccc gtc ttc gcc gcc gca ctc aac gac atc tgc acc cac ctc     8016
   Thr His Pro Val Phe Ala Ala Ala Leu Asn Asp Ile Cys Thr His Leu
               2660                2665                2670
   gac ccc cac ctc gac cac ccc ctc ctc ccc ctc ctc acc caa aac gac     8064
   Asp Pro His Leu Asp His Pro Leu Leu Pro Leu Leu Thr Gln Asn Asp
           2675                2680                2685
   aac gac aac gag gac gcg gcc gca ctg ctc cag cag acc cgc tac gcc     8112
   Asn Asp Asn Glu Asp Ala Ala Ala Leu Leu Gln Gln Thr Arg Tyr Ala
       2690                2695                2700
   cag ccc gcc ctc ttc gcc ttc cag gtc gcc ctc cac cgc ctc ctc acc     8160
   Gln Pro Ala Leu Phe Ala Phe Gln Val Ala Leu His Arg Leu Leu Thr
   2705                2710                2715                2720
   gac ggc tac cac atc acc ccc cac tac tac gcc gga cac tcc ctc ggc     8208
   Asp Gly Tyr His Ile Thr Pro His Tyr Tyr Ala Gly His Ser Leu Gly
                   2725                2730                2735
   gaa atc acc gcc gcc cac ctc gcc ggc atc ctc acc ctc acc gac gcc     8256
   Glu Ile Thr Ala Ala His Leu Ala Gly Ile Leu Thr Leu Thr Asp Ala
               2740                2745                2750
   acc acc ctc atc acc caa cgc gcc acc ctc atg caa acc atg ccc ccc     8304
   Thr Thr Leu Ile Thr Gln Arg Ala Thr Leu Met Gln Thr Met Pro Pro
           2755                2760                2765
   ggc acc atg acc acc ctc cac acc acc ccc cac cac atc acc cac cac     8352
   Gly Thr Met Thr Thr Leu His Thr Thr Pro His His Ile Thr His His
       2770                2775                2780
   ctc acc gcc cac gaa aac gac ctc gcc atc gcc gcc atc aac acc ccc     8400
   Leu Thr Ala His Glu Asn Asp Leu Ala Ile Ala Ala Ile Asn Thr Pro
   2785                2790                2795                2800
   acc tcc ctc gtc atc agc ggc acc ccc cac acc gtc caa cac atc acc     8448
   Thr Ser Leu Val Ile Ser Gly Thr Pro His Thr Val Gln His Ile Thr
                   2805                2810                2815
   acc ctc tgc caa caa caa ggc atc aaa acc aaa acc ctc ccc acc aac     8496
   Thr Leu Cys Gln Gln Gln Gly Ile Lys Thr Lys Thr Leu Pro Thr Asn
               2820                2825                2830
   cac gcc ttc cac tcc ccc cac acc aac ccc atc ctc aac caa ctc cac     8544
   His Ala Phe His Ser Pro His Thr Asn Pro Ile Leu Asn Gln Leu His
           2835                2840                2845
   cag cac acc caa acc ctc acc tac cac cca ccc cac acc ccc ctc atc    8592
   Gln His Thr Gln Thr Leu Thr Tyr His Pro Pro His Thr Pro Leu Ile
       2850                2855                2860
   acc gcc aac acc cca ccc gac caa ctc ctc acc ccc cac tac tgg acc    8640
   Thr Ala Asn Thr Pro Pro Asp Gln Leu Leu Thr Pro His Tyr Trp Thr
   2865                2870                2875                2880
   caa caa gcc cgc aac acc gtc gac tac gcc acc acc acc caa acc ctc    8688
   Gln Gln Ala Arg Asn Thr Val Asp Tyr Ala Thr Thr Thr Gln Thr Leu
                   2885                2890                2895
   cac caa cac ggc gtc acc acc tac atc gaa ctc gga ccc gac aac acc    8736
   His Gln His Gly Val Thr Thr Tyr Ile Glu Leu Gly Pro Asp Asn Thr
               2900                2905                2910
   ctc acc acc ctc acc cac cac aac ctc ccc aac ccc ccc acc acc acc    8784
   Leu Thr Thr Leu Thr His His Asn Leu Pro Asn Pro Pro Thr Thr Thr
           2915                2920                2925
   ctc acc ctc acc cac ccc cac cac cac ccc caa acc cac ctc ctc acc    8832
   Leu Thr Leu Thr His Pro His His His Pro Gln Thr His Leu Leu Thr
       2930                2935                2940
   aac ctc gcc aaa acc acc acc acc tgg cac ccc cac cac tac acc cac    8880
   Asn Leu Ala Lys Thr Thr Thr Thr Trp His Pro His His Tyr Thr His
   2945                2950                2955                2960
   cac gac aac caa ccc cac acc cac acc cac ctc gac ctc ccc acc tac    8928
   His Asp Asn Gln Pro His Thr His Thr His Leu Asp Leu Pro Thr Tyr
                   2965                2970                2975
   ccc ttc caa cac cac cac tac tgg ctc gaa agc aca cag ccc ggt gcc    8976
   Pro Phe Gln His His His Tyr Trp Leu Glu Ser Thr Gln Pro Gly Ala
               2980                2985                2990
   ggc aac gtg tca gca gcc gga ctc gac ccc acc gaa cac ccc cta ctc    9024
   Gly Asn Val Ser Ala Ala Gly Leu Asp Pro Thr Glu His Pro Leu Leu
           2995                3000                3005
   ggc gcc aca ttg gaa ctg gcg act gac ggt gga gcg ctt ctt gca ggg    9072
   Gly Ala Thr Leu Glu Leu Ala Thr Asp Gly Gly Ala Leu Leu Ala Gly
       3010                3015                3020
   cgc ttg tct ttg agg tcg cat ccg tgg ctg gct gac cat gcc gtc ggc    9120
   Arg Leu Ser Leu Arg Ser His Pro Trp Leu Ala Asp His Ala Val Gly
   3025                3030                3035                3040
   ggc acg gtg ctg ctg tcg ggc gcc acc ttc ctc gaa ctc gcc ctt cat    9168
   Gly Thr Val Leu Leu Ser Gly Ala Thr Phe Leu Glu Leu Ala Leu His
                   3045                3050                3055
   gcg ggc aca tac gtg ggc tgc gac cga gtg gat gag ctg acg ctg cat    9216
   Ala Gly Thr Tyr Val Gly Cys Asp Arg Val Asp Glu Leu Thr Leu His
               3060                3065                3070
   gcg ccg ctg gtg gtt cct gtg gat ggg ggt gtg agt gtg cag gtt ggg    9264
   Ala Pro Leu Val Val Pro Val Asp Gly Gly Val Ser Val Gln Val Gly
           3075                3080                3085
   gtt gcg gct gcg gat ggg gag ggg cgg cgt ttg gtg agt gtg tat gcg    9312
   Val Ala Ala Ala Asp Gly Glu Gly Arg Arg Leu Val Ser Val Tyr Ala
       3090                3095                3100
   cgg ggt ggg agt gct tgt ggt ggg ggt ggt gcg tcg ggt ggg gtg tgg    9360
   Arg Gly Gly Ser Ala Cys Gly Gly Gly Gly Ala Ser Gly Gly Val Trp
   3105                3110                3115                3120
   acg tgt cat gcc tcg ggg gtg ctg gtt gag gct gct gct ggt ggt gtg    9408
   Thr Cys His Ala Ser Gly Val Leu Val Glu Ala Ala Ala Gly Gly Val
                   3125                3130                3135
   gtg gtg gat ggt ctg gcg ggg gtg tgg ccg ccg cgg ggt gcg gtg gcg    9456
   Val Val Asp Gly Leu Ala Gly Val Trp Pro Pro Arg Gly Ala Val Ala
               3140                3145                3150
   gtg gat gtc gat ggt gtc cgt gac cgt ttg gct ggg gct ggt tgt gtt    9504
   Val Asp Val Asp Gly Val Arg Asp Arg Leu Ala Gly Ala Gly Cys Val
           3155                3160                3165
   ttg ggg ccg gtg ttt tcg ggg ctg cgt gcg gtg tgg cgt gat ggg ggg    9552
   Leu Gly Pro Val Phe Ser Gly Leu Arg Ala Val Trp Arg Asp Gly Gly
       3170                3175                3180
   gat ttg ctg gct gag gtg tgt ctg ccg gag gag gcg tgg ggt gat gcg    9600
   Asp Leu Leu Ala Glu Val Cys Leu Pro Glu Glu Ala Trp Gly Asp Ala
   3185                3190                3195                3200
   gct ggt ttt ggg ctg cat ccg gcg ttg ctg gat ggt gtg gtc cag ccg    9648
   Ala Gly Phe Gly Leu His Pro Ala Leu Leu Asp Gly Val Val Gln Pro
                   3205                3210                3215
   ttg tcg gtg ttg ctt ccg ggt ggg acg ggg ttt ggg gag ggg gcg ggg    9696
   Leu Ser Val Leu Leu Pro Gly Gly Thr Gly Phe Gly Glu Gly Ala Gly
               3220                3225                3230
   ttc ggg gag ggt gtt cgg gtg ccg gct gtg tgg ggt ggt gtg tcg ctt    9744
   Phe Gly Glu Gly Val Arg Val Pro Ala Val Trp Gly Gly Val Ser Leu
           3235                3240                3245
   cac cgg gcg ggt gtg acc ggt gtg cgg gtg cgt gtg tcg gct gtc ggg    9792
   His Arg Ala Gly Val Thr Gly Val Arg Val Arg Val Ser Ala Val Gly
       3250                3255                3260
   cgg ggc ggc ggg cgt gag gcg gtg tcg gtc gtg gtc ggg gat gag gcg    9840
   Arg Gly Gly Gly Arg Glu Ala Val Ser Val Val Val Gly Asp Glu Ala
   3265                3270                3275                3280
   ggt gtg ccg gtg gcg tcg gtc gat cgt ctt gag ttg cgg cct gtg gat    9888
   Gly Val Pro Val Ala Ser Val Asp Arg Leu Glu Leu Arg Pro Val Asp
                   3285                3290                3295
   atg ggt cag ttg cgt gct gtc tcg gtt tcg gcg ggg cgg cgg ggt tcg    9936
   Met Gly Gln Leu Arg Ala Val Ser Val Ser Ala Gly Arg Arg Gly Ser
   3300 3305 3310
   ctg tat gcg gtg cag tgg gct gag gtg ggt cct gtg ccg gtg tgt ggg 9984
   Leu Tyr Ala Val Gln Trp Ala Glu Val Gly Pro Val Pro Val Cys Gly
   3315 3320 3325
   cag gcg tgg gcg tgg cac gag gac gtg ggt gag agc ggt ggt ggg cct 10032
   Gln Ala Trp Ala Trp His Glu Asp Val Gly Glu Ser Gly Gly Gly Pro
   3330 3335 3340
   gtg ccg ggg gtg gtg gtg ttg cgg tgc ccg gat gcc ggt gcc ggt ggc 10080
   Val Pro Gly Val Val Val Leu Arg Cys Pro Asp Ala Gly Ala Gly Gly
   3345 3350 3355 3360
   ggt ggc ggt ggc ggt ggt ggc ggt ggt gtg ggt gag gtt gtt ggt ggg 10128
   Gly Gly Gly Gly Gly Gly Gly Gly Gly Val Gly Glu Val Val Gly Gly
   3365 3370 3375
   gtg ttg ggt gtg gtg cag ggg tgg ctg ggg ctg gag cgg ttt gcg ggt 10176
   Val Leu Gly Val Val Gln Gly Trp Leu Gly Leu Glu Arg Phe Ala Gly
   3380 3385 3390
   tcg cgg ctg gtg gtg gtg acc cgg ggt gcg gtg gtg gcc ggc ccg gag 10224
   Ser Arg Leu Val Val Val Thr Arg Gly Ala Val Val Ala Gly Pro Glu
   3395 3400 3405
   gac ggc ccg gtg gat gtg gtg ggt gcg tcg gtg tgg ggg ctg gtg cgt 10272
   Asp Gly Pro Val Asp Val Val Gly Ala Ser Val Trp Gly Leu Val Arg
   3410 3415 3420
   tcg gcg cag gct gag cat ccg gac cgg ttt gtc ctc ctc gac ctc gac 10320
   Ser Ala Gln Ala Glu His Pro Asp Arg Phe Val Leu Leu Asp Leu Asp
   3425 3430 3435 3440
   acc gac acc ggc acc gac ctc gac acc ggt gct ggt gct ggt tgg ggc 10368
   Thr Asp Thr Gly Thr Asp Leu Asp Thr Gly Ala Gly Ala Gly Trp Gly
   3445 3450 3455
   gtg gat ggt ggg cgt gtg gcg gcg gtg gtg gcg tgt ggt gag ccg cag 10416
   Val Asp Gly Gly Arg Val Ala Ala Val Val Ala Cys Gly Glu Pro Gln
   3460 3465 3470
   ttg gcg gtg cgt ggg gag cgg ttg ctg gcc gca cgc ctg aaa cga ctt 10464
   Leu Ala Val Arg Gly Glu Arg Leu Leu Ala Ala Arg Leu Lys Arg Leu
   3475 3480 3485
   gag tca tcc ggt gat gtt cca gcc cag cgg tcc ggt gac aca cga gcc 10512
   Glu Ser Ser Gly Asp Val Pro Ala Gln Arg Ser Gly Asp Thr Arg Ala
   3490 3495 3500
   cgg cgg tcc gac gtg cct gcc cag cgc tcc ggt ggc gtg cct gct cgg 10560
   Arg Arg Ser Asp Val Pro Ala Gln Arg Ser Gly Gly Val Pro Ala Arg
   3505 3510 3515 3520
   cgg tcg gtt gat gta tcg ggt cgg gag gtg ttg ccg tgg ttg tcg ggt 10608
   Arg Ser Val Asp Val Ser Gly Arg Glu Val Leu Pro Trp Leu Ser Gly
   3525 3530 3535
   ggg tcg gtg ttg gtg acg ggt ggg acg ggt gtg ctg ggt gcg gcg gtg     10656
   Gly Ser Val Leu Val Thr Gly Gly Thr Gly Val Leu Gly Ala Ala Val
                   3540                3545                3550
   gcg cgg cat ctg gct ggt gtg tgt ggg gtg cgg gat ctg ctg ttg gtg     10704
   Ala Arg His Leu Ala Gly Val Cys Gly Val Arg Asp Leu Leu Leu Val
               3555                3560                3565
   agc cgg cgt ggt ccg gat gct ccg ggt gcg gag ggt ctg cgg gcg gag     10752
   Ser Arg Arg Gly Pro Asp Ala Pro Gly Ala Glu Gly Leu Arg Ala Glu
           3570                3575                3580
   ctg gcc gcg ttg ggg gcg gag gtg cgg att gtt gcg tgt gat gtg ggg     10800
   Leu Ala Ala Leu Gly Ala Glu Val Arg Ile Val Ala Cys Asp Val Gly
   3585                3590                3595                3600
   gag cgg cgg gag gtg gtc cgg ctg ctg gag ggt gtt cct gcc ggg tgt     10848
   Glu Arg Arg Glu Val Val Arg Leu Leu Glu Gly Val Pro Ala Gly Cys
                   3605                3610                3615
   ccg ctg acg ggt gtc gtg cat gcg gct ggt gtg ctg gac gat gcg acg     10896
   Pro Leu Thr Gly Val Val His Ala Ala Gly Val Leu Asp Asp Ala Thr
               3620                3625                3630
   atc gcc tct ctc acg ccc gag cgg ctg ggc acg gtg ttc gcg gcc aag     10944
   Ile Ala Ser Leu Thr Pro Glu Arg Leu Gly Thr Val Phe Ala Ala Lys
           3635                3640                3645
   gtg gat gcc gct ctt ttg ctg gat gag ctg acg cgg ggt atg gag ctg     10992
   Val Asp Ala Ala Leu Leu Leu Asp Glu Leu Thr Arg Gly Met Glu Leu
       3650                3655                3660
   tcg gcg ttc gtg ctg ttc tcc tcg gcc gcg ggg atc ctg ggg tcg gcc     11040
   Ser Ala Phe Val Leu Phe Ser Ser Ala Ala Gly Ile Leu Gly Ser Ala
   3665                3670                3675                3680
   ggg cag ggc aac tac gcc gcg gcc aat gcc gct ctg gac gcg ctg gcg     11088
   Gly Gln Gly Asn Tyr Ala Ala Ala Asn Ala Ala Leu Asp Ala Leu Ala
                   3685                3690                3695
   tac cgg cgg cgg gcg gcg ggt ctg ccg ggg gtg tcg ctg gcg tgg ggg     11136
   Tyr Arg Arg Arg Ala Ala Gly Leu Pro Gly Val Ser Leu Ala Trp Gly
               3700                3705                3710
   ctg tgg gaa gag gcc agc ggg atg acc ggg cac ctg gcc ggc acc gac     11184
   Leu Trp Glu Glu Ala Ser Gly Met Thr Gly His Leu Ala Gly Thr Asp
           3715                3720                3725
   cac cgg cgc atc atc cgt tcc ggt ctg cat ccc atg tcg acc ccg gac     11232
   His Arg Arg Ile Ile Arg Ser Gly Leu His Pro Met Ser Thr Pro Asp
       3730                3735                3740
   gca ctg gcc ctc ttc gat gcg gcc ctg gct ctg gac cgg ccg gtc ctg     11280
   Ala Leu Ala Leu Phe Asp Ala Ala Leu Ala Leu Asp Arg Pro Val Leu
   3745                3750                3755                3760
   ctg ccc gcc gac ctg cgt ccc gcc ccg ccc ctg ccg ccc ctg ctg cag     11328
   Leu Pro Ala Asp Leu Arg Pro Ala Pro Pro Leu Pro Pro Leu Leu Gln
                   3765                3770                3775
   gac ctc ctg ccc gcc acc cgc cgc cgc acc acc cgc acc acc act acc     11376
   Asp Leu Leu Pro Ala Thr Arg Arg Arg Thr Thr Arg Thr Thr Thr Thr
               3780                3785                3790
   ggt ggt gcg gac aac ggc gcc cag ctg cac gcc cgg ctg gcc ggc cag     11424
   Gly Gly Ala Asp Asn Gly Ala Gln Leu His Ala Arg Leu Ala Gly Gln
           3795                3800                3805
   aca cac gaa caa cag cac acc acc ctc ctc gcc ctg gtc cgc tcc cac     11472
   Thr His Glu Gln Gln His Thr Thr Leu Leu Ala Leu Val Arg Ser His
       3810                3815                3820
   atc gcc acc gtc ctg ggc cac acc acc ccc gac acc atc ccc ccc gac     11520
   Ile Ala Thr Val Leu Gly His Thr Thr Pro Asp Thr Ile Pro Pro Asp
   3825                3830                3835                3840
   cgc gcg ttc cgc gac ctc ggc ttc gac tcc ctc acc gcc gtc gaa cta     11568
   Arg Ala Phe Arg Asp Leu Gly Phe Asp Ser Leu Thr Ala Val Glu Leu
                   3845                3850                3855
   cgc aac cgg ctc tcc cgc acc acc gga ctc cgc ctc ccc acc acc ctc     11616
   Arg Asn Arg Leu Ser Arg Thr Thr Gly Leu Arg Leu Pro Thr Thr Leu
               3860                3865                3870
   gcc ttc gac cac ccc aac ccc acc acc ctc acc cac cac ctc cac aca     11664
   Ala Phe Asp His Pro Asn Pro Thr Thr Leu Thr His His Leu His Thr
           3875                3880                3885
   caa ctc cag cca caa ccg gac aac gct gtc gcc ccc gtg ttg gcg gag     11712
   Gln Leu Gln Pro Gln Pro Asp Asn Ala Val Ala Pro Val Leu Ala Glu
       3890                3895                3900
   ctc gac aaa ctc gaa tcc gcc ctc tcc gcc ctc gac aaa acc gac agc     11760
   Leu Asp Lys Leu Glu Ser Ala Leu Ser Ala Leu Asp Lys Thr Asp Ser
   3905                3910                3915                3920
   gcc agc gaa aga gtc acc ctg cgg ctg aag tca ctc atg ttg agg tgg     11808
   Ala Ser Glu Arg Val Thr Leu Arg Leu Lys Ser Leu Met Leu Arg Trp
                   3925                3930                3935
   aac gca ccc cag cat ccg aca gcc gaa agc gct gat gac gac gag aag     11856
   Asn Ala Pro Gln His Pro Thr Ala Glu Ser Ala Asp Asp Asp Glu Lys
               3940                3945                3950
   ttc aca tcg gca aca gag gct gag att ttc aaa ttc att gac aac gac     11904
   Phe Thr Ser Ala Thr Glu Ala Glu Ile Phe Lys Phe Ile Asp Asn Asp
           3955                3960                3965
   ctc ggc ctg tcc tgaaccggac gcctgccact ccgcccgtat ccgctgggcc         11956
   Leu Gly Leu Ser
       3970
   ctgctaggac gtga atg caa ttg gcg aat gaa gcg aag ctc ctg gaa tac     12006
   Met Gln Leu Ala Asn Glu Ala Lys Leu Leu Glu Tyr
   3975 3980
   ctc aag cgc gtc act gcg gac ctg gac cgc act cgc cgt cgc ctg tac 12054
   Leu Lys Arg Val Thr Ala Asp Leu Asp Arg Thr Arg Arg Arg Leu Tyr
   3985 3990 3995 4000
   gag gtg gtc gag cgt gag cag gag ccg atc gcg att gtg ggg atg gcg 12102
   Glu Val Val Glu Arg Glu Gln Glu Pro Ile Ala Ile Val Gly Met Ala
   4005 4010 4015
   tgt cgt tac cca ggc ggg gcg acg tca ccc acg cga ctg tgg cat ctc 12150
   Cys Arg Tyr Pro Gly Gly Ala Thr Ser Pro Thr Arg Leu Trp His Leu
   4020 4025 4030
   gtc aag tcc cag acg gac gct atc ggg gag ttc ccg acc gac cgt gga 12198
   Val Lys Ser Gln Thr Asp Ala Ile Gly Glu Phe Pro Thr Asp Arg Gly
   4035 4040 4045
   tgg aac ctg gag cag ctc tac gac ccg gac ccc gac cgc tca gga acc 12246
   Trp Asn Leu Glu Gln Leu Tyr Asp Pro Asp Pro Asp Arg Ser Gly Thr
   4050 4055 4060
   agt tac acg cgc agc gga ggg ttt ctc tat gac gcg ggc gac ttc gac 12294
   Ser Tyr Thr Arg Ser Gly Gly Phe Leu Tyr Asp Ala Gly Asp Phe Asp
   4065 4070 4075 4080
   gcc gcg ttc ttc gag ttg tca ccg cgt gag gcg ctg gca atg gac ccg 12342
   Ala Ala Phe Phe Glu Leu Ser Pro Arg Glu Ala Leu Ala Met Asp Pro
   4085 4090 4095
   cag cag cgc ctg ctg ctc gaa acc act tgg gaa acg ttc gaa cag ggc 12390
   Gln Gln Arg Leu Leu Leu Glu Thr Thr Trp Glu Thr Phe Glu Gln Gly
   4100 4105 4110
   gga atc gac ccg agg tcc atg cgc gga agc cgg acc ggg gtt ttc gtg 12438
   Gly Ile Asp Pro Arg Ser Met Arg Gly Ser Arg Thr Gly Val Phe Val
   4115 4120 4125
   ggg atc aat ccg gag gac tac acc acc gga tac aca cat cag ccc tca 12486
   Gly Ile Asn Pro Glu Asp Tyr Thr Thr Gly Tyr Thr His Gln Pro Ser
   4130 4135 4140
   aac gca gtc gag ggc tac ctg ctc act ggc agc gcg gca agc att gcg 12534
   Asn Ala Val Glu Gly Tyr Leu Leu Thr Gly Ser Ala Ala Ser Ile Ala
   4145 4150 4155 4160
   tca ggc cgt atc tcc tac aac ttc ggg ctc gaa ggc cct gcg atc act 12582
   Ser Gly Arg Ile Ser Tyr Asn Phe Gly Leu Glu Gly Pro Ala Ile Thr
   4165 4170 4175
   atc gac acc gcg tgt tcc tcc tcg ctc gtc gcc ctg cat ctg gcc tgc 12630
   Ile Asp Thr Ala Cys Ser Ser Ser Leu Val Ala Leu His Leu Ala Cys
   4180 4185 4190
   caa gcg ctc cgg tcc ggt gaa tgc acc atg gcg ctc gca ggc ggc gcc 12678
   Gln Ala Leu Arg Ser Gly Glu Cys Thr Met Ala Leu Ala Gly Gly Ala
   4195 4200 4205
   tcc gtc atg gcc act ccc ttc gtc ttc acc gag ttc tct cgc cag cgg 12726
   Ser Val Met Ala Thr Pro Phe Val Phe Thr Glu Phe Ser Arg Gln Arg
   4210 4215 4220
   ggc ctg gcc gca gac ggc cgg tgc aag gcg ttt tcg gcg gcg gcg gac 12774
   Gly Leu Ala Ala Asp Gly Arg Cys Lys Ala Phe Ser Ala Ala Ala Asp
   4225 4230 4235 4240
   ggg acc ggc tgg tcc gag ggt gtg ggg atg ctg ctg gtg gag cgg ctc 12822
   Gly Thr Gly Trp Ser Glu Gly Val Gly Met Leu Leu Val Glu Arg Leu
   4245 4250 4255
   tcc gac gcc cgc cgc aac ggt cac cgt gtc ctg gcc gtc gtc cgc ggc 12870
   Ser Asp Ala Arg Arg Asn Gly His Arg Val Leu Ala Val Val Arg Gly
   4260 4265 4270
   agc gcc gtc aac cag gac ggc gca agc aac ggc ctg acc gca ccc aac 12918
   Ser Ala Val Asn Gln Asp Gly Ala Ser Asn Gly Leu Thr Ala Pro Asn
   4275 4280 4285
   ggt cgt tca caa gtc aag gtc atc cgc cag gct ttg gcc aac gca cac 12966
   Gly Arg Ser Gln Val Lys Val Ile Arg Gln Ala Leu Ala Asn Ala His
   4290 4295 4300
   ctc tcc cct gcc gat gtc gat gcg gtg gag gcc cac ggc acg ggg acc 13014
   Leu Ser Pro Ala Asp Val Asp Ala Val Glu Ala His Gly Thr Gly Thr
   4305 4310 4315 4320
   acc ctg ggc gac ccg atc gag gct caa gcc ctc gtc gaa gcc tac ggt 13062
   Thr Leu Gly Asp Pro Ile Glu Ala Gln Ala Leu Val Glu Ala Tyr Gly
   4325 4330 4335
   cag gac cgc ccc aac ggc cgc ccc ctc tgg ctc gga acc ctc aag tcc 13110
   Gln Asp Arg Pro Asn Gly Arg Pro Leu Trp Leu Gly Thr Leu Lys Ser
   4340 4345 4350
   aac atc ggg cac tcc atg gcc gct gcg ggt gtg ggc ggg gtc atc aag 13158
   Asn Ile Gly His Ser Met Ala Ala Ala Gly Val Gly Gly Val Ile Lys
   4355 4360 4365
   atg gtg atg gcg ctg cgg aat ggt ctg ctg ccg cgg acg ttg cat gtg 13206
   Met Val Met Ala Leu Arg Asn Gly Leu Leu Pro Arg Thr Leu His Val
   4370 4375 4380
   gat gag ccg tcg ccg cat gtg gac tgg tcc gcg ggt gcg gtg cag ctg 13254
   Asp Glu Pro Ser Pro His Val Asp Trp Ser Ala Gly Ala Val Gln Leu
   4385 4390 4395 4400
   ctg acg gag acg gtg ccc tgg ccc ggc ggg gag ggg cgg cta cgg cgg 13302
   Leu Thr Glu Thr Val Pro Trp Pro Gly Gly Glu Gly Arg Leu Arg Arg
   4405 4410 4415
   gca gga gtg tca tca ttc ggc gtc agc ggc acc aac gcc cac gtc atc 13350
   Ala Gly Val Ser Ser Phe Gly Val Ser Gly Thr Asn Ala His Val Ile
   4420 4425 4430
   ctc gag gaa gca ccc gcc cac aac atc ccg tca gac aca ccc gcc gac    13398
   Leu Glu Glu Ala Pro Ala His Asn Ile Pro Ser Asp Thr Pro Ala Asp
               4435                4440                4445
   gac gtc ccg gga gaa tca gcc gcc gac gag gat gcc ggt agt ggc gat    13446
   Asp Val Pro Gly Glu Ser Ala Ala Asp Glu Asp Ala Gly Ser Gly Asp
       4450                4455                4460
   gag gct gct gcc ggc agt cca ggg gtg tgg ccg tgg ctg gtg tcg gcc    13494
   Glu Ala Ala Ala Gly Ser Pro Gly Val Trp Pro Trp Leu Val Ser Ala
   4465                4470                4475                4480
   aag tcg cag ccg gcc ctg cgc gcc cag gcc cag gcc ctg cac gcc cac    13542
   Lys Ser Gln Pro Ala Leu Arg Ala Gln Ala Gln Ala Leu His Ala His
                   4485                4490                4495
   ctc acc gac cac ccc ggc ctc gac ctc gcc gac gtc ggg tac acc ctc    13590
   Leu Thr Asp His Pro Gly Leu Asp Leu Ala Asp Val Gly Tyr Thr Leu
               4500                4505                4510
   gcc cac gcc cgc gcc gtg ttc gac cac cgc gcc acc ctc atc gcc gcc    13638
   Ala His Ala Arg Ala Val Phe Asp His Arg Ala Thr Leu Ile Ala Ala
           4515                4520                4525
   gac cgc gac acc ttc ctg caa gca ctc cag gca ctc gcc gca ggc gaa    13686
   Asp Arg Asp Thr Phe Leu Gln Ala Leu Gln Ala Leu Ala Ala Gly Glu
       4530                4535                4540
   ccc cac ccc gcc gtc atc cac agc agc gcc cca ggc ggg acc ggg acc    13734
   Pro His Pro Ala Val Ile His Ser Ser Ala Pro Gly Gly Thr Gly Thr
   4545                4550                4555                4560
   ggg gag gcc gca gga aag acc gca ttc atc tgc tcc gga cag ggc acc    13782
   Gly Glu Ala Ala Gly Lys Thr Ala Phe Ile Cys Ser Gly Gln Gly Thr
                   4565                4570                4575
   caa cgc ccc ggc atg gcc cac ggc ctc tac cac acc cac ccc gtc ttc    13830
   Gln Arg Pro Gly Met Ala His Gly Leu Tyr His Thr His Pro Val Phe
               4580                4585                4590
   gcc gcc gca ctc aac gac atc tgc acc cac ctc gac ccc cac ctc gac    13878
   Ala Ala Ala Leu Asn Asp Ile Cys Thr His Leu Asp Pro His Leu Asp
           4595                4600                4605
   cac ccc ctc ctc ccc ctc ctc acc cag gac ccc aac acc cag gac acc    13926
   His Pro Leu Leu Pro Leu Leu Thr Gln Asp Pro Asn Thr Gln Asp Thr
       4610                4615                4620
   acc acc ctc gaa gaa gcg gcc gca ctg ctc cag cag acc cgc tac gcc    13974
   Thr Thr Leu Glu Glu Ala Ala Ala Leu Leu Gln Gln Thr Arg Tyr Ala
   4625                4630                4635                4640
   cag ccc gcc ctc ttc gcc ttc cag gtc gcc ctc cac cgc ctc ctc acc    14022
   Gln Pro Ala Leu Phe Ala Phe Gln Val Ala Leu His Arg Leu Leu Thr
                   4645                4650                4655
   gac ggc tac cac atc acc ccc cac tac tac gcc gga cac tcc ctc ggc    14070
   Asp Gly Tyr His Ile Thr Pro His Tyr Tyr Ala Gly His Ser Leu Gly
               4660                4665                     4670
   gaa atc acc gcc gcc cac ctc gcc ggc atc ctc acc ctc acc gac gcc    14118
   Glu Ile Thr Ala Ala His Leu Ala Gly Ile Leu Thr Leu Thr Asp Ala
           4675                4680                     4685
   acc acc ctc atc acc caa cgc gcc acc ctc atg caa acc atg ccc ccc    14166
   Thr Thr Leu Ile Thr Gln Arg Ala Thr Leu Met Gln Thr Met Pro Pro
       4690                4695                     4700
   ggc acc atg acc acc ctc cac acc acc ccc cac cac atc acc cac cac    14214
   Gly Thr Met Thr Thr Leu His Thr Thr Pro His His Ile Thr His His
   4705                4710                     4715                4720
   ctc acc gcc cac gaa aac gac ctc gcc atc gcc gcc atc aac acc ccc    14262
   Leu Thr Ala His Glu Asn Asp Leu Ala Ile Ala Ala Ile Asn Thr Pro
                   4725                     4730                4735
   acc tcc ctc gtc atc agc ggc acc ccc cac acc gtc caa cac atc acc    14310
   Thr Ser Leu Val Ile Ser Gly Thr Pro His Thr Val Gln His Ile Thr
               4740                4745                     4750
   acc ctc tgc caa caa caa ggc atc aaa acc aaa acc ctc ccc acc aac    14358
   Thr Leu Cys Gln Gln Gln Gly Ile Lys Thr Lys Thr Leu Pro Thr Asn
           4755                4760                     4765
   cac gcc ttc cac tcc ccc cac acc aac ccc atc ctc aac caa ctc cac    14406
   His Ala Phe His Ser Pro His Thr Asn Pro Ile Leu Asn Gln Leu His
       4770                4775                     4780
   cag cac acc caa acc ctc acc tac cac cca ccc cac acc ccc ctc atc    14454
   Gln His Thr Gln Thr Leu Thr Tyr His Pro Pro His Thr Pro Leu Ile
   4785                4790                     4795                4800
   acc gcc aac acc cca ccc gac caa ctc ctc acc ccc cac tac tgg acc    14502
   Thr Ala Asn Thr Pro Pro Asp Gln Leu Leu Thr Pro His Tyr Trp Thr
                   4805                     4810                4815
   caa caa gcc cgc aac acc gtc gac tac gcc acc acc acc caa acc ctc    14550
   Gln Gln Ala Arg Asn Thr Val Asp Tyr Ala Thr Thr Thr Gln Thr Leu
               4820                4825                     4830
   cac caa cac ggc gtc acc acc tac atc gaa ctc gga ccc gac aac acc    14598
   His Gln His Gly Val Thr Thr Tyr Ile Glu Leu Gly Pro Asp Asn Thr
           4835                4840                     4845
   ctc acc acc ctc acc cac gac aac ctc ccc aac acc ccc acc acc acc    14646
   Leu Thr Thr Leu Thr His Asp Asn Leu Pro Asn Thr Pro Thr Thr Thr
       4850                4855                     4860
   ctc acc ctc acc cac ccc cac cac cac ccc caa acc cac ctc ctc acc    14694
   Leu Thr Leu Thr His Pro His His His Pro Gln Thr His Leu Leu Thr
   4865                4870                     4875                4880
   aac ctc gcc aaa acc acc acc acc tgg cac ccc cac cac tac acc cac    14742
   Asn Leu Ala Lys Thr Thr Thr Thr Trp His Pro His His Tyr Thr His
   4885 4890 4895
   cac cac aac caa ccc cac acc cac acc cac ctc gac ctc ccc acc tac 14790
   His His Asn Gln Pro His Thr His Thr His Leu Asp Leu Pro Thr Tyr
   4900 4905 4910
   ccc ttc caa cac cac cac tac tgg ctc caa cca ccc ggc aag ccg agc 14838
   Pro Phe Gln His His His Tyr Trp Leu Gln Pro Pro Gly Lys Pro Ser
   4915 4920 4925
   gac ccg tca ccg agc gaa ggc cgt gag caa gcc acg acc cca tca acc 14886
   Asp Pro Ser Pro Ser Glu Gly Arg Glu Gln Ala Thr Thr Pro Ser Thr
   4930 4935 4940
   ccg ctg cgt gat gtc ctc gtg ggc aag tct ccg cag gag cga gac gaa 14934
   Pro Leu Arg Asp Val Leu Val Gly Lys Ser Pro Gln Glu Arg Asp Glu
   4945 4950 4955 4960
   gag ctg ttg cgc ctg gtg cgc acc cat gcg gcc gct gtg ctg ggc cat 14982
   Glu Leu Leu Arg Leu Val Arg Thr His Ala Ala Ala Val Leu Gly His
   4965 4970 4975
   gcc act ccc gaa gtg atc gtt ccg aac aag gcc ttc aaa gag ctg ggt 15030
   Ala Thr Pro Glu Val Ile Val Pro Asn Lys Ala Phe Lys Glu Leu Gly
   4980 4985 4990
   ttt gat tct ctc gcc gca att cag ctt cgt aat cga ctg ctt gct gac 15078
   Phe Asp Ser Leu Ala Ala Ile Gln Leu Arg Asn Arg Leu Leu Ala Asp
   4995 5000 5005
   gtt gac ctg ccg ctt ccg gcc acg ctg atc ttc gat tac ccc act ccg 15126
   Val Asp Leu Pro Leu Pro Ala Thr Leu Ile Phe Asp Tyr Pro Thr Pro
   5010 5015 5020
   atg gcg ctt tgc cag ttc ctc cgg gcg gcg atc gtc gga gcg gac aca 15174
   Met Ala Leu Cys Gln Phe Leu Arg Ala Ala Ile Val Gly Ala Asp Thr
   5025 5030 5035 5040
   ggc acg acc act cgt ctg ccg cta act gcg gtc ccc gcc gac gag ccg 15222
   Gly Thr Thr Thr Arg Leu Pro Leu Thr Ala Val Pro Ala Asp Glu Pro
   5045 5050 5055
   atc gcc atc gtc ggc atg gcc tgt cgg tac ccc ggt gat gta cgg acg 15270
   Ile Ala Ile Val Gly Met Ala Cys Arg Tyr Pro Gly Asp Val Arg Thr
   5060 5065 5070
   gtc gat gat ctc tgg cag gtg gtc agt ggt ggc cat gac gcg atc ggc 15318
   Val Asp Asp Leu Trp Gln Val Val Ser Gly Gly His Asp Ala Ile Gly
   5075 5080 5085
   gga ttc ccg acg aac cgt ggg tgg gac ctc gac acg ctg tac aac ccg 15366
   Gly Phe Pro Thr Asn Arg Gly Trp Asp Leu Asp Thr Leu Tyr Asn Pro
   5090 5095 5100
   gac ccg gac cac cac gga acc agc tac acc cgg agc ggc gga ttc ctt 15414
   Asp Pro Asp His His Gly Thr Ser Tyr Thr Arg Ser Gly Gly Phe Leu
   5105                5110                  5115                  5120
   tac gac gca ggc aat ttc gat ccc gac ttc ttc ggt atc agt ccg cgt      15462
   Tyr Asp Ala Gly Asn Phe Asp Pro Asp Phe Phe Gly Ile Ser Pro Arg
                   5125                5130                5135
   gag gca ctg gcg atg gac ccg cag cag cgg ctg ctg ctg gaa aca gcg      15510
   Glu Ala Leu Ala Met Asp Pro Gln Gln Arg Leu Leu Leu Glu Thr Ala
               5140                5145                5150
   tgg gag agc atc gaa cac gcc tgc atc aac ccc gac agc ctc cgt ggc      15558
   Trp Glu Ser Ile Glu His Ala Cys Ile Asn Pro Asp Ser Leu Arg Gly
           5155                5160                5165
   aca cca acc ggc gtc ttc gcc ggg ctg acc tac cac gac tac gcc gcg      15606
   Thr Pro Thr Gly Val Phe Ala Gly Leu Thr Tyr His Asp Tyr Ala Ala
       5170                5175                5180
   cgc ttt ccc aca gct ccg gca ggg ttc gag ggg tat ctc ggg cac gga      15654
   Arg Phe Pro Thr Ala Pro Ala Gly Phe Glu Gly Tyr Leu Gly His Gly
   5185                5190                5195                5200
   agc gca ggc agt atc gcc tcg ggt cgt gtc gcc tac gct ctc ggc ctg      15702
   Ser Ala Gly Ser Ile Ala Ser Gly Arg Val Ala Tyr Ala Leu Gly Leu
                   5205                5210                5215
   gaa ggt ccg gcc ctc aca gtc gac act gcc tgc tct tcg tcc ctg gtc      15750
   Glu Gly Pro Ala Leu Thr Val Asp Thr Ala Cys Ser Ser Ser Leu Val
               5220                5225                5230
   gct ctg cac ctg gcc tgt cag gcg ctg cgg tcc ggc gag tgt tcc atg      15798
   Ala Leu His Leu Ala Cys Gln Ala Leu Arg Ser Gly Glu Cys Ser Met
           5235                5240                5245
   gcc ctc gcg ggt ggc gtc acg gtg atg tca acc ccg gcc ggg ttc gtg      15846
   Ala Leu Ala Gly Gly Val Thr Val Met Ser Thr Pro Ala Gly Phe Val
       5250                5255                5260
   gag ttt tcg cgg cag cgg ggc ctg gcc gtg gac ggg cgg tgc aag gcg      15894
   Glu Phe Ser Arg Gln Arg Gly Leu Ala Val Asp Gly Arg Cys Lys Ala
   5265                5270                5275                5280
   ttc tcg gca gcg gct gac ggc acc ggc tgg ggt gag ggt gtc gga atg      15942
   Phe Ser Ala Ala Ala Asp Gly Thr Gly Trp Gly Glu Gly Val Gly Met
                   5285                5290                5295
   ctg ctg gtg gag cgg ctg tcg gac gcg cgg cgg ctc ggt cac cga atc      15990
   Leu Leu Val Glu Arg Leu Ser Asp Ala Arg Arg Leu Gly His Arg Ile
               5300                5305                5310
   ctc gcg gtg gtg cgt ggc agt gcg gtc aat cag gac ggt gcg agc aac      16038
   Leu Ala Val Val Arg Gly Ser Ala Val Asn Gln Asp Gly Ala Ser Asn
           5315                5320                5325
   ggg ctg acg gcg ccc aac ggg ccg tcc cag gag cgt gtc atc cgc ctg      16086
   Gly Leu Thr Ala Pro Asn Gly Pro Ser Gln Glu Arg Val Ile Arg Leu
       5330                5335                5340
   gcc ctg gcc aac gcg gac ctg acc ccc gcc gac gtc gat gcg gtg gag      16134
   Ala Leu Ala Asn Ala Asp Leu Thr Pro Ala Asp Val Asp Ala Val Glu
   5345                5350                5355                5360
   gcc cac ggc acc ggc acc act ttg ggc gac ccg atc gag gcc cag gcc      16182
   Ala His Gly Thr Gly Thr Thr Leu Gly Asp Pro Ile Glu Ala Gln Ala
                   5365                5370                5375
   ctc ctc gcc acc tac gga cag gac cgc ccc ggc aac gaa ccg ctg tgg      16230
   Leu Leu Ala Thr Tyr Gly Gln Asp Arg Pro Gly Asn Glu Pro Leu Trp
               5380                5385                5390
   ctg ggc tcg atg aag tcg aac atc ggc cac gcg cag gct gcc gca ggt      16278
   Leu Gly Ser Met Lys Ser Asn Ile Gly His Ala Gln Ala Ala Ala Gly
           5395                5400                5405
   gtg ggc ggg gtc atc aag atg gtg atg gcg ctg cgg aat ggt ctg ctg      16326
   Val Gly Gly Val Ile Lys Met Val Met Ala Leu Arg Asn Gly Leu Leu
       5410                5415                5420
   ccg cgg acg ttg cat gtg gat gag ccg tcg ccg cat gtg gac tgg tcc      16374
   Pro Arg Thr Leu His Val Asp Glu Pro Ser Pro His Val Asp Trp Ser
   5425                5430                5435                5440
   gcg ggg gcg gtg cag ctg ctg acg gag acg gtg ccc tgg ccc ggc ggg      16422
   Ala Gly Ala Val Gln Leu Leu Thr Glu Thr Val Pro Trp Pro Gly Gly
                   5445                5450                5455
   gag ggg cgg ctg cgg cgg gca gga gtg tca tcg ttc ggc gtc agc ggc      16470
   Glu Gly Arg Leu Arg Arg Ala Gly Val Ser Ser Phe Gly Val Ser Gly
               5460                5465                5470
   acc aac gcc cac gtc atc ctc gaa gaa gca ccc gcc cac aac atc ccg      16518
   Thr Asn Ala His Val Ile Leu Glu Glu Ala Pro Ala His Asn Ile Pro
           5475                5480                5485
   tca gac aca ccc gcc gac gac gcc ccg gga gaa gca gcc gcc gac gat      16566
   Ser Asp Thr Pro Ala Asp Asp Ala Pro Gly Glu Ala Ala Ala Asp Asp
       5490                5495                5500
   gtt ccg ggg gaa gcg gcc ggc gac gac gcc ggt acc ggc ggg gaa gcg      16614
   Val Pro Gly Glu Ala Ala Gly Asp Asp Ala Gly Thr Gly Gly Glu Ala
   5505                5510                5515                5520
   act ggt cct gct gcc ggc agt cca ggg gtg tgg ccg tgg ctg gtg tcg      16662
   Thr Gly Pro Ala Ala Gly Ser Pro Gly Val Trp Pro Trp Leu Val Ser
                   5525                5530                5535
   gcc aag tcg cag ccg gcc ctg cgc gcc cag gcc cag gcc ctg cac gcc      16710
   Ala Lys Ser Gln Pro Ala Leu Arg Ala Gln Ala Gln Ala Leu His Ala
               5540                5545                5550
   cac ctc acc gac cac ccc ggc ctc gac ctc gcc gac gtc ggg tac acc      16758
   His Leu Thr Asp His Pro Gly Leu Asp Leu Ala Asp Val Gly Tyr Thr
           5555                5560                5565
   ctc gcc cac gcc cgc gcc gtg ttc gac cac cgc gcc acc ctc atc gcc    16806
   Leu Ala His Ala Arg Ala Val Phe Asp His Arg Ala Thr Leu Ile Ala
      5570                5575                5580
   gcc gac cgc gac acc ttc ctg caa gca ctc cag gca ctc gcc gca ggc    16854
   Ala Asp Arg Asp Thr Phe Leu Gln Ala Leu Gln Ala Leu Ala Ala Gly
   5585                5590                5595                5600
   gaa ccc cac ccc gcc gtc atc cac agc agc gcc cca ggc ggg acc ggg    16902
   Glu Pro His Pro Ala Val Ile His Ser Ser Ala Pro Gly Gly Thr Gly
                   5605                5610                5615
   acc ggg gag gcc gca gga aag acc gca ttc atc tgc tcc gga cag ggc    16950
   Thr Gly Glu Ala Ala Gly Lys Thr Ala Phe Ile Cys Ser Gly Gln Gly
               5620                5625                5630
   acc caa cgc ccc ggc atg gcc cac ggc ctc tac cac acc cac ccc gtc    16998
   Thr Gln Arg Pro Gly Met Ala His Gly Leu Tyr His Thr His Pro Val
           5635                5640                5645
   ttc gcc gcc gca ctc aac gac atc tgc acc cac ctc gac ccc cac ctc    17046
   Phe Ala Ala Ala Leu Asn Asp Ile Cys Thr His Leu Asp Pro His Leu
       5650                5655                5660
   gac cac ccc ctc ctc ccc ctc ctc acc cag gac ccc aac acc cag gac    17094
   Asp His Pro Leu Leu Pro Leu Leu Thr Gln Asp Pro Asn Thr Gln Asp
   5665                5670                5675                5680
   acc acc acc ctc gaa gaa gcg gcc gca ctg ctc cag cag acc ccg tac    17142
   Thr Thr Thr Leu Glu Glu Ala Ala Ala Leu Leu Gln Gln Thr Pro Tyr
                   5685                5690                5695
   gcc cag ccc gcc ctc ttc gcc ttc cag gtc gcc ctc cac cgc ctc ctc    17190
   Ala Gln Pro Ala Leu Phe Ala Phe Gln Val Ala Leu His Arg Leu Leu
               5700                5705                5710
   acc gac ggc tac cac atc acc ccc cac tac tac gcc gga cac tcc ctc    17238
   Thr Asp Gly Tyr His Ile Thr Pro His Tyr Tyr Ala Gly His Ser Leu
           5715                5720                5725
   ggc gaa atc acc gcc gcc cac ctc gcc ggc atc ctc acc ctc acc gac    17286
   Gly Glu Ile Thr Ala Ala His Leu Ala Gly Ile Leu Thr Leu Thr Asp
       5730                5735                5740
   gcc acc acc ctc atc acc caa cgc gcc acc ctc atg caa acc atg ccc    17334
   Ala Thr Thr Leu Ile Thr Gln Arg Ala Thr Leu Met Gln Thr Met Pro
   5745                5750                5755                5760
   ccc ggc acc atg acc acc ctc cac acc acc ccc cac cac atc acc cac    17382
   Pro Gly Thr Met Thr Thr Leu His Thr Thr Pro His His Ile Thr His
                   5765                5770                5775
   cac ctc acc gcc cac gaa aac gac ctc gcc atc gcc gcc atc aac acc    17430
   His Leu Thr Ala His Glu Asn Asp Leu Ala Ile Ala Ala Ile Asn Thr
               5780                5785                5790
   ccc acc tcc ctc gtc atc agc ggc acc ccc cac acc gtc caa cac atc    17478
   Pro Thr Ser Leu Val Ile Ser Gly Thr Pro His Thr Val Gln His Ile
           5795                5800                5805
   acc acc ctc tgc caa caa caa ggc atc aaa acc aaa acc ctc ccc acc    17526
   Thr Thr Leu Cys Gln Gln Gln Gly Ile Lys Thr Lys Thr Leu Pro Thr
       5810                5815                5820
   aaa aac gcc ttc cac tcc ccc cac acc aac ccc atc ctc aac caa ctc    17574
   Lys Asn Ala Phe His Ser Pro His Thr Asn Pro Ile Leu Asn Gln Leu
   5825                5830                5835                5840
   cac cag cac acc caa acc ctc acc tac cac cca ccc cac acc ccc ctc    17622
   His Gln His Thr Gln Thr Leu Thr Tyr His Pro Pro His Thr Pro Leu
                   5845                5850                5855
   atc acc gcc aac acc cca ccc gac caa ctc ctc acc ccc cac tac tgg    17670
   Ile Thr Ala Asn Thr Pro Pro Asp Gln Leu Leu Thr Pro His Tyr Trp
               5860                5865                5870
   acc caa caa gcc cgc aac acc gtc gac tac gcc acc acc acc caa acc    17718
   Thr Gln Gln Ala Arg Asn Thr Val Asp Tyr Ala Thr Thr Thr Gln Thr
           5875                5880                5885
   ctc cac caa cac ggc gtc acc acc tac atc gaa ctc gga ccc gac aac    17766
   Leu His Gln His Gly Val Thr Thr Tyr Ile Glu Leu Gly Pro Asp Asn
       5890                5895                5900
   acc ctc acc acc ctc acc cac cac aac ctc ccc aac acc ccc acc acc    17814
   Thr Leu Thr Thr Leu Thr His His Asn Leu Pro Asn Thr Pro Thr Thr
   5905                5910                5915                5920
   acc ctc acc ctc acc cac ccc cac cac cac ccc caa acc cac ctc ctc    17862
   Thr Leu Thr Leu Thr His Pro His His His Pro Gln Thr His Leu Leu
                   5925                5930                5935
   acc aac ctc gcc aaa acc acc acc acc tgg cac ccc cac cac tac acc    17910
   Thr Asn Leu Ala Lys Thr Thr Thr Thr Trp His Pro His His Tyr Thr
               5940                5945                5950
   cac cac cac aac caa ccc cac acc cac acc cac ctc gac ctc ccc acc    17958
   His His His Asn Gln Pro His Thr His Thr His Leu Asp Leu Pro Thr
           5955                5960                5965
   tac ccc ttc caa cac cag cac tac tgg ctc gaa agc aca cag ccg ggt    18006
   Tyr Pro Phe Gln His Gln His Tyr Trp Leu Glu Ser Thr Gln Pro Gly
       5970                5975                5980
   gcc gga tcc ggt tcg ggt tcc ggt tcc ggg cgg gca ggg act gcg ggc    18054
   Ala Gly Ser Gly Ser Gly Ser Gly Ser Gly Arg Ala Gly Thr Ala Gly
   5985                5990                5995                6000
   ggg acg gca gag gtg gag tcg cgg ttc tgg gac gcg gtg gcc cgc cag    18102
   Gly Thr Ala Glu Val Glu Ser Arg Phe Trp Asp Ala Val Ala Arg Gln
                   6005                6010                6015
   gac ctg gaa acg gtc gcg acc acg ctc gcc gtg ccc ccc tcc gcc ggc    18150
   Asp Leu Glu Thr Val Ala Thr Thr Leu Ala Val Pro Pro Ser Ala Gly
   6020 6025 6030
   ctg gac acg gtg gtg ccc gca ctc tcc gcc tgg cac cgc cac caa cac 18198
   Leu Asp Thr Val Val Pro Ala Leu Ser Ala Trp His Arg His Gln His
   6035 6040 6045
   gac caa gcc cgc atc aac acc tgg acc tac cag gaa acc tgg aaa ccc 18246
   Asp Gln Ala Arg Ile Asn Thr Trp Thr Tyr Gln Glu Thr Trp Lys Pro
   6050 6055 6060
   ctc acc ctc ccc acc acc cac caa ccc cac caa acc tgg ctc atc gcc 18294
   Leu Thr Leu Pro Thr Thr His Gln Pro His Gln Thr Trp Leu Ile Ala
   6065 6070 6075 6080
   atc ccc gaa acc cag acc cac cac ccc cac atc acc aac atc ctc acc 18342
   Ile Pro Glu Thr Gln Thr His His Pro His Ile Thr Asn Ile Leu Thr
   6085 6090 6095
   aac ctc cac cac cac ggc atc acc ccc atc ccc ctc acc ctc aac cac 18390
   Asn Leu His His His Gly Ile Thr Pro Ile Pro Leu Thr Leu Asn His
   6100 6105 6110
   acc cac acc aac ccc caa cac ctc cac cac acc cga caa caa gcc caa 18438
   Thr His Thr Asn Pro Gln His Leu His His Thr Arg Gln Gln Ala Gln
   6115 6120 6125
   aac cac acc acc gga ccc atc acc ggc ctg ctc tcc ctc ctc gcc ctc 18486
   Asn His Thr Thr Gly Pro Ile Thr Gly Leu Leu Ser Leu Leu Ala Leu
   6130 6135 6140
   gac gaa aca ccc cac ccc cac cac ccc cac aca ccc acc ggc acc ctc 18534
   Asp Glu Thr Pro His Pro His His Pro His Thr Pro Thr Gly Thr Leu
   6145 6150 6155 6160
   ctc aac ctc acc ctc acc caa acc cac acc caa acc cac cca cca acc 18582
   Leu Asn Leu Thr Leu Thr Gln Thr His Thr Gln Thr His Pro Pro Thr
   6165 6170 6175
   ccc ctc tgg tac gcc acc acc aac gcc acc acc acc cac ccc aac gac 18630
   Pro Leu Trp Tyr Ala Thr Thr Asn Ala Thr Thr Thr His Pro Asn Asp
   6180 6185 6190
   ccc ctc aca cac ccc acc caa gcc caa acc tgg gga ctc gcc cgc acc 18678
   Pro Leu Thr His Pro Thr Gln Ala Gln Thr Trp Gly Leu Ala Arg Thr
   6195 6200 6205
   acc ctc ctc gaa cac ccc acc cac acc gcc gga atc atc gac ctc ccc 18726
   Thr Leu Leu Glu His Pro Thr His Thr Ala Gly Ile Ile Asp Leu Pro
   6210 6215 6220
   acc acc ccc acc ccc cac acc ctc cac cac ctc acc caa acc ctc acc 18774
   Thr Thr Pro Thr Pro His Thr Leu His His Leu Thr Gln Thr Leu Thr
   6225 6230 6235 6240
   caa ccc cac cac caa acc caa ctc gcc atc cgc acc acc ggc acc cac 18822
   Gln Pro His His Gln Thr Gln Leu Ala Ile Arg Thr Thr Gly Thr His
   6245 6250 6255
   acc cgc cgc ctc acc ccc acc acc ctc acc ccc aca cac caa cca ccc    18870
   Thr Arg Arg Leu Thr Pro Thr Thr Leu Thr Pro Thr His Gln Pro Pro
               6260                6265                6270
   acc ccc acc ccc cac gga acc acc ctc atc acc ggc gga acc ggc gcc    18918
   Thr Pro Thr Pro His Gly Thr Thr Leu Ile Thr Gly Gly Thr Gly Ala
           6275                6280                6285
   ctc gcc acc cac ctc acc cac cac ctc acc acc cac caa ccc acc caa    18966
   Leu Ala Thr His Leu Thr His His Leu Thr Thr His Gln Pro Thr Gln
       6290                6295                6300
   cac ctc ctc ctc acc agc cga acc ggc ccc cac acc ccc cac gca caa    19014
   His Leu Leu Leu Thr Ser Arg Thr Gly Pro His Thr Pro His Ala Gln
   6305                6310                6315                6320
   cac ctc acc acc caa ctc caa caa aaa ggc atc cac ctc acc atc acc    19062
   His Leu Thr Thr Gln Leu Gln Gln Lys Gly Ile His Leu Thr Ile Thr
                   6325                6330                6335
   acc tgc gac acc agc aac cca gac caa ctc caa caa ctc ctc aac acc    19110
   Thr Cys Asp Thr Ser Asn Pro Asp Gln Leu Gln Gln Leu Leu Asn Thr
               6340                6345                6350
   atc ccc cca caa cac ccc ctc acc acc gtc atc cac acc gca ggc atc    19158
   Ile Pro Pro Gln His Pro Leu Thr Thr Val Ile His Thr Ala Gly Ile
           6355                6360                6365
   ctc gac gac gcc acc ctc acc aac ctc acc ccc acc caa ctc aac aac    19206
   Leu Asp Asp Ala Thr Leu Thr Asn Leu Thr Pro Thr Gln Leu Asn Asn
       6370                6375                6380
   gtc ctc cgc gcc aaa gcc cac agc gcc cac ctc ctc cac caa ctc acc    19254
   Val Leu Arg Ala Lys Ala His Ser Ala His Leu Leu His Gln Leu Thr
   6385                6390                6395                6400
   caa cac acc ccc ctc aac gcc ttc gtc ctc tac tcc tcc gcc gcc gcc    19302
   Gln His Thr Pro Leu Asn Ala Phe Val Leu Tyr Ser Ser Ala Ala Ala
                   6405                6410                6415
   acc ttc ggc gca ccc ggc caa gcc aac tac gcc gca gcc aac gcc tac    19350
   Thr Phe Gly Ala Pro Gly Gln Ala Asn Tyr Ala Ala Ala Asn Ala Tyr
               6420                6425                6430
   ctc gac gcc ctc gcc cac cac cgc cac acc cac cac ctc ccc gcc acc    19398
   Leu Asp Ala Leu Ala His His Arg His Thr His His Leu Pro Ala Thr
           6435                6440                6445
   agc atc gcc tgg ggc acc tgg caa gga aac gga ctg gcg act ggt caa    19446
   Ser Ile Ala Trp Gly Thr Trp Gln Gly Asn Gly Leu Ala Thr Gly Gln
       6450                6455                6460
   gtc agc gaa cat ctc cgc cgc cgc ggg atg ttc gcc atg ccg ccc gag    19494
   Val Ser Glu His Leu Arg Arg Arg Gly Met Phe Ala Met Pro Pro Glu
   6465                6470                6475                6480
   ttg gcg gtc aca gct gtt gac ggc gcg atc gcg agc ggg cgc ccg agt    19542
   Leu Ala Val Thr Ala Val Asp Gly Ala Ile Ala Ser Gly Arg Pro Ser
                   6485                6490                6495
   ctc ctc gtc gcc gat atc gac tgg aag aaa ttg gga ccg gtt ctc tcc    19590
   Leu Leu Val Ala Asp Ile Asp Trp Lys Lys Leu Gly Pro Val Leu Ser
               6500                6505                6510
   agc aag tcg tcg gtc ttg ctc gag gac ctt ccc cag gca cag gga act    19638
   Ser Lys Ser Ser Val Leu Leu Glu Asp Leu Pro Gln Ala Gln Gly Thr
           6515                6520                6525
   gag gag gcg cgc agt acc gtt gag cag acg gag agc aca aac ctc cgg    19686
   Glu Glu Ala Arg Ser Thr Val Glu Gln Thr Glu Ser Thr Asn Leu Arg
       6530                6535                6540
   caa ctc ctc atg ggt cgg tca cgt tcc gag cag gaa gaa gag ctg ctc    19734
   Gln Leu Leu Met Gly Arg Ser Arg Ser Glu Gln Glu Glu Glu Leu Leu
   6545                6550                6555                6560
   agc ctc gtc cgc atc cac tcc gcg gca gtg ctc ggg cgc gac gac tcc    19782
   Ser Leu Val Arg Ile His Ser Ala Ala Val Leu Gly Arg Asp Asp Ser
                   6565                6570                6575
   gag gcc atc ccg ccc ggt cgg ctg ttc agg gat cta ggg ttc gac tcg    19830
   Glu Ala Ile Pro Pro Gly Arg Leu Phe Arg Asp Leu Gly Phe Asp Ser
               6580                6585                6590
   ctt gcg gcg gtg gag ctt cgc aac cac ctc gca gca cag acg gag ctg    19878
   Leu Ala Ala Val Glu Leu Arg Asn His Leu Ala Ala Gln Thr Glu Leu
           6595                6600                6605
   gct ctg ccg acg act ctc gtc ttc gat tac ccc agc ccc acc aag ctc    19926
   Ala Leu Pro Thr Thr Leu Val Phe Asp Tyr Pro Ser Pro Thr Lys Leu
       6610                6615                6620
   gcc caa ttt ctg ctc tcc gag atc gcg gag ttc cag ccc gac aac tca    19974
   Ala Gln Phe Leu Leu Ser Glu Ile Ala Glu Phe Gln Pro Asp Asn Ser
   6625                6630                6635                6640
   act ccg ctt ccg cga ccc cgg gca gag ctc gat gag ccg atc gcc atc    20022
   Thr Pro Leu Pro Arg Pro Arg Ala Glu Leu Asp Glu Pro Ile Ala Ile
                   6645                6650                6655
   gtt ggc atg gcc tgt cgc ttc ccc ggc gga gtg acc tcg gcg gac gac    20070
   Val Gly Met Ala Cys Arg Phe Pro Gly Gly Val Thr Ser Ala Asp Asp
               6660                6665                6670
   ttc tgg gat ctg atc tcc tcc gag cag gac gcg atc ggc gga ttc ccc    20118
   Phe Trp Asp Leu Ile Ser Ser Glu Gln Asp Ala Ile Gly Gly Phe Pro
           6675                6680                6685
   acc gac cgc ggc tgg gac ctg gac acg ctc tac gac ccc gac ccc gac    20166
   Thr Asp Arg Gly Trp Asp Leu Asp Thr Leu Tyr Asp Pro Asp Pro Asp
       6690                6695                6700
   cac ccc ggc acc tgc tac acc cga aac ggc gga ttc ctc tac gac gca    20214
   His Pro Gly Thr Cys Tyr Thr Arg Asn Gly Gly Phe Leu Tyr Asp Ala
   6705                6710                6715                6720
   ggc cac ttc gac gcc gaa ttc ttc ggc atc agc ccc cgc gaa gcc ctc     20262
   Gly His Phe Asp Ala Glu Phe Phe Gly Ile Ser Pro Arg Glu Ala Leu
                   6725                6730                6735
   gcc atg gac ccc cag caa cga ctc ctc ctc gaa acc gcc tgg gaa acc     20310
   Ala Met Asp Pro Gln Gln Arg Leu Leu Leu Glu Thr Ala Trp Glu Thr
               6740                6745                6750
   atc gaa cac gcc ggc atc aac ccc cac acc ctc cac ggc acc ccc acc     20358
   Ile Glu His Ala Gly Ile Asn Pro His Thr Leu His Gly Thr Pro Thr
           6755                6760                6765
   gga gtc ttc acc ggc acc aac gga cag gac cac gcg gca cac atc cgt     20406
   Gly Val Phe Thr Gly Thr Asn Gly Gln Asp His Ala Ala His Ile Arg
       6770                6775                6780
   cag gcc ccg agc ggt acc gag gga ttc gtc ctg acc ggg gca gcc acc     20454
   Gln Ala Pro Ser Gly Thr Glu Gly Phe Val Leu Thr Gly Ala Ala Thr
   6785                6790                6795                6800
   agc atc gcc tcc ggc cga atc tcc tac atc ctc ggg ttg gaa ggg cct     20502
   Ser Ile Ala Ser Gly Arg Ile Ser Tyr Ile Leu Gly Leu Glu Gly Pro
                   6805                6810                6815
   gcg gtc acc ctc gac aca gcg tgt tcc tcc tcg ctc gtc gcc ctg cac     20550
   Ala Val Thr Leu Asp Thr Ala Cys Ser Ser Ser Leu Val Ala Leu His
               6820                6825                6830
   ctc gcc tgc cag tcc ctc agg tcc ggt gaa tgc acc atg gcc ttg gcc     20598
   Leu Ala Cys Gln Ser Leu Arg Ser Gly Glu Cys Thr Met Ala Leu Ala
           6835                6840                6845
   ggc ggg gcc acg gtc atg acc acc ccg atc acc ttc acc gaa ttc gcc     20646
   Gly Gly Ala Thr Val Met Thr Thr Pro Ile Thr Phe Thr Glu Phe Ala
       6850                6855                6860
   cgc caa cgc gga ctc gcc ccc gac ggg cgt tgc aag gcg ttc tcg gcg     20694
   Arg Gln Arg Gly Leu Ala Pro Asp Gly Arg Cys Lys Ala Phe Ser Ala
   6865                6870                6875                6880
   gcg gct gac ggt acc ggc tgg ggt gag ggt gtg ggg atg ctg ctg gtg     20742
   Ala Ala Asp Gly Thr Gly Trp Gly Glu Gly Val Gly Met Leu Leu Val
                   6885                6890                6895
   gag cgg ctc tcc gac gcc cgc cgc aac ggt cac cgt gtc ctg gcc gtg     20790
   Glu Arg Leu Ser Asp Ala Arg Arg Asn Gly His Arg Val Leu Ala Val
               6900                6905                6910
   gtg cgt ggc agt gcg gtc aac cag gac ggt gcg agc aac ggt ctg acc     20838
   Val Arg Gly Ser Ala Val Asn Gln Asp Gly Ala Ser Asn Gly Leu Thr
           6915                6920                6925
   gcg ccc aac ggg ccc tcc cag cag cgc gtc atc cgc cag gcc ctc gcc     20886
   Ala Pro Asn Gly Pro Ser Gln Gln Arg Val Ile Arg Gln Ala Leu Ala
   6930 6935 6940
   aac gcg gac ctg acc ccc gcc gac gtc gat gcg gtg gag gcc cac ggc 20934
   Asn Ala Asp Leu Thr Pro Ala Asp Val Asp Ala Val Glu Ala His Gly
   6945 6950 6955 6960
   acc ggc acc act ttg ggc gac ccg atc gag gcc cag gcc atc ctc gcg 20982
   Thr Gly Thr Thr Leu Gly Asp Pro Ile Glu Ala Gln Ala Ile Leu Ala
   6965 6970 6975
   acc tac gga cag gac cgt ccc ggc aac ggg ccg ttg tgg ctg ggc tcc 21030
   Thr Tyr Gly Gln Asp Arg Pro Gly Asn Gly Pro Leu Trp Leu Gly Ser
   6980 6985 6990
   gtc aag tcc aac gtc gga cac aca cag gcc gcg gcg ggc gtg gcc gga 21078
   Val Lys Ser Asn Val Gly His Thr Gln Ala Ala Ala Gly Val Ala Gly
   6995 7000 7005
   gtg atc aag atg gtg atg gcc ctc cgc cac cgg aca ctc cca ccg act 21126
   Val Ile Lys Met Val Met Ala Leu Arg His Arg Thr Leu Pro Pro Thr
   7010 7015 7020
   ctc cac gcg gat gag ccg tcg ccg cat gtg gac tgg tcc gcg ggt gcg 21174
   Leu His Ala Asp Glu Pro Ser Pro His Val Asp Trp Ser Ala Gly Ala
   7025 7030 7035 7040
   gtg cag ctg ctg acg gag acg gtg ccc tgg ccc ggc ggg gag ggg cgg 21222
   Val Gln Leu Leu Thr Glu Thr Val Pro Trp Pro Gly Gly Glu Gly Arg
   7045 7050 7055
   ccg cgg cgg gca gga gtg tca tca ttc ggc gtc agc ggc acc aac gcc 21270
   Pro Arg Arg Ala Gly Val Ser Ser Phe Gly Val Ser Gly Thr Asn Ala
   7060 7065 7070
   cac gtc atc ctc gaa gaa gca ccc gcc gac gac gtt ccg ggg gga cca 21318
   His Val Ile Leu Glu Glu Ala Pro Ala Asp Asp Val Pro Gly Gly Pro
   7075 7080 7085
   ccc gcc gac gag gat gcc ggt agt ggc gag gag gct gct gcc ggc agt 21366
   Pro Ala Asp Glu Asp Ala Gly Ser Gly Glu Glu Ala Ala Ala Gly Ser
   7090 7095 7100
   cct ggg gtg tgg ccg tgg ctg gtg tcg gcc aag tcg cag ccg gcc ctg 21414
   Pro Gly Val Trp Pro Trp Leu Val Ser Ala Lys Ser Gln Pro Ala Leu
   7105 7110 7115 7120
   cgc gcc cag gcc cag gcc ctg cac gcc cac ctc acc gac cac ccc ggc 21462
   Arg Ala Gln Ala Gln Ala Leu His Ala His Leu Thr Asp His Pro Gly
   7125 7130 7135
   ctc gac ctc gcc gac gtc gga tac acc ctc gcc cac gcc cgc gcc gtg 21510
   Leu Asp Leu Ala Asp Val Gly Tyr Thr Leu Ala His Ala Arg Ala Val
   7140 7145 7150
   ttc gac cac cgc gcc acc ctc atc gcc gcc gac cgc gac acc ttc ctg 21558
   Phe Asp His Arg Ala Thr Leu Ile Ala Ala Asp Arg Asp Thr Phe Leu
   7155 7160 7165
   caa gca ctc cag gca ctc gcc gca ggc gaa ccc cac ccc gcc gtc atc    21606
   Gln Ala Leu Gln Ala Leu Ala Ala Gly Glu Pro His Pro Ala Val Ile
       7170                7175                7180
   cac agc agc gcc cca ggc ggg acc ggg acc ggg gag gcc gca gga aag    21654
   His Ser Ser Ala Pro Gly Gly Thr Gly Thr Gly Glu Ala Ala Gly Lys
   7185                7190                7195                7200
   acc gca ttc atc tgc tcc gga cag ggc acc caa cgc ccc ggc atg gcc    21702
   Thr Ala Phe Ile Cys Ser Gly Gln Gly Thr Gln Arg Pro Gly Met Ala
                   7205                7210                7215
   cac ggc ctc tac cac acc cac ccc gtc ttc gcc gcc gca ctc aac gac    21750
   His Gly Leu Tyr His Thr His Pro Val Phe Ala Ala Ala Leu Asn Asp
               7220                7225                7230
   atc tgc acc cac ctc gac ccc cac ctc gac cac ccc ctc ctc ccc ctc    21798
   Ile Cys Thr His Leu Asp Pro His Leu Asp His Pro Leu Leu Pro Leu
           7235                7240                7245
   ctc acc caa aac gac aac gac aac gac aac gag gac gcg gcc gca ctg    21846
   Leu Thr Gln Asn Asp Asn Asp Asn Asp Asn Glu Asp Ala Ala Ala Leu
       7250                7255                7260
   ctc cag cag acc ccg tac gcc cag ccc gcc ctc ttc gcc ttc cag gtc    21894
   Leu Gln Gln Thr Pro Tyr Ala Gln Pro Ala Leu Phe Ala Phe Gln Val
   7265                7270                7275                7280
   gcc ctc cac cgc ctc ctc acc gac ggc tac cac atc acc ccc cac tac    21942
   Ala Leu His Arg Leu Leu Thr Asp Gly Tyr His Ile Thr Pro His Tyr
                   7285                7290                7295
   tac gcc gga cac tcc ctc ggc gaa atc acc gcc gcc cac ctc gcc ggc    21990
   Tyr Ala Gly His Ser Leu Gly Glu Ile Thr Ala Ala His Leu Ala Gly
               7300                7305                7310
   atc ctc acc ctc acc gac gcc acc acc ctc atc acc caa cgc gcc acc    22038
   Ile Leu Thr Leu Thr Asp Ala Thr Thr Leu Ile Thr Gln Arg Ala Thr
           7315                7320                7325
   ctc atg caa acc atg ccc ccc ggc acc atg acc acc ctc cac acc acc    22086
   Leu Met Gln Thr Met Pro Pro Gly Thr Met Thr Thr Leu His Thr Thr
       7330                7335                7340
   cca cac cac atc acc cac cac ctc acc gcc cac gaa aac gac ctc gcc    22134
   Pro His His Ile Thr His His Leu Thr Ala His Glu Asn Asp Leu Ala
   7345                7350                7355                7360
   atc gcc gcc atc aac acc ccc acc tcc ctc gtc atc agc ggc acc ccc    22182
   Ile Ala Ala Ile Asn Thr Pro Thr Ser Leu Val Ile Ser Gly Thr Pro
                   7365                7370                7375
   cac acc gtc caa cac atc acc acc ctc tgc caa caa caa ggc atc aaa    22230
   His Thr Val Gln His Ile Thr Thr Leu Cys Gln Gln Gln Gly Ile Lys
               7380                7385                7390
   acc aaa acc ctc ccc acc aac cac gcc ttc cac tcc ccc cac acc aac    22278
   Thr Lys Thr Leu Pro Thr Asn His Ala Phe His Ser Pro His Thr Asn
           7395                7400                7405
   ccc atc ctc aac caa ctc cac cag cac acc caa acc ctc acc tac cac    22326
   Pro Ile Leu Asn Gln Leu His Gln His Thr Gln Thr Leu Thr Tyr His
       7410                7415                7420
   cca ccc cac acc ccc ctc atc acc gcc aac acc cca ccc gac caa ctc    22374
   Pro Pro His Thr Pro Leu Ile Thr Ala Asn Thr Pro Pro Asp Gln Leu
   7425                7430                7435                7440
   ctc acc ccc cac tac tgg acc caa caa gcc cgc aac acc gtc gac tac    22422
   Leu Thr Pro His Tyr Trp Thr Gln Gln Ala Arg Asn Thr Val Asp Tyr
                   7445                7450                7455
   gcc acc acc acc caa acc ctc cac caa cac ggc gtc acc acc tac atc    22470
   Ala Thr Thr Thr Gln Thr Leu His Gln His Gly Val Thr Thr Tyr Ile
               7460                7465                7470
   gaa ctc gga ccc gac aac acc ctc acc acc ctc acc cac cac aac ctc    22518
   Glu Leu Gly Pro Asp Asn Thr Leu Thr Thr Leu Thr His His Asn Leu
           7475                7480                7485
   ccc aac acc ccc acc acc acc ctc acc ctc acc cac ccc cac cac cac    22566
   Pro Asn Thr Pro Thr Thr Thr Leu Thr Leu Thr His Pro His His His
       7490                7495                7500
   ccc caa acc cac ctc ctc acc aac ctc gcc aaa acc acc acc acc tgg    22614
   Pro Gln Thr His Leu Leu Thr Asn Leu Ala Lys Thr Thr Thr Thr Trp
   7505                7510                7515                7520
   cac ccc cac cac tac acc cac cac cac aac caa ccc cac acc cac acc    22662
   His Pro His His Tyr Thr His His His Asn Gln Pro His Thr His Thr
                   7525                7530                7535
   cac ctc gac ctc ccc acc tac ccc ttc caa cac cac cac tac tgg ctc    22710
   His Leu Asp Leu Pro Thr Tyr Pro Phe Gln His His His Tyr Trp Leu
               7540                7545                7550
   gaa cta ccc agc gcc caa acc agc ccc ggt caa agg cgt tct cgc cgc    22758
   Glu Leu Pro Ser Ala Gln Thr Ser Pro Gly Gln Arg Arg Ser Arg Arg
           7555                7560                7565
   tcg gct cca gac acc gcc gag tcg gag ttc tgg gac gcg gtg aac gag    22806
   Ser Ala Pro Asp Thr Ala Glu Ser Glu Phe Trp Asp Ala Val Asn Glu
       7570                7575                7580
   gaa gac ctc cag agc ctc gcc gaa acc ctc gac atc gac gcc tct gct    22854
   Glu Asp Leu Gln Ser Leu Ala Glu Thr Leu Asp Ile Asp Ala Ser Ala
   7585                7590                7595                7600
   ctg gac acg gtg gtg ccc gca ctc tcc gcc tgg cac cgc cac caa cac    22902
   Leu Asp Thr Val Val Pro Ala Leu Ser Ala Trp His Arg His Gln His
                   7605                7610                7615
   gac caa gcc cgc atc aac acc tgg acc tac cag gaa acc tgg aaa ccc    22950
   Asp Gln Ala Arg Ile Asn Thr Trp Thr Tyr Gln Glu Thr Trp Lys Pro
               7620                7625                7630
   ctc acc ctc ccc acc acc cac caa ccc cac caa acc tgg ctc atc gcc    22998
   Leu Thr Leu Pro Thr Thr His Gln Pro His Gln Thr Trp Leu Ile Ala
           7635                7640                7645
   atc ccc gaa acc cag acc cac cac ccc cac atc acc aac atc ctc acc    23046
   Ile Pro Glu Thr Gln Thr His His Pro His Ile Thr Asn Ile Leu Thr
       7650                7655                7660
   aac ctc cac cac cac ggc atc acc ccc atc ccc ctc act gtc aac cac    23094
   Asn Leu His His His Gly Ile Thr Pro Ile Pro Leu Thr Val Asn His
   7665                7670                7675                7680
   acc cac acc aac ccc caa cac ctc cac cac acc ctc cac cac acc cga    23142
   Thr His Thr Asn Pro Gln His Leu His His Thr Leu His His Thr Arg
                   7685                7690                7695
   caa caa gcc caa aac cac acc acc gga ccc atc acc ggc ctg ctc tcc    23190
   Gln Gln Ala Gln Asn His Thr Thr Gly Pro Ile Thr Gly Leu Leu Ser
               7700                7705                7710
   ctc ctc gcc ctc gac gaa aca ccc cac ccc cac cac ccc cac aca ccc    23238
   Leu Leu Ala Leu Asp Glu Thr Pro His Pro His His Pro His Thr Pro
           7715                7720                7725
   acc ggc acc ctc ctc aac ctc acc ctc ccc caa acc cac acc caa acc    23286
   Thr Gly Thr Leu Leu Asn Leu Thr Leu Pro Gln Thr His Thr Gln Thr
       7730                7735                7740
   cac cca cca acc ccc ctc tgg tac gcc acc acc aac gcc acc acc acc    23334
   His Pro Pro Thr Pro Leu Trp Tyr Ala Thr Thr Asn Ala Thr Thr Thr
   7745                7750                7755                7760
   cac ccc aac gac ccc ctc aca cac ccc acc caa gcc caa acc tgg gga    23382
   His Pro Asn Asp Pro Leu Thr His Pro Thr Gln Ala Gln Thr Trp Gly
                   7765                7770                7775
   ctc gcc cgc acc acc ctc ctc gaa cac ccc acc cac acc gcc gga atc    23430
   Leu Ala Arg Thr Thr Leu Leu Glu His Pro Thr His Thr Ala Gly Ile
               7780                7785                7790
   atc gac ctc ccc acc acc ccc acc ccc cac acc ctc cac cac ctc acc    23478
   Ile Asp Leu Pro Thr Thr Pro Thr Pro His Thr Leu His His Leu Thr
           7795                7800                7805
   caa acc ctc acc caa ccc cac cac caa acc caa ctc gcc atc cgc acc    23526
   Gln Thr Leu Thr Gln Pro His His Gln Thr Gln Leu Ala Ile Arg Thr
       7810                7815                7820
   acc ggc acc cac acc cgc cgc ctc acc ccc acc acc ctc acc ccc aca    23574
   Thr Gly Thr His Thr Arg Arg Leu Thr Pro Thr Thr Leu Thr Pro Thr
   7825                7830                7835                7840
   cac caa cca ccc acc ccc acc ccc cac gga acc acc ctc atc acc ggc    23622
   His Gln Pro Pro Thr Pro Thr Pro His Gly Thr Thr Leu Ile Thr Gly
   7845 7850 7855
   gga acc ggc gcc ctc gcc acc cac ctc acc cac cac ctc acc acc cac 23670
   Gly Thr Gly Ala Leu Ala Thr His Leu Thr His His Leu Thr Thr His
   7860 7865 7870
   caa ccc acc caa cac ctc ctc ctc acc agc cga acc ggc ccc cac acc 23718
   Gln Pro Thr Gln His Leu Leu Leu Thr Ser Arg Thr Gly Pro His Thr
   7875 7880 7885
   ccc cac gca caa cac ctc acc acc caa ctc caa caa aaa ggc atc cac 23766
   Pro His Ala Gln His Leu Thr Thr Gln Leu Gln Gln Lys Gly Ile His
   7890 7895 7900
   ctc acc atc acc acc tgc gac acc agc aac cca gac caa ctc caa caa 23814
   Leu Thr Ile Thr Thr Cys Asp Thr Ser Asn Pro Asp Gln Leu Gln Gln
   7905 7910 7915 7920
   ctc ctc aac acc atc ccc cca caa cac ccc ctc acc acc gtc atc cac 23862
   Leu Leu Asn Thr Ile Pro Pro Gln His Pro Leu Thr Thr Val Ile His
   7925 7930 7935
   acc gca ggc gtc aat ctc ttc gcc ccc gtg tcg gaa acc gat gcc gaa 23910
   Thr Ala Gly Val Asn Leu Phe Ala Pro Val Ser Glu Thr Asp Ala Glu
   7940 7945 7950
   tcc ttc tct tcc gtt acg gca gcg aag gca acg ggc gcg gcg att ctg 23958
   Ser Phe Ser Ser Val Thr Ala Ala Lys Ala Thr Gly Ala Ala Ile Leu
   7955 7960 7965
   cat gag ttg ctg ctg gac cat gaa acg ctt gaa cac ttc att ctc ttc 24006
   His Glu Leu Leu Leu Asp His Glu Thr Leu Glu His Phe Ile Leu Phe
   7970 7975 7980
   tcg tcg ggc gcc ggc gct tgg ggc agc ggg aat cag tgc gca tac tcg 24054
   Ser Ser Gly Ala Gly Ala Trp Gly Ser Gly Asn Gln Cys Ala Tyr Ser
   7985 7990 7995 8000
   gcg gcc aac gca tac ctg gac gcg ctc gcg acg cat cgt cag aca cat 24102
   Ala Ala Asn Ala Tyr Leu Asp Ala Leu Ala Thr His Arg Gln Thr His
   8005 8010 8015
   gga ctt ccc ggg gca tcg atc gcc tgg ggc ccc tgg gcc gga aag ggc 24150
   Gly Leu Pro Gly Ala Ser Ile Ala Trp Gly Pro Trp Ala Gly Lys Gly
   8020 8025 8030
   atg tcg gcc ggt gat gcg gct cat ggt tac ctg gaa aag cgc ggc att 24198
   Met Ser Ala Gly Asp Ala Ala His Gly Tyr Leu Glu Lys Arg Gly Ile
   8035 8040 8045
   ctg ccg atg gag cca cgc atg gcg ctc gcg gca ttc cat cgt gcg cgg 24246
   Leu Pro Met Glu Pro Arg Met Ala Leu Ala Ala Phe His Arg Ala Arg
   8050 8055 8060
   gcg cag cgg ccg aat tcc aac ctg atc atc gcg gac atc gac tgg gag 24294
   Ala Gln Arg Pro Asn Ser Asn Leu Ile Ile Ala Asp Ile Asp Trp Glu
   8065 8070 8075 8080
   cgc ttc gtc ccc gcc ttc acc gct cga cgc cac agc ccg ctc atc gag     24342
   Arg Phe Val Pro Ala Phe Thr Ala Arg Arg His Ser Pro Leu Ile Glu
                   8085                8090                8095
   gac att ccg gag gtt cgg caa gcg gct cag gag ctg gaa gca gct gcg     24390
   Asp Ile Pro Glu Val Arg Gln Ala Ala Gln Glu Leu Glu Ala Ala Ala
               8100                8105                8110
   tcg acg gca aag acg acc aca gct cag ccg att gcg acg tct ctc cgt     24438
   Ser Thr Ala Lys Thr Thr Thr Ala Gln Pro Ile Ala Thr Ser Leu Arg
           8115                8120                8125
   gag cga ttg gcc cga ctg acg tcc tca aag cag aac cag gtg ctg ctc     24486
   Glu Arg Leu Ala Arg Leu Thr Ser Ser Lys Gln Asn Gln Val Leu Leu
       8130                8135                8140
   ggc ctg att cgg aca ggc atc tgc acc gtt ctc ggc ctt cgt aat ccg     24534
   Gly Leu Ile Arg Thr Gly Ile Cys Thr Val Leu Gly Leu Arg Asn Pro
   8145                8150                8155                8160
   gaa ggc atc gag gac caa cga gcc ttc cgc gac ctc ggc ttc gac tcg     24582
   Glu Gly Ile Glu Asp Gln Arg Ala Phe Arg Asp Leu Gly Phe Asp Ser
                   8165                8170                8175
   ctg acg tcg gct cag ttc agc aag gaa ctc gcc aag gaa acc gga ctg     24630
   Leu Thr Ser Ala Gln Phe Ser Lys Glu Leu Ala Lys Glu Thr Gly Leu
               8180                8185                8190
   cca ctc ccc ccg tcc ctg gtc ttc gac tat ccc acc ccg cag gaa tgt     24678
   Pro Leu Pro Pro Ser Leu Val Phe Asp Tyr Pro Thr Pro Gln Glu Cys
           8195                8200                8205
   gct gcc cat ctg cgc aca caa ctc gtc gac cta gac gac gaa gag gac     24726
   Ala Ala His Leu Arg Thr Gln Leu Val Asp Leu Asp Asp Glu Glu Asp
       8210                8215                8220
   gcg gca ctg tcg aat gct ctc ccg caa gtg gcc cat cgg cgt acc gtc     24774
   Ala Ala Leu Ser Asn Ala Leu Pro Gln Val Ala His Arg Arg Thr Val
   8225                8230                8235                8240
   gag gac gaa ccg atc gcc atc atc ggt atg gca tgt cgc ttc ccc ggc     24822
   Glu Asp Glu Pro Ile Ala Ile Ile Gly Met Ala Cys Arg Phe Pro Gly
                   8245                8250                8255
   ggc gta cgt tct gcc gac gac ctg tgg gaa ttg ctc gct tcg ggt aag     24870
   Gly Val Arg Ser Ala Asp Asp Leu Trp Glu Leu Leu Ala Ser Gly Lys
               8260                8265                8270
   gac gct atc ggc gtc ttc ccg acc gac cgc ggc tgg gac ctg gac acg     24918
   Asp Ala Ile Gly Val Phe Pro Thr Asp Arg Gly Trp Asp Leu Asp Thr
           8275                8280                8285
   ctc tac gac ccc gac ccc gac cac ccc ggc acc tgc tac acc cga aac     24966
   Leu Tyr Asp Pro Asp Pro Asp His Pro Gly Thr Cys Tyr Thr Arg Asn
       8290                8295                8300
   ggc gga ttc ctc tac ggc gca ggc cac ttc gac gcc gaa ttc ttc ggc     25014
   Gly Gly Phe Leu Tyr Gly Ala Gly His Phe Asp Ala Glu Phe Phe Gly
   8305                8310                8315                8320
   atc agc ccc cgc gaa gcc ctc gcc atg gac ccc cag caa cga ctc ctc     25062
   Ile Ser Pro Arg Glu Ala Leu Ala Met Asp Pro Gln Gln Arg Leu Leu
                   8325                8330                8335
   ctc gaa acc gcc tgg gaa acc atc gaa cac gcc ggc atc aac ccc cac     25110
   Leu Glu Thr Ala Trp Glu Thr Ile Glu His Ala Gly Ile Asn Pro His
               8340                8345                8350
   acc ctc cac ggc acc ccc acc gga gtc ttc gcc gga atc aac gct caa     25158
   Thr Leu His Gly Thr Pro Thr Gly Val Phe Ala Gly Ile Asn Ala Gln
           8355                8360                8365
   gac cac gcc gcg cat atc cgc caa agc cgt gat gtg gag acc atc gag     25206
   Asp His Ala Ala His Ile Arg Gln Ser Arg Asp Val Glu Thr Ile Glu
       8370                8375                8380
   ggc tac gcc ctg acc ggc agt tcg gga agt gtg gcg tcc ggc cgg gtg     25254
   Gly Tyr Ala Leu Thr Gly Ser Ser Gly Ser Val Ala Ser Gly Arg Val
   8385                8390                8395                8400
   gcc tac acg ctc ggg ctc gaa ggc ccc gcg gtg tcg gtg gat acg gcg     25302
   Ala Tyr Thr Leu Gly Leu Glu Gly Pro Ala Val Ser Val Asp Thr Ala
                   8405                8410                8415
   tgt tcg tcg tcg ttg gtg gcg ttg cat tgg gcg gcg cag gcg ttg cgt     25350
   Cys Ser Ser Ser Leu Val Ala Leu His Trp Ala Ala Gln Ala Leu Arg
               8420                8425                8430
   gcg ggt gag tgt tcg atg gcg ctt gcc ggg ggt gtg acg gtg atg tcg     25398
   Ala Gly Glu Cys Ser Met Ala Leu Ala Gly Gly Val Thr Val Met Ser
           8435                8440                8445
   tct ccg ggt acg ttt gtg gag ttc tca cgt cag cgg ggt ctg gcc gcg     25446
   Ser Pro Gly Thr Phe Val Glu Phe Ser Arg Gln Arg Gly Leu Ala Ala
       8450                8455                8460
   gac ggg cgg tgc aag gcc tat tcg gcg gct gct gac ggt acc ggc tgg     25494
   Asp Gly Arg Cys Lys Ala Tyr Ser Ala Ala Ala Asp Gly Thr Gly Trp
   8465                8470                8475                8480
   gcc gag ggt gtg ggg atg ctg ctg gtg gag cgg ctc tcc gac gcc cgt     25542
   Ala Glu Gly Val Gly Met Leu Leu Val Glu Arg Leu Ser Asp Ala Arg
                   8485                8490                8495
   cgc aac ggt cac cgt gtc ctg gcc gtg gtg cgt ggc agt gcg gtc aac     25590
   Arg Asn Gly His Arg Val Leu Ala Val Val Arg Gly Ser Ala Val Asn
               8500                8505                8510
   cag gac ggt gcg agc aac ggt ctg acc gcg ccc aac ggg ccc tcc cag     25638
   Gln Asp Gly Ala Ser Asn Gly Leu Thr Ala Pro Asn Gly Pro Ser Gln
           8515                8520                8525
   cag cgt gtc atc cgt cag gcc ctg gcc aat gcg gga ctg acc ccg gcc     25686
   Gln Arg Val Ile Arg Gln Ala Leu Ala Asn Ala Gly Leu Thr Pro Ala
       8530                8535                8540
   gat gtc gac gca gtg gag ggc cac ggc acc ggg acc act ctg ggg gac    25734
   Asp Val Asp Ala Val Glu Gly His Gly Thr Gly Thr Thr Leu Gly Asp
   8545                8550                8555                8560
   ccg atc gag gcc cag gca ctc ctg gcc gcc tac gga caa cac cgc ccc    25782
   Pro Ile Glu Ala Gln Ala Leu Leu Ala Ala Tyr Gly Gln His Arg Pro
                   8565                8570                8575
   cac cac cgc ccc ttg tgg ctg gga tcc ctc aaa tcc aac atc ggg cac    25830
   His His Arg Pro Leu Trp Leu Gly Ser Leu Lys Ser Asn Ile Gly His
               8580                8585                8590
   gca cag gcc gcc gcg ggc gtg ggc gga gtc atc aag atg gtg atg gcc    25878
   Ala Gln Ala Ala Ala Gly Val Gly Gly Val Ile Lys Met Val Met Ala
           8595                8600                8605
   ctg cgc aac ggg ctg ctg cca cag acc ctc cac gtg gac gag ccc acc    25926
   Leu Arg Asn Gly Leu Leu Pro Gln Thr Leu His Val Asp Glu Pro Thr
       8610                8615                8620
   ccc cag gtc gac tgg tcc aca ggc gca gta caa ctc ctg aca caa ccg    25974
   Pro Gln Val Asp Trp Ser Thr Gly Ala Val Gln Leu Leu Thr Gln Pro
   8625                8630                8635                8640
   gtg ccc tgg ccc gcc gac ccg gcc ggc cgg cca cgc cac gcc ggc gtg    26022
   Val Pro Trp Pro Ala Asp Pro Ala Gly Arg Pro Arg His Ala Gly Val
                   8645                8650                8655
   tca tca ttc ggc gtc agc ggc acc aac gcc cac atc atc ctc gaa gaa    26070
   Ser Ser Phe Gly Val Ser Gly Thr Asn Ala His Ile Ile Leu Glu Glu
               8660                8665                8670
   gca ccc act ccc cag gac agc gat acc gac gac gaa ccg cct gcc aac    26118
   Ala Pro Thr Pro Gln Asp Ser Asp Thr Asp Asp Glu Pro Pro Ala Asn
           8675                8680                8685
   gca cca gcc ctg ccc cat ccc ctc cct ctt ccc gtg ccg gtg tcg gcg    26166
   Ala Pro Ala Leu Pro His Pro Leu Pro Leu Pro Val Pro Val Ser Ala
       8690                8695                8700
   agg tct gag gcc ggg ttg cgg gcg cag gca cag gcg ttg cgc cag tac    26214
   Arg Ser Glu Ala Gly Leu Arg Ala Gln Ala Gln Ala Leu Arg Gln Tyr
   8705                8710                8715                8720
   gtg gca gcc cgc ccg gac atg tca cct gcc gac att ggt gcg ggt ctg    26262
   Val Ala Ala Arg Pro Asp Met Ser Pro Ala Asp Ile Gly Ala Gly Leu
                   8725                8730                8735
   gcc cgc ggc cgg gcc gta ctg gaa cac cgc gcc gtc atc ctg gcc gcg    26310
   Ala Arg Gly Arg Ala Val Leu Glu His Arg Ala Val Ile Leu Ala Ala
               8740                8745                8750
   gac cgc gag gaa ctg gcg cag gca ctg aca gcc ctg gca gcc ggc gaa    26358
   Asp Arg Glu Glu Leu Ala Gln Ala Leu Thr Ala Leu Ala Ala Gly Glu
   8755 8760 8765
   ccc cac ccc cac atc acc aca ggc cac acc cgg ggc ggt gac cgc ggc 26406
   Pro His Pro His Ile Thr Thr Gly His Thr Arg Gly Gly Asp Arg Gly
   8770 8775 8780
   ggc gtc gtc ttc gtc ttc ccc gga cag ggc ggc cag tgg gcc ggg atg 26454
   Gly Val Val Phe Val Phe Pro Gly Gln Gly Gly Gln Trp Ala Gly Met
   8785 8790 8795 8800
   ggc ctg acc ctg ctc acc tcc tca ccc gtg ttc gcc gaa cac atc gac 26502
   Gly Leu Thr Leu Leu Thr Ser Ser Pro Val Phe Ala Glu His Ile Asp
   8805 8810 8815
   gca tgc gag aaa gcc ctc acc ccc tgg gtg ccc tgg tcc ctg acc gac 26550
   Ala Cys Glu Lys Ala Leu Thr Pro Trp Val Pro Trp Ser Leu Thr Asp
   8820 8825 8830
   atc ctg cac cgc gac ccc gac gac ccc gca tgg caa caa gcc gac gtg 26598
   Ile Leu His Arg Asp Pro Asp Asp Pro Ala Trp Gln Gln Ala Asp Val
   8835 8840 8845
   gtc cag ccc gtg ctc ttc agc atc atg gtc tcc ctc gcc gcc ctg tgg 26646
   Val Gln Pro Val Leu Phe Ser Ile Met Val Ser Leu Ala Ala Leu Trp
   8850 8855 8860
   cgc tcc tac ggc atc gaa ccc gac gcg gtc ctc ggc cac tcc cag gga 26694
   Arg Ser Tyr Gly Ile Glu Pro Asp Ala Val Leu Gly His Ser Gln Gly
   8865 8870 8875 8880
   gaa atc gcc gcc gcc cac atc tgc ggc gca ctc agc ctg aaa gac gcc 26742
   Glu Ile Ala Ala Ala His Ile Cys Gly Ala Leu Ser Leu Lys Asp Ala
   8885 8890 8895
   gcc aaa acc gtt gca ctg cgc agc cgc gca ctg gcc gcc gta cga ggc 26790
   Ala Lys Thr Val Ala Leu Arg Ser Arg Ala Leu Ala Ala Val Arg Gly
   8900 8905 8910
   cgg ggc gcc atg gcc tca ctg ccc ctg ccc gcc cag gac gtg cag cag 26838
   Arg Gly Ala Met Ala Ser Leu Pro Leu Pro Ala Gln Asp Val Gln Gln
   8915 8920 8925
   ctc att tcc gaa cgg tgg gaa ggg cag ttg tgg gtg gca gcc ctc aac 26886
   Leu Ile Ser Glu Arg Trp Glu Gly Gln Leu Trp Val Ala Ala Leu Asn
   8930 8935 8940
   ggc ccc cac tcc acc acc gtc tcc ggc gac acc aag gcg gtg gat gag 26934
   Gly Pro His Ser Thr Thr Val Ser Gly Asp Thr Lys Ala Val Asp Glu
   8945 8950 8955 8960
   gtg ctg gcg cac tgc acc gac acc ggc cta cgg gcc aaa cgc atc ccc 26982
   Val Leu Ala His Cys Thr Asp Thr Gly Leu Arg Ala Lys Arg Ile Pro
   8965 8970 8975
   gtc gac tac gcc tcc cac tgc ccc cac gtc caa ccc ctc cac gac gaa 27030
   Val Asp Tyr Ala Ser His Cys Pro His Val Gln Pro Leu His Asp Glu
   8980 8985 8990
   ctc ctg cac ctg ctg gga gac atc acc ccc cag ccg tcc acc gtg ccg    27078
   Leu Leu His Leu Leu Gly Asp Ile Thr Pro Gln Pro Ser Thr Val Pro
           8995                9000                9005
   ttc ttc tcc acc gtg gaa ggc acc tgg ctg gac acc aca acc ctg gac    27126
   Phe Phe Ser Thr Val Glu Gly Thr Trp Leu Asp Thr Thr Thr Leu Asp
       9010                9015                9020
   gcc gcc tac tgg tac cgc aac ctc cac cag ccc gtc cgc ttc agc cac    27174
   Ala Ala Tyr Trp Tyr Arg Asn Leu His Gln Pro Val Arg Phe Ser His
   9025                9030                9035                9040
   gcc atc cag acc ctg acc gac gac gga cac cgc gcc ttc atc gaa atc    27222
   Ala Ile Gln Thr Leu Thr Asp Asp Gly His Arg Ala Phe Ile Glu Ile
                   9045                9050                9055
   agc ccc cac ccc acc ctc gtc ccc gcc atc gaa gac acc acc gaa aac    27270
   Ser Pro His Pro Thr Leu Val Pro Ala Ile Glu Asp Thr Thr Glu Asn
               9060                9065                9070
   acc acc gaa aac atc acc gcg acc ggc agc ctc cgc cgc ggc gac aac    27318
   Thr Thr Glu Asn Ile Thr Ala Thr Gly Ser Leu Arg Arg Gly Asp Asn
           9075                9080                9085
   gac acc cac cgc ttc ctc acc gcc ctc gcc cac acc cac acc acc ggc    27366
   Asp Thr His Arg Phe Leu Thr Ala Leu Ala His Thr His Thr Thr Gly
       9090                9095                9100
   atc ggc aca ccc acc acc tgg cac cac cac tac acc caa acc cac ccc    27414
   Ile Gly Thr Pro Thr Thr Trp His His His Tyr Thr Gln Thr His Pro
   9105                9110                9115                9120
   cac ccc aac ccc cac acc cac ctc gac ctg ccc acc tac ccc ttc caa    27462
   His Pro Asn Pro His Thr His Leu Asp Leu Pro Thr Tyr Pro Phe Gln
                   9125                9130                9135
   cac cag cac tac tgg ctc caa cca ccc acc aca aca acc gac ctc acc    27510
   His Gln His Tyr Trp Leu Gln Pro Pro Thr Thr Thr Thr Asp Leu Thr
               9140                9145                9150
   acc acc ggc ctc acc ccc acc cac cac ccc ctc ctc acc gcc aca ctc    27558
   Thr Thr Gly Leu Thr Pro Thr His His Pro Leu Leu Thr Ala Thr Leu
           9155                9160                9165
   acc ctc gcc gac aac aac aca caa cta ctc acc ggc cgc ctc tcc cta    27606
   Thr Leu Ala Asp Asn Asn Thr Gln Leu Leu Thr Gly Arg Leu Ser Leu
       9170                9175                9180
   cgc acc cac ccc tgg ctc acc gac cac acc gtc gcc ggc atg gtc ctc    27654
   Arg Thr His Pro Trp Leu Thr Asp His Thr Val Ala Gly Met Val Leu
   9185                9190                9195                9200
   ctg ccg ggc acc gcg ctc ctc gaa ctc gcc ctc caa gcc ggc gaa cgg    27702
   Leu Pro Gly Thr Ala Leu Leu Glu Leu Ala Leu Gln Ala Gly Glu Arg
                   9205                9210                9215
   gtg gac tgc cct cgg gtg gag gaa ctg acc ctg cac gca ccg ttg gtg 27750
   Val Asp Cys Pro Arg Val Glu Glu Leu Thr Leu His Ala Pro Leu Val
   9220 9225 9230
   atc ccg cac acc gag gac gtg acg ttg cag gtc acc gtt cgg gca gcc 27798
   Ile Pro His Thr Glu Asp Val Thr Leu Gln Val Thr Val Arg Ala Ala
   9235 9240 9245
   gat gag agt ggc cat cgc gcc ctc gcg atc cac tcg tac tcc ggc acc 27846
   Asp Glu Ser Gly His Arg Ala Leu Ala Ile His Ser Tyr Ser Gly Thr
   9250 9255 9260
   gcg tcg tcg gcg gac cgg gag tgg acc cgt cac gcc acg ggc ctc ctc 27894
   Ala Ser Ser Ala Asp Arg Glu Trp Thr Arg His Ala Thr Gly Leu Leu
   9265 9270 9275 9280
   aca cac cac gcc gac acc gat cac cgt gcc gac acg cac acg gac gcg 27942
   Thr His His Ala Asp Thr Asp His Arg Ala Asp Thr His Thr Asp Ala
   9285 9290 9295
   tgc ctt ggc ggg agc tgg ccc ccg ccc ggc gcg cag ccc atc gaa ctg 27990
   Cys Leu Gly Gly Ser Trp Pro Pro Pro Gly Ala Gln Pro Ile Glu Leu
   9300 9305 9310
   ggc gac gtc tac ggt cgt atg gcg gcg gac tcg gac atc gcc tac ggg 28038
   Gly Asp Val Tyr Gly Arg Met Ala Ala Asp Ser Asp Ile Ala Tyr Gly
   9315 9320 9325
   ccg gtc ttc cag ggg ctg cac gcc gcc tgg agg ttc ggc gac gat gtc 28086
   Pro Val Phe Gln Gly Leu His Ala Ala Trp Arg Phe Gly Asp Asp Val
   9330 9335 9340
   ctg gcc gag gtg cgt ctg ccg gaa gag gct ctg cgc gat gct ccg gcg 28134
   Leu Ala Glu Val Arg Leu Pro Glu Glu Ala Leu Arg Asp Ala Pro Ala
   9345 9350 9355 9360
   gcg gcc ttc ggt gtt cac ccg gcc ttg ctc gac gcg gcc ctg cac gcc 28182
   Ala Ala Phe Gly Val His Pro Ala Leu Leu Asp Ala Ala Leu His Ala
   9365 9370 9375
   acg gcg ctc acc ccc cag aac ggg gac ggc tcg acg gag aac gtc gcc 28230
   Thr Ala Leu Thr Pro Gln Asn Gly Asp Gly Ser Thr Glu Asn Val Ala
   9380 9385 9390
   cag gag agc atg cct gac cgc gca gcc cac cag gcg cga ctg ccg ttc 28278
   Gln Glu Ser Met Pro Asp Arg Ala Ala His Gln Ala Arg Leu Pro Phe
   9395 9400 9405
   agc tgg agc ggc gtg tcc ctg cac acg gcg ggc agt tcc gtg ttg cgc 28326
   Ser Trp Ser Gly Val Ser Leu His Thr Ala Gly Ser Ser Val Leu Arg
   9410 9415 9420
   gta cgg ctg tcg cgc agt ccg cag cac ggt aat gcc gtg gcc ctc acc 28374
   Val Arg Leu Ser Arg Ser Pro Gln His Gly Asn Ala Val Ala Leu Thr
   9425 9430 9435 9440
   gcg gcc gac gag gac ggt cgg ccg gtg gtg acg atc gag tcg ctc gcg 28422
   Ala Ala Asp Glu Asp Gly Arg Pro Val Val Thr Ile Glu Ser Leu Ala
   9445 9450 9455
   ctg cgg ccg gtg tcc acc gag gag ctg cgc gcg gcc gcg gat cgt acg 28470
   Leu Arg Pro Val Ser Thr Glu Glu Leu Arg Ala Ala Ala Asp Arg Thr
   9460 9465 9470
   ccc gag cac gag tcg ctc ttc cga ctg gac tgg gtt tcc gta cca gtg 28518
   Pro Glu His Glu Ser Leu Phe Arg Leu Asp Trp Val Ser Val Pro Val
   9475 9480 9485
   ccc gcc aac gcc cct tcg ccc acc gcg gac cgg ccc tgg gcg gtc atc 28566
   Pro Ala Asn Ala Pro Ser Pro Thr Ala Asp Arg Pro Trp Ala Val Ile
   9490 9495 9500
   ggc gcg ggc ctt ccc cac ctg ccc ggc ctg acg gag cac gag cac gtg 28614
   Gly Ala Gly Leu Pro His Leu Pro Gly Leu Thr Glu His Glu His Val
   9505 9510 9515 9520
   acc gcg tat gac gag ccg gcg gac ctg ctt ctg gct ctg gac cgc ggt 28662
   Thr Ala Tyr Asp Glu Pro Ala Asp Leu Leu Leu Ala Leu Asp Arg Gly
   9525 9530 9535
   gct ccg ccg ccc ggt gtg ctg gtc gta ggt ggt gtc gcc cac acc gaa 28710
   Ala Pro Pro Pro Gly Val Leu Val Val Gly Gly Val Ala His Thr Glu
   9540 9545 9550
   gcc cgg gag tat tcc gcc gaa gcc ccc ggg gag cgc ggg acc gag gcc 28758
   Ala Arg Glu Tyr Ser Ala Glu Ala Pro Gly Glu Arg Gly Thr Glu Ala
   9555 9560 9565
   tgc gag gcc cgg ccg gac gtc gtg cac gtg ggc gtc gtg cac acg gct 28806
   Cys Glu Ala Arg Pro Asp Val Val His Val Gly Val Val His Thr Ala
   9570 9575 9580
   gcc gtg cac gcg gct gcc gcg cag atg ttg gcc agg ctc cag gcc tgg 28854
   Ala Val His Ala Ala Ala Ala Gln Met Leu Ala Arg Leu Gln Ala Trp
   9585 9590 9595 9600
   ctg ggc gac gag cgc ctc gca gac agc cgg ctg ctc gtc ctg acg tgc 28902
   Leu Gly Asp Glu Arg Leu Ala Asp Ser Arg Leu Leu Val Leu Thr Cys
   9605 9610 9615
   ggc gcg gtc gcc cgc gcc tcc ggc gac gat gcg acg gac ctg ccc ggg 28950
   Gly Ala Val Ala Arg Ala Ser Gly Asp Asp Ala Thr Asp Leu Pro Gly
   9620 9625 9630
   gcc gcc gtg tgg ggg ctg gtg cgt tcg gcg cag tcc gag cac ccg gac 28998
   Ala Ala Val Trp Gly Leu Val Arg Ser Ala Gln Ser Glu His Pro Asp
   9635 9640 9645
   cgc atc acg ctg ctg gac ttc gag cgg ggc aca gag gcg gag ccc ggt 29046
   Arg Ile Thr Leu Leu Asp Phe Glu Arg Gly Thr Glu Ala Glu Pro Gly
   9650 9655 9660
   cag ctg gcg acg gcg ctg aac tgc ggg gag cgg cag ctt gcc gtc cgc 29094
   Gln Leu Ala Thr Ala Leu Asn Cys Gly Glu Arg Gln Leu Ala Val Arg
   9665                9670                 9675                9680
   ccc gga ggg ctg ttc acg cca cgg ctg gtg cgc gcg cca cgt gtc gcc    29142
   Pro Gly Gly Leu Phe Thr Pro Arg Leu Val Arg Ala Pro Arg Val Ala
                   9685                 9690                9695
   gac gcc gta ccc gcc gta ccc gcc gtg gcc gta ccg tca gcg ggt cac    29190
   Asp Ala Val Pro Ala Val Pro Ala Val Ala Val Pro Ser Ala Gly His
               9700                9705                 9710
   gca gcc gta ccg gca gcg ggt ccc ttc ctt ccg ggc gga acg gtg ctg    29238
   Ala Ala Val Pro Ala Ala Gly Pro Phe Leu Pro Gly Gly Thr Val Leu
           9715                9720                 9725
   atc acc ggc gga acc ggt gtc ctg ggc cgg ctc gtg gcc cgg cat ctg    29286
   Ile Thr Gly Gly Thr Gly Val Leu Gly Arg Leu Val Ala Arg His Leu
       9730                9735                 9740
   gtg gag gcg cac ggc gta cgg cat ctg ttg ctg gcg ggt cgg cgc gga    29334
   Val Glu Ala His Gly Val Arg His Leu Leu Leu Ala Gly Arg Arg Gly
   9745                9750                 9755                9760
   ccg gac gcc gag ggt gcg ccg gag ttg cgg gcg gag ctc ggt ggg ctc    29382
   Pro Asp Ala Glu Gly Ala Pro Glu Leu Arg Ala Glu Leu Gly Gly Leu
                   9765                 9770                9775
   ggc gcg acg gtg gag gtc gtc gcc tgc gac gcg gcg gac cgg cag cag    29430
   Gly Ala Thr Val Glu Val Val Ala Cys Asp Ala Ala Asp Arg Gln Gln
               9780                9785                 9790
   ctg gcc gac ctg ctg aca cgg atc ccc gac gat cgg ccg ctg acc ggt    29478
   Leu Ala Asp Leu Leu Thr Arg Ile Pro Asp Asp Arg Pro Leu Thr Gly
           9795                9800                 9805
   gtc gtg cac agt gcg ggc atc ctg gac gac ggc gtg atc acg tcg ctg    29526
   Val Val His Ser Ala Gly Ile Leu Asp Asp Gly Val Ile Thr Ser Leu
       9810                9815                 9820
   tcg ccg gag cgg ctc ggg gcc gtc ctc cgg gcc aag gcg gac gct gcg    29574
   Ser Pro Glu Arg Leu Gly Ala Val Leu Arg Ala Lys Ala Asp Ala Ala
   9825                9830                 9835                9840
   ctg ctt ctc gac gag ctg acg cgc ggg gca gag ctg tcg gct ttc gtc    29622
   Leu Leu Leu Asp Glu Leu Thr Arg Gly Ala Glu Leu Ser Ala Phe Val
                   9845                 9850                9855
   atg ttc tcc tcc gcg tcg gcg gtg gtc ggc tcg ccc ggg cag ggc aac    29670
   Met Phe Ser Ser Ala Ser Ala Val Val Gly Ser Pro Gly Gln Gly Asn
               9860                9865                 9870
   tac gcc gcc gcc aac gcc gtc ctc gac ttc ctt gct cat cgc cgc cgc    29718
   Tyr Ala Ala Ala Asn Ala Val Leu Asp Phe Leu Ala His Arg Arg Arg
           9875                9880                 9885
   gcc gag ggg ctg ccc gcc gtc tct ctc gcc tgg ggc ctg tgg gaa gag    29766
   Ala Glu Gly Leu Pro Ala Val Ser Leu Ala Trp Gly Leu Trp Glu Glu
       9890                9895                 9900
   ggc aca ggg atg acg ggc cac ctc gac gtc gac gac cat gcg cgg atc 29814
   Gly Thr Gly Met Thr Gly His Leu Asp Val Asp Asp His Ala Arg Ile
   9905 9910 9915 9920
   agc cgc gcg gga atg cgg ccg ctg ccg act gcc gag gct ctg gcg ctg 29862
   Ser Arg Ala Gly Met Arg Pro Leu Pro Thr Ala Glu Ala Leu Ala Leu
   9925 9930 9935
   ttc gac gcg gcc ttg gcc gac ggc gag ccg ttc ctg atg ccg gct cgg 29910
   Phe Asp Ala Ala Leu Ala Asp Gly Glu Pro Phe Leu Met Pro Ala Arg
   9940 9945 9950
   ctc gac ctc acg gcc gta cgg tct ggt gcc gcg tcc gca ccg gtg ccg 29958
   Leu Asp Leu Thr Ala Val Arg Ser Gly Ala Ala Ser Ala Pro Val Pro
   9955 9960 9965
   ccg ctg ctg caa ggt ctg ctt cag ctg cct cgg tcc cgc tcg gcc gcc 30006
   Pro Leu Leu Gln Gly Leu Leu Gln Leu Pro Arg Ser Arg Ser Ala Ala
   9970 9975 9980
   gcg gcc ccc ggc cat ggg gcc ccg gcg gcg gac gag gcg gcg gcc tgg 30054
   Ala Ala Pro Gly His Gly Ala Pro Ala Ala Asp Glu Ala Ala Ala Trp
   9985 9990 9995 10000
   cgt gag cgt ctg gcc cgg cag agt gcc ggt gag cgc agg cag gcg ctg 30102
   Arg Glu Arg Leu Ala Arg Gln Ser Ala Gly Glu Arg Arg Gln Ala Leu
   10005 10010 10015
   ctg cgc ctg gtg cgg tcg cat gtc gcg gcg gtg ctc ggc cat agc ggt 30150
   Leu Arg Leu Val Arg Ser His Val Ala Ala Val Leu Gly His Ser Gly
   10020 10025 10030
   gcc gac gga atc gac gca tcg cgg gcg ttc cgc gag ctg ggg ttc gac 30198
   Ala Asp Gly Ile Asp Ala Ser Arg Ala Phe Arg Glu Leu Gly Phe Asp
   10035 10040 10045
   tcg ctc acg gcg gtc gag ctg cgc aac cgt ctc acg gcc gcg acg ggc 30246
   Ser Leu Thr Ala Val Glu Leu Arg Asn Arg Leu Thr Ala Ala Thr Gly
   10050 10055 10060
   ctg cgg ctg cgg gcc acg ctg gcc ttc gat ttc ccg acc ccg gca gcg 30294
   Leu Arg Leu Arg Ala Thr Leu Ala Phe Asp Phe Pro Thr Pro Ala Ala
   10065 10070 10075 10080
   ctg gcc gag cac ttg ggc gag cgt ctg ctt ccc gac cag gag gcc acg 30342
   Leu Ala Glu His Leu Gly Glu Arg Leu Leu Pro Asp Gln Glu Ala Thr
   10085 10090 10095
   ggc gag caa gcc ggc gat cag ctc tcc ggc ggc agc gag gag gac gta 30390
   Gly Glu Gln Ala Gly Asp Gln Leu Ser Gly Gly Ser Glu Glu Asp Val
   10100 10105 10110
   cgc agc ctc ctg acg tcc att ccg atc ggc agg ctg cgg gac gcg ggg 30438
   Arg Ser Leu Leu Thr Ser Ile Pro Ile Gly Arg Leu Arg Asp Ala Gly
   10115 10120 10125
   ctc ctc ggg ccc ctg ctc acg ctc gcg gac acg ggc cgc ggc gcc tcg      30486
   Leu Leu Gly Pro Leu Leu Thr Leu Ala Asp Thr Gly Arg Gly Ala Ser
     10130                10135                10140
   ggc gcc gcc gca ggt ccg gag gac gcg ccg ccc tcc ggc cag gac aca      30534
   Gly Ala Ala Ala Gly Pro Glu Asp Ala Pro Pro Ser Gly Gln Asp Thr
   10145               10150               10155               10160
   ccg gct ccc gtc tcg atc gac gag atg gac atc gac gac ctg atg gat      30582
   Pro Ala Pro Val Ser Ile Asp Glu Met Asp Ile Asp Asp Leu Met Asp
               10165               10170               10175
   ctg gcg cac ggg cat ggc acc gca ccc gcc cgt gag ccc gcc gac gca      30630
   Leu Ala His Gly His Gly Thr Ala Pro Ala Arg Glu Pro Ala Asp Ala
           10180               10185               10190
   gag gac tcg tcg tca tca cga aac cgg aca cac cac aca cac gaa ggt      30678
   Glu Asp Ser Ser Ser Ser Arg Asn Arg Thr His His Thr His Glu Gly
       10195               10200               10205
   gag aca gcg tga                                                       30690
   Glu Thr Ala
     10210
<210> 2
   <211> 31422
   <212> DNA
   <213> Streptomyces avermitilis
<220>
   <221> CDS
   <222> (1)..(14643)
<220>
   <221> CDS
   <222> (14824) .. (31419)
<400> 2
   atg gct aac gag gaa aag ctc cgc gac tat ctc aag cgc gtt act gcc      48
   Met Ala Asn Glu Glu Lys Leu Arg Asp Tyr Leu Lys Arg Val Thr Ala
   1               5                   10                  15
   gat ctc ctc aat gtg cgg cgt cga ctt cag cag att gaa tcg ggc gag      96
   Asp Leu Leu Asn Val Arg Arg Arg Leu Gln Gln Ile Glu Ser Gly Glu
               20                  25                  30
   cag gag ccg att gca att gtg ggg atg gcg tgc cgt ttt ccg ggg ggt      144
   Gln Glu Pro Ile Ala Ile Val Gly Met Ala Cys Arg Phe Pro Gly Gly
           35                  40                  45
   gtg gag tcg gcg gag gat ttc tgg gag ttg att gcg tcg ggt cgg gat      192
   Val Glu Ser Ala Glu Asp Phe Trp Glu Leu Ile Ala Ser Gly Arg Asp
       50                  55                  60
   gcg gtg ggg gag ttt ccg gtc gac cgg ggt tgg gac gtg gag gct ttc      240
   Ala Val Gly Glu Phe Pro Val Asp Arg Gly Trp Asp Val Glu Ala Phe
   65 70 75 80
   tat gat ccg gag ccg ggg cgg gcg ggt tcg tcg tat acg cgc cgg ggc 288
   Tyr Asp Pro Glu Pro Gly Arg Ala Gly Ser Ser Tyr Thr Arg Arg Gly
   85 90 95
   ggt ttc ctg gag ggt gcg gcg gag ttc gat gcg ggg ttt ttc ggg atc 336
   Gly Phe Leu Glu Gly Ala Ala Glu Phe Asp Ala Gly Phe Phe Gly Ile
   100 105 110
   agt ccg cgt gag gcg ttg gcg atg gat ccg cag cag cgg ttg atg ctg 384
   Ser Pro Arg Glu Ala Leu Ala Met Asp Pro Gln Gln Arg Leu Met Leu
   115 120 125
   gag gtg tcc tgg gag gcg ttg gag cgg gcg ggc atc gac ccc gcc acg 432
   Glu Val Ser Trp Glu Ala Leu Glu Arg Ala Gly Ile Asp Pro Ala Thr
   130 135 140
   ttg cgc ggc agc cgg acg ggc gtc ttc gcc ggc ctc atg tcc cag gac 480
   Leu Arg Gly Ser Arg Thr Gly Val Phe Ala Gly Leu Met Ser Gln Asp
   145 150 155 160
   tac gcg acc cgt ctg ctc tcg gtc ccc gac gac ctg gcc ggc tac ctg 528
   Tyr Ala Thr Arg Leu Leu Ser Val Pro Asp Asp Leu Ala Gly Tyr Leu
   165 170 175
   ggc aac ggc aac gcg gga agc atc ctg tcc gga cgc gtc gcc tac acc 576
   Gly Asn Gly Asn Ala Gly Ser Ile Leu Ser Gly Arg Val Ala Tyr Thr
   180 185 190
   ttc ggc ttc gag ggc ccc gcg gtg acg gtc gac acg gcg tgc tcg tcg 624
   Phe Gly Phe Glu Gly Pro Ala Val Thr Val Asp Thr Ala Cys Ser Ser
   195 200 205
   tcg ctg gtg gca ctg cac ctc gcc tgc cag tca ctg cgc acc ggt gag 672
   Ser Leu Val Ala Leu His Leu Ala Cys Gln Ser Leu Arg Thr Gly Glu
   210 215 220
   tcc tcc ttc gcc ctc gcc gga ggc gtg acg gtc atg tcc acc ccg ggc 720
   Ser Ser Phe Ala Leu Ala Gly Gly Val Thr Val Met Ser Thr Pro Gly
   225 230 235 240
   atg ttc gtg gag ttc tcg cgg cag cgg ggt ctg tcg ccg gac ggc cgg 768
   Met Phe Val Glu Phe Ser Arg Gln Arg Gly Leu Ser Pro Asp Gly Arg
   245 250 255
   tgc aag gcg tac gcg tcg gct gcc gac ggc acc ggc atg tcc gag ggc 816
   Cys Lys Ala Tyr Ala Ser Ala Ala Asp Gly Thr Gly Met Ser Glu Gly
   260 265 270
   gtg ggg att ttg ctg ctg gag cgg ctg tcc gag gct gaa cgt cgt ggt 864
   Val Gly Ile Leu Leu Leu Glu Arg Leu Ser Glu Ala Glu Arg Arg Gly
   275 280 285
   cat cgg gtt ttg gcg gtg gtg cgg ggg agt gcg gtg aat cag gac ggt 912
   His Arg Val Leu Ala Val Val Arg Gly Ser Ala Val Asn Gln Asp Gly
           290                 295                 300
   gcg tcg aat ggg ttg acg gcg ccg aat ggt ccg tcg cag cag cgg gtg     960
   Ala Ser Asn Gly Leu Thr Ala Pro Asn Gly Pro Ser Gln Gln Arg Val
   305                 310                 315                 320
   att cgg cag gcg ttg gcg tgt gcg ggg ttg tct gtg gcg gat gtg gat    1008
   Ile Arg Gln Ala Leu Ala Cys Ala Gly Leu Ser Val Ala Asp Val Asp
                   325                 330                 335
   gtg gtg gag ggg cac ggg acg ggc acg acg ctg ggt gat ccg atc gag    1056
   Val Val Glu Gly His Gly Thr Gly Thr Thr Leu Gly Asp Pro Ile Glu
               340                 345                 350
   gcg cag gcg ttg ctc gcc acg tac ggg cag cgg gcc ggt gac acg ccg    1104
   Ala Gln Ala Leu Leu Ala Thr Tyr Gly Gln Arg Ala Gly Asp Thr Pro
           355                 360                 365
   gtg tgg ttg ggg tcg gtg aag tcg aac atc ggg cat gcg cag gct gct    1152
   Val Trp Leu Gly Ser Val Lys Ser Asn Ile Gly His Ala Gln Ala Ala
       370                 375                 380
   gcg ggt gtg gcg ggt gtg atc aag atg gtg atg gcg ttg cgg gcg ggg    1200
   Ala Gly Val Ala Gly Val Ile Lys Met Val Met Ala Leu Arg Ala Gly
   385                 390                 395                 400
   gtg ttg ccg cgg acg ttg cat gtg gat gag ccg tcg tcg cag gtg gat    1248
   Val Leu Pro Arg Thr Leu His Val Asp Glu Pro Ser Ser Gln Val Asp
                   405                 410                 415
   tgg tcg agt ggg tcg gtt cgt gtg ttg gcg gat gag gtg gag tgg ccg    1296
   Trp Ser Ser Gly Ser Val Arg Val Leu Ala Asp Glu Val Glu Trp Pro
               420                 425                 430
   ggg gtg gag ggt cgg ctg cgg cgt gcg ggg gtg tct gcg ttc ggg gtg    1344
   Gly Val Glu Gly Arg Leu Arg Arg Ala Gly Val Ser Ala Phe Gly Val
           435                 440                 445
   agt ggg acg aat gcg cat gtg att ttg gag gag gcg tcg ggg ggc gcg    1392
   Ser Gly Thr Asn Ala His Val Ile Leu Glu Glu Ala Ser Gly Gly Ala
       450                 455                 460
   ggt ggg ggt gcg ggc cgg ctg cag gag ttg ggt ccg ggg gtg gtg tcg    1440
   Gly Gly Gly Ala Gly Arg Leu Gln Glu Leu Gly Pro Gly Val Val Ser
   465                 470                 475                 480
   ggt tcg ggg gtg gtg ccg tgg gtg gtg tcg gcg cgg tcg gag ttg gcg    1488
   Gly Ser Gly Val Val Pro Trp Val Val Ser Ala Arg Ser Glu Leu Ala
                   485                 490                 495
   ttg cgg ggg cag gcg cgt cgg ttg cgt ggg gtt gtg gcg gtt ggt ggg    1536
   Leu Arg Gly Gln Ala Arg Arg Leu Arg Gly Val Val Ala Val Gly Gly
               500                 505                 510
   ggt gcg gat ggt gtg ggg gtg agt ccg gct ggg gtc ggg cgg gct ttg    1584
   Gly Ala Asp Gly Val Gly Val Ser Pro Ala Gly Val Gly Arg Ala Leu
           515                 520                 525
   gtg tcg gag cgg tcg gtg ttc gag cat cgt gcg gtg gtc gtg gcc gag     1632
   Val Ser Glu Arg Ser Val Phe Glu His Arg Ala Val Val Val Ala Glu
       530                 535                 540
   gac cgc gac gag ttc ctg cac gca ctc gac gca ctg gcc ggc ggc cgc     1680
   Asp Arg Asp Glu Phe Leu His Ala Leu Asp Ala Leu Ala Gly Gly Arg
   545                 550                 555                 560
   ccc gtg ccc ggc gtc gtc gag gga cga acc acc tcg ggc gaa ctc gcc     1728
   Pro Val Pro Gly Val Val Glu Gly Arg Thr Thr Ser Gly Glu Leu Ala
                   565                 570                 575
   gta ctc ttc gcc ggg cag gga acc cag cgc gca ggc atg ggc cgc gaa     1776
   Val Leu Phe Ala Gly Gln Gly Thr Gln Arg Ala Gly Met Gly Arg Glu
               580                 585                 590
   ctg tac gag gcg tac ccc gtc ttc gcc cag gcc atc gac gag atc tgc     1824
   Leu Tyr Glu Ala Tyr Pro Val Phe Ala Gln Ala Ile Asp Glu Ile Cys
           595                 600                 605
   gcg gag gcc gac acc gcc cgc acc gac ccc ggt gcc cct ggg ctg cgg     1872
   Ala Glu Ala Asp Thr Ala Arg Thr Asp Pro Gly Ala Pro Gly Leu Arg
       610                 615                 620
   gac gta ctc ttc gca ccg cag gac tct ccc gaa ggc cgg ctg atc gag     1920
   Asp Val Leu Phe Ala Pro Gln Asp Ser Pro Glu Gly Arg Leu Ile Glu
   625                 630                 635                 640
   gac acg ggt ttc gcc cag ccc gcc ctg ttc gcc ttc gag gtg gcg ctg     1968
   Asp Thr Gly Phe Ala Gln Pro Ala Leu Phe Ala Phe Glu Val Ala Leu
                   645                 650                 655
   ttc cgg ctg ctg gag acc tgg ggt ctg acg ccc gac tac gtc ctc ggc     2016
   Phe Arg Leu Leu Glu Thr Trp Gly Leu Thr Pro Asp Tyr Val Leu Gly
               660                 665                 670
   cat tcc gtc ggt gaa ctg gcg gcc gcc cat gtc gcc ggg atg ctc tgc     2064
   His Ser Val Gly Glu Leu Ala Ala Ala His Val Ala Gly Met Leu Cys
           675                 680                 685
   ctt gcc gac gcg gtg gca ctg gtg gtc gca cga ggc cgc ctg atg caa     2112
   Leu Ala Asp Ala Val Ala Leu Val Val Ala Arg Gly Arg Leu Met Gln
       690                 695                 700
   ggg ctc ccg tcc ggc gga gcc atg gtg gcc atc gag gcg tcc gag gac     2160
   Gly Leu Pro Ser Gly Gly Ala Met Val Ala Ile Glu Ala Ser Glu Asp
   705                 710                 715                 720
   gag atc ctc ccg ctg ccc gac gaa tac gca tcc cgg gtc gcg cac gcc     2208
   Glu Ile Leu Pro Leu Pro Asp Glu Tyr Ala Ser Arg Val Ala His Ala
                   725                 730                 735
   gcg gtg aac ggg ccg cgg tcg atc gtc ctc tcc ggg gac gag gac gcg     2256
   Ala Val Asn Gly Pro Arg Ser Ile Val Leu Ser Gly Asp Glu Asp Ala
               740                 745                 750
   gtc ctg gac ctc gcg cag caa tgg gcg gca cga ggc cgc cgc acc cgg    2304
   Val Leu Asp Leu Ala Gln Gln Trp Ala Ala Arg Gly Arg Arg Thr Arg
           755                 760                 765
   cgg ctg cgg acc agc cac gcc ttc cac tcg ccg cac atg gac gcc atg    2352
   Arg Leu Arg Thr Ser His Ala Phe His Ser Pro His Met Asp Ala Met
       770                 775                 780
   ttg ggc gac ttc cgc cgc gcg gcc gag cag gtc acc ttc agc gcc ccg    2400
   Leu Gly Asp Phe Arg Arg Ala Ala Glu Gln Val Thr Phe Ser Ala Pro
   785                 790                 795                 800
   cgg att ccc gtc gtc tcc aac gtc acc ggc gcg ccc ctc ccc gcc gag    2448
   Arg Ile Pro Val Val Ser Asn Val Thr Gly Ala Pro Leu Pro Ala Glu
                   805                 810                 815
   acc atg tgc acc ccg gac tac tgg gtc gaa cac gcc cgc agc acg gtc    2496
   Thr Met Cys Thr Pro Asp Tyr Trp Val Glu His Ala Arg Ser Thr Val
               820                 825                 830
   cgt ttc gcg gac ggc atc tca tgg ctt cag gaa cag ggc gtc acc acc    2544
   Arg Phe Ala Asp Gly Ile Ser Trp Leu Gln Glu Gln Gly Val Thr Thr
           835                 840                 845
   tgc ctc gaa atc ggc ccc gac ggc acg ctg tcg gcc ctc gca cag gac    2592
   Cys Leu Glu Ile Gly Pro Asp Gly Thr Leu Ser Ala Leu Ala Gln Asp
       850                 855                 860
   tcg ctc agt gca ccg gcc cgc gcc atc ccc gcc ctg cgg ccg gac cag    2640
   Ser Leu Ser Ala Pro Ala Arg Ala Ile Pro Ala Leu Arg Pro Asp Gln
   865                 870                 875                 880
   ccg gag gca cgg tcg gtc atg acc gcc ctg gcg gag ttg ttc gtg gct    2688
   Pro Glu Ala Arg Ser Val Met Thr Ala Leu Ala Glu Leu Phe Val Ala
                   885                 890                 895
   ggg acg gcg gtt gag tgg gcc ggt gtg ttc gag ggg act gct cgc gag    2736
   Gly Thr Ala Val Glu Trp Ala Gly Val Phe Glu Gly Thr Ala Arg Glu
               900                 905                 910
   gtc ggt gat gga tgc ggg gtg gag ctg ccg acg tat gcg ttt gag cgg    2784
   Val Gly Asp Gly Cys Gly Val Glu Leu Pro Thr Tyr Ala Phe Glu Arg
           915                 920                 925
   gag cga ttt tgg ctg gac gtg gag gag gga tct gcg gga ggt tcc ggg    2832
   Glu Arg Phe Trp Leu Asp Val Glu Glu Gly Ser Ala Gly Gly Ser Gly
       930                 935                 940
   gtt tcc ggg atg tgg ggt ggt ccg ttg tgg gag gcg gtc gag tgt ggt    2880
   Val Ser Gly Met Trp Gly Gly Pro Leu Trp Glu Ala Val Glu Cys Gly
   945                 950                 955                 960
   gat gcg ggg gtg gtg gca tcg ctc ctt ggg gtg gat gag ggg gcg tcg    2928
   Asp Ala Gly Val Val Ala Ser Leu Leu Gly Val Asp Glu Gly Ala Ser
                   965                 970                 975
   ctg ggt gcg gtg gtg tcg gcg ttg ggg gaa tgg ggg cgg gta cgg cac    2976
   Leu Gly Ala Val Val Ser Ala Leu Gly Glu Trp Gly Arg Val Arg His
               980                 985                 990
   gag cgt gaa gtg gtg gac ggg tgg cgc tat cgg gag gtg tgg cga ccc      3024
   Glu Arg Glu Val Val Asp Gly Trp Arg Tyr Arg Glu Val Trp Arg Pro
           995                 1000                1005
   gtt tcg ggc ggt ggt gta ggg ggg ctg tcg ggc gcg tgg ctg gtg gtg      3072
   Val Ser Gly Gly Gly Val Gly Gly Leu Ser Gly Ala Trp Leu Val Val
       1010                1015                1020
   tcc gag ggc gag gcg ggc ccg gtt gat gtg gtg gcg gag ggg ttg gag      3120
   Ser Glu Gly Glu Ala Gly Pro Val Asp Val Val Ala Glu Gly Leu Glu
   1025                1030                1035                1040
   cgg tgt ggg gcg cga gtg gtt cgg gtg gag gtg gaa gcg ggg tgt gtg      3168
   Arg Cys Gly Ala Arg Val Val Arg Val Glu Val Glu Ala Gly Cys Val
                   1045                1050                1055
   agc agg gaa gtg ttg gcc ggc cac ctg cgt gag gcg gtc gat ggt gag      3216
   Ser Arg Glu Val Leu Ala Gly His Leu Arg Glu Ala Val Asp Gly Glu
               1060                1065                1070
   gct gtc ggc ggt gtc gtc tcc ctt gtg ggc tgg ggg agt ggc gtc gtg      3264
   Ala Val Gly Gly Val Val Ser Leu Val Gly Trp Gly Ser Gly Val Val
           1075                1080                1085
   cag gcg gga gtg gcg tct gtg ggg ttg gtg cag gcg ctg ggt gat gtg      3312
   Gln Ala Gly Val Ala Ser Val Gly Leu Val Gln Ala Leu Gly Asp Val
       1090                1095                1100
   ggc gtg ggg gcg cgg ctg tgg tgt gtg acg ggc ggg gcc gtg tcg gtg      3360
   Gly Val Gly Ala Arg Leu Trp Cys Val Thr Gly Gly Ala Val Ser Val
   1105                1110                1115                1120
   ggg ggc cgg gat gct gtg tgg ggg ccg gcc tcg ggt gtg gtg tgg ggg      3408
   Gly Gly Arg Asp Ala Val Trp Gly Pro Ala Ser Gly Val Val Trp Gly
                   1125                1130                1135
   ctg ggc cgt gtg gtg ggg gcg gag gca ccg gac cgc tgg ggt ggg ctg      3456
   Leu Gly Arg Val Val Gly Ala Glu Ala Pro Asp Arg Trp Gly Gly Leu
               1140                1145                1150
   gtt gat gtg ccg gag ctc gtg gat gag cgg gtg gtc gat ggg ttg gta      3504
   Val Asp Val Pro Glu Leu Val Asp Glu Arg Val Val Asp Gly Leu Val
           1155                1160                1165
   ggt gtg ctg gcg ggt gtg ggg gga ggg ggt gag agt gag ttt gcc gtg      3552
   Gly Val Leu Ala Gly Val Gly Gly Gly Gly Glu Ser Glu Phe Ala Val
       1170                1175                1180
   cgg tct tcg ggg gcg ttt gtg cgg cgg ttg gtg cgg gcg ccg ttg gag      3600
   Arg Ser Ser Gly Ala Phe Val Arg Arg Leu Val Arg Ala Pro Leu Glu
   1185                1190                1195                1200
   gag gcc gtc gcg gag cgg gag tgg cgg ccc cgc ggc acc gta ctc gtc 3648
   Glu Ala Val Ala Glu Arg Glu Trp Arg Pro Arg Gly Thr Val Leu Val
   1205 1210 1215
   acc gga ggc acc ggc gag ttg ggt gcg cac gtc gcc cgg tgg atg gcc 3696
   Thr Gly Gly Thr Gly Glu Leu Gly Ala His Val Ala Arg Trp Met Ala
   1220 1225 1230
   cgg cgt ggc gcc gaa cac ctg ctg ctg gtg agc cga cgc ggg gag agc 3744
   Arg Arg Gly Ala Glu His Leu Leu Leu Val Ser Arg Arg Gly Glu Ser
   1235 1240 1245
   gcc cag gga gtc gaa gaa ctc cga gcg gac ttg atg ggc ttg ggc gcg 3792
   Ala Gln Gly Val Glu Glu Leu Arg Ala Asp Leu Met Gly Leu Gly Ala
   1250 1255 1260
   cgg gtg tcg gtg gtg gcg tgt gat gcg gcg gac cgt gag gcg ttg gcg 3840
   Arg Val Ser Val Val Ala Cys Asp Ala Ala Asp Arg Glu Ala Leu Ala
   1265 1270 1275 1280
   gag gtg ttg cgg tcg gcc gtt ccg gcg gag tgc ccg ctg ggt gtg gtg 3888
   Glu Val Leu Arg Ser Ala Val Pro Ala Glu Cys Pro Leu Gly Val Val
   1285 1290 1295
   gtg cat gcc gcg gga gtt gtg gat gac ggg gtg ttg gag ggg ttg tcg 3936
   Val His Ala Ala Gly Val Val Asp Asp Gly Val Leu Glu Gly Leu Ser
   1300 1305 1310
   tcc gag cgt gtc acg ggg gtg ctg cgg gcg aag gcg ctg gcg gcc tgg 3984
   Ser Glu Arg Val Thr Gly Val Leu Arg Ala Lys Ala Leu Ala Ala Trp
   1315 1320 1325
   aat ctg cat gag ttg acg cgg ggg gcg gat ctt tcg ggg ttc gtg gtg 4032
   Asn Leu His Glu Leu Thr Arg Gly Ala Asp Leu Ser Gly Phe Val Val
   1330 1335 1340
   ttc tcg tcg gct gcg gcg acg ttc ggg ccg gcg gga cag ggg agt tac 4080
   Phe Ser Ser Ala Ala Ala Thr Phe Gly Pro Ala Gly Gln Gly Ser Tyr
   1345 1350 1355 1360
   gcg gcg gcg aac gcg tat gtg gag gca atc gtt cgg cac cgg cgt ggt 4128
   Ala Ala Ala Asn Ala Tyr Val Glu Ala Ile Val Arg His Arg Arg Gly
   1365 1370 1375
   gag ggc ctg ccg ggg ttg gcg gtg gcg tgg ggt ccg tgg gct ggt ggg 4176
   Glu Gly Leu Pro Gly Leu Ala Val Ala Trp Gly Pro Trp Ala Gly Gly
   1380 1385 1390
   ggg atg gcg gag ggg gcc gtg ggg cag atg cgg cgt cgg ggt ctg gcg 4224
   Gly Met Ala Glu Gly Ala Val Gly Gln Met Arg Arg Arg Gly Leu Ala
   1395 1400 1405
   gcg atg acg ccg gag acg gcg ctg gtg gca ctg ggc cag gcg ttg gac 4272
   Ala Met Thr Pro Glu Thr Ala Leu Val Ala Leu Gly Gln Ala Leu Asp
   1410 1415 1420
   cat gac gag acc tgt gtg acg gtc gcc gac atc gac tgg gac cga ttc 4320
   His Asp Glu Thr Cys Val Thr Val Ala Asp Ile Asp Trp Asp Arg Phe
   1425                1430                1435                1440
   acc gcc aac tcc ctc ccc ggc tcc cga ctc tcg ccc ctc atc agc gac     4368
   Thr Ala Asn Ser Leu Pro Gly Ser Arg Leu Ser Pro Leu Ile Ser Asp
               1445                1450                1455
   atc ccc gaa gca cgc ctc gcc cgg gaa acc acc gga ctc gac acc gcc     4416
   Ile Pro Glu Ala Arg Leu Ala Arg Glu Thr Thr Gly Leu Asp Thr Ala
           1460                1465                1470
   acc gca tcc ccc gac tcg ttc tcc gca cgg ctc aag gcc atg gac acc     4464
   Thr Ala Ser Pro Asp Ser Phe Ser Ala Arg Leu Lys Ala Met Asp Thr
       1475                1480                1485
   gcc gag cag gaa cgt gcg ctt ctc gac ctg gtc cgt acg tac gcg gcg     4512
   Ala Glu Gln Glu Arg Ala Leu Leu Asp Leu Val Arg Thr Tyr Ala Ala
       1490                1495                1500
   acc gtg ctc gga cac agc acc ccc acc gcc gta cgc cct gag cga gcc     4560
   Thr Val Leu Gly His Ser Thr Pro Thr Ala Val Arg Pro Glu Arg Ala
   1505                1510                1515                1520
   ttc cgc gac ctg ggc ttc gtc tcc gtg agc gcc gtc gaa ctg cgc aac     4608
   Phe Arg Asp Leu Gly Phe Val Ser Val Ser Ala Val Glu Leu Arg Asn
               1525                1530                1535
   cgc ctc aac gcc gtc acc ggg ctc ctc ctg ccc acc acg ctg atc ttc     4656
   Arg Leu Asn Ala Val Thr Gly Leu Leu Leu Pro Thr Thr Leu Ile Phe
           1540                1545                1550
   gac tac ccc act ccc tcc gcg ctg gcc gga tac ctc aag gaa cag ctg     4704
   Asp Tyr Pro Thr Pro Ser Ala Leu Ala Gly Tyr Leu Lys Glu Gln Leu
       1555                1560                1565
   gag gag ggc gcg ggc ggc cag cgt gac att gct cct ccg gtc ccg gcg     4752
   Glu Glu Gly Ala Gly Gly Gln Arg Asp Ile Ala Pro Pro Val Pro Ala
       1570                1575                1580
   tcg cgt gtc gac gtt gac gag ccg att gcg att gtg ggg atg gcg tgc     4800
   Ser Arg Val Asp Val Asp Glu Pro Ile Ala Ile Val Gly Met Ala Cys
   1585                1590                1595                1600
   cgt ttt ccg ggg ggt gtg gag tcg gcg gag gac ttg tgg gaa ctg gtc     4848
   Arg Phe Pro Gly Gly Val Glu Ser Ala Glu Asp Leu Trp Glu Leu Val
               1605                1610                1615
   gcg tcg ggt cgg gat gcg gtg gga gag ttt ccg gtc gac cgg ggt tgg     4896
   Ala Ser Gly Arg Asp Ala Val Gly Glu Phe Pro Val Asp Arg Gly Trp
           1620                1625                1630
   gac gtg gag gct ttc tat gat ccg gag ccg ggg cgg gcg ggt tcg tcg     4944
   Asp Val Glu Ala Phe Tyr Asp Pro Glu Pro Gly Arg Ala Gly Ser Ser
       1635                1640                1645
   tat acg cgc cgg ggc ggt ttc ctg gag ggt gcg gcg gag ttc gat gcg     4992
   Tyr Thr Arg Arg Gly Gly Phe Leu Glu Gly Ala Ala Glu Phe Asp Ala
   1650 1655 1660
   ggg ttt ttc ggg atc agt ccg cgt gag gcg ttg gcg atg gat ccg cag 5040
   Gly Phe Phe Gly Ile Ser Pro Arg Glu Ala Leu Ala Met Asp Pro Gln
   1665 1670 1675 1680
   cag cgg ttg atg ctg gag gtg tcc tgg gag gcg ttg gag cgg gcg ggc 5088
   Gln Arg Leu Met Leu Glu Val Ser Trp Glu Ala Leu Glu Arg Ala Gly
   1685 1690 1695
   atc gac ccc gcc acg ttg cgc ggg tcc acg acc ggt gtc ttc gcc ggc 5136
   Ile Asp Pro Ala Thr Leu Arg Gly Ser Thr Thr Gly Val Phe Ala Gly
   1700 1705 1710
   atg tgc agt cag gac tac gcc gac ctc gtg cgc cgg gcc acc gag gac 5184
   Met Cys Ser Gln Asp Tyr Ala Asp Leu Val Arg Arg Ala Thr Glu Asp
   1715 1720 1725
   ctc gag ggc tac gcc atg acg ggc ctg tcc agc agc gtc aca tcc gga 5232
   Leu Glu Gly Tyr Ala Met Thr Gly Leu Ser Ser Ser Val Thr Ser Gly
   1730 1735 1740
   cgc gtc gcc tac acc ctg ggg ctc gag ggt ccg gcg gtg acg gtg gat 5280
   Arg Val Ala Tyr Thr Leu Gly Leu Glu Gly Pro Ala Val Thr Val Asp
   1745 1750 1755 1760
   acg gcg tgt tcg tcg tcg ttg gtg gcg ctg cat ctg gcg tgt cag gcg 5328
   Thr Ala Cys Ser Ser Ser Leu Val Ala Leu His Leu Ala Cys Gln Ala
   1765 1770 1775
   ttg agg tcg ggg gag tgt tcg ctg gcg ttg gcg ggg ggt gtg acg gtg 5376
   Leu Arg Ser Gly Glu Cys Ser Leu Ala Leu Ala Gly Gly Val Thr Val
   1780 1785 1790
   atg tcg acg ccg ggt gcg ttt gtg gag ttc tcg cgg cag cgg ggt ctg 5424
   Met Ser Thr Pro Gly Ala Phe Val Glu Phe Ser Arg Gln Arg Gly Leu
   1795 1800 1805
   tcg ccg gac ggc cgg tgc aag gcg tac ggg tcg ggg gcc gat ggg gtc 5472
   Ser Pro Asp Gly Arg Cys Lys Ala Tyr Gly Ser Gly Ala Asp Gly Val
   1810 1815 1820
   ggc tgg gcc gag ggt gtg ggt gtg ctg ttg gtg gag cgg ctg tcc gag 5520
   Gly Trp Ala Glu Gly Val Gly Val Leu Leu Val Glu Arg Leu Ser Glu
   1825 1830 1835 1840
   gct gaa cgt cgt ggt cat cgg gtt ttg gcg gtg gtg cgg ggg agt gcg 5568
   Ala Glu Arg Arg Gly His Arg Val Leu Ala Val Val Arg Gly Ser Ala
   1845 1850 1855
   gtg aat cag gac ggt gcg tcg aat ggg ttg acg gcg ccg aat ggt ccg 5616
   Val Asn Gln Asp Gly Ala Ser Asn Gly Leu Thr Ala Pro Asn Gly Pro
   1860 1865 1870
   tcg cag cag cgg gtg att cgg cag gcg ttg gcg tgt gcg ggg ttg tcc 5664
   Ser Gln Gln Arg Val Ile Arg Gln Ala Leu Ala Cys Ala Gly Leu Ser
   1875 1880 1885
   gtg gcg gat gtg gat gtg gtg gag ggg cac ggg acg ggt acg acg ttg 5712
   Val Ala Asp Val Asp Val Val Glu Gly His Gly Thr Gly Thr Thr Leu
   1890 1895 1900
   ggt gat ccg atc gag gcg cag gcg ttg ctc gcc act tat ggg cag ggt 5760
   Gly Asp Pro Ile Glu Ala Gln Ala Leu Leu Ala Thr Tyr Gly Gln Gly
   1905 1910 1915 1920
   cgt tcg ggg gag cgg ccg gtg tgg ttg ggg tcg gtg aag tcg aac atc 5808
   Arg Ser Gly Glu Arg Pro Val Trp Leu Gly Ser Val Lys Ser Asn Ile
   1925 1930 1935
   ggg cat gcg cag gct gct gcg ggt gtg gcg ggt gtg atc aag atg gtg 5856
   Gly His Ala Gln Ala Ala Ala Gly Val Ala Gly Val Ile Lys Met Val
   1940 1945 1950
   atg gcg ttg cgg gcg ggg gtg ttg ccg cgg acg ttg cat gtg gat gag 5904
   Met Ala Leu Arg Ala Gly Val Leu Pro Arg Thr Leu His Val Asp Glu
   1955 1960 1965
   ccg tcg tcg cag gtg gat tgg tcg agt ggg tcg gtt cgt gtg ttg gcg 5952
   Pro Ser Ser Gln Val Asp Trp Ser Ser Gly Ser Val Arg Val Leu Ala
   1970 1975 1980
   gat gag gtg gag tgg ccg ggg gtg gag ggt cgg ctg cgg cgt gcg ggg 6000
   Asp Glu Val Glu Trp Pro Gly Val Glu Gly Arg Leu Arg Arg Ala Gly
   1985 1990 1995 2000
   gtg tct gcg ttc ggg gtg agt ggg acg aat gcg cat gtg att ttg gag 6048
   Val Ser Ala Phe Gly Val Ser Gly Thr Asn Ala His Val Ile Leu Glu
   2005 2010 2015
   gag gcg tcc ggg ggc gcg gat ggg ggt gcg ggc cgg ctg cag gag ttg 6096
   Glu Ala Ser Gly Gly Ala Asp Gly Gly Ala Gly Arg Leu Gln Glu Leu
   2020 2025 2030
   ggt ccg ggg gtg gtg tcg ggt tcg ggg gtg gtg ccg tgg gtg gtg tcg 6144
   Gly Pro Gly Val Val Ser Gly Ser Gly Val Val Pro Trp Val Val Ser
   2035 2040 2045
   gcg cgg tcg gag ttg gcg ttg cgg ggg cag gcg cgt cgg ttg cgt ggg 6192
   Ala Arg Ser Glu Leu Ala Leu Arg Gly Gln Ala Arg Arg Leu Arg Gly
   2050 2055 2060
   gtt gtg gcg gtt ggt ggg ggt gcg gat ggt gtg ggg gtg agt ccg gct 6240
   Val Val Ala Val Gly Gly Gly Ala Asp Gly Val Gly Val Ser Pro Ala
   2065 2070 2075 2080
   ggg gtc ggg cgg gct ttg gtg tcg gag cgg tcg gtg ttc gag cat cgt 6288
   Gly Val Gly Arg Ala Leu Val Ser Glu Arg Ser Val Phe Glu His Arg
   2085 2090 2095
   gcg gtg gtc gtg gcc gag gac cgc gac gag ttc ctg cac gca ctc gac 6336
   Ala Val Val Val Ala Glu Asp Arg Asp Glu Phe Leu His Ala Leu Asp
   2100 2105 2110
   gca ctg gcc gag ggg gca ccc acc gcg ggg gtg gta cag ggt gtg gcc    6384
   Ala Leu Ala Glu Gly Ala Pro Thr Ala Gly Val Val Gln Gly Val Ala
           2115                2120                2125
   gga ccg gcg gcc gac gga aag atc gcc atg ctg ttc gga gga cag ggc    6432
   Gly Pro Ala Ala Asp Gly Lys Ile Ala Met Leu Phe Gly Gly Gln Gly
       2130                2135                2140
   acc cac tgg gaa ggc atg gcg cag gaa ctc ctc ggc tcc tca ccg gtc    6480
   Thr His Trp Glu Gly Met Ala Gln Glu Leu Leu Gly Ser Ser Pro Val
   2145                2150                2155                2160
   ttc gcc cag cag atg tcc gac tgc gcc caa gcc ctc gaa ccg tac ctg    6528
   Phe Ala Gln Gln Met Ser Asp Cys Ala Gln Ala Leu Glu Pro Tyr Leu
                   2165                2170                2175
   gac tgg tct ctc ctc gac gtc ctg cgc ggc gca ccg gac gca ccc cct    6576
   Asp Trp Ser Leu Leu Asp Val Leu Arg Gly Ala Pro Asp Ala Pro Pro
               2180                2185                2190
   ctg caa cgc gtc gat gtc gtc cag ccc gtc ctc ttc gcg gtg atg gtc    6624
   Leu Gln Arg Val Asp Val Val Gln Pro Val Leu Phe Ala Val Met Val
           2195                2200                2205
   tcg ctg gcg gcg ctc tgg cgc tcg tac ggt gta cac ccg gac gcg gtg    6672
   Ser Leu Ala Ala Leu Trp Arg Ser Tyr Gly Val His Pro Asp Ala Val
       2210                2215                2220
   gcc ggg cac tcg cag ggc gag atc gca gcg gcc tac gtc gcc ggt gca    6720
   Ala Gly His Ser Gln Gly Glu Ile Ala Ala Ala Tyr Val Ala Gly Ala
   2225                2230                2235                2240
   ctc tcc ctc gac gac gcc gcc cgg gtc acc gcc ctg cgc agc cag gcg    6768
   Leu Ser Leu Asp Asp Ala Ala Arg Val Thr Ala Leu Arg Ser Gln Ala
                   2245                2250                2255
   ctg gcc gca ctg gcc ggg cag ggg gcg atg gca tcg gtc ggt ctg ccg    6816
   Leu Ala Ala Leu Ala Gly Gln Gly Ala Met Ala Ser Val Gly Leu Pro
               2260                2265                2270
   gtc gag aag ctg gag ccg cgt ctt gcg aca tgg ggc gac cgt ctg gtc    6864
   Val Glu Lys Leu Glu Pro Arg Leu Ala Thr Trp Gly Asp Arg Leu Val
           2275                2280                2285
   atc gcc gcc gtg aac ggg gcg cgt tcg gcc gtg gtc tcc ggg gag ccg    6912
   Ile Ala Ala Val Asn Gly Ala Arg Ser Ala Val Val Ser Gly Glu Pro
       2290                2295                2300
   gaa gcg gtc gac gcc ctg gtg gag gag ctg tca cac gaa gac gta ccg    6960
   Glu Ala Val Asp Ala Leu Val Glu Glu Leu Ser His Glu Asp Val Pro
   2305                2310                2315                2320
   gcc cgc agg ctc atg gtc gac tgg gcg tcg cac tcc ccg cag gtc gag    7008
   Ala Arg Arg Leu Met Val Asp Trp Ala Ser His Ser Pro Gln Val Glu
                   2325                2330                2335
   gcg atc cag ggg cgg ctg ctc gaa ctc ctc gcc ccc atc cgc gcg agg    7056
   Ala Ile Gln Gly Arg Leu Leu Glu Leu Leu Ala Pro Ile Arg Ala Arg
               2340                2345                2350
   acc ggc gac gtg ccc ttc tac tcc acc gtc acc ggc gaa cgc atc gac    7104
   Thr Gly Asp Val Pro Phe Tyr Ser Thr Val Thr Gly Glu Arg Ile Asp
           2355                2360                2365
   ggc acc gaa ctc gac gcc gac tac tgg tac cgc aac ctg cgc cag gtc    7152
   Gly Thr Glu Leu Asp Ala Asp Tyr Trp Tyr Arg Asn Leu Arg Gln Val
       2370                2375                2380
   gtc cgc ttc cgg gac gcg aca cag gcg ctg gtc cgc gcc ggc cac acc    7200
   Val Arg Phe Arg Asp Ala Thr Gln Ala Leu Val Arg Ala Gly His Thr
   2385                2390                2395                2400
   gtc ttc atc gag gcg tgc ccg cat ccg gcc gtc gcg gtc ggt gtg cag    7248
   Val Phe Ile Glu Ala Cys Pro His Pro Ala Val Ala Val Gly Val Gln
                   2405                2410                2415
   gaa acc ctg gac gag atg ggt gac ttg gac agc ctg gtc gtc gga tct    7296
   Glu Thr Leu Asp Glu Met Gly Asp Leu Asp Ser Leu Val Val Gly Ser
               2420                2425                2430
   ctg cgc cgg ggc gaa ggc ggc ttg cga cgc ttc ctg atg tcc gtg gcc    7344
   Leu Arg Arg Gly Glu Gly Gly Leu Arg Arg Phe Leu Met Ser Val Ala
           2435                2440                2445
   gag ttg ttc gtg ggt ggg gtg gcg gtt gag tgg tcc ggt gtg ttc ggg    7392
   Glu Leu Phe Val Gly Gly Val Ala Val Glu Trp Ser Gly Val Phe Gly
       2450                2455                2460
   agt gtt ggt cgc ggg gtc gct ggt ggt tgc ggg gtg gag ctg ccg acg    7440
   Ser Val Gly Arg Gly Val Ala Gly Gly Cys Gly Val Glu Leu Pro Thr
   2465                2470                2475                2480
   tat gcg ttc gag cga gag cgc ttt tgg ctg gat gtg gag ggg gcg ccg    7488
   Tyr Ala Phe Glu Arg Glu Arg Phe Trp Leu Asp Val Glu Gly Ala Pro
                   2485                2490                2495
   cgg ggt tcc ggg gtc tct ggg cag tgg ggt ggt cag ttg tcg gag gcg    7536
   Arg Gly Ser Gly Val Ser Gly Gln Trp Gly Gly Gln Leu Ser Glu Ala
               2500                2505                2510
   gtg gac acc gtg cgc ggc ggc atg ctg cgc gac tgc ctc gcc gga ctc    7584
   Val Asp Thr Val Arg Gly Gly Met Leu Arg Asp Cys Leu Ala Gly Leu
           2515                2520                2525
   gac ccc gcc gca cag gcc gag acc gtg ctg gac ctg gtc ctt acc cat    7632
   Asp Pro Ala Ala Gln Ala Glu Thr Val Leu Asp Leu Val Leu Thr His
       2530                2535                2540
   gcc gcg gcc gtc ctt gga cac ggc acc gcc gat gcg gtg gtg ccc gag    7680
   Ala Ala Ala Val Leu Gly His Gly Thr Ala Asp Ala Val Val Pro Glu
   2545                2550                2555                2560
   cgc gcc ttc cgc gac ctc ggt ttc gac tcc ctc acc gcc gtc gaa cta    7728
   Arg Ala Phe Arg Asp Leu Gly Phe Asp Ser Leu Thr Ala Val Glu Leu
   2565 2570 2575
   cgc aac cgc ctc aac acc gcc acg ggc ctg cgc ttc ccg agg acc ctg 7776
   Arg Asn Arg Leu Asn Thr Ala Thr Gly Leu Arg Phe Pro Arg Thr Leu
   2580 2585 2590
   gtg ttc gac cat ccc cgc ccg gtg gca ctc gcg gca cac atc cac gag 7824
   Val Phe Asp His Pro Arg Pro Val Ala Leu Ala Ala His Ile His Glu
   2595 2600 2605
   cag ctg agc ggc gga agc ccg acc acc ggc act gcc ctt gcc ctt gcc 7872
   Gln Leu Ser Gly Gly Ser Pro Thr Thr Gly Thr Ala Leu Ala Leu Ala
   2610 2615 2620
   ctt cgg gcc ccg gca ccg cgt gtg gat gtc gac gag ccg att gcc att 7920
   Leu Arg Ala Pro Ala Pro Arg Val Asp Val Asp Glu Pro Ile Ala Ile
   2625 2630 2635 2640
   gtg ggg atg gcg tgc cgt ttt ccg ggg ggt gtg gag tcg gcg gag gat 7968
   Val Gly Met Ala Cys Arg Phe Pro Gly Gly Val Glu Ser Ala Glu Asp
   2645 2650 2655
   ttc tgg gag ttg atc gcg tcg ggt cgg gat gcg gtg ggg gag ttt ccg 8016
   Phe Trp Glu Leu Ile Ala Ser Gly Arg Asp Ala Val Gly Glu Phe Pro
   2660 2665 2670
   gtc gac cgg ggt tgg gac gtg gag gct ttc tat gat ccg gag ccg ggg 8064
   Val Asp Arg Gly Trp Asp Val Glu Ala Phe Tyr Asp Pro Glu Pro Gly
   2675 2680 2685
   cgg gcg ggt acg tcc tac acg cgg tgt ggt ggg ttt ttg cag ggt gcg 8112
   Arg Ala Gly Thr Ser Tyr Thr Arg Cys Gly Gly Phe Leu Gln Gly Ala
   2690 2695 2700
   gcg gag ttc gat gcg ggg ttt ttc ggg atc agt ccg cgt gag gcg ttg 8160
   Ala Glu Phe Asp Ala Gly Phe Phe Gly Ile Ser Pro Arg Glu Ala Leu
   2705 2710 2715 2720
   gcg atg gat ccg cag cag cgg ttg atg ctg gag gtg tcc tgg gag gcg 8208
   Ala Met Asp Pro Gln Gln Arg Leu Met Leu Glu Val Ser Trp Glu Ala
   2725 2730 2735
   ttg gag cgg gcg ggc atc gac ccc gcc acg ctg cac ggg tcc acg acc 8256
   Leu Glu Arg Ala Gly Ile Asp Pro Ala Thr Leu His Gly Ser Thr Thr
   2740 2745 2750
   ggt gtc ttc gcc ggc gtc tcg cag cag gac tac gcc gag ctc ctg cgc 8304
   Gly Val Phe Ala Gly Val Ser Gln Gln Asp Tyr Ala Glu Leu Leu Arg
   2755 2760 2765
   cgc ggc acc cag gac cac gag ggg tac gcg ctc acc ggc gtc tcc aac 8352
   Arg Gly Thr Gln Asp His Glu Gly Tyr Ala Leu Thr Gly Val Ser Asn
   2770 2775 2780
   agc gtc gtc tcc ggg cgg ctt tcc tac acc ttc ggc ttc gag ggt ccg 8400
   Ser Val Val Ser Gly Arg Leu Ser Tyr Thr Phe Gly Phe Glu Gly Pro
   2785 2790 2795 2800
   gcg gtg acg gtg gat acg gcg tgt tcg tcg tcg ttg gtg gcg ctg cat     8448
   Ala Val Thr Val Asp Thr Ala Cys Ser Ser Ser Leu Val Ala Leu His
                   2805                2810                2815
   ctg gcg tgt cag gcg ttg agg tcg ggg gag tgt tcg ctg gcg ttg gcg     8496
   Leu Ala Cys Gln Ala Leu Arg Ser Gly Glu Cys Ser Leu Ala Leu Ala
               2820                2825                2830
   ggg ggt gtg acg gtg atg tcg acg ccg ggt gcg ttt gtg gag ttc tcg     8544
   Gly Gly Val Thr Val Met Ser Thr Pro Gly Ala Phe Val Glu Phe Ser
           2835                2840                2845
   cgg cag cgg ggt ctg tcg ccg gac ggc cgg tgc aag gcg tac ggg tcg     8592
   Arg Gln Arg Gly Leu Ser Pro Asp Gly Arg Cys Lys Ala Tyr Gly Ser
       2850                2855                2860
   ggg gcc gat ggg gtc ggc tgg gcc gag ggt gtg ggt gtg ctg ttg gtg     8640
   Gly Ala Asp Gly Val Gly Trp Ala Glu Gly Val Gly Val Leu Leu Val
   2865                2870                2875                2880
   gag cgg ctg tcc gag gct gaa cgt cgt ggt cat cgg gtt ttg gcg gtg     8688
   Glu Arg Leu Ser Glu Ala Glu Arg Arg Gly His Arg Val Leu Ala Val
                   2885                2890                2895
   gtg cgg ggg agt gcg gtg aat cag gac ggt gcg tcg aat ggg ttg acg     8736
   Val Arg Gly Ser Ala Val Asn Gln Asp Gly Ala Ser Asn Gly Leu Thr
               2900                2905                2910
   gcg ccg aat ggt ccg tcg cag cag cgg gtg att cgg cag gcg ttg gcg     8784
   Ala Pro Asn Gly Pro Ser Gln Gln Arg Val Ile Arg Gln Ala Leu Ala
           2915                2920                2925
   tgt gcg ggg ttg tcc gtg gcg gat gtg gat gtg gtg gag ggg cac ggg     8832
   Cys Ala Gly Leu Ser Val Ala Asp Val Asp Val Val Glu Gly His Gly
       2930                2935                2940
   acg ggt acg acg ttg ggt gat ccg atc gag gcg cag gcg ttg ctc gcc     8880
   Thr Gly Thr Thr Leu Gly Asp Pro Ile Glu Ala Gln Ala Leu Leu Ala
   2945                2950                2955                2960
   acg tac ggg cag ggt cgt tcg ggg gag cgg ccg gtg tgg ttg ggg tcg     8928
   Thr Tyr Gly Gln Gly Arg Ser Gly Glu Arg Pro Val Trp Leu Gly Ser
                   2965                2970                2975
   gtg aag tcg aac atc ggg cat gcg cag gct gcc gcg ggt gtg gcc ggt     8976
   Val Lys Ser Asn Ile Gly His Ala Gln Ala Ala Ala Gly Val Ala Gly
               2980                2985                2990
   gtg atc aag atg gtc atg gcc ctg aac cac gaa ctg ttg ccg acc agc     9024
   Val Ile Lys Met Val Met Ala Leu Asn His Glu Leu Leu Pro Thr Ser
           2995                3000                3005
   ctg cac atc gac gaa ccc tcc ccc cac atc gac tgg tcg agc ggc ggc     9072
   Leu His Ile Asp Glu Pro Ser Pro His Ile Asp Trp Ser Ser Gly Gly
       3010                3015                3020
   gtc cgg ctt ctc acc gag ccc gta ccg tgg cag cag aac ggc cgg ccc 9120
   Val Arg Leu Leu Thr Glu Pro Val Pro Trp Gln Gln Asn Gly Arg Pro
   3025 3030 3035 3040
   agg cgc gcg ggc gtc tcc gcg ttc gga gtc agc ggg acc aac gcc cac 9168
   Arg Arg Ala Gly Val Ser Ala Phe Gly Val Ser Gly Thr Asn Ala His
   3045 3050 3055
   gtc atc atc gag cag gcg ccg gtc gag gcg cac gtc atc agt gag ccg 9216
   Val Ile Ile Glu Gln Ala Pro Val Glu Ala His Val Ile Ser Glu Pro
   3060 3065 3070
   gta ccg gct gag gcg cac gtc atc gtc gag cag gcg ccg gtc gag gcg 9264
   Val Pro Ala Glu Ala His Val Ile Val Glu Gln Ala Pro Val Glu Ala
   3075 3080 3085
   ccc cac gtg gtc gac gcc acc gga ccg gcg gac ctc acc gag ccg caa 9312
   Pro His Val Val Asp Ala Thr Gly Pro Ala Asp Leu Thr Glu Pro Gln
   3090 3095 3100
   gag gag gcg gct gaa ccg gag tgc gtc gct gac gcc gtg acc gag atg 9360
   Glu Glu Ala Ala Glu Pro Glu Cys Val Ala Asp Ala Val Thr Glu Met
   3105 3110 3115 3120
   tcg gct gaa ccg gag tgc gtc gcc gac gcc atg tcc gag atg tcg gct 9408
   Ser Ala Glu Pro Glu Cys Val Ala Asp Ala Met Ser Glu Met Ser Ala
   3125 3130 3135
   gag tgc gtc gcc gag gcc gtg tcc gac aag tcg gct gaa ccg gag tgc 9456
   Glu Cys Val Ala Glu Ala Val Ser Asp Lys Ser Ala Glu Pro Glu Cys
   3140 3145 3150
   gtc gcc gac gcc atg tcc gac aag ccg gcc ctc ctg ccc atc ccg tgg 9504
   Val Ala Asp Ala Met Ser Asp Lys Pro Ala Leu Leu Pro Ile Pro Trp
   3155 3160 3165
   ctg ctc tcc gcc aag tcc gag cga gcg ctg cgg ggc cag gcg cga cgg 9552
   Leu Leu Ser Ala Lys Ser Glu Arg Ala Leu Arg Gly Gln Ala Arg Arg
   3170 3175 3180
   ttg cgg cag ttc gct gcc agg gca tcc gat gcc cgg ccg gcc gac gtg 9600
   Leu Arg Gln Phe Ala Ala Arg Ala Ser Asp Ala Arg Pro Ala Asp Val
   3185 3190 3195 3200
   gcg cac gcc ctg gcg gca cag cgg tcc gtg ttc gat cac cgg gcc gtc 9648
   Ala His Ala Leu Ala Ala Gln Arg Ser Val Phe Asp His Arg Ala Val
   3205 3210 3215
   gtc gtg gcc gag gac cgc gac ggc ttc ctt cag gcc ctc gac gcg ctg 9696
   Val Val Ala Glu Asp Arg Asp Gly Phe Leu Gln Ala Leu Asp Ala Leu
   3220 3225 3230
   gcc gag ggc cgg tcg gcg gac ggc ctg atc gaa ggg tcg gtc ggc ccg 9744
   Ala Glu Gly Arg Ser Ala Asp Gly Leu Ile Glu Gly Ser Val Gly Pro
   3235 3240 3245
   cgt ggc ggc cac tca ggc cgc cgg cgc gga aag acc gcc atg ctg ttc 9792
   Arg Gly Gly His Ser Gly Arg Arg Arg Gly Lys Thr Ala Met Leu Phe
       3250                3255                3260
   gcc gga cag ggc acg caa cgc gtg gga atg ggc cgt cag ctg tat gcg     9840
   Ala Gly Gln Gly Thr Gln Arg Val Gly Met Gly Arg Gln Leu Tyr Ala
   3265                3270                3275                3280
   gct cac ccg gcc tac gcg gac gcg ctg gac cag gta ctg gcg gaa ctg     9888
   Ala His Pro Ala Tyr Ala Asp Ala Leu Asp Gln Val Leu Ala Glu Leu
                   3285                3290                3295
   gac ggt cac ctg gac cag ccc ctg cgc ccg ctg atc cac gcc agt gcg     9936
   Asp Gly His Leu Asp Gln Pro Leu Arg Pro Leu Ile His Ala Ser Ala
               3300                3305                3310
   gat ctt gcg gat gtc gcg gat gcc gcg gat gtt ctg gac cgt acg cgg     9984
   Asp Leu Ala Asp Val Ala Asp Ala Ala Asp Val Leu Asp Arg Thr Arg
           3315                3320                3325
   tac gcc cag ccg gcg ctg ttc gcc gtc cag gtc gcg ctc ttc cgg cac     10032
   Tyr Ala Gln Pro Ala Leu Phe Ala Val Gln Val Ala Leu Phe Arg His
       3330                3335                3340
   ctg gaa cgt ctc ggc gtg cgc gcg gac ttc gtg gcc ggg cac tcg atc     10080
   Leu Glu Arg Leu Gly Val Arg Ala Asp Phe Val Ala Gly His Ser Ile
   3345                3350                3355                3360
   ggc gag ctc gcg gcc gcc cac gtc gcc ggg gtg ctt ccc ctg gca gca     10128
   Gly Glu Leu Ala Ala Ala His Val Ala Gly Val Leu Pro Leu Ala Ala
                   3365                3370                3375
   gcc tgc cgc ctg gtg gcg gcc cgc ggg cgc ctg atg gag cag ctc gca     10176
   Ala Cys Arg Leu Val Ala Ala Arg Gly Arg Leu Met Glu Gln Leu Ala
               3380                3385                3390
   cca ggc ggc gcc atg gtc gcc gta cgg gcg agc gaa gcc gag gcg cga     10224
   Pro Gly Gly Ala Met Val Ala Val Arg Ala Ser Glu Ala Glu Ala Arg
           3395                3400                3405
   cag gcg ctc gac ggc cgg gaa gcc cgg gtg tcg gtc gcg gcc gtg aac     10272
   Gln Ala Leu Asp Gly Arg Glu Ala Arg Val Ser Val Ala Ala Val Asn
       3410                3415                3420
   gga ccc gcc tcg gtg gtg ttc tcc ggc gcc gag gac gag gtg ggg aac     10320
   Gly Pro Ala Ser Val Val Phe Ser Gly Ala Glu Asp Glu Val Gly Asn
   3425                3430                3435                3440
   atg gcg gac tgg ttc gcc gag cgc ggg cgg aga gtc aag cgc ctg cga     10368
   Met Ala Asp Trp Phe Ala Glu Arg Gly Arg Arg Val Lys Arg Leu Arg
                   3445                3450                3455
   acc ggg cat gcc ttc cac tca ccg ctg atg gac ccg atg ctg gag gag     10416
   Thr Gly His Ala Phe His Ser Pro Leu Met Asp Pro Met Leu Glu Glu
               3460                3465                3470
   ttc cag cag gtc gcg gcc tcg ctg acc tac agc gaa cca gcc att ccc     10464
   Phe Gln Gln Val Ala Ala Ser Leu Thr Tyr Ser Glu Pro Ala Ile Pro
   3475 3480 3485
   atg gtg tcg acg ctc acc ggc gac atc gtg gcg gcg gga gaa ctg agc 10512
   Met Val Ser Thr Leu Thr Gly Asp Ile Val Ala Ala Gly Glu Leu Ser
   3490 3495 3500
   gac ccc gag tac tgg gtc cgg cag gta cgg cgg acc gtg cgc ttc ggc 10560
   Asp Pro Glu Tyr Trp Val Arg Gln Val Arg Arg Thr Val Arg Phe Gly
   3505 3510 3515 3520
   gac gcg atc agc cgc ctg cac acc gac gga gtc cgc acc ttc atg gaa 10608
   Asp Ala Ile Ser Arg Leu His Thr Asp Gly Val Arg Thr Phe Met Glu
   3525 3530 3535
   ctg ggc cca gac ggg acc ctg tcg gca ctg gcc gag gaa tgc cta gag 10656
   Leu Gly Pro Asp Gly Thr Leu Ser Ala Leu Ala Glu Glu Cys Leu Glu
   3540 3545 3550
   gcc acc gcc gac agc cac ccc gcc gac gac gac acc ggc acc ccg caa 10704
   Ala Thr Ala Asp Ser His Pro Ala Asp Asp Asp Thr Gly Thr Pro Gln
   3555 3560 3565
   gag aac ctg ctc atc ccg ctc cta cgg ccg gac agc ccg gaa ccc ggc 10752
   Glu Asn Leu Leu Ile Pro Leu Leu Arg Pro Asp Ser Pro Glu Pro Gly
   3570 3575 3580
   acc ctg ctc acc ggc ttg gcc cgg ctg cat acg cac gga gcg gcg gcg 10800
   Thr Leu Leu Thr Gly Leu Ala Arg Leu His Thr His Gly Ala Ala Ala
   3585 3590 3595 3600
   gtc aac tgg ccc gcc gcc ctg ccc gaa cgc gat cga gcc cgc cac ctc 10848
   Val Asn Trp Pro Ala Ala Leu Pro Glu Arg Asp Arg Ala Arg His Leu
   3605 3610 3615
   gac ctg ccg acc tac gcc ttc gat cac cac cgc tac tgg gtc gac acc 10896
   Asp Leu Pro Thr Tyr Ala Phe Asp His His Arg Tyr Trp Val Asp Thr
   3620 3625 3630
   tcg gcc ggc cac ccg ggg gac ctg tcg gca gcg ggg ctc ggc acc gcc 10944
   Ser Ala Gly His Pro Gly Asp Leu Ser Ala Ala Gly Leu Gly Thr Ala
   3635 3640 3645
   ggg cat ccc ctg ctc ggt tcc gcg gtg gca ctg gcc gag tcg cag gaa 10992
   Gly His Pro Leu Leu Gly Ser Ala Val Ala Leu Ala Glu Ser Gln Glu
   3650 3655 3660
   ctc ctc ttc acc ggc cgt ctc tcc ctg cgc aca cac ccg tgg ctg gcc 11040
   Leu Leu Phe Thr Gly Arg Leu Ser Leu Arg Thr His Pro Trp Leu Ala
   3665 3670 3675 3680
   gac cac gcc atc ttc ggt acc gtc ctg ctg ccc ggc acg gcc atc ctg 11088
   Asp His Ala Ile Phe Gly Thr Val Leu Leu Pro Gly Thr Ala Ile Leu
   3685 3690 3695
   gaa ctg gcc gtg cgc gca ggc gac gag gtc gac tgc ggc acc gtc gag 11136
   Glu Leu Ala Val Arg Ala Gly Asp Glu Val Asp Cys Gly Thr Val Glu
   3700 3705 3710
   gaa ctc acc ctg cgg aca ccg ctc gtc ctt ccc gaa cag ggc tcg gtg     11184
   Glu Leu Thr Leu Arg Thr Pro Leu Val Leu Pro Glu Gln Gly Ser Val
               3715                3720                3725
   atc ctg caa ctc tcc gtc ggg gca ccc cag ggc ccc cag acg ccc gag     11232
   Ile Leu Gln Leu Ser Val Gly Ala Pro Gln Gly Pro Gln Thr Pro Glu
           3730                3735                3740
   gag ccc gaa cgg cgc acc ttc gcc ctg tac gcc cgc gaa gac gac gga     11280
   Glu Pro Glu Arg Arg Thr Phe Ala Leu Tyr Ala Arg Glu Asp Asp Gly
   3745                3750                3755                3760
   ctg tcg tcc tcg tcc gcg gcg gcg acc ggc acc gag tgg acc tgc cac     11328
   Leu Ser Ser Ser Ser Ala Ala Ala Thr Gly Thr Glu Trp Thr Cys His
                   3765                3770                3775
   gcc acc ggc gtc ctg acc ggc acc gcc cgg ccc gcg gag gag cac aca     11376
   Ala Thr Gly Val Leu Thr Gly Thr Ala Arg Pro Ala Glu Glu His Thr
               3780                3785                3790
   cag gaa ccg tgg ccg ccc gcc gac gca gca ccg gtg gac ctg gac ggc     11424
   Gln Glu Pro Trp Pro Pro Ala Asp Ala Ala Pro Val Asp Leu Asp Gly
           3795                3800                3805
   tgg tac gag cag ctg gcc ggc gcc ggc ctg gga tac ggg ccg gtg ttc     11472
   Trp Tyr Glu Gln Leu Ala Gly Ala Gly Leu Gly Tyr Gly Pro Val Phe
       3810                3815                3820
   cag ggg ctg cgc gag gtc tgg cgg cgc ggg gac gag gtg ttc gcc gtc     11520
   Gln Gly Leu Arg Glu Val Trp Arg Arg Gly Asp Glu Val Phe Ala Val
   3825                3830                3835                3840
   gtc acc ctg ccc gag agc acg gag gga cag gcg gcc gac gcc gcc cgg     11568
   Val Thr Leu Pro Glu Ser Thr Glu Gly Gln Ala Ala Asp Ala Ala Arg
                   3845                3850                3855
   tac gcc ctg cac ccg gcc ctg ctg gac gcg gca ctg cac ccg gtc gtt     11616
   Tyr Ala Leu His Pro Ala Leu Leu Asp Ala Ala Leu His Pro Val Val
               3860                3865                3870
   ctg cgc cac gag ggc gat gcc gcc gcc gac gga cac ggc tgg ctg ccg     11664
   Leu Arg His Glu Gly Asp Ala Ala Ala Asp Gly His Gly Trp Leu Pro
           3875                3880                3885
   ttc tcc tgg acc ggc gtc acg gtc gcc gcc tcc ggc gcc tcc acc ctg     11712
   Phe Ser Trp Thr Gly Val Thr Val Ala Ala Ser Gly Ala Ser Thr Leu
       3890                3895                3900
   cac gtc cgt ctc acc gtc cgc acg gac gag gac gcg gtc gga ctg ctg     11760
   His Val Arg Leu Thr Val Arg Thr Asp Glu Asp Ala Val Gly Leu Leu
   3905                3910                3915                3920
   gcc acc gac gca tcg gga cgc atc gtc atc tcc gcg ggg tcc ctc gcc     11808
   Ala Thr Asp Ala Ser Gly Arg Ile Val Ile Ser Ala Gly Ser Leu Ala
                   3925                3930                3935
   ttc cgg ccc gtc tcc gcc gag cag ctc cag gcc gcg cgc acc ggc tac     11856
   Phe Arg Pro Val Ser Ala Glu Gln Leu Gln Ala Ala Arg Thr Gly Tyr
           3940                3945                3950
   cac gac cac ctc ttc cgc atc gaa tgg cgg ccg ctg cac ctc ccc acc     11904
   His Asp His Leu Phe Arg Ile Glu Trp Arg Pro Leu His Leu Pro Thr
       3955                3960                3965
   aca ccg gca cgg aca gcc gac tgg gcc cta atc ggc ccc ggt gcc cgg     11952
   Thr Pro Ala Arg Thr Ala Asp Trp Ala Leu Ile Gly Pro Gly Ala Arg
   3970                3975                3980
   cgg acg gcc gcc gtc ctg gag cgc aac ggc gcc tcc tgg cag gcc tac     12000
   Arg Thr Ala Ala Val Leu Glu Arg Asn Gly Ala Ser Trp Gln Ala Tyr
   3985                3990                3995                4000
   ccg gac ccg gcg gct ctc gca gaa gcc ctg gcg gcc ggc gcc ccg gca     12048
   Pro Asp Pro Ala Ala Leu Ala Glu Ala Leu Ala Ala Gly Ala Pro Ala
                   4005                4010                4015
   ccg ggc atg gtc gtc atc tcg tgc gag ccg gac ggc gca tcc gcc ccc     12096
   Pro Gly Met Val Val Ile Ser Cys Glu Pro Asp Gly Ala Ser Ala Pro
               4020                4025                4030
   acc gat tcc gcc ctc acc gat tcc gcc ctc acc gat tcc gcc ccg gcc     12144
   Thr Asp Ser Ala Leu Thr Asp Ser Ala Leu Thr Asp Ser Ala Pro Ala
           4035                4040                4045
   ggc tcg gcc ccg gcc gac tcc acc gcc ctc gcc gac gcc acc cgg caa     12192
   Gly Ser Ala Pro Ala Asp Ser Thr Ala Leu Ala Asp Ala Thr Arg Gln
       4050                4055                4060
   gcc acc acc cgc gtc ctc gcc ctg ctc cag gaa tgg gtc gcc gac gaa     12240
   Ala Thr Thr Arg Val Leu Ala Leu Leu Gln Glu Trp Val Ala Asp Glu
   4065                4070                4075                4080
   cgg ctc gcg gcc tgc cgc ctg gcc ctc ctc acg cac ggc tcg gtc acc     12288
   Arg Leu Ala Ala Cys Arg Leu Ala Leu Leu Thr His Gly Ser Val Thr
                   4085                4090                4095
   gcg acc ccc gac gag ccc gtg tcc gac ctc gca cac gcc gcc gtc tgg     12336
   Ala Thr Pro Asp Glu Pro Val Ser Asp Leu Ala His Ala Ala Val Trp
               4100                4105                4110
   gga ctg gtc cgc tcc gtg cag acc gag aac ccc gac cgg ttc ctg ctg     12384
   Gly Leu Val Arg Ser Val Gln Thr Glu Asn Pro Asp Arg Phe Leu Leu
           4115                4120                4125
   gcc gac acc gac gac acc gac gcc tcc cgc aac gcc ctt ccc ctg ctg     12432
   Ala Asp Thr Asp Asp Thr Asp Ala Ser Arg Asn Ala Leu Pro Leu Leu
       4130                4135                4140
   gcc ggg gaa ccg cag atc gcc ctg cga aat ggt gcc gtc cgc atc ccg     12480
   Ala Gly Glu Pro Gln Ile Ala Leu Arg Asn Gly Ala Val Arg Ile Pro
   4145                4150                4155                4160
   cgg atg aca cga gtg ccc gtc cgg cag cca cag ccg agc acc acc gac     12528
   Arg Met Thr Arg Val Pro Val Arg Gln Pro Gln Pro Ser Thr Thr Asp
                   4165                4170                4175
   gcc gac tgg gac ccg gag gcc acg gtc ctc atc acg ggc ggt acc ggc    12576
   Ala Asp Trp Asp Pro Glu Ala Thr Val Leu Ile Thr Gly Gly Thr Gly
               4180                4185                4190
   gtc ctc ggc cgg ctc gtc gcc cgt cat ctc gcc acg gcc cac ggg gta    12624
   Val Leu Gly Arg Leu Val Ala Arg His Leu Ala Thr Ala His Gly Val
           4195                4200                4205
   cgg cac ctg ctg ctg gcc acc cgc cgc ggc acg gcc gcg gac ggc gcc    12672
   Arg His Leu Leu Leu Ala Thr Arg Arg Gly Thr Ala Ala Asp Gly Ala
       4210                4215                4220
   gcc gac ctg gtc gcc gaa ctc gcc ggc ctc ggc gcc gag gcc acg gtc    12720
   Ala Asp Leu Val Ala Glu Leu Ala Gly Leu Gly Ala Glu Ala Thr Val
   4225                4230                4235                4240
   gcg gcc tgc gac atc ggg gac cgg gcg gcc gtc gcc gcg ctc ctc gac    12768
   Ala Ala Cys Asp Ile Gly Asp Arg Ala Ala Val Ala Ala Leu Leu Asp
                   4245                4250                4255
   caa gtg ccc gcg cag cac ccc ctg aaa gcc gtg atc cac acg gcc ggt    12816
   Gln Val Pro Ala Gln His Pro Leu Lys Ala Val Ile His Thr Ala Gly
               4260                4265                4270
   gtg gtc gac gac ggc atc ctc acc tcg ctc act ccg gag cgc atg gag    12864
   Val Val Asp Asp Gly Ile Leu Thr Ser Leu Thr Pro Glu Arg Met Glu
           4275                4280                4285
   gcc gtc ctg cac gcg aag gcg ttc ggc gcc gcg cac ctg cac gac ctg    12912
   Ala Val Leu His Ala Lys Ala Phe Gly Ala Ala His Leu His Asp Leu
       4290                4295                4300
   acc cgc gac gcc ggc ctc acc acc ttc acc gtc ttc tcc tcg gcc gcc    12960
   Thr Arg Asp Ala Gly Leu Thr Thr Phe Thr Val Phe Ser Ser Ala Ala
   4305                4310                4315                4320
   gcc tcc ttc ggc agt ccc gga cag ggc aac tac acc gcg gcg aac gcc    13008
   Ala Ser Phe Gly Ser Pro Gly Gln Gly Asn Tyr Thr Ala Ala Asn Ala
                   4325                4330                4335
   ttt ctg gac gcc ctg atg cag cac cgc cac acc cag gca ctg ccg ggc    13056
   Phe Leu Asp Ala Leu Met Gln His Arg His Thr Gln Ala Leu Pro Gly
               4340                4345                4350
   cgg tcg ctc gcc tgg ggc ctt tgg ggc gag gcc gac ggc atg acc cgc    13104
   Arg Ser Leu Ala Trp Gly Leu Trp Gly Glu Ala Asp Gly Met Thr Arg
           4355                4360                4365
   aac ctc gcc ggc acc gac ttc gcg cgc atg gcc cgc ggc ggc ctg ctc    13152
   Asn Leu Ala Gly Thr Asp Phe Ala Arg Met Ala Arg Gly Gly Leu Leu
       4370                4375                4380
   ccc ctg tcc aac gca cag gga ctc gcg ctc ctc gac aca gcg gat cgc    13200
   Pro Leu Ser Asn Ala Gln Gly Leu Ala Leu Leu Asp Thr Ala Asp Arg
   4385                 4390                  4395                  4400
   ctc ggc cct ttc ggt gac ggg ctg ctc ctc gcc acc cgg ctc gac gcg      13248
   Leu Gly Pro Phe Gly Asp Gly Leu Leu Leu Ala Thr Arg Leu Asp Ala
                   4405                  4410                  4415
   gcc acc ctc cac gca cag gcc acg gcc ggc gcc ctg ccg cgc atc ctg      13296
   Ala Thr Leu His Ala Gln Ala Thr Ala Gly Ala Leu Pro Arg Ile Leu
               4420                  4425                  4430
   cac ggg ctg atc cgc atc ccg gcc cgg cgg tcc gcc gac cac ggc atc      13344
   His Gly Leu Ile Arg Ile Pro Ala Arg Arg Ser Ala Asp His Gly Ile
           4435                  4440                  4445
   gcg acc gac acc ccc gcc acg ctg cgc gag cgc ctg gcc gga ctc acc      13392
   Ala Thr Asp Thr Pro Ala Thr Leu Arg Glu Arg Leu Ala Gly Leu Thr
       4450                  4455                  4460
   atc ccc gcg cag cgc acc ggt ctc ctc ctg gaa ctc gta cgg acc cat      13440
   Ile Pro Ala Gln Arg Thr Gly Leu Leu Leu Glu Leu Val Arg Thr His
   4465                 4470                  4475                  4480
   gcc gcc gcc gtc ctc ggc cac ccc acc agc gcc gtc aca gcc gcg gac      13488
   Ala Ala Ala Val Leu Gly His Pro Thr Ser Ala Val Thr Ala Ala Asp
                   4485                  4490                  4495
   ggc gca ctc ccg gac gat ctg gtc ccg gcc gac acc gag ttc cgc gac      13536
   Gly Ala Leu Pro Asp Asp Leu Val Pro Ala Asp Thr Glu Phe Arg Asp
               4500                  4505                  4510
   ctc ggc ttc gac tcg ctg acc gcc gtc gaa ctc cgc aac cgg atc aac      13584
   Leu Gly Phe Asp Ser Leu Thr Ala Val Glu Leu Arg Asn Arg Ile Asn
           4515                  4520                  4525
   gcc gtc acc ggc ctg cgc ctc ccg gca acg ctc atc ttc gac cag ccc      13632
   Ala Val Thr Gly Leu Arg Leu Pro Ala Thr Leu Ile Phe Asp Gln Pro
       4530                  4535                  4540
   agc ccc gcg gca ctc gcc gat cac ctc gcg acc cgc ctg acg gcc gag      13680
   Ser Pro Ala Ala Leu Ala Asp His Leu Ala Thr Arg Leu Thr Ala Glu
   4545                 4550                  4555                  4560
   gcg ggc acg ccg gac gag ccg gcc cct gcc gcc gcg gca gcc ggg gcc      13728
   Ala Gly Thr Pro Asp Glu Pro Ala Pro Ala Ala Ala Ala Ala Gly Ala
                   4565                  4570                  4575
   ggg agc gca ggg agt gcc gag acc gga cag cag cgc agt acg ggg agc      13776
   Gly Ser Ala Gly Ser Ala Glu Thr Gly Gln Gln Arg Ser Thr Gly Ser
               4580                  4585                  4590
   gag aag cag cag acc agg ggc ggc acc tcc acc gaa acc gtc gaa tcc      13824
   Glu Lys Gln Gln Thr Arg Gly Gly Thr Ser Thr Glu Thr Val Glu Ser
           4595                  4600                  4605
   ctg ttc tgg atc gga cac gac acc cgc cgc atc gag gag tcc atg gcc      13872
   Leu Phe Trp Ile Gly His Asp Thr Arg Arg Ile Glu Glu Ser Met Ala
       4610                  4615                  4620
   ctg ctc tcg gcg gcc tcc ttc ttc cgg ccc gcc ttc acg gac ccc tcg    13920
   Leu Leu Ser Ala Ala Ser Phe Phe Arg Pro Ala Phe Thr Asp Pro Ser
   4625                4630                4635                4640
   gac atc ccg gag ccg acg ttc gtc cgg ctc gcc cag ggt gaa gcg cgc    13968
   Asp Ile Pro Glu Pro Thr Phe Val Arg Leu Ala Gln Gly Glu Ala Arg
                   4645                4650                4655
   gcc caa ggt gaa gca ctc gcc cgg ggc gaa aca cgg ccc gcc ctc atc    14016
   Ala Gln Gly Glu Ala Leu Ala Arg Gly Glu Thr Arg Pro Ala Leu Ile
               4660                4665                4670
   tgc ctg ccc acc gtc gcc gcc gtg tcg agc gtg tac cag tac tca cgt    14064
   Cys Leu Pro Thr Val Ala Ala Val Ser Ser Val Tyr Gln Tyr Ser Arg
           4675                4680                4685
   ttc gcg gcg gga ctg aac gga cac cga gac gtc tgg tac gtt cct gcg    14112
   Phe Ala Ala Gly Leu Asn Gly His Arg Asp Val Trp Tyr Val Pro Ala
       4690                4695                4700
   cca ggg ttc ctg gag ggc gaa ccc ctg ccg tcc gga atc ggc gcg gtg    14160
   Pro Gly Phe Leu Glu Gly Glu Pro Leu Pro Ser Gly Ile Gly Ala Val
   4705                4710                4715                4720
   acc cgc atg ttc gcc gac gcg atc gtc cgg ttc acc gac ggc gcg cct    14208
   Thr Arg Met Phe Ala Asp Ala Ile Val Arg Phe Thr Asp Gly Ala Pro
                   4725                4730                4735
   ttt gcg ctc gcc ggg cat tcc gcg ggc gga tgg ttc gtc tac gcg gtg    14256
   Phe Ala Leu Ala Gly His Ser Ala Gly Gly Trp Phe Val Tyr Ala Val
               4740                4745                4750
   acg agt cat ctg gag cgt cta ggc gtc cgt ccg gaa gcg gtg gtg acc    14304
   Thr Ser His Leu Glu Arg Leu Gly Val Arg Pro Glu Ala Val Val Thr
           4755                4760                4765
   atg gac gcc tat ctc ccg gac gac ggc atc gca cct gtc gcg tcc gcg    14352
   Met Asp Ala Tyr Leu Pro Asp Asp Gly Ile Ala Pro Val Ala Ser Ala
       4770                4775                4780
   ctg aca agt gaa atc ttc gac cgc gtc acg cag ttt gtg gac gtg gac    14400
   Leu Thr Ser Glu Ile Phe Asp Arg Val Thr Gln Phe Val Asp Val Asp
   4785                4790                4795                4800
   tac aca cgc ctg gtc gcc atg ggc gga tac ttc cgc atc ttc tcc ggc    14448
   Tyr Thr Arg Leu Val Ala Met Gly Gly Tyr Phe Arg Ile Phe Ser Gly
                   4805                4810                4815
   tgg agt cct ccg gac atc acc aca ccc gcc ctc ttc ctg cgc ggc cgg    14496
   Trp Ser Pro Pro Asp Ile Thr Thr Pro Ala Leu Phe Leu Arg Gly Arg
               4820                4825                4830
   gac gga gaa cag atg ccg ccg ccg tgg gga gtt ccg cac acc gtt ctg    14544
   Asp Gly Glu Gln Met Pro Pro Pro Trp Gly Val Pro His Thr Val Leu
           4835                4840                4845
   gac atc cag ggg aat cac ttc acg atg ctg gaa cag ttt gcg gat tcg   14592
   Asp Ile Gln Gly Asn His Phe Thr Met Leu Glu Gln Phe Ala Asp Ser
       4850                4855                4860
   act gct cgg cat gtc gac gaa tgg ctg aca gaa atc gca tca gtg cgg   14640
   Thr Ala Arg His Val Asp Glu Trp Leu Thr Glu Ile Ala Ser Val Arg
   4865                4870                4875                4880
   cgc tgatcgcgcc tctgatcgcg gtcctgatcg cggccctgat cggcgggtcg        14693
   Arg
   ggcacagccc ggtcggccgg tcggccagtc ggccagtcgg tggtatccgg tcggctccgg 14753
   catcgatcag tgctttcccc cttacggcca tacgggcctt tctgagactt cttgaatttg 14813
   ggagacagtg atg gac acg tcc agc gaa aag ctc gtc gac gcg ctt agg    14862
              Met Asp Thr Ser Ser Glu Lys Leu Val Asp Ala Leu Arg
                          4885                4890
   gcg tct ctg aag gcg aac cag acc ctg cgg gca cgt aat gag caa ctg   14910
   Ala Ser Leu Lys Ala Asn Gln Thr Leu Arg Ala Arg Asn Glu Gln Leu
   4895                4900                4905                4910
   gca gcc gcc atg gag gcg tcc agc gag ccg att gcg att gtg ggg atg   14958
   Ala Ala Ala Met Glu Ala Ser Ser Glu Pro Ile Ala Ile Val Gly Met
                   4915                4920                4925
   gcg tgt cgt ttt ccg ggt ggg gtg tgt tcg ccg gag gag ttg tgg gag   15006
   Ala Cys Arg Phe Pro Gly Gly Val Cys Ser Pro Glu Glu Leu Trp Glu
               4930                4935                4940
   ctg gtt gcg tcg ggt ggg gat gcg att ggt gaa ttt ccg gcc ggt cgg   15054
   Leu Val Ala Ser Gly Gly Asp Ala Ile Gly Glu Phe Pro Ala Gly Arg
           4945                4950                4955
   ggg tgg gat ctg gag ggg ttg ttt gat tcg gac cct gac cgg tcg ggg   15102
   Gly Trp Asp Leu Glu Gly Leu Phe Asp Ser Asp Pro Asp Arg Ser Gly
       4960                4965                4970
   acg tcg tac gcg cgg tat ggc ggg ttt ttg tat gag gcg ggg gag ttc   15150
   Thr Ser Tyr Ala Arg Tyr Gly Gly Phe Leu Tyr Glu Ala Gly Glu Phe
   4975                4980                4985                4990
   gat gcg gac ttc ttc ggg atc agt ccg cgt gag gcg ttg gcg atg gat   15198
   Asp Ala Asp Phe Phe Gly Ile Ser Pro Arg Glu Ala Leu Ala Met Asp
                   4995                5000                5005
   ccg cag cag cgg ttg ttg ctg gag acg tcg tgg gag gcg ttc gag cgg   15246
   Pro Gln Gln Arg Leu Leu Leu Glu Thr Ser Trp Glu Ala Phe Glu Arg
               5010                5015                5020
   gcg ggt atc gat ccg ctg tcg atg cgt ggc tcc cgt acg ggt gtc ttc   15294
   Ala Gly Ile Asp Pro Leu Ser Met Arg Gly Ser Arg Thr Gly Val Phe
           5025                5030                5035
   gcc ggg gtg atg tac cac gac tac gga tcc cgc ctg ggt acc atc ccc   15342
   Ala Gly Val Met Tyr His Asp Tyr Gly Ser Arg Leu Gly Thr Ile Pro
   5040 5045 5050
   gag gga ttc gag ggc tac atc ggc aac ggt agc ggc ggc gcc gtc gcg 15390
   Glu Gly Phe Glu Gly Tyr Ile Gly Asn Gly Ser Gly Gly Ala Val Ala
   5055 5060 5065 5070
   tcg ggc cgc gtc gcc tac acg ctc ggt ctc gag ggc cct gcc gtc tcg 15438
   Ser Gly Arg Val Ala Tyr Thr Leu Gly Leu Glu Gly Pro Ala Val Ser
   5075 5080 5085
   gtg gac acg gca tgt tcg tcg tcg ttg gtg gcg ctg cat ctg gcg tgc 15486
   Val Asp Thr Ala Cys Ser Ser Ser Leu Val Ala Leu His Leu Ala Cys
   5090 5095 5100
   cag tcg ctg cgg tcg ggt gag tgc acg ctc gcg ctg gcc ggc ggt gtg 15534
   Gln Ser Leu Arg Ser Gly Glu Cys Thr Leu Ala Leu Ala Gly Gly Val
   5105 5110 5115
   acg gtg atg tcg acc ccg cac ctc ttc gtc gag ttc tca cgc cag cgc 15582
   Thr Val Met Ser Thr Pro His Leu Phe Val Glu Phe Ser Arg Gln Arg
   5120 5125 5130
   gga ctg tcg gtg gac ggc cgc tgc aag tcc ttc gcg ggt gga gcc gac 15630
   Gly Leu Ser Val Asp Gly Arg Cys Lys Ser Phe Ala Gly Gly Ala Asp
   5135 5140 5145 5150
   ggc acc ggc atg ggc gag ggc gtc ggg atg ctg ttg gtg gag cgg ttg 15678
   Gly Thr Gly Met Gly Glu Gly Val Gly Met Leu Leu Val Glu Arg Leu
   5155 5160 5165
   tcg gat gcg gtg cgg ctg ggg cat cgg gtg ctg gcg gtg ctg cgc ggc 15726
   Ser Asp Ala Val Arg Leu Gly His Arg Val Leu Ala Val Leu Arg Gly
   5170 5175 5180
   agt gcg gtc aat cag gac ggt gcg tcg aat ggg ttg acg gcg ccg aat 15774
   Ser Ala Val Asn Gln Asp Gly Ala Ser Asn Gly Leu Thr Ala Pro Asn
   5185 5190 5195
   ggt ccg gct cag gag cgg gtg atc cgg cag gcg ttg gcg aac gcg ggg 15822
   Gly Pro Ala Gln Glu Arg Val Ile Arg Gln Ala Leu Ala Asn Ala Gly
   5200 5205 5210
   ttg tcc gtg gcg gat gtg gat gtg gtg gag ggg cat ggg acg ggc acg 15870
   Leu Ser Val Ala Asp Val Asp Val Val Glu Gly His Gly Thr Gly Thr
   5215 5220 5225 5230
   acg ctg ggt gat ccg atc gag gcg cag gcg ttg ctc gcc acg tac ggg 15918
   Thr Leu Gly Asp Pro Ile Glu Ala Gln Ala Leu Leu Ala Thr Tyr Gly
   5235 5240 5245
   cag cgg gcc ggt aac agg ccg ctg tgg ctg gga tcg gtg aag tcg aac 15966
   Gln Arg Ala Gly Asn Arg Pro Leu Trp Leu Gly Ser Val Lys Ser Asn
   5250 5255 5260
   atc ggc cat gcg cag gct gcc gcg ggt gtg ggt ggg gtc atc aag atg 16014
   Ile Gly His Ala Gln Ala Ala Ala Gly Val Gly Gly Val Ile Lys Met
   5265 5270 5275
   gtg atg gcg ttg cgg gag ggg gtg ttg ccg cgg acg ttg cat gtg gat     16062
   Val Met Ala Leu Arg Glu Gly Val Leu Pro Arg Thr Leu His Val Asp
       5280                5285                5290
   gag ccg tcg ccg cag gtg gac tgg tcc gcg ggg gcg gtg cgg ctg ctg     16110
   Glu Pro Ser Pro Gln Val Asp Trp Ser Ala Gly Ala Val Arg Leu Leu
   5295                5300                5305                5310
   acg gag gcg gtg ccg tgg ccg ggg gac gcg gca ggg cgg ttg cgg cgg     16158
   Thr Glu Ala Val Pro Trp Pro Gly Asp Ala Ala Gly Arg Leu Arg Arg
                   5315                5320                5325
   gcg gga gtg tcg tcg ttc ggg gtc agt ggc acg aat gcg cat gtg att     16206
   Ala Gly Val Ser Ser Phe Gly Val Ser Gly Thr Asn Ala His Val Ile
               5330                5335                5340
   ttg gag gag gcg ccg gcg gcg ggg ggc tgt gtt gcc ggg ggt ggg gtg     16254
   Leu Glu Glu Ala Pro Ala Ala Gly Gly Cys Val Ala Gly Gly Gly Val
           5345                5350                5355
   ttg gag ggt gct ccg ggt ctt gcc att tcg gtg gct gag tcg gtg gcc     16302
   Leu Glu Gly Ala Pro Gly Leu Ala Ile Ser Val Ala Glu Ser Val Ala
       5360                5365                5370
   gct cca gtg gct gtg tct gcg ccg gtg gct gag tcg gtg ccg gtg ccg     16350
   Ala Pro Val Ala Val Ser Ala Pro Val Ala Glu Ser Val Pro Val Pro
   5375                5380                5385                5390
   gtg ccg gtg ccg gtt cct gtg ccg gtg tcg gct agg tct gag gct ggg     16398
   Val Pro Val Pro Val Pro Val Pro Val Ser Ala Arg Ser Glu Ala Gly
                   5395                5400                5405
   ttg cgg gcg cag gcg gag gcg ttg cgt cag tac gtg gca gtc cgg ccg     16446
   Leu Arg Ala Gln Ala Glu Ala Leu Arg Gln Tyr Val Ala Val Arg Pro
               5410                5415                5420
   gac gtt tcg ctt gcc gat gtg ggt gcg ggt ctg gcc tgt ggg cgg gct     16494
   Asp Val Ser Leu Ala Asp Val Gly Ala Gly Leu Ala Cys Gly Arg Ala
           5425                5430                5435
   gtg ctg gag cat cgt gcg gtc gtc ctg gcc gcg gac cgt gag gag ctg     16542
   Val Leu Glu His Arg Ala Val Val Leu Ala Ala Asp Arg Glu Glu Leu
       5440                5445                5450
   gtg caa ggg ttg ggg gcg ctg gcg gcg ggt gag ccg gat cgg cgg gtg     16590
   Val Gln Gly Leu Gly Ala Leu Ala Ala Gly Glu Pro Asp Arg Arg Val
   5455                5460                5465                5470
   acc acg ggt cat gcg ccg ggt ggt gac cgg ggc ggt gtc gtc ttc gtg     16638
   Thr Thr Gly His Ala Pro Gly Gly Asp Arg Gly Gly Val Val Phe Val
                   5475                5480                5485
   ttt ccc gga cag ggt ggg cag tgg gcc ggg atg ggt gtg cgt ctg ctc     16686
   Phe Pro Gly Gln Gly Gly Gln Trp Ala Gly Met Gly Val Arg Leu Leu
               5490                5495                5500
   gcc tcc tct ccg gtg ttc gcc cgg cgg atg cag gcg tgc gag gag gct 16734
   Ala Ser Ser Pro Val Phe Ala Arg Arg Met Gln Ala Cys Glu Glu Ala
   5505 5510 5515
   ctg gcg ccg tgg gtg gac tgg tct gtg gtg gac atc ctg cgc cgg gac 16782
   Leu Ala Pro Trp Val Asp Trp Ser Val Val Asp Ile Leu Arg Arg Asp
   5520 5525 5530
   gcg ggg gat gcg gtg tgg gag cgg gcc gat gtg gtc cag cct gtg ctg 16830
   Ala Gly Asp Ala Val Trp Glu Arg Ala Asp Val Val Gln Pro Val Leu
   5535 5540 5545 5550
   ttc agc gtc atg gtg tct ttg gct gct ctg tgg cgt tcc tac ggt atc 16878
   Phe Ser Val Met Val Ser Leu Ala Ala Leu Trp Arg Ser Tyr Gly Ile
   5555 5560 5565
   gaa ccc gac gcg gtc ctt ggc cat tcc cag ggc gag atc gcg gcc gcg 16926
   Glu Pro Asp Ala Val Leu Gly His Ser Gln Gly Glu Ile Ala Ala Ala
   5570 5575 5580
   cat gtg tgt ggg gcg ctg agc ctg aag gac gcg gcg aag act gtt gcg 16974
   His Val Cys Gly Ala Leu Ser Leu Lys Asp Ala Ala Lys Thr Val Ala
   5585 5590 5595
   ctg cgc agc cgg gcg ctg gcc gct gtg cgg ggc cgg ggc ggc atg gcc 17022
   Leu Arg Ser Arg Ala Leu Ala Ala Val Arg Gly Arg Gly Gly Met Ala
   5600 5605 5610
   tca gtg ccg ctg cct gcc cag gag gtg gag cag ctc att ggt gag cgg 17070
   Ser Val Pro Leu Pro Ala Gln Glu Val Glu Gln Leu Ile Gly Glu Arg
   5615 5620 5625 5630
   tgg gcg ggg cgg ttg tgg gtg gcg gcg gtc aac ggc ccc cgc tcc acc 17118
   Trp Ala Gly Arg Leu Trp Val Ala Ala Val Asn Gly Pro Arg Ser Thr
   5635 5640 5645
   gcc gtc tcg ggg gat gcc gag gcg gtg gac gag gtg ctg gcg tac tgt 17166
   Ala Val Ser Gly Asp Ala Glu Ala Val Asp Glu Val Leu Ala Tyr Cys
   5650 5655 5660
   gcc ggc acc ggg gtg cgg gcc cgg cgg atc ccg gtc gac tat gcc tcg 17214
   Ala Gly Thr Gly Val Arg Ala Arg Arg Ile Pro Val Asp Tyr Ala Ser
   5665 5670 5675
   cac tgc ccc cat gtg cag ccc ctg cgg gag gag ttg ctg gag ctg ctg 17262
   His Cys Pro His Val Gln Pro Leu Arg Glu Glu Leu Leu Glu Leu Leu
   5680 5685 5690
   ggg gac atc agc ccg cag ccg tcc ggc gtg ccg ttc ttc tcc acg gtg 17310
   Gly Asp Ile Ser Pro Gln Pro Ser Gly Val Pro Phe Phe Ser Thr Val
   5695 5700 5705 5710
   gag ggc acc tgg ctg gac acc aca acc ctg gac gcc gcc tac tgg tac 17358
   Glu Gly Thr Trp Leu Asp Thr Thr Thr Leu Asp Ala Ala Tyr Trp Tyr
   5715 5720 5725
   cgc aac ctg cac cag cct gtc cgt ttc agc gat gcc gtc cag gcc ctg 17406
   Arg Asn Leu His Gln Pro Val Arg Phe Ser Asp Ala Val Gln Ala Leu
               5730                5735                5740
   gcg gat gac gga cac cgc gtc ttc gtc gaa gtc agc ccc cac ccc acc     17454
   Ala Asp Asp Gly His Arg Val Phe Val Glu Val Ser Pro His Pro Thr
           5745                5750                5755
   ctc gtc ccc gcc atc gaa gac acc acc gaa gac acc gcc gaa gac gtc     17502
   Leu Val Pro Ala Ile Glu Asp Thr Thr Glu Asp Thr Ala Glu Asp Val
       5760                5765                5770
   acc gcg atc ggc agc ctc cgc cgc ggc gac aac gac acc cgc cgc ttc     17550
   Thr Ala Ile Gly Ser Leu Arg Arg Gly Asp Asn Asp Thr Arg Arg Phe
   5775                5780                5785                5790
   ctc acc gcc ctc gcc cac acc cac acc acc ggc atc ggc aca ccc acc     17598
   Leu Thr Ala Leu Ala His Thr His Thr Thr Gly Ile Gly Thr Pro Thr
                   5795                5800                5805
   acc tgg cac cac cac tac acc cac cac cac acc cac ccc cac aac cac     17646
   Thr Trp His His His Tyr Thr His His His Thr His Pro His Asn His
               5810                5815                5820
   cac ctc gac ctc ccc act tat ccc ttc caa cgc cag cac tac tgg ctc     17694
   His Leu Asp Leu Pro Thr Tyr Pro Phe Gln Arg Gln His Tyr Trp Leu
           5825                5830                5835
   gac gct ccc acg gga gca ggt gac gtc gcc gct gct ggc ttg gag ccg     17742
   Asp Ala Pro Thr Gly Ala Gly Asp Val Ala Ala Ala Gly Leu Glu Pro
       5840                5845                5850
   gcc gaa cac cct ctg ctc gcg gca aca gtc caa ctc gca gac acg gac     17790
   Ala Glu His Pro Leu Leu Ala Ala Thr Val Gln Leu Ala Asp Thr Asp
   5855                5860                5865                5870
   ggc tgc cta ctg acg ggt cgc ctg tcc ttg cgc tcg cat ccg tgg ctg     17838
   Gly Cys Leu Leu Thr Gly Arg Leu Ser Leu Arg Ser His Pro Trp Leu
                   5875                5880                5885
   ggc gat tac gag gtg ggg ggt gcg gtc ctg ctg tcg ggg tcg gcg ttc     17886
   Gly Asp Tyr Glu Val Gly Gly Ala Val Leu Leu Ser Gly Ser Ala Phe
               5890                5895                5900
   gtg gag ctg gcg gtc cag gtt ggc gaa cgc gtg ggc tgc acc cga atc     17934
   Val Glu Leu Ala Val Gln Val Gly Glu Arg Val Gly Cys Thr Arg Ile
           5905                5910                5915
   gag caa ctc act gtg cat gcg ccg ctg gtg gtt cct gtg ggt ggg ggt     17982
   Glu Gln Leu Thr Val His Ala Pro Leu Val Val Pro Val Gly Gly Gly
       5920                5925                5930
   gtg agt gtg cag gtt ggg gtt gcg gct gcg gat ggg gag ggg cgg cgt     18030
   Val Ser Val Gln Val Gly Val Ala Ala Ala Asp Gly Glu Gly Arg Arg
   5935                5940                5945                5950
   ttg gtg agt gtg tat gcg cgg ggt ggg agt gct tgt ggt ggg ggt ggt     18078
   Leu Val Ser Val Tyr Ala Arg Gly Gly Ser Ala Cys Gly Gly Gly Gly
   5955 5960 5965
   gcg tcg ggt ggg gtg tgg acg tgt cat gcc tcg ggg gtg ctg gtt gag 18126
   Ala Ser Gly Gly Val Trp Thr Cys His Ala Ser Gly Val Leu Val Glu
   5970 5975 5980
   gct gct gct ggt ggt ggt gtg gtg gtg gat ggt ctg gcg ggg gtg tgg 18174
   Ala Ala Ala Gly Gly Gly Val Val Val Asp Gly Leu Ala Gly Val Trp
   5985 5990 5995
   ccg ccg cgg ggt gcg gtg gcg gtg gat gtc gat ggt gtc cgt gac cgt 18222
   Pro Pro Arg Gly Ala Val Ala Val Asp Val Asp Gly Val Arg Asp Arg
   6000 6005 6010
   ttg gct ggg gct ggt tgt gtt ttg ggg ccg gtg ttt tcg ggg ctg cgt 18270
   Leu Ala Gly Ala Gly Cys Val Leu Gly Pro Val Phe Ser Gly Leu Arg
   6015 6020 6025 6030
   gcg gtg tgg cgt gat ggg ggg gat ttg ctg gct gag gtg tgt ctg ccg 18318
   Ala Val Trp Arg Asp Gly Gly Asp Leu Leu Ala Glu Val Cys Leu Pro
   6035 6040 6045
   gag gag gcg tgg ggt gat gcg gct ggt ttt ggg ctg cat ccg gcg ttg 18366
   Glu Glu Ala Trp Gly Asp Ala Ala Gly Phe Gly Leu His Pro Ala Leu
   6050 6055 6060
   ctg gat ggt gtg gtc cag ccg ttg tcg gtg ttg ctt ccg ggt ggg acg 18414
   Leu Asp Gly Val Val Gln Pro Leu Ser Val Leu Leu Pro Gly Gly Thr
   6065 6070 6075
   ggg ttt ggg gag ggg gcg ggg ttc ggg gag ggt gtt cgg gtg ccg gct 18462
   Gly Phe Gly Glu Gly Ala Gly Phe Gly Glu Gly Val Arg Val Pro Ala
   6080 6085 6090
   gtg tgg ggt ggt gtg tcg ctt cac cgg gcg ggt gtg acc ggt gtg cgg 18510
   Val Trp Gly Gly Val Ser Leu His Arg Ala Gly Val Thr Gly Val Arg
   6095 6100 6105 6110
   gtg cgt gtg tgg gct gta ggg cgg ggc ggc ggg cgt gag gcg gtg tcg 18558
   Val Arg Val Trp Ala Val Gly Arg Gly Gly Gly Arg Glu Ala Val Ser
   6115 6120 6125
   gtc gtg gtc ggg gat gag gcg ggt gtg ccg gtg gcg tcg gtc gat cgt 18606
   Val Val Val Gly Asp Glu Ala Gly Val Pro Val Ala Ser Val Asp Arg
   6130 6135 6140
   ctt gag ttg cgg cct gtg gat atg ggt cag ttg cgt gct gtc tcg gtt 18654
   Leu Glu Leu Arg Pro Val Asp Met Gly Gln Leu Arg Ala Val Ser Val
   6145 6150 6155
   tcg gcg ggg cgg cgg ggt tcg ctg tat gcg gtg cag tgg gct gag gtg 18702
   Ser Ala Gly Arg Arg Gly Ser Leu Tyr Ala Val Gln Trp Ala Glu Val
   6160 6165 6170
   ggt cct gtg ccg gtg tgt ggg cag gcg tgg gcg tgg cac gag gac gtg 18750
   Gly Pro Val Pro Val Cys Gly Gln Ala Trp Ala Trp His Glu Asp Val
   6175 6180 6185 6190
   ggt gag agc ggt ggt ggg cct gtg ccg ggg gtg gtg gtg ttg cgg tgc    18798
   Gly Glu Ser Gly Gly Gly Pro Val Pro Gly Val Val Val Leu Arg Cys
                   6195                6200                6205
   ccg gat gcc ggt gcc ggt ggc ggc ggt ggc ggt ggt gtg ggt gag gtt    18846
   Pro Asp Ala Gly Ala Gly Gly Gly Gly Gly Gly Gly Val Gly Glu Val
               6210                6215                6220
   gtt ggt ggg gtg ttg ggt gtg gtg cag ggg tgg ctg ggg ctg gag cgg    18894
   Val Gly Gly Val Leu Gly Val Val Gln Gly Trp Leu Gly Leu Glu Arg
           6225                6230                6235
   ttt gcg ggt tcg cgg ctg gtg gtg gtg acc cgg ggt gcg gtg gtg gcc    18942
   Phe Ala Gly Ser Arg Leu Val Val Val Thr Arg Gly Ala Val Val Ala
       6240                6245                6250
   ggc caa gaa gac ggc ccg gtg gat gtg gtg ggt gcg gcg gtg tgg ggg    18990
   Gly Gln Glu Asp Gly Pro Val Asp Val Val Gly Ala Ala Val Trp Gly
   6255                6260                6265                6270
   ctg gtg cgg tcg gcg cag gct gag cat ccg gac cgg ttt gtc ctc ctc    19038
   Leu Val Arg Ser Ala Gln Ala Glu His Pro Asp Arg Phe Val Leu Leu
                   6275                6280                6285
   gac ctc gac acc gac acc gac acc ggc acc gac ctc gac acc ggt gct    19086
   Asp Leu Asp Thr Asp Thr Asp Thr Gly Thr Asp Leu Asp Thr Gly Ala
               6290                6295                6300
   ggt gct ggt gct ggt gct ggt tgg ggc gtg gat ggt ggg cat gtg gcg    19134
   Gly Ala Gly Ala Gly Ala Gly Trp Gly Val Asp Gly Gly His Val Ala
           6305                6310                6315
   gcg gtg gtg gcg tgt ggt gag ccg cag ttg gcg gtg cgt ggt gag cgg    19182
   Ala Val Val Ala Cys Gly Glu Pro Gln Leu Ala Val Arg Gly Glu Arg
       6320                6325                6330
   gtg ctg gcc gca cgc ctg acg cga ctt gag tcg tcc gtt gat gta cct    19230
   Val Leu Ala Ala Arg Leu Thr Arg Leu Glu Ser Ser Val Asp Val Pro
   6335                6340                6345                6350
   gct cag cgg tcc ggt gat gtt gct ggt cgg gag gtg ttg ccg tgg ttg    19278
   Ala Gln Arg Ser Gly Asp Val Ala Gly Arg Glu Val Leu Pro Trp Leu
                   6355                6360                6365
   tcg ggt ggg tcg gtg ttg gtg acg ggt ggg acg ggt gtg ctg ggt gcg    19326
   Ser Gly Gly Ser Val Leu Val Thr Gly Gly Thr Gly Val Leu Gly Ala
               6370                6375                6380
   gcg gtg gcg cgg cat ctg gct ggt gtg tgt ggg gtg cgg gat ctg ctg    19374
   Ala Val Ala Arg His Leu Ala Gly Val Cys Gly Val Arg Asp Leu Leu
           6385                6390                6395
   ttg gtg agc cgg cgt ggt ccg gat gct ccg ggt gcg gag ggt ttg cgg    19422
   Leu Val Ser Arg Arg Gly Pro Asp Ala Pro Gly Ala Glu Gly Leu Arg
       6400                6405                6410
   gcg gag ctg gcc gcg ttg ggg gcg gag gtg cgg att gtt gcg tgt gat    19470
   Ala Glu Leu Ala Ala Leu Gly Ala Glu Val Arg Ile Val Ala Cys Asp
   6415                6420                6425                6430
   gtg ggg gag cgg cgg gag gtg gtc cgg ctg ctg gag ggt gtt cct gcc    19518
   Val Gly Glu Arg Arg Glu Val Val Arg Leu Leu Glu Gly Val Pro Ala
               6435                6440                6445
   ggg tgt ccg ctg acg ggt gtc gtg cat gcg gct ggt gtg ctg gac gat    19566
   Gly Cys Pro Leu Thr Gly Val Val His Ala Ala Gly Val Leu Asp Asp
           6450                6455                6460
   gcg acg atc gcc tct ctc acg ccc gag cgg ctg ggc acg gtg ttc gcg    19614
   Ala Thr Ile Ala Ser Leu Thr Pro Glu Arg Leu Gly Thr Val Phe Ala
       6465                6470                6475
   gcc aag gtg gat gcc gct ctt ttg ctg gat gag ctg acg cgg ggt atg    19662
   Ala Lys Val Asp Ala Ala Leu Leu Leu Asp Glu Leu Thr Arg Gly Met
   6480                6485                6490
   gag ctg tcg gcg ttc gtg ctg ttc tcc tcg gcc gcg ggg atc ctg ggg    19710
   Glu Leu Ser Ala Phe Val Leu Phe Ser Ser Ala Ala Gly Ile Leu Gly
   6495                6500                6505                6510
   tcg gcc ggg cag ggc aac tac gcc gcg gcc aat gcc gct ctg gac gcg    19758
   Ser Ala Gly Gln Gly Asn Tyr Ala Ala Ala Asn Ala Ala Leu Asp Ala
               6515                6520                6525
   ctg gcg tac cgg cgg cgg gcg gcg ggt ctg ccg ggg gtg tcg ctg gcg    19806
   Leu Ala Tyr Arg Arg Arg Ala Ala Gly Leu Pro Gly Val Ser Leu Ala
           6530                6535                6540
   tgg ggg ctg tgg gaa gag gcc agc ggg atg acc ggg cac ctg gcc ggc    19854
   Trp Gly Leu Trp Glu Glu Ala Ser Gly Met Thr Gly His Leu Ala Gly
       6545                6550                6555
   acc gac cac cgg cgc atc atc cgt tcc ggt ctg cat ccc atg tcg acc    19902
   Thr Asp His Arg Arg Ile Ile Arg Ser Gly Leu His Pro Met Ser Thr
   6560                6565                6570
   ccg gac gca ctg gct ctc ttc gat gcg gcc ctg gct ctg gac cgg ccg    19950
   Pro Asp Ala Leu Ala Leu Phe Asp Ala Ala Leu Ala Leu Asp Arg Pro
   6575                6580                6585                6590
   gtc ctg ctg ccc gcc gac ctg cgt ccc gcc ccg ccc ctg ccg ccc ctg    19998
   Val Leu Leu Pro Ala Asp Leu Arg Pro Ala Pro Pro Leu Pro Pro Leu
               6595                6600                6605
   ctg cag gac ctc ctg ccc gcc acc cgc cgc cgc acc acc cgc acc acc    20046
   Leu Gln Asp Leu Leu Pro Ala Thr Arg Arg Arg Thr Thr Arg Thr Thr
           6610                6615                6620
   act acc ggt ggt gcg gac aac ggc gcc cag ctg cat gcc cgg ctg gcc    20094
   Thr Thr Gly Gly Ala Asp Asn Gly Ala Gln Leu His Ala Arg Leu Ala
       6625                6630                6635
   ggc cag aca cac gaa caa cag cac acc acc ctc ctc gcc ctg gtc cgc    20142
   Gly Gln Thr His Glu Gln Gln His Thr Thr Leu Leu Ala Leu Val Arg
   6640 6645 6650
   tcc cac atc gcc acc gtc ctc ggc cac acc acc ccc gac acc atc ccc 20190
   Ser His Ile Ala Thr Val Leu Gly His Thr Thr Pro Asp Thr Ile Pro
   6655 6660 6665 6670
   ccc gac cgc gcg ttc cgc gac ctc ggc ttc gac tcc ctc acc gcc gtc 20238
   Pro Asp Arg Ala Phe Arg Asp Leu Gly Phe Asp Ser Leu Thr Ala Val
   6675 6680 6685
   gaa cta cgc aac cgg ctc tcc cgc acc acc gga ctc cgc ctc ccc acc 20286
   Glu Leu Arg Asn Arg Leu Ser Arg Thr Thr Gly Leu Arg Leu Pro Thr
   6690 6695 6700
   acc ctc gcc ttc gac cac ccc aac ccc acc acc ctc acc cac cac ctc 20334
   Thr Leu Ala Phe Asp His Pro Asn Pro Thr Thr Leu Thr His His Leu
   6705 6710 6715
   cac aca caa ctt ctg ggc tcg gac agc act gcc tcc atc cca gct ccc 20382
   His Thr Gln Leu Leu Gly Ser Asp Ser Thr Ala Ser Ile Pro Ala Pro
   6720 6725 6730
   cgt gct gcg gct gtg cct gca gac cag gac gag ccc gtc gcg atc att 20430
   Arg Ala Ala Ala Val Pro Ala Asp Gln Asp Glu Pro Val Ala Ile Ile
   6735 6740 6745 6750
   ggc atg gcg tgc cgc tat ccc gga ggc gtc acc tca gcc gag gag ctg 20478
   Gly Met Ala Cys Arg Tyr Pro Gly Gly Val Thr Ser Ala Glu Glu Leu
   6755 6760 6765
   tgg gaa ctg ctc gca tcg ggg agg gac acg gtc ggc gag ttt ccg acg 20526
   Trp Glu Leu Leu Ala Ser Gly Arg Asp Thr Val Gly Glu Phe Pro Thr
   6770 6775 6780
   gac cgt ggg tgg gac ctg gaa gca ctg ttc gat ccg gaa ccg ggt cgg 20574
   Asp Arg Gly Trp Asp Leu Glu Ala Leu Phe Asp Pro Glu Pro Gly Arg
   6785 6790 6795
   ccg ggc acc tcg tac acc cgc tgt ggg agt ttc ctc tac gac gcg ggg 20622
   Pro Gly Thr Ser Tyr Thr Arg Cys Gly Ser Phe Leu Tyr Asp Ala Gly
   6800 6805 6810
   gag ttc gac gcc ggc ttc ttc ggg atc agt ccg cgt gag gca ctg gcg 20670
   Glu Phe Asp Ala Gly Phe Phe Gly Ile Ser Pro Arg Glu Ala Leu Ala
   6815 6820 6825 6830
   atg gac ccg cag cag cga ttg ctg ctg gag gcc tca tgg gag gcc atg 20718
   Met Asp Pro Gln Gln Arg Leu Leu Leu Glu Ala Ser Trp Glu Ala Met
   6835 6840 6845
   gag cag gca ggt att gac cct acg acc gta cgc ggg agc cag aca ggc 20766
   Glu Gln Ala Gly Ile Asp Pro Thr Thr Val Arg Gly Ser Gln Thr Gly
   6850 6855 6860
   gtg ttc gcg ggc ctc att ccg cag gcc tat gga ccc agg ctg cac gaa 20814
   Val Phe Ala Gly Leu Ile Pro Gln Ala Tyr Gly Pro Arg Leu His Glu
   6865 6870 6875
   aac gcc gca gcc gac acc gag ggc tat gtc ctg acc ggc aca tcc ggg 20862
   Asn Ala Ala Ala Asp Thr Glu Gly Tyr Val Leu Thr Gly Thr Ser Gly
   6880 6885 6890
   agt gtg gcc tcc ggt cgt atc tcg tac acg ttt ggt ttt gag ggt cct 20910
   Ser Val Ala Ser Gly Arg Ile Ser Tyr Thr Phe Gly Phe Glu Gly Pro
   6895 6900 6905 6910
   gcg gtg tcg gtg gac acg gct tgt tcc tcg tcg ttg gtg gct tta cat 20958
   Ala Val Ser Val Asp Thr Ala Cys Ser Ser Ser Leu Val Ala Leu His
   6915 6920 6925
   ctg gcc tgt cag gcg ttg cgt gcg ggt gag tgc tcg atg gcg ctt gcc 21006
   Leu Ala Cys Gln Ala Leu Arg Ala Gly Glu Cys Ser Met Ala Leu Ala
   6930 6935 6940
   ggg ggt gtg acg gtg atg tcg tct ccg ggt gcc ttc gtg gag ttt tcg 21054
   Gly Gly Val Thr Val Met Ser Ser Pro Gly Ala Phe Val Glu Phe Ser
   6945 6950 6955
   cgg cag cgg ggt ctg gcc gcg gac ggg cat tgc aag gcg ttc tcg gcg 21102
   Arg Gln Arg Gly Leu Ala Ala Asp Gly His Cys Lys Ala Phe Ser Ala
   6960 6965 6970
   gcg gcg gac ggg acc ggc tgg ggt gag ggt gtg ggg atg ctg ctg gtg 21150
   Ala Ala Asp Gly Thr Gly Trp Gly Glu Gly Val Gly Met Leu Leu Val
   6975 6980 6985 6990
   gag cgg ctc tcc gac gcc cgt cgc aac ggt cac cgt gtc ctg gcc gtg 21198
   Glu Arg Leu Ser Asp Ala Arg Arg Asn Gly His Arg Val Leu Ala Val
   6995 7000 7005
   gtg cgt ggc agt gcg gtc aac cag gac ggt gcg agc aac ggg ctg acc 21246
   Val Arg Gly Ser Ala Val Asn Gln Asp Gly Ala Ser Asn Gly Leu Thr
   7010 7015 7020
   gcg ccc aac ggg ccc tcc cag cag cgt gtc atc cgc cag gcc ctc gcc 21294
   Ala Pro Asn Gly Pro Ser Gln Gln Arg Val Ile Arg Gln Ala Leu Ala
   7025 7030 7035
   aac gcc ggc ttg tcg gcc ggt gat gtc gat gcg gtg gag gcc cac ggc 21342
   Asn Ala Gly Leu Ser Ala Gly Asp Val Asp Ala Val Glu Ala His Gly
   7040 7045 7050
   acc ggc acc act ttg ggc gac ccg atc gag gcc cag gcc ctc ctt gcg 21390
   Thr Gly Thr Thr Leu Gly Asp Pro Ile Glu Ala Gln Ala Leu Leu Ala
   7055 7060 7065 7070
   acc tac ggg cag gac cgt gcc ggc gag ggg ccg ctg tgg ctg ggc tcg 21438
   Thr Tyr Gly Gln Asp Arg Ala Gly Glu Gly Pro Leu Trp Leu Gly Ser
   7075 7080 7085
   gtc aag tcc aat gtc ggt cac aca cag gct gcc gcg ggc gtc gcc ggg 21486
   Val Lys Ser Asn Val Gly His Thr Gln Ala Ala Ala Gly Val Ala Gly
   7090 7095 7100
   gtg atc aag atg gtg atg gcg ctg cgg aat ggt ctg ctg ccg cgg acg    21534
   Val Ile Lys Met Val Met Ala Leu Arg Asn Gly Leu Leu Pro Arg Thr
           7105                7110                7115
   ttg cat gtg gat gag ccg tcg ccg cat gtg gac tgg tcc gcg ggt gcg    21582
   Leu His Val Asp Glu Pro Ser Pro His Val Asp Trp Ser Ala Gly Ala
       7120                7125                7130
   gtg cag ctg ctg acg gag acg gtg ccc tgg ccc ggc ggg gag ggg cgg    21630
   Val Gln Leu Leu Thr Glu Thr Val Pro Trp Pro Gly Gly Glu Gly Arg
   7135                7140                7145                7150
   cta cgg cgg gca gga gtg tca tca ttc ggc gtc agc ggc acc aac gcc    21678
   Leu Arg Arg Ala Gly Val Ser Ser Phe Gly Val Ser Gly Thr Asn Ala
                   7155                7160                7165
   cac gtc atc ctc gaa gaa gca ccc gcc cac aac atc ccg tca gac aca    21726
   His Val Ile Leu Glu Glu Ala Pro Ala His Asn Ile Pro Ser Asp Thr
               7170                7175                7180
   ccc gcc gac gac gtt ccg ggg gga cca ccc gcc ggc gag gat gcc ggt    21774
   Pro Ala Asp Asp Val Pro Gly Gly Pro Pro Ala Gly Glu Asp Ala Gly
           7185                7190                7195
   agt ggc gag gag gct gct gcc ggc agt cca ggg gtg tgg ccg tgg ctg    21822
   Ser Gly Glu Glu Ala Ala Ala Gly Ser Pro Gly Val Trp Pro Trp Leu
       7200                7205                7210
   gtg tcg gcc aag tcg cag ccg gcc ctg cgc gcc cag gcc cag gcc ctg    21870
   Val Ser Ala Lys Ser Gln Pro Ala Leu Arg Ala Gln Ala Gln Ala Leu
   7215                7220                7225                7230
   cac gcc cac ctc acc gac cac ccc ggc ctc gac ctc gcc gac gtc gga    21918
   His Ala His Leu Thr Asp His Pro Gly Leu Asp Leu Ala Asp Val Gly
                   7235                7240                7245
   tac acc ctc gcc cac gcc cgc gcc gtg ttc gac cac cgc gcc acc ctc    21966
   Tyr Thr Leu Ala His Ala Arg Ala Val Phe Asp His Arg Ala Thr Leu
               7250                7255                7260
   atc gcc gcc gac cgc gac acc ttc ctg caa gca ctc cag gca ctc gcc    22014
   Ile Ala Ala Asp Arg Asp Thr Phe Leu Gln Ala Leu Gln Ala Leu Ala
           7265                7270                7275
   gca ggc gaa ccc cac ccc gcc gtc atc cac agc agc gcc cca ggc ggg    22062
   Ala Gly Glu Pro His Pro Ala Val Ile His Ser Ser Ala Pro Gly Gly
       7280                7285                7290
   acc ggg acc ggg gag gcc gca gga aag acc gca ttc atc tgc tcc gga    22110
   Thr Gly Thr Gly Glu Ala Ala Gly Lys Thr Ala Phe Ile Cys Ser Gly
   7295                7300                7305                7310
   cag ggc acc caa cgc ccc ggc atg gcc cac ggc ctc tac cac acc cac    22158
   Gln Gly Thr Gln Arg Pro Gly Met Ala His Gly Leu Tyr His Thr His
                   7315                7320                7325
   ccc gtc ttc gcc gcc gca ctc aac gac atc tgc acc cac ctc gac ccc 22206
   Pro Val Phe Ala Ala Ala Leu Asn Asp Ile Cys Thr His Leu Asp Pro
   7330 7335 7340
   cac ctc gac cac ccc ctc ctc ccc ctc ctc acc cag gac ccc aac acc 22254
   His Leu Asp His Pro Leu Leu Pro Leu Leu Thr Gln Asp Pro Asn Thr
   7345 7350 7355
   cag gac acc acc acc ctc gaa gaa gcg gcc gca ctg ctc cag cag acc 22302
   Gln Asp Thr Thr Thr Leu Glu Glu Ala Ala Ala Leu Leu Gln Gln Thr
   7360 7365 7370
   ccg tac gcc cag ccc gcc ctc ttc gcc ttc cag gtc gcc ctc cac cgc 22350
   Pro Tyr Ala Gln Pro Ala Leu Phe Ala Phe Gln Val Ala Leu His Arg
   7375 7380 7385 7390
   ctc ctc acc gac ggc tac cac atc acc ccc cac tac tac gcc gga cac 22398
   Leu Leu Thr Asp Gly Tyr His Ile Thr Pro His Tyr Tyr Ala Gly His
   7395 7400 7405
   tcc ctc ggc gaa atc acc gcc gcc cac ctc gcc ggc atc ctc acc ctc 22446
   Ser Leu Gly Glu Ile Thr Ala Ala His Leu Ala Gly Ile Leu Thr Leu
   7410 7415 7420
   acc gac gcc acc acc ctc atc acc caa cgc gcc acc ctc atg caa acc 22494
   Thr Asp Ala Thr Thr Leu Ile Thr Gln Arg Ala Thr Leu Met Gln Thr
   7425 7430 7435
   atg ccc ccc ggc acc atg acc acc ctc cac acc acc ccc cac cac atc 22542
   Met Pro Pro Gly Thr Met Thr Thr Leu His Thr Thr Pro His His Ile
   7440 7445 7450
   acc cac cac atc acc gcc cac gaa aac gac ctc gcc atc gcc gcc atc 22590
   Thr His His Ile Thr Ala His Glu Asn Asp Leu Ala Ile Ala Ala Ile
   7455 7460 7465 7470
   aac acc ccc acc tcc ctc gtc atc agc ggc acc ccc cac acc gtc caa 22638
   Asn Thr Pro Thr Ser Leu Val Ile Ser Gly Thr Pro His Thr Val Gln
   7475 7480 7485
   cac atc acc acc ctc tgc caa caa caa ggc atc aaa acc aaa acc ctc 22686
   His Ile Thr Thr Leu Cys Gln Gln Gln Gly Ile Lys Thr Lys Thr Leu
   7490 7495 7500
   ccc acc aac cac gcc ttc cac tcc ccc cac acc aac ccc atc ctc aac 22734
   Pro Thr Asn His Ala Phe His Ser Pro His Thr Asn Pro Ile Leu Asn
   7505 7510 7515
   caa ctc cac cag cac acc caa acc ctc acc tac cac cca ccc cac acc 22782
   Gln Leu His Gln His Thr Gln Thr Leu Thr Tyr His Pro Pro His Thr
   7520 7525 7530
   ccc ctc atc acc gcc aac acc cca ccc gac caa ctc ctc acc ccc cac 22830
   Pro Leu Ile Thr Ala Asn Thr Pro Pro Asp Gln Leu Leu Thr Pro His
   7535 7540 7545 7550
   tac tgg acc caa caa gcc cgc aac acc gtc gac ata gcc acc acc acc 22878
   Tyr Trp Thr Gln Gln Ala Arg Asn Thr Val Asp Ile Ala Thr Thr Thr
                   7555                7560                7565
   caa acc ctc cac caa cac ggc gtc acc acc tac atc gaa ctc gga ccc    22926
   Gln Thr Leu His Gln His Gly Val Thr Thr Tyr Ile Glu Leu Gly Pro
               7570                7575                7580
   gac aac acc ctc acc acc ctc acc cac cac aac ctc ccc aac acc ccc    22974
   Asp Asn Thr Leu Thr Thr Leu Thr His His Asn Leu Pro Asn Thr Pro
           7585                7590                7595
   acc acc acc ctc acc ctc acc cac ccc cac cac cac ccc caa acc cac    23022
   Thr Thr Thr Leu Thr Leu Thr His Pro His His His Pro Gln Thr His
       7600                7605                7610
   ctc ctc acc aac ctc gcc aaa acc acc acc acc tgg cac ccc cac cac    23070
   Leu Leu Thr Asn Leu Ala Lys Thr Thr Thr Thr Trp His Pro His His
   7615                7620                7625                7630
   tac acc cac cac cac aac caa ccc cac acc cac acc cac ctc gac ctc    23118
   Tyr Thr His His His Asn Gln Pro His Thr His Thr His Leu Asp Leu
                   7635                7640                7645
   ccc acc tac ccc ttc caa cac cac cac tac tgg ctc gaa agc aca cag    23166
   Pro Thr Tyr Pro Phe Gln His His His Tyr Trp Leu Glu Ser Thr Gln
               7650                7655                7660
   ccc ggt gcc ggc aac gtg tca gca gcc gga ctc gac ccc acc gaa cac    23214
   Pro Gly Ala Gly Asn Val Ser Ala Ala Gly Leu Asp Pro Thr Glu His
           7665                7670                7675
   ccc cta ctc ggc gcc aca ttg gaa ctg gcc gaa ggg gac ggc tgc cta    23262
   Pro Leu Leu Gly Ala Thr Leu Glu Leu Ala Glu Gly Asp Gly Cys Leu
       7680                7685                7690
   ctg acg ggg cgc ctc tcg ttg cgc acg cat ccc tgg ctc gcc ggc cat    23310
   Leu Thr Gly Arg Leu Ser Leu Arg Thr His Pro Trp Leu Ala Gly His
   7695                7700                7705                7710
   gcg gta ggc ggt gtc gtg ctg ctg ccg ggt acg gcc ttc gcg gaa ctg    23358
   Ala Val Gly Gly Val Val Leu Leu Pro Gly Thr Ala Phe Ala Glu Leu
                   7715                7720                7725
   gcc ctt cat gcc gga gaa agt gtg ggt tgc gac cac gtg gac gag ctg    23406
   Ala Leu His Ala Gly Glu Ser Val Gly Cys Asp His Val Asp Glu Leu
               7730                7735                7740
   acg ctc cac aca ccg ttg gtc att cct gag gtc gga gac gtg acc ctt    23454
   Thr Leu His Thr Pro Leu Val Ile Pro Glu Val Gly Asp Val Thr Leu
           7745                7750                7755
   cag gtt gcc att gcg gcg ccg gac gag tcg ggt cgc cgc atg atg acc    23502
   Gln Val Ala Ile Ala Ala Pro Asp Glu Ser Gly Arg Arg Met Met Thr
       7760                7765                7770
   atc cac tca cgc ggt gag ggc ggc agt ggt gga gcc gat gcg tcg gcc    23550
   Ile His Ser Arg Gly Glu Gly Gly Ser Gly Gly Ala Asp Ala Ser Ala
   7775 7780 7785 7790
   agt gcg tgg acg cgt cat gcc gcg ggt gtg ctg agc cct gcc aag gac 23598
   Ser Ala Trp Thr Arg His Ala Ala Gly Val Leu Ser Pro Ala Lys Asp
   7795 7800 7805
   gat gac act gcc tcg tac gag ctg ctt gcg gga ccc tgg cct ccc gtt 23646
   Asp Asp Thr Ala Ser Tyr Glu Leu Leu Ala Gly Pro Trp Pro Pro Val
   7810 7815 7820
   gga gct acg cct gtc gac ctg aac acg gct tac gat caa atg gcc gac 23694
   Gly Ala Thr Pro Val Asp Leu Asn Thr Ala Tyr Asp Gln Met Ala Asp
   7825 7830 7835
   gcc ggc ttt gct tat ggc ctg gca ttc caa ggg ttg cgc gcg gcc tgg 23742
   Ala Gly Phe Ala Tyr Gly Leu Ala Phe Gln Gly Leu Arg Ala Ala Trp
   7840 7845 7850
   cgc tac ggc gac gac atc ctc gtc gag gca cgt ctt ccc gaa gaa gtg 23790
   Arg Tyr Gly Asp Asp Ile Leu Val Glu Ala Arg Leu Pro Glu Glu Val
   7855 7860 7865 7870
   tcg gga gac gcg gcg gcg tac ggt ctg cac ccg gcc ctg ctc gac gct 23838
   Ser Gly Asp Ala Ala Ala Tyr Gly Leu His Pro Ala Leu Leu Asp Ala
   7875 7880 7885
   gcc ctt cag ggc acc ggc ctg ctt tct gtg gcg ggt ccg ggg acg ccc 23886
   Ala Leu Gln Gly Thr Gly Leu Leu Ser Val Ala Gly Pro Gly Thr Pro
   7890 7895 7900
   gtc gtg ccc cat gtg tgg aac ggt ctg cgg ttc cgt acg cat ggt gca 23934
   Val Val Pro His Val Trp Asn Gly Leu Arg Phe Arg Thr His Gly Ala
   7905 7910 7915
   gtc tcc gtg cgc gcg tgc ctg tcg acg ctt gga gcg aca ggg gcg gcc 23982
   Val Ser Val Arg Ala Cys Leu Ser Thr Leu Gly Ala Thr Gly Ala Ala
   7920 7925 7930
   gtg tgc gtg cgc atc acc gac gac acc ggg gtg ccg gtg gcg tcg gtc 24030
   Val Cys Val Arg Ile Thr Asp Asp Thr Gly Val Pro Val Ala Ser Val
   7935 7940 7945 7950
   gat cgt ctt gag ttg cgg cct gtg gat atg ggt cag ttg cgt gct gtc 24078
   Asp Arg Leu Glu Leu Arg Pro Val Asp Met Gly Gln Leu Arg Ala Val
   7955 7960 7965
   tcg gtt tcg gcg ggg cgg cgg ggt tcg ctg tat gcg gtg cag tgg gct 24126
   Ser Val Ser Ala Gly Arg Arg Gly Ser Leu Tyr Ala Val Gln Trp Ala
   7970 7975 7980
   gag gtg ggt cct gtg ccg gtg tgt ggg cag gcg tgg gcg tgg cac gag 24174
   Glu Val Gly Pro Val Pro Val Cys Gly Gln Ala Trp Ala Trp His Glu
   7985 7990 7995
   gac gtg ggt gag agc ggt ggt ggg cct gtg ccg ggg gtg gtg gtg ttg 24222
   Asp Val Gly Glu Ser Gly Gly Gly Pro Val Pro Gly Val Val Val Leu
   8000 8005 8010
   cgg tgc ccg gat gcc ggt gcc gat ggc ggc ggt ggc ggt ggt gtg ggt    24270
   Arg Cys Pro Asp Ala Gly Ala Asp Gly Gly Gly Gly Gly Gly Val Gly
   8015                8020                8025                8030
   gag gtt gtt ggt ggg gtg ttg ggt gtg gtg cag ggg tgg ctg ggg ctg    24318
   Glu Val Val Gly Gly Val Leu Gly Val Val Gln Gly Trp Leu Gly Leu
                   8035                8040                8045
   gag cgg ttt gcg ggt tcg cgg ctg gtg gtg gtg acc cgg ggt gcg gtg    24366
   Glu Arg Phe Ala Gly Ser Arg Leu Val Val Val Thr Arg Gly Ala Val
               8050                8055                8060
   gtg gcc ggc ccg gag gac ggc ccg gtg gat gtg gtg ggt gcg gcg gtg    24414
   Val Ala Gly Pro Glu Asp Gly Pro Val Asp Val Val Gly Ala Ala Val
           8065                8070                8075
   tgg ggg ctg gtg cgg tcg gcg cag gct gag cat ccg gac cgg ttt gtc    24462
   Trp Gly Leu Val Arg Ser Ala Gln Ala Glu His Pro Asp Arg Phe Val
       8080                8085                8090
   ctc ctc gac ctg gac acc gac ctc gac agc ggc gct gac gcc gat gcc    24510
   Leu Leu Asp Leu Asp Thr Asp Leu Asp Ser Gly Ala Asp Ala Asp Ala
   8095                8100                8105                8110
   ggc aac gag gcc ggt atg ggg tct ggt ctg gat ggt ggg cgt gtg gct    24558
   Gly Asn Glu Ala Gly Met Gly Ser Gly Leu Asp Gly Gly Arg Val Ala
                   8115                8120                8125
   gcg gtg gtg gcg tgt ggt gag ccg cag ttg gcg gtg cgt ggt gag cgg    24606
   Ala Val Val Ala Cys Gly Glu Pro Gln Leu Ala Val Arg Gly Glu Arg
               8130                8135                8140
   gtg ctg gcc gca cgc ctg aca cga ctt gag tcg ccg gtt gat gta tcg    24654
   Val Leu Ala Ala Arg Leu Thr Arg Leu Glu Ser Pro Val Asp Val Ser
           8145                8150                8155
   ggt cgg gag gtg ttg ccg tgg ttg tcg ggt ggg tcg gtg ttg gtg acg    24702
   Gly Arg Glu Val Leu Pro Trp Leu Ser Gly Gly Ser Val Leu Val Thr
       8160                8165                8170
   ggt ggg acg ggt gtg ctg ggt gcg gcg gtg gcg cgg cat ctg gct ggt    24750
   Gly Gly Thr Gly Val Leu Gly Ala Ala Val Ala Arg His Leu Ala Gly
   8175                8180                8185                8190
   gtg tgt ggg gtg cgg gat ctg ttg ttg gtg agc cgg cgt ggt ccg gat    24798
   Val Cys Gly Val Arg Asp Leu Leu Leu Val Ser Arg Arg Gly Pro Asp
                   8195                8200                8205
   gct ccg ggt gcg gag ggt ttg cgg gcg gag ctg gcc gcg ttg ggg gcg    24846
   Ala Pro Gly Ala Glu Gly Leu Arg Ala Glu Leu Ala Ala Leu Gly Ala
               8210                8215                8220
   gag gtg cgg att gtt gcg tgt gat gtg ggg gag cgg cgg gag gtg gtc    24894
   Glu Val Arg Ile Val Ala Cys Asp Val Gly Glu Arg Arg Glu Val Val
           8225                8230                8235
   cgg ctg ctg gag ggt gtt cct gcc ggg tgt ccg ctg acg ggt gtc gtg 24942
   Arg Leu Leu Glu Gly Val Pro Ala Gly Cys Pro Leu Thr Gly Val Val
   8240 8245 8250
   cat gcg gct ggt gtg ctg gac gat gcg acg atc gcc tct ctc acg ccc 24990
   His Ala Ala Gly Val Leu Asp Asp Ala Thr Ile Ala Ser Leu Thr Pro
   8255 8260 8265 8270
   gag cgg ctg ggc acg gtg ttc gcg gcc aag gtg gat gcc gct ctt ttg 25038
   Glu Arg Leu Gly Thr Val Phe Ala Ala Lys Val Asp Ala Ala Leu Leu
   8275 8280 8285
   ctg gat gag ctg acg cgg ggt atg gag ctg tcg gcg ttc gtg ctg ttc 25086
   Leu Asp Glu Leu Thr Arg Gly Met Glu Leu Ser Ala Phe Val Leu Phe
   8290 8295 8300
   tcc tcg gcc gcg ggg atc ctg ggg tcg gcc ggg cag ggc aac tac gcc 25134
   Ser Ser Ala Ala Gly Ile Leu Gly Ser Ala Gly Gln Gly Asn Tyr Ala
   8305 8310 8315
   gcg gcc aat gcc gct ctg gac gcg ctg gcg tac cgg cgg cgg gcg gcg 25182
   Ala Ala Asn Ala Ala Leu Asp Ala Leu Ala Tyr Arg Arg Arg Ala Ala
   8320 8325 8330
   ggt ctg ccg ggg gtg tcg ctg gcg tgg ggg ctg tgg gaa gag gcc agc 25230
   Gly Leu Pro Gly Val Ser Leu Ala Trp Gly Leu Trp Glu Glu Ala Ser
   8335 8340 8345 8350
   ggg atg acc ggg cac ctg gcc ggc acc gac cac cgg cgc atc atc cgt 25278
   Gly Met Thr Gly His Leu Ala Gly Thr Asp His Arg Arg Ile Ile Arg
   8355 8360 8365
   tcc ggt ctg cat ccc atg tcg acc ccg gac gca ctg gct ctc ttc gat 25326
   Ser Gly Leu His Pro Met Ser Thr Pro Asp Ala Leu Ala Leu Phe Asp
   8370 8375 8380
   gcg gcc ctg gct ctg gac cgg ccg gtc ctg ctg ccc gcc gac ctg cgt 25374
   Ala Ala Leu Ala Leu Asp Arg Pro Val Leu Leu Pro Ala Asp Leu Arg
   8385 8390 8395
   ccc gcc ccg ccc ctg ccg ccc ctg ctg cag gac ctc ctg ccc gcc acc 25422
   Pro Ala Pro Pro Leu Pro Pro Leu Leu Gln Asp Leu Leu Pro Ala Thr
   8400 8405 8410
   cgc cgc cgc acc acc cgc acc acc act acc ggt ggt gcg gac aac ggc 25470
   Arg Arg Arg Thr Thr Arg Thr Thr Thr Thr Gly Gly Ala Asp Asn Gly
   8415 8420 8425 8430
   gcc cag ctg cat gcc cgg ctg gcc ggc cag aca cac gaa caa cag cac 25518
   Ala Gln Leu His Ala Arg Leu Ala Gly Gln Thr His Glu Gln Gln His
   8435 8440 8445
   acc acc ctc ctc gcc ctg gtc cgc tcc cac atc gcc acc gtc ctc ggc 25566
   Thr Thr Leu Leu Ala Leu Val Arg Ser His Ile Ala Thr Val Leu Gly
   8450 8455 8460
   cac aac gcg ccg gag atg atc ccc gtt gac tcg gcg ttc cgc gac cta 25614
   His Asn Ala Pro Glu Met Ile Pro Val Asp Ser Ala Phe Arg Asp Leu
   8465 8470 8475
   ggc ttc gac tcc ttg aca gcg gtg gaa ctc cgt aac cgc ctg ggt gag 25662
   Gly Phe Asp Ser Leu Thr Ala Val Glu Leu Arg Asn Arg Leu Gly Glu
   8480 8485 8490
   gca acg gga ctg cga ctg ccg acc agt ctg gtc ttc gac cag ccg aat 25710
   Ala Thr Gly Leu Arg Leu Pro Thr Ser Leu Val Phe Asp Gln Pro Asn
   8495 8500 8505 8510
   gca gcg acc ctg gcg cgt cac cta cgt cgt gag ctg atg ggc gac gac 25758
   Ala Ala Thr Leu Ala Arg His Leu Arg Arg Glu Leu Met Gly Asp Asp
   8515 8520 8525
   gcg gaa ggc gag acg cca tcg cag gtc gca ctt cat cag gtt gcc gcg 25806
   Ala Glu Gly Glu Thr Pro Ser Gln Val Ala Leu His Gln Val Ala Ala
   8530 8535 8540
   gat gag ccg att gcg att gtg ggg atg gcg tgt cgt ttt ccg ggt ggg 25854
   Asp Glu Pro Ile Ala Ile Val Gly Met Ala Cys Arg Phe Pro Gly Gly
   8545 8550 8555
   gtg tgt tcg ccg gag gag ttg tgg gag ctg gtt gcg tcg ggt ggg gat 25902
   Val Cys Ser Pro Glu Glu Leu Trp Glu Leu Val Ala Ser Gly Gly Asp
   8560 8565 8570
   gcg att ggt gaa ttt ccg gcc ggt cgg ggg tgg gat ctg gag ggg ttg 25950
   Ala Ile Gly Glu Phe Pro Ala Gly Arg Gly Trp Asp Leu Glu Gly Leu
   8575 8580 8585 8590
   ttt gat tcg gac cct gac cgg tcg ggg acg tcg tac gcg cgg tat ggc 25998
   Phe Asp Ser Asp Pro Asp Arg Ser Gly Thr Ser Tyr Ala Arg Tyr Gly
   8595 8600 8605
   ggg ttt ttg tat gag gcg ggg gag ttc gat gcg gac ttc ttc ggg atc 26046
   Gly Phe Leu Tyr Glu Ala Gly Glu Phe Asp Ala Asp Phe Phe Gly Ile
   8610 8615 8620
   agt ccg cgt gag gcg ttg gcg atg gat ccg cag cag cgg ttg ttg ctg 26094
   Ser Pro Arg Glu Ala Leu Ala Met Asp Pro Gln Gln Arg Leu Leu Leu
   8625 8630 8635
   gag acg tcg tgg gag gcg ttc gag cgg gcg ggt atc gat ccg ctg tcg 26142
   Glu Thr Ser Trp Glu Ala Phe Glu Arg Ala Gly Ile Asp Pro Leu Ser
   8640 8645 8650
   atg cgt ggc tcc cgt acg ggt gtc ttc gcc ggg gtg atg tac cac gac 26190
   Met Arg Gly Ser Arg Thr Gly Val Phe Ala Gly Val Met Tyr His Asp
   8655 8660 8665 8670
   tac gcc gcg cgt ctc cac cat gtc ccc gag ggt ttc gaa ggc ctc atc 26238
   Tyr Ala Ala Arg Leu His His Val Pro Glu Gly Phe Glu Gly Leu Ile
   8675 8680 8685
   gcc aac ggc agc gca ggc agc gtc gcg acc ggc cgg gtg gcc tac agc 26286
   Ala Asn Gly Ser Ala Gly Ser Val Ala Thr Gly Arg Val Ala Tyr Ser
                8690                8695                8700
   ttt ggc ctt gag ggt ccg gcc gtg acc gtc gat acg gcg tgt tcg tcg     26334
   Phe Gly Leu Glu Gly Pro Ala Val Thr Val Asp Thr Ala Cys Ser Ser
           8705                8710                8715
   tcg ttg gtg gcg ttg cat tgg gcg gcg cag gcg ttg cgt gcg ggt gag     26382
   Ser Leu Val Ala Leu His Trp Ala Ala Gln Ala Leu Arg Ala Gly Glu
       8720                8725                8730
   tgt tcg atg gcg ctt gcc ggg ggt gtg acg gtg atg tcg tct ccg ggt     26430
   Cys Ser Met Ala Leu Ala Gly Gly Val Thr Val Met Ser Ser Pro Gly
   8735                8740                8745                8750
   acg ttt gtg gag ttc tca cgt cag cgg ggt ctg gcc gcg gac ggg cgg     26478
   Thr Phe Val Glu Phe Ser Arg Gln Arg Gly Leu Ala Ala Asp Gly Arg
                   8755                8760                8765
   tgc aag gcc tat tcg gcg gct gct gac ggt acc ggc tgg gcc gag ggt     26526
   Cys Lys Ala Tyr Ser Ala Ala Ala Asp Gly Thr Gly Trp Ala Glu Gly
               8770                8775                8780
   gtg ggg atg ctg ctg gtg gag cgg ctc tcc gac gcc cgt cgc aac ggt     26574
   Val Gly Met Leu Leu Val Glu Arg Leu Ser Asp Ala Arg Arg Asn Gly
           8785                8790                8795
   cac cgt gtc ctg gcc gtg gtg cgt ggc agt gcg gtc aac cag gac ggt     26622
   His Arg Val Leu Ala Val Val Arg Gly Ser Ala Val Asn Gln Asp Gly
       8800                8805                8810
   gcg agc aac ggt ctg acc gcg ccc aac ggg ccc tcc cag cag cgt gtc     26670
   Ala Ser Asn Gly Leu Thr Ala Pro Asn Gly Pro Ser Gln Gln Arg Val
   8815                8820                8825                8830
   atc cgt cag gcc ctg gcc aat gcg gga ctg acc ccg gcc gat gtc gac     26718
   Ile Arg Gln Ala Leu Ala Asn Ala Gly Leu Thr Pro Ala Asp Val Asp
                   8835                8840                8845
   gca gtg gag ggc cac ggc acc ggg acc act ctg ggg gac ccg atc gag     26766
   Ala Val Glu Gly His Gly Thr Gly Thr Thr Leu Gly Asp Pro Ile Glu
               8850                8855                8860
   gcc cag gca ctc ctg gcc gcc tac gga caa cac cgc ccc cac cac cgc     26814
   Ala Gln Ala Leu Leu Ala Ala Tyr Gly Gln His Arg Pro His His Arg
           8865                8870                8875
   ccc ttg tgg ctg gga tcc ctc aaa tcc aac atc ggg cac gca cag gcc     26862
   Pro Leu Trp Leu Gly Ser Leu Lys Ser Asn Ile Gly His Ala Gln Ala
       8880                8885                8890
   gcc gcg ggc gtg ggc gga gtc atc aag atg gtg atg gcc ctg cgc aac     26910
   Ala Ala Gly Val Gly Gly Val Ile Lys Met Val Met Ala Leu Arg Asn
   8895                8900                8905                8910
   ggg ctg ctg cca cag acc ctc cac gtg gac gag ccc acc ccc cag gtc     26958
   Gly Leu Leu Pro Gln Thr Leu His Val Asp Glu Pro Thr Pro Gln Val
                   8915                8920                8925
   gac tgg tcc aca ggc gca gta caa ctc ctg aca caa ccg gtg ccc tgg    27006
   Asp Trp Ser Thr Gly Ala Val Gln Leu Leu Thr Gln Pro Val Pro Trp
                   8930                 8935                 8940
   ccc gcc gac ccg gcc ggc cgg cca cgc cac gcc ggc gtg tca tca ttc    27054
   Pro Ala Asp Pro Ala Gly Arg Pro Arg His Ala Gly Val Ser Ser Phe
           8945                 8950                 8955
   ggc gtc agc ggc acc aac gcc cat gtg att ttg gag gag gcg cct gcg    27102
   Gly Val Ser Gly Thr Asn Ala His Val Ile Leu Glu Glu Ala Pro Ala
       8960                 8965                 8970
   gcg gcg ggc ggt gct gcc ggt ggt ggg gtg tcg gtg ggt gct ccg aat    27150
   Ala Ala Gly Gly Ala Ala Gly Gly Gly Val Ser Val Gly Ala Pro Asn
   8975                 8980                 8985                 8990
   cca gcc ctt ccg gtg gct gag tct gag ccg gtg ccg gtg ccg gtg ccg    27198
   Pro Ala Leu Pro Val Ala Glu Ser Glu Pro Val Pro Val Pro Val Pro
                   8995                 9000                 9005
   gtg tcg gcg agg tct gag gcc ggg ttg cgg gcg cag gca cag gcg ttg    27246
   Val Ser Ala Arg Ser Glu Ala Gly Leu Arg Ala Gln Ala Gln Ala Leu
               9010                 9015                 9020
   cgc cag tac gtg gca gcc cgc ccg gac atg tca cct gcc gac atc ggt    27294
   Arg Gln Tyr Val Ala Ala Arg Pro Asp Met Ser Pro Ala Asp Ile Gly
           9025                 9030                 9035
   gcg ggt ctg gcc cgc ggc cgg gcc gta ctg gaa cac cgc gcc gtc atc    27342
   Ala Gly Leu Ala Arg Gly Arg Ala Val Leu Glu His Arg Ala Val Ile
       9040                 9045                 9050
   ctg gcc gcg gac cgc gag gaa ctg gcg cag gca ctg aca gcc ctg gca    27390
   Leu Ala Ala Asp Arg Glu Glu Leu Ala Gln Ala Leu Thr Ala Leu Ala
   9055                 9060                 9065                 9070
   gcc ggc gaa ccc cac ccc cac atc acc aca ggc cac acc cgg ggc agt    27438
   Ala Gly Glu Pro His Pro His Ile Thr Thr Gly His Thr Arg Gly Ser
                   9075                 9080                 9085
   gac cgc ggc ggc gtc gtc ttc gtc ttc ccc gga cag ggc ggc cag tgg    27486
   Asp Arg Gly Gly Val Val Phe Val Phe Pro Gly Gln Gly Gly Gln Trp
               9090                 9095                 9100
   gcc ggg atg ggc ctg acc ctg ctc acc tcc tca ccc gtg ttc gcc gaa    27534
   Ala Gly Met Gly Leu Thr Leu Leu Thr Ser Ser Pro Val Phe Ala Glu
           9105                 9110                 9115
   cac atc gac gca tgc gag aaa gcc ctc acc ccc tgg gtg ccc tgg tcc    27582
   His Ile Asp Ala Cys Glu Lys Ala Leu Thr Pro Trp Val Pro Trp Ser
       9120                 9125                 9130
   ctg acc gac atc ctg cac cgc gac ccc gac gac ccc gca tgg caa caa    27630
   Leu Thr Asp Ile Leu His Arg Asp Pro Asp Asp Pro Ala Trp Gln Gln
   9135                 9140                 9145                 9150
   gcc gac gtg gtc cag ccc gtg ctc ttc agc atc atg gtc tcc ctc gcc 27678
   Ala Asp Val Val Gln Pro Val Leu Phe Ser Ile Met Val Ser Leu Ala
   9155 9160 9165
   gcc ctg tgg cgc tcc tac ggc atc gaa ccc gac gcg gtc ctc ggc cac 27726
   Ala Leu Trp Arg Ser Tyr Gly Ile Glu Pro Asp Ala Val Leu Gly His
   9170 9175 9180
   tcc cag gga gaa atc gcc gcc gcc cac atc tgc ggc gca ctc agc ctg 27774
   Ser Gln Gly Glu Ile Ala Ala Ala His Ile Cys Gly Ala Leu Ser Leu
   9185 9190 9195
   aaa gac gcc gcc aaa acc gtt gca ctg cgc agc cag gca ctg gcc gcc 27822
   Lys Asp Ala Ala Lys Thr Val Ala Leu Arg Ser Gln Ala Leu Ala Ala
   9200 9205 9210
   gta cga ggc cgg ggc gcc atg gtc tca ctg ccc ctg ccc gcc cag gac 27870
   Val Arg Gly Arg Gly Ala Met Val Ser Leu Pro Leu Pro Ala Gln Asp
   9215 9220 9225 9230
   gtg cag cag ctc att tcc gaa cgg tgg gaa ggg cag ttg tgg gtg gca 27918
   Val Gln Gln Leu Ile Ser Glu Arg Trp Glu Gly Gln Leu Trp Val Ala
   9235 9240 9245
   gcc ctc aac ggc ccc cac tcc acc acc gtc tcc ggc gac acc acc gca 27966
   Ala Leu Asn Gly Pro His Ser Thr Thr Val Ser Gly Asp Thr Thr Ala
   9250 9255 9260
   gta gaa gaa ctc ctc acc cac tgt gcc gac acc ggc cta cgg gcc aaa 28014
   Val Glu Glu Leu Leu Thr His Cys Ala Asp Thr Gly Leu Arg Ala Lys
   9265 9270 9275
   cgc atc ccc gtc gac tac gcc tcc cac tgc ccc cac gtc caa ccc ctc 28062
   Arg Ile Pro Val Asp Tyr Ala Ser His Cys Pro His Val Gln Pro Leu
   9280 9285 9290
   cac gac gaa ctc ctg cac ctg ctg gga gac atc acc ccc cag ccg tcc 28110
   His Asp Glu Leu Leu His Leu Leu Gly Asp Ile Thr Pro Gln Pro Ser
   9295 9300 9305 9310
   acc atg ccg ttc ttc tcc acc gtc gta ggg cac ctg gtc tgg tac acc 28158
   Thr Met Pro Phe Phe Ser Thr Val Val Gly His Leu Val Trp Tyr Thr
   9315 9320 9325
   aca acc ctg gac gcc gcc tac tgg tac cgc aac ctc cac cag ccc gtc 28206
   Thr Thr Leu Asp Ala Ala Tyr Trp Tyr Arg Asn Leu His Gln Pro Val
   9330 9335 9340
   cgc ttc agc cac gcc atc cag acc ctg acc gac gac gga cac cgc ccc 28254
   Arg Phe Ser His Ala Ile Gln Thr Leu Thr Asp Asp Gly His Arg Pro
   9345 9350 9355
   ttc atc gaa atc agt ccc cac ccc acc ctc gtc ccc gcc atc gaa gac 28302
   Phe Ile Glu Ile Ser Pro His Pro Thr Leu Val Pro Ala Ile Glu Asp
   9360 9365 9370
   acc acc gaa aac acc acc gaa aac atc acc gcg acc ggc agc ctc cgc 28350
   Thr Thr Glu Asn Thr Thr Glu Asn Ile Thr Ala Thr Gly Ser Leu Arg
   9375                9380                9385                9390
   cgc ggc gac aac gac acc cac cgc ttc ctc acc gcc ctc gcc cac acc    28398
   Arg Gly Asp Asn Asp Thr His Arg Phe Leu Thr Ala Leu Ala His Thr
                   9395                9400                9405
   cac acc acc ggc att cgg aca ccc acc acc tgg cac cac cac tac acc    28446
   His Thr Thr Gly Ile Arg Thr Pro Thr Thr Trp His His His Tyr Thr
               9410                9415                9420
   caa acc cac ccc cac ccc cac aac cac cac ctc gac ctg ccc acc tac    28494
   Gln Thr His Pro His Pro His Asn His His Leu Asp Leu Pro Thr Tyr
           9425                9430                9435
   ccc ttc caa cac cag cac tac tgg ctc caa cca ccc acc acg aca acc    28542
   Pro Phe Gln His Gln His Tyr Trp Leu Gln Pro Pro Thr Thr Thr Thr
       9440                9445                9450
   gac ctc acc acc acc ggc ctc acc ccc acc cac cac ccc ctc ctc acc    28590
   Asp Leu Thr Thr Thr Gly Leu Thr Pro Thr His His Pro Leu Leu Thr
   9455                9460                9465                9470
   gca aca ctc acc ctc gcc aac aac aac aca caa cta ctc acc ggc cgc    28638
   Ala Thr Leu Thr Leu Ala Asn Asn Asn Thr Gln Leu Leu Thr Gly Arg
                   9475                9480                9485
   ctc tcc cta cgc acc cac ccc tgg ctc acc gac cac acc gtc gtc ggt    28686
   Leu Ser Leu Arg Thr His Pro Trp Leu Thr Asp His Thr Val Val Gly
               9490                9495                9500
   acc act ctt gtg cca gga acc gcc ctc ctc gaa ctc gcc ctc caa gca    28734
   Thr Thr Leu Val Pro Gly Thr Ala Leu Leu Glu Leu Ala Leu Gln Ala
           9505                9510                9515
   acc acg acc gac cac ctc gaa gaa ctc gcc ctc cac acg cct ctc gtc    28782
   Thr Thr Thr Asp His Leu Glu Glu Leu Ala Leu His Thr Pro Leu Val
       9520                9525                9530
   atc ccc cgt gag ggt gcc gtc gac gtt cag gtg cac atc aat cca ccg    28830
   Ile Pro Arg Glu Gly Ala Val Asp Val Gln Val His Ile Asn Pro Pro
   9535                9540                9545                9550
   gac gac acc gac act cgt tca ctg acg atc tac tcg cga agc gag aac    28878
   Asp Asp Thr Asp Thr Arg Ser Leu Thr Ile Tyr Ser Arg Ser Glu Asn
                   9555                9560                9565
   gcc ccc gca gcg gct ccc tgg cgt cat cac gcc acg gcc gtt ctg gga    28926
   Ala Pro Ala Ala Ala Pro Trp Arg His His Ala Thr Ala Val Leu Gly
               9570                9575                9580
   acc aag acc tcg cgc att gag aca ggc cgt agc cac gat gat ctg tcg    28974
   Thr Lys Thr Ser Arg Ile Glu Thr Gly Arg Ser His Asp Asp Leu Ser
           9585                9590                9595
   atg tgg ccg cca gcg ggc gca gtt cgc tgt gct gat gag gaa ttg gca    29022
   Met Trp Pro Pro Ala Gly Ala Val Arg Cys Ala Asp Glu Glu Leu Ala
   9600 9605 9610
   gcc ttg tat ggc gac tac gag gca aat ggc ttt gtc tat ggc ccc gca 29070
   Ala Leu Tyr Gly Asp Tyr Glu Ala Asn Gly Phe Val Tyr Gly Pro Ala
   9615 9620 9625 9630
   ttc cgg ggg ctg act gct gcc tgg cgt ctg gga gac gag gtg ttt gcc 29118
   Phe Arg Gly Leu Thr Ala Ala Trp Arg Leu Gly Asp Glu Val Phe Ala
   9635 9640 9645
   gag gtt cgc ctt cca gaa cag gtg cac ggc gag gca tcc gcg tac aac 29166
   Glu Val Arg Leu Pro Glu Gln Val His Gly Glu Ala Ser Ala Tyr Asn
   9650 9655 9660
   ctg cac ccg gca ctg ctg gat gct gcc ttg cac gca gcg gcc ttt gcg 29214
   Leu His Pro Ala Leu Leu Asp Ala Ala Leu His Ala Ala Ala Phe Ala
   9665 9670 9675
   ccg tcg ggc agt ctg ccg cag gga tcc gta ccg ttc tcc ttc acc ggt 29262
   Pro Ser Gly Ser Leu Pro Gln Gly Ser Val Pro Phe Ser Phe Thr Gly
   9680 9685 9690
   gtg acg ctg cac gcc gcc aat gcg tcg tcg ttg cgc gtg cga ctc tcg 29310
   Val Thr Leu His Ala Ala Asn Ala Ser Ser Leu Arg Val Arg Leu Ser
   9695 9700 9705 9710
   ccg gcc gat ccg aac agc ggc cac gcc gca gtt tcc gtg ctg gtc acg 29358
   Pro Ala Asp Pro Asn Ser Gly His Ala Ala Val Ser Val Leu Val Thr
   9715 9720 9725
   gat gac acc ggt acg ccc gtg gcg tcc gtc gag gcg ttg gcg gtg cgc 29406
   Asp Asp Thr Gly Thr Pro Val Ala Ser Val Glu Ala Leu Ala Val Arg
   9730 9735 9740
   ccg ttg gcg gcg gac gaa ttg cga gct gcc gag cgc gcc gta cag cgc 29454
   Pro Leu Ala Ala Asp Glu Leu Arg Ala Ala Glu Arg Ala Val Gln Arg
   9745 9750 9755
   gct gag ctc ttc gac atg aag tgg gtt gag gtg ccc tca gat gta ctg 29502
   Ala Glu Leu Phe Asp Met Lys Trp Val Glu Val Pro Ser Asp Val Leu
   9760 9765 9770
   gtg tcg ggc ggg gca tcg gtg gtg gtg ctg gat ggt gcc gac gac ctc 29550
   Val Ser Gly Gly Ala Ser Val Val Val Leu Asp Gly Ala Asp Asp Leu
   9775 9780 9785 9790
   gtt ggt ctg gcg gct gag gag gat ggt gtg ccg ggg gtg gtg gtg ttg 29598
   Val Gly Leu Ala Ala Glu Glu Asp Gly Val Pro Gly Val Val Val Leu
   9795 9800 9805
   cgg tgc ccg gat gcc ggt gcc gat ggc ggc ggt ggt ggc ggt ggt gtg 29646
   Arg Cys Pro Asp Ala Gly Ala Asp Gly Gly Gly Gly Gly Gly Gly Val
   9810 9815 9820
   ggt gag gtt gtt ggt ggg gtg ttg ggt gtg gtg cag ggg tgg ctg ggg 29694
   Gly Glu Val Val Gly Gly Val Leu Gly Val Val Gln Gly Trp Leu Gly
   9825 9830 9835
   ctg gag cgg ttt gcg ggt tcg cgg ctg gtg gtg gtg acc cgg ggt gcg    29742
   Leu Glu Arg Phe Ala Gly Ser Arg Leu Val Val Val Thr Arg Gly Ala
       9840                9845                9850
   gtg gtg gcc ggc ccg gag gac ggc ccg gtg gat ggc ccg gtg gat gtg    29790
   Val Val Ala Gly Pro Glu Asp Gly Pro Val Asp Gly Pro Val Asp Val
   9855                9860                9865                9870
   gtg ggt gcg gcg gtg tgg ggg ctg gtg cgg tcg gcg cag gct gag cat    29838
   Val Gly Ala Ala Val Trp Gly Leu Val Arg Ser Ala Gln Ala Glu His
                   9875                9880                9885
   ccg gac cgg ttt gtc ctc ctc gac ctg gac acc gac ctc gac agc ggc    29886
   Pro Asp Arg Phe Val Leu Leu Asp Leu Asp Thr Asp Leu Asp Ser Gly
               9890                9895                9900
   gct gac cgc gat gcc ggc aac gag gcc ggt atg ggg tct ggt ctg gat    29934
   Ala Asp Arg Asp Ala Gly Asn Glu Ala Gly Met Gly Ser Gly Leu Asp
           9905                9910                9915
   ggt ggg cgt gtg gct gcg gtg gtg gcg tgt ggt gag ccg cag ttg gcg    29982
   Gly Gly Arg Val Ala Ala Val Val Ala Cys Gly Glu Pro Gln Leu Ala
       9920                9925                9930
   gtg cgt ggt gag cgg gtg ctg gcc gca cgc ctg aca cga ctt gag tcg    30030
   Val Arg Gly Glu Arg Val Leu Ala Ala Arg Leu Thr Arg Leu Glu Ser
   9935                9940                9945                9950
   ccg gtt gat gta tcg ggt cgg gag gtg ttg ccg tgg ttg tcg ggt ggg    30078
   Pro Val Asp Val Ser Gly Arg Glu Val Leu Pro Trp Leu Ser Gly Gly
                   9955                9960                9965
   tcg gtg ttg gtg acg ggt ggg acg ggt gtg ctg ggt gcg gcg gtg gcg    30126
   Ser Val Leu Val Thr Gly Gly Thr Gly Val Leu Gly Ala Ala Val Ala
               9970                9975                9980
   cgg cat ctg gct ggt gtg tgt ggg gtg cgg gat ctg ttg ttg gtg agc    30174
   Arg His Leu Ala Gly Val Cys Gly Val Arg Asp Leu Leu Leu Val Ser
           9985                9990                9995
   cgg cgt ggt ccg gat gct ccg ggt gcg gag ggt ttg cgg gcg gag ctg    30222
   Arg Arg Gly Pro Asp Ala Pro Gly Ala Glu Gly Leu Arg Ala Glu Leu
       10000               10005               10010
   gcc gcg ttg ggg gcg gag gtg cgg att gtt gcg tgt gat gtg ggg gag    30270
   Ala Ala Leu Gly Ala Glu Val Arg Ile Val Ala Cys Asp Val Gly Glu
   10015               10020               10025               10030
   cgg cgg gag gtg gtc cgg ctg ctg gag ggt gtt cct gcc ggg tgt ccg    30318
   Arg Arg Glu Val Val Arg Leu Leu Glu Gly Val Pro Ala Gly Cys Pro
                   10035               10040               10045
   ctg acg ggt gtc gtg cat gcg gct ggt gtg ctg gac gat gcg acg atc    30366
   Leu Thr Gly Val Val His Ala Ala Gly Val Leu Asp Asp Ala Thr Ile
               10050               10055               10060
   gcc tct ctc acg ccc gag cgg ctg ggc acg gtg ttc gcg gcc aag gtg    30414
   Ala Ser Leu Thr Pro Glu Arg Leu Gly Thr Val Phe Ala Ala Lys Val
           10065                   10070                   10075
   gat gcc gct ctt ttg ctg gat gag ctg acg cgg ggt atg gag ctg tcg    30462
   Asp Ala Ala Leu Leu Leu Asp Glu Leu Thr Arg Gly Met Glu Leu Ser
       10080                   10085                   10090
   gcg ttc gtg ctg ttc tcc tcg gcc gcg ggg atc ctg ggg tcg gcc ggg    30510
   Ala Phe Val Leu Phe Ser Ser Ala Ala Gly Ile Leu Gly Ser Ala Gly
   10095                   10100                   10105               10110
   cag ggc aac tac gcc gcg gcc aat gcc gct ctg gac gcg ctg gcg tac    30558
   Gln Gly Asn Tyr Ala Ala Ala Asn Ala Ala Leu Asp Ala Leu Ala Tyr
                   10115                   10120                   10125
   cgg cgg cgg gcg gcg ggt ctg ccg ggg gtg tcg ctg gcg tgg ggg ctg    30606
   Arg Arg Arg Ala Ala Gly Leu Pro Gly Val Ser Leu Ala Trp Gly Leu
               10130                   10135                   10140
   tgg gaa gag gcc agc ggg atg acc ggg cat ctg gcc ggc acc gac cac    30654
   Trp Glu Glu Ala Ser Gly Met Thr Gly His Leu Ala Gly Thr Asp His
           10145                   10150                   10155
   cgg cgc atc atc cgt tcc ggt ctg cat ccc atg tcg acc ccg gac gca    30702
   Arg Arg Ile Ile Arg Ser Gly Leu His Pro Met Ser Thr Pro Asp Ala
       10160                   10165                   10170
   ctg gcc ctc ttc gat gcg gcc ctg gct ctg gac cgg ccg gtc ctg ctg    30750
   Leu Ala Leu Phe Asp Ala Ala Leu Ala Leu Asp Arg Pro Val Leu Leu
   10175                   10180                   10185               10190
   ccc gcc gac ctg cgt ccc gcc ccg ccc ctg ccg ccc ctg ctg cag gac    30798
   Pro Ala Asp Leu Arg Pro Ala Pro Pro Leu Pro Pro Leu Leu Gln Asp
                   10195                   10200                   10205
   ctc ctg ccc gcc acc cgc cgc cgc acc acc cgc acc acc act acc ggt    30846
   Leu Leu Pro Ala Thr Arg Arg Arg Thr Thr Arg Thr Thr Thr Thr Gly
               10210                   10215                   10220
   ggt gcg gac aac ggc gcc cag ctg cac ggc cgg ctg gcc ggc cag aca    30894
   Gly Ala Asp Asn Gly Ala Gln Leu His Gly Arg Leu Ala Gly Gln Thr
           10225                   10230                   10235
   cac gaa caa cag cac acc acc ctc ctc gcc ctg gtc cgc tcc cac atc    30942
   His Glu Gln Gln His Thr Thr Leu Leu Ala Leu Val Arg Ser His Ile
       10240                   10245                   10250
   gcc acc gtc ctg ggc cac acc acc ccc gac acc atc ccc ccc gac cgc    30990
   Ala Thr Val Leu Gly His Thr Thr Pro Asp Thr Ile Pro Pro Asp Arg
   10255                   10260                   10265               10270
   gcg ttc cgc gac ctc ggc ttc gac tcc ctc acc gcc gtc gaa cta cgc    31038
   Ala Phe Arg Asp Leu Gly Phe Asp Ser Leu Thr Ala Val Glu Leu Arg
                   10275                   10280                   10285
   aac cgg ctc tcc cac acc acc gga ctc cgc ctc ccc acc acc ctc gcc    31086
   Asn Arg Leu Ser His Thr Thr Gly Leu Arg Leu Pro Thr Thr Leu Ala
   10290 10295 10300
   ttc gac cac ccc aac ccc acc acc ctc acc cac cac ctc cac aca caa 31134
   Phe Asp His Pro Asn Pro Thr Thr Leu Thr His His Leu His Thr Gln
   10305 10310 10315
   ctc gtc agc aag gga ctc acc gcc gcg gcc gag ccg gac gcc gca acg 31182
   Leu Val Ser Lys Gly Leu Thr Ala Ala Ala Glu Pro Asp Ala Ala Thr
   10320 10325 10330
   aca ccc ccg ggg ctg ccc tcg ctg ctc tcg gag ctc gag cgg ctg gag 31230
   Thr Pro Pro Gly Leu Pro Ser Leu Leu Ser Glu Leu Glu Arg Leu Glu
   10335 10340 10345 10350
   gcg gta gtg ctc tcc tcc acc aca tcc tcc gct gcc ccg ctg gac gac 31278
   Ala Val Val Leu Ser Ser Thr Thr Ser Ser Ala Ala Pro Leu Asp Asp
   10355 10360 10365
   ggc gcg cgc acg cgg ctg gcc tcc cga ctg cat tcc ctc gcc cag aag 31326
   Gly Ala Arg Thr Arg Leu Ala Ser Arg Leu His Ser Leu Ala Gln Lys
   10370 10375 10380
   ttg aac ggc gac gac acc gcc ccc gac ctc gca gag aca tcg gac gag 31374
   Leu Asn Gly Asp Asp Thr Ala Pro Asp Leu Ala Glu Thr Ser Asp Glu
   10385 10390 10395
   gag atg ttc gct ctc atc gac agg gaa gtc gga ttc gaa tct caa tga 31422
   Glu Met Phe Ala Leu Ile Asp Arg Glu Val Gly Phe Glu Ser Gln
   10400 10405 10410
<210> 3
   <211> 11916
   <212> DNA
   <213> Artificial Sequence
   <223> Description of Artificial Sequence;In vitro mutagenized DNA
<220>
   <221> CDS
   <222> (1)..(11916)
<400> 3
   gtg cag agg atg gac ggc ggg gaa gaa ccc cgc cct gcg gca ggg gag 48
   Val Gln Arg Met Asp Gly Gly Glu Glu Pro Arg Pro Ala Ala Gly Glu
   1 5 10 15
   gtc ctc gga gtg gcc gac gag gcg gac ggc ggc gtc gtc ttc gtt ttt 96
   Val Leu Gly Val Ala Asp Glu Ala Asp Gly Gly Val Val Phe Val Phe
   20 25 30
   ccc ggg cag ggc ccg caa tgg ccg ggc atg gga agg gaa ctt ctc gac 144
   Pro Gly Gln Gly Pro Gln Trp Pro Gly Met Gly Arg Glu Leu Leu Asp
   35 40 45
   gct tcc gac gtc ttc cgg gag agc gtc cgc gcc tgc gaa gcc gcg ttc 192
   Ala Ser Asp Val Phe Arg Glu Ser Val Arg Ala Cys Glu Ala Ala Phe
       50                       55                       60
   gcg ccc tac gtc gac tgg tcg gtg gag cag gtg ttg cgg gac tcg ccg      240
   Ala Pro Tyr Val Asp Trp Ser Val Glu Gln Val Leu Arg Asp Ser Pro
    65                  70                  75                  80
   gac gct ccc ggg ctg gac cgg gtg gac gtc gtc cag ccg acc ctg ttc      288
   Asp Ala Pro Gly Leu Asp Arg Val Asp Val Val Gln Pro Thr Leu Phe
                    85                  90                  95
   gcc gtc atg atc tcc ctg gcc gcc ctc tgg cgc tcg caa ggg gtc gag      336
   Ala Val Met Ile Ser Leu Ala Ala Leu Trp Arg Ser Gln Gly Val Glu
               100                 105                 110
   ccg tgc gcg gtg ctg gga cac agc ctg ggc gag atc gcg gca gcc cac      384
   Pro Cys Ala Val Leu Gly His Ser Leu Gly Glu Ile Ala Ala Ala His
           115                 120                 125
   gtc tcg gga ggc ctg tcc ctg gcc gac gcc gca cgc gtg gtg acg ctt      432
   Val Ser Gly Gly Leu Ser Leu Ala Asp Ala Ala Arg Val Val Thr Leu
       130                 135                 140
   tgg agc cag gca cag acc acc ctt gcc ggg acc ggc gcg ctc gtc tcc      480
   Trp Ser Gln Ala Gln Thr Thr Leu Ala Gly Thr Gly Ala Leu Val Ser
   145                 150                 155                 160
   gtc gcc gcc acg ccg gat gag ctc ctg ccc cga atc gct ccg tgg acc      528
   Val Ala Ala Thr Pro Asp Glu Leu Leu Pro Arg Ile Ala Pro Trp Thr
                   165                 170                 175
   gag gac aac ccg gcg cgg ctc gcc gtc gca gcc gtc aac gga ccc cgg      576
   Glu Asp Asn Pro Ala Arg Leu Ala Val Ala Ala Val Asn Gly Pro Arg
               180                 185                 190
   agc aca gtc gtt tcc ggt gcc cgc gag gcc gtc gcg gac ctg gtg gcc      624
   Ser Thr Val Val Ser Gly Ala Arg Glu Ala Val Ala Asp Leu Val Ala
           195                 200                 205
   gac ctc acc gcc gcg cag gtg cgc acg cgc atg atc ccg gtg gac gtt      672
   Asp Leu Thr Ala Ala Gln Val Arg Thr Arg Met Ile Pro Val Asp Val
       210                 215                 220
   ccc gcc cac tcc ccc ctg atg tac gcc atc gag gaa cgg gtc gtc agc      720
   Pro Ala His Ser Pro Leu Met Tyr Ala Ile Glu Glu Arg Val Val Ser
   225                 230                 235                 240
   ggc ctg ctg ccc atc acc cca cgc ccc tcc cgc atc ccc ttc cac tcc      768
   Gly Leu Leu Pro Ile Thr Pro Arg Pro Ser Arg Ile Pro Phe His Ser
                   245                 250                 255
   tcg gtg acc ggc ggc cgc ctc gac acc cgc gag cta gac gcg gcg tac      816
   Ser Val Thr Gly Gly Arg Leu Asp Thr Arg Glu Leu Asp Ala Ala Tyr
               260                 265                 270
   tgg tac cgc aac atg tcg agc acg gtc cgg ttc gag ccc gcc gcc cgg      864
   Trp Tyr Arg Asn Met Ser Ser Thr Val Arg Phe Glu Pro Ala Ala Arg
           275                 280                 285
   ctg ctt ctg cag cag ggg ccc aag acg ttc gtc gag atg agc ccg cac    912
   Leu Leu Leu Gln Gln Gly Pro Lys Thr Phe Val Glu Met Ser Pro His
       290                 295                 300
   ccg gtg ctg acc atg ggc ctc cag gag ctc gcc ccg gac ctg ggc gac    960
   Pro Val Leu Thr Met Gly Leu Gln Glu Leu Ala Pro Asp Leu Gly Asp
   305                 310                 315                     320
   acc acc ggc acc gcc gac acc gtg atc atg ggc acg ctg cgc cgc ggc    1008
   Thr Thr Gly Thr Ala Asp Thr Val Ile Met Gly Thr Leu Arg Arg Gly
                   325                 330                 335
   cag ggc acc ctg gac cac ttc ctg acg tct ctc gcc caa cta cgg ggg    1056
   Gln Gly Thr Leu Asp His Phe Leu Thr Ser Leu Ala Gln Leu Arg Gly
               340                 345                 350
   cat ggt gag acg tcg gcg acc acc gtc ctc tcg gca cgc ctg acc gcg    1104
   His Gly Glu Thr Ser Ala Thr Thr Val Leu Ser Ala Arg Leu Thr Ala
           355                 360                 365
   ctg tcc ccc acg cag cag cag tcg ctg ctc ctg gac ctg gtg cgc gcc    1152
   Leu Ser Pro Thr Gln Gln Gln Ser Leu Leu Leu Asp Leu Val Arg Ala
       370                 375                 380
   cac acc atg gcg gtg ctg aac gac gac gga aac gag cgc acc gcg tcg    1200
   His Thr Met Ala Val Leu Asn Asp Asp Gly Asn Glu Arg Thr Ala Ser
   385                 390                 395                     400
   gat gcc ggc cca tcg gcg agt ttc gcc cac ctc ggc ttc gac tcc gtc    1248
   Asp Ala Gly Pro Ser Ala Ser Phe Ala His Leu Gly Phe Asp Ser Val
                   405                 410                 415
   atg ggt gtc gaa ctg cgc aac cgc ctc agc aag gcc acg ggc ctg cgg    1296
   Met Gly Val Glu Leu Arg Asn Arg Leu Ser Lys Ala Thr Gly Leu Arg
               420                 425                 430
   ttg ccc gtg acg ctc atc ttc gac cac acc acg ccg gcc gcg gtc gcc    1344
   Leu Pro Val Thr Leu Ile Phe Asp His Thr Thr Pro Ala Ala Val Ala
           435                 440                 445
   gcg cgc ctt cgg acc gcg gcg ctc ggc cac ctc gac gag gac acc gcg    1392
   Ala Arg Leu Arg Thr Ala Ala Leu Gly His Leu Asp Glu Asp Thr Ala
       450                 455                 460
   ccc gta ccg gac tca ccc agc ggc cac gga ggc acg gca gcg gcg gac    1440
   Pro Val Pro Asp Ser Pro Ser Gly His Gly Gly Thr Ala Ala Ala Asp
   465                 470                 475                     480
   gac ccg atc gcc atc atc ggc atg gca tgc cgt ttc ccg ggc gga gtc    1488
   Asp Pro Ile Ala Ile Ile Gly Met Ala Cys Arg Phe Pro Gly Gly Val
                   485                 490                 495
   cgg tcc ccg aag gac ctg tgg gag ctg gcc gcc tcg ggc gga gac gcc    1536
   Arg Ser Pro Lys Asp Leu Trp Glu Leu Ala Ala Ser Gly Gly Asp Ala
               500                 505                 510
   atc ggg ccg ttc ccc acc gac cgc gga tgg ccc acg gaa cag cgt cac     1584
   Ile Gly Pro Phe Pro Thr Asp Arg Gly Trp Pro Thr Glu Gln Arg His
           515                 520                 525
   gcc cag gac ccc acg cag ccc ggc acg ttc tat ccg cag gga ggc ggg     1632
   Ala Gln Asp Pro Thr Gln Pro Gly Thr Phe Tyr Pro Gln Gly Gly Gly
       530                 535                 540
   ttc ctt cac gac gcg gcg cac ttc gac gcc ggc ttc ttc gga atc agt     1680
   Phe Leu His Asp Ala Ala His Phe Asp Ala Gly Phe Phe Gly Ile Ser
   545                 550                 555                 560
   cca cgt gag gca ctg gcg atg gat ccg cag cag cgg ctg ctg ctg gag     1728
   Pro Arg Glu Ala Leu Ala Met Asp Pro Gln Gln Arg Leu Leu Leu Glu
                   565                 570                 575
   acg tcc tgg gag gcg ttc gag cgg gcg gga atc gat ccg ctg tcg gta     1776
   Thr Ser Trp Glu Ala Phe Glu Arg Ala Gly Ile Asp Pro Leu Ser Val
               580                 585                 590
   cgc ggg tcc cgt acg ggc gtc ttc gcg ggc gcc ctc tcc ttc gac tac     1824
   Arg Gly Ser Arg Thr Gly Val Phe Ala Gly Ala Leu Ser Phe Asp Tyr
           595                 600                 605
   ggc ccg cgt atg gac acc gcg tcg tcg gag ggc gcc gcg gac gtg gag     1872
   Gly Pro Arg Met Asp Thr Ala Ser Ser Glu Gly Ala Ala Asp Val Glu
       610                 615                 620
   ggc cac atc ctc acc ggt acc acg ggc agc gtc ctg tcg ggc cgt atc     1920
   Gly His Ile Leu Thr Gly Thr Thr Gly Ser Val Leu Ser Gly Arg Ile
   625                 630                 635                 640
   gcc tac agc ttc ggg ctg gaa ggg ccg gcg atc acc gtg gac acg ggg     1968
   Ala Tyr Ser Phe Gly Leu Glu Gly Pro Ala Ile Thr Val Asp Thr Gly
                   645                 650                 655
   ggc tcg gca tcg ctc gtg acg ctg cat ctg gcg tgc cag tcg ctg cgg     2016
   Gly Ser Ala Ser Leu Val Thr Leu His Leu Ala Cys Gln Ser Leu Arg
               660                 665                 670
   tcg ggt gag tgc acg ctc gcg ctg gcc ggc ggc gtc tcg gtc atg tcc     2064
   Ser Gly Glu Cys Thr Leu Ala Leu Ala Gly Gly Val Ser Val Met Ser
           675                 680                 685
   acc ctc ggc atg ttc atc gag ttc tcc cgg cag cgc ggg ctg tcg gtg     2112
   Thr Leu Gly Met Phe Ile Glu Phe Ser Arg Gln Arg Gly Leu Ser Val
       690                 695                 700
   gac ggc agg tgc aag gcg tac tcg gct gca gcc gac ggc acc ggc tgg     2160
   Asp Gly Arg Cys Lys Ala Tyr Ser Ala Ala Ala Asp Gly Thr Gly Trp
   705                 710                 715                 720
   ggc gag ggc gtc ggg atg ctg ttg gtg gag cgg ttg tcg gat gcg gtg     2208
   Gly Glu Gly Val Gly Met Leu Leu Val Glu Arg Leu Ser Asp Ala Val
                   725                 730                 735
   cgg ctg ggg cat cgg gtg ctg gcg gtg gta cgc ggc agt gcg gtc aac     2256
   Arg Leu Gly His Arg Val Leu Ala Val Val Arg Gly Ser Ala Val Asn
               740                 745                 750
   cag gac ggt gcg tcg aat ggg ctg acg gcg ccg aac ggt ccg gct cag     2304
   Gln Asp Gly Ala Ser Asn Gly Leu Thr Ala Pro Asn Gly Pro Ala Gln
           755                 760                 765
   gag cgg gtg atc cgg cag gcg ttg gcg aac gcg ggg ttg tcc gtg gcg     2352
   Glu Arg Val Ile Arg Gln Ala Leu Ala Asn Ala Gly Leu Ser Val Ala
       770                 775                 780
   gat gtg gat gtg gtg gag ggg cac ggg acg ggc acg acg ctg ggt gat     2400
   Asp Val Asp Val Val Glu Gly His Gly Thr Gly Thr Thr Leu Gly Asp
   785                 790                 795                 800
   ccg atc gag gca cag gcg ttg ctc gcc acg tac ggg cag cgg gcc ggt     2448
   Pro Ile Glu Ala Gln Ala Leu Leu Ala Thr Tyr Gly Gln Arg Ala Gly
               805                 810                 815
   gac agg ccg ctg tgg ctg ggg tct ctg aag tcc aac atc ggg cac acc     2496
   Asp Arg Pro Leu Trp Leu Gly Ser Leu Lys Ser Asn Ile Gly His Thr
               820                 825                 830
   atg gct gcc gcg ggt gtg ggt ggg gtc atc aag atg gtg atg gcg ttg     2544
   Met Ala Ala Ala Gly Val Gly Gly Val Ile Lys Met Val Met Ala Leu
           835                 840                 845
   cgg gag ggg gtg ttg ccg cgg acg ttg cat gtg gat aag ccg tcg ccg     2592
   Arg Glu Gly Val Leu Pro Arg Thr Leu His Val Asp Lys Pro Ser Pro
       850                 855                 860
   cag gtg gac tgg tcc gcg ggg gcg gtg cgg ctg ctg acg gag gcg gtg     2640
   Gln Val Asp Trp Ser Ala Gly Ala Val Arg Leu Leu Thr Glu Ala Val
   865                 870                 875                 880
   ccg tgg ccg ggg gac gcg gca ggg cgg ttg cgg cgg gcg gga gtg tcg     2688
   Pro Trp Pro Gly Asp Ala Ala Gly Arg Leu Arg Arg Ala Gly Val Ser
                   885                 890                 895
   tcg ttc ggg atc ggc ggc acg aat gcg cat gtg att ttg gag gag gcg     2736
   Ser Phe Gly Ile Gly Gly Thr Asn Ala His Val Ile Leu Glu Glu Ala
               900                 905                 910
   ccg gcg gcg ggg ggc tgt gtt gcc ggg ggt ggg gtg ttg gag ggt gct     2784
   Pro Ala Ala Gly Gly Cys Val Ala Gly Gly Gly Val Leu Glu Gly Ala
           915                 920                 925
   ccg ggt ctt gcc att tcg gtg gct gag tcg gtg gcc gct cca gtg gct     2832
   Pro Gly Leu Ala Ile Ser Val Ala Glu Ser Val Ala Ala Pro Val Ala
       930                 935                 940
   gtg tct gcg ccg gtg gct gag tcg gtg ccg gtg ccg gtg ccg gtg ccg     2880
   Val Ser Ala Pro Val Ala Glu Ser Val Pro Val Pro Val Pro Val Pro
   945                 950                 955                 960
   gtt cct gtg ccg gtg tcg gct agg tct gag gct ggg ttg cgg gcg cag     2928
   Val Pro Val Pro Val Ser Ala Arg Ser Glu Ala Gly Leu Arg Ala Gln
   965 970 975
   gcg gag gcg ttg cgt cag tac gtg gca gtc cgg ccg gac gtt tcg ctt 2976
   Ala Glu Ala Leu Arg Gln Tyr Val Ala Val Arg Pro Asp Val Ser Leu
   980 985 990
   gcc gat gtg ggt gcg ggt ctg gcc tgt ggg cgg gct gtg ctg gag cat 3024
   Ala Asp Val Gly Ala Gly Leu Ala Cys Gly Arg Ala Val Leu Glu His
   995 1000 1005
   cgt gcg gtc gtc ctg gcc gcg gac cgt gag gag ctg gtg caa ggg ttg 3072
   Arg Ala Val Val Leu Ala Ala Asp Arg Glu Glu Leu Val Gln Gly Leu
   1010 1015 1020
   ggg gcg ctg gcg gcg ggt gag ccg gat cgg cgg gtg acc acg ggt cat 3120
   Gly Ala Leu Ala Ala Gly Glu Pro Asp Arg Arg Val Thr Thr Gly His
   1025 1030 1035 1040
   gcg ccg ggt ggt gac cgg ggc ggt gtc gtc ttc gtg ttt ccc gga cag 3168
   Ala Pro Gly Gly Asp Arg Gly Gly Val Val Phe Val Phe Pro Gly Gln
   1045 1050 1055
   ggt ggg cag tgg gcc ggg atg ggt gtg cgt ctg ctc gcc tcc tct ccg 3216
   Gly Gly Gln Trp Ala Gly Met Gly Val Arg Leu Leu Ala Ser Ser Pro
   1060 1065 1070
   gtg ttc gcc cgg cgg atg cag gcg tgc gag gag gct ctg gcg ccg tgg 3264
   Val Phe Ala Arg Arg Met Gln Ala Cys Glu Glu Ala Leu Ala Pro Trp
   1075 1080 1085
   gtg gac tgg tct gtg gtg gac atc ctg cgc cgg gac gcg ggg gat gcg 3312
   Val Asp Trp Ser Val Val Asp Ile Leu Arg Arg Asp Ala Gly Asp Ala
   1090 1095 1100
   gtg tgg gag cgg gcc gat gtg gtc cag cct gtg ctg ttc agc gtc atg 3360
   Val Trp Glu Arg Ala Asp Val Val Gln Pro Val Leu Phe Ser Val Met
   1105 1110 1115 1120
   gtg tct ttg gct gct ctg tgg cgt tcc tac ggt atc gaa ccc gac gcg 3408
   Val Ser Leu Ala Ala Leu Trp Arg Ser Tyr Gly Ile Glu Pro Asp Ala
   1125 1130 1135
   gtc ctt ggc cat tcc cag ggc gag atc gcg gcc gcg cat gtg tgt ggg 3456
   Val Leu Gly His Ser Gln Gly Glu Ile Ala Ala Ala His Val Cys Gly
   1140 1145 1150
   gcg ctg agc ctg aag gac gcg gcg aag act gtt gcg ctg cgc agc cgg 3504
   Ala Leu Ser Leu Lys Asp Ala Ala Lys Thr Val Ala Leu Arg Ser Arg
   1155 1160 1165
   gcg ctg gcc gct gtg cgg ggc cgg ggc ggc atg gcc tca gtg ccg ctg 3552
   Ala Leu Ala Ala Val Arg Gly Arg Gly Gly Met Ala Ser Val Pro Leu
   1170 1175 1180
   cct gcc cag gag gtg gag cag ctc att ggt gag cgg tgg gcg ggg cgg 3600
   Pro Ala Gln Glu Val Glu Gln Leu Ile Gly Glu Arg Trp Ala Gly Arg
   1185 1190 1195 1200
   ttg tgg gtg gcg gcg gtc aac ggc ccc cgc tcc acc gcc gtc tcg ggg     3648
   Leu Trp Val Ala Ala Val Asn Gly Pro Arg Ser Thr Ala Val Ser Gly
                   1205                1210                1215
   gat gcc gag gcg gtg gac gag gtg ctg gcg tac tgt gcc ggc acc ggg     3696
   Asp Ala Glu Ala Val Asp Glu Val Leu Ala Tyr Cys Ala Gly Thr Gly
               1220                1225                1230
   gtg cgg gcc cgg cgg atc ccg gtc gac tat gcc tcg cac tgc ccc cat     3744
   Val Arg Ala Arg Arg Ile Pro Val Asp Tyr Ala Ser His Cys Pro His
           1235                1240                1245
   gtg cag ccc ctg cgg gag gag ttg ctg gag ctg ctg ggg gac atc agc     3792
   Val Gln Pro Leu Arg Glu Glu Leu Leu Glu Leu Leu Gly Asp Ile Ser
       1250                1255                1260
   ccg cag ccg tcc ggc gtg ccg ttc ttc tcc acg gtg gag ggc acc tgg     3840
   Pro Gln Pro Ser Gly Val Pro Phe Phe Ser Thr Val Glu Gly Thr Trp
   1265                1270                1275                1280
   ctg gac acc aca acc ctg gac gcc gcc tac tgg tac cgc aac ctg cac     3888
   Leu Asp Thr Thr Thr Leu Asp Ala Ala Tyr Trp Tyr Arg Asn Leu His
                   1285                1290                1295
   cag ccg gtc cgt ttc agc gat gcc gtc cag gcc ctg gcg gat gac gga     3936
   Gln Pro Val Arg Phe Ser Asp Ala Val Gln Ala Leu Ala Asp Asp Gly
               1300                1305                1310
   cac cgc gtc ttc gtc gaa gtc agc ccc cac ccc acc ctc gtc ccc gcc     3984
   His Arg Val Phe Val Glu Val Ser Pro His Pro Thr Leu Val Pro Ala
           1315                1320                1325
   atc gaa gac acc acc gaa gac acc gcc gaa gac gtc acc gcg atc ggc     4032
   Ile Glu Asp Thr Thr Glu Asp Thr Ala Glu Asp Val Thr Ala Ile Gly
       1330                1335                1340
   agc ctc cgc cgc ggc gac aac gac acc cgc cgc ttc ctc acc gcc ctc     4080
   Ser Leu Arg Arg Gly Asp Asn Asp Thr Arg Arg Phe Leu Thr Ala Leu
   1345                1350                1355                1360
   gcc cac acc cat acc acc ggc atc ggc aca ccc acc acc tgg cac cac     4128
   Ala His Thr His Thr Thr Gly Ile Gly Thr Pro Thr Thr Trp His His
                   1365                1370                1375
   cac tac acc cac cac cac acc cac ccc cac ccc cac acg cac ctc gac     4176
   His Tyr Thr His His His Thr His Pro His Pro His Thr His Leu Asp
               1380                1385                1390
   ctg ccc acc tac ccc ttc caa cac cag cac tac tgg ctc gag agc tca     4224
   Leu Pro Thr Tyr Pro Phe Gln His Gln His Tyr Trp Leu Glu Ser Ser
           1395                1400                1405
   cag ccg ggt gcc gga tcc ggt tcg ggt gcc ggt gcc ggt tcg ggt gcc     4272
   Gln Pro Gly Ala Gly Ser Gly Ser Gly Ala Gly Ala Gly Ser Gly Ala
       1410                1415                1420
   ggt tcc ggg cgg gca ggg act gcg ggc ggg acg gca gag gtg gag tcg    4320
   Gly Ser Gly Arg Ala Gly Thr Ala Gly Gly Thr Ala Glu Val Glu Ser
   1425                1430                1435                1440
   cgg ttc tgg gac gcg gtg gcc cgc cag gac ctg gaa acg gtc gcg acc    4368
   Arg Phe Trp Asp Ala Val Ala Arg Gln Asp Leu Glu Thr Val Ala Thr
                   1445                1450                1455
   aca ctc gcc gtg ccc ccc tcc gcc ggc ctg gac acg gtg gtg ccc gca    4416
   Thr Leu Ala Val Pro Pro Ser Ala Gly Leu Asp Thr Val Val Pro Ala
               1460                1465                1470
   ctc tcc gcc tgg cac cgc cac caa cac gac caa gcc cgc atc aac acc    4464
   Leu Ser Ala Trp His Arg His Gln His Asp Gln Ala Arg Ile Asn Thr
           1475                1480                1485
   tgg acc tac cag gaa acc tgg aaa ccc ctc acc ctc ccc acc acc cac    4512
   Trp Thr Tyr Gln Glu Thr Trp Lys Pro Leu Thr Leu Pro Thr Thr His
       1490                1495                1500
   caa ccc cac caa acc tgg ctc atc gcc atc ccc gaa acc cag acc cac    4560
   Gln Pro His Gln Thr Trp Leu Ile Ala Ile Pro Glu Thr Gln Thr His
   1505                1510                1515                1520
   cac ccc cac atc acc aac atc ctc acc aac ctc cac cac cac ggc atc    4608
   His Pro His Ile Thr Asn Ile Leu Thr Asn Leu His His His Gly Ile
                   1525                1530                1535
   acc ccc atc ccc ctc acc ctc aac cac acc cac acc aac ccc caa cac    4656
   Thr Pro Ile Pro Leu Thr Leu Asn His Thr His Thr Asn Pro Gln His
               1540                1545                1550
   ctc cac cac acc ctc cac cac acc cga caa caa gcc caa aac cac acc    4704
   Leu His His Thr Leu His His Thr Arg Gln Gln Ala Gln Asn His Thr
           1555                1560                1565
   acc gga gcc atc acc ggc ctg ctc tcc ctc ctc gcc ctc gac gaa aca    4752
   Thr Gly Ala Ile Thr Gly Leu Leu Ser Leu Leu Ala Leu Asp Glu Thr
       1570                1575                1580
   ccc cac ccc cac cac ccc cac aca ccc acc ggc acc ctc ctc aac ctc    4800
   Pro His Pro His His Pro His Thr Pro Thr Gly Thr Leu Leu Asn Leu
   1585                1590                1595                1600
   acc ctc acc caa acc cac acc caa acc cac cca cca acc ccc ctc tgg    4848
   Thr Leu Thr Gln Thr His Thr Gln Thr His Pro Pro Thr Pro Leu Trp
                   1605                1610                1615
   tac gcc acc acc aac gcc acc acc acc cac ccc aac gac ccc ctc aca    4896
   Tyr Ala Thr Thr Asn Ala Thr Thr Thr His Pro Asn Asp Pro Leu Thr
               1620                1625                1630
   cac ccc acc caa gcc caa acc tgg gga ctc gcc cgc acc acc ctc ctc    4944
   His Pro Thr Gln Ala Gln Thr Trp Gly Leu Ala Arg Thr Thr Leu Leu
           1635                1640                1645
   gaa cac ccc acc cac acc gcc gga atc atc gac ctc ccc acc acc ccc    4992
   Glu His Pro Thr His Thr Ala Gly Ile Ile Asp Leu Pro Thr Thr Pro
   1650 1655 1660
   acc ccc cac acc ctc cag cac ctc acc caa acc ctc acc caa ccc cac 5040
   Thr Pro His Thr Leu Gln His Leu Thr Gln Thr Leu Thr Gln Pro His
   1665 1670 1675 1680
   cac caa acc caa ctc gcc atc cgc acc acc ggc acc cac acc cgc cgc 5088
   His Gln Thr Gln Leu Ala Ile Arg Thr Thr Gly Thr His Thr Arg Arg
   1685 1690 1695
   ctc acc ccc acc acc ctc acc ccc aca cac caa cca ccc acc ccc acc 5136
   Leu Thr Pro Thr Thr Leu Thr Pro Thr His Gln Pro Pro Thr Pro Thr
   1700 1705 1710
   ccc cac gga acc acc ctc atc acc ggc gga acc ggc gcc ctc gcc acc 5184
   Pro His Gly Thr Thr Leu Ile Thr Gly Gly Thr Gly Ala Leu Ala Thr
   1715 1720 1725
   cac ctc acc cac cac ctc acc acc cac caa ccc acc caa cac ctc ctc 5232
   His Leu Thr His His Leu Thr Thr His Gln Pro Thr Gln His Leu Leu
   1730 1735 1740
   ctc acc agc cga acc ggc ccc cac acc ccc cac gca caa cac ctc acc 5280
   Leu Thr Ser Arg Thr Gly Pro His Thr Pro His Ala Gln His Leu Thr
   1745 1750 1755 1760
   acc caa ctc caa caa aaa ggc atc cac ctc acc atc acc acc tgc gac 5328
   Thr Gln Leu Gln Gln Lys Gly Ile His Leu Thr Ile Thr Thr Cys Asp
   1765 1770 1775
   acc agc aac cca gac caa ctc caa caa ctc ctc aac acc atc ccc cca 5376
   Thr Ser Asn Pro Asp Gln Leu Gln Gln Leu Leu Asn Thr Ile Pro Pro
   1780 1785 1790
   caa cac ccc ctc acc acc gtc atc cac acc gca ggc atc ctc gac gac 5424
   Gln His Pro Leu Thr Thr Val Ile His Thr Ala Gly Ile Leu Asp Asp
   1795 1800 1805
   gcc acc ctc acc aac ctc acc ccc acc caa ctc aac aac gtc ctc cgc 5472
   Ala Thr Leu Thr Asn Leu Thr Pro Thr Gln Leu Asn Asn Val Leu Arg
   1810 1815 1820
   gcc aaa gcc cac agc gcc cac ctc ctc cac caa ctc acc caa cac acc 5520
   Ala Lys Ala His Ser Ala His Leu Leu His Gln Leu Thr Gln His Thr
   1825 1830 1835 1840
   ccc ctc acc gcc ttc gtc ctc tac tcc tcc gcc gcc gcc acc ttc ggc 5568
   Pro Leu Thr Ala Phe Val Leu Tyr Ser Ser Ala Ala Ala Thr Phe Gly
   1845 1850 1855
   gca ccc ggc caa gcc aac tac gcc gca gcc aac gcc tac ctc gac gcc 5616
   Ala Pro Gly Gln Ala Asn Tyr Ala Ala Ala Asn Ala Tyr Leu Asp Ala
   1860 1865 1870
   ctc gcc cac cac cgc cac acc cac cac ctc ccc gcc acc agc atc gcc 5664
   Leu Ala His His Arg His Thr His His Leu Pro Ala Thr Ser Ile Ala
   1875 1880 1885
   tgg ggc acc tgg caa gga aac gga ctc gct gat tcg gac aag gcc cgc 5712
   Trp Gly Thr Trp Gln Gly Asn Gly Leu Ala Asp Ser Asp Lys Ala Arg
   1890 1895 1900
   gca tat ctc gac cgc cgc ggg ttt cga ccc atg tca ccc gag ttg gcc 5760
   Ala Tyr Leu Asp Arg Arg Gly Phe Arg Pro Met Ser Pro Glu Leu Ala
   1905 1910 1915 1920
   acg gca gcg gtc acg cag gcg atc gcg gac acc gaa cgg ccg tat gtc 5808
   Thr Ala Ala Val Thr Gln Ala Ile Ala Asp Thr Glu Arg Pro Tyr Val
   1925 1930 1935
   gtc atc gcc gac atc gac tgg agc aag atc gaa cac acc tct cag acc 5856
   Val Ile Ala Asp Ile Asp Trp Ser Lys Ile Glu His Thr Ser Gln Thr
   1940 1945 1950
   agc gac ctg gtg agc gcg gcc cgg gaa agg gag cca gct gtc cag cgc 5904
   Ser Asp Leu Val Ser Ala Ala Arg Glu Arg Glu Pro Ala Val Gln Arg
   1955 1960 1965
   ccc act cca ccg gcg gag ttg cac aaa acg ctg gcc cat cag acg tcg 5952
   Pro Thr Pro Pro Ala Glu Leu His Lys Thr Leu Ala His Gln Thr Ser
   1970 1975 1980
   gcc gac caa cgg gcc gca ttg ctc gag ctc gta cga gac cat gtg gcg 6000
   Ala Asp Gln Arg Ala Ala Leu Leu Glu Leu Val Arg Asp His Val Ala
   1985 1990 1995 2000
   gca gtg ctc cgg cac gcg gac ccg aaa gcc atc gcg ccc gac cag tcg 6048
   Ala Val Leu Arg His Ala Asp Pro Lys Ala Ile Ala Pro Asp Gln Ser
   2005 2010 2015
   ttc cgt gca ctc ggc ttc gat tca ctc acg gcc gtc gag ttc cga aac 6096
   Phe Arg Ala Leu Gly Phe Asp Ser Leu Thr Ala Val Glu Phe Arg Asn
   2020 2025 2030
   ctg ctg atc aag gca aca gga ctc cgc ctt cct gtc tcg ctg gtc ttc 6144
   Leu Leu Ile Lys Ala Thr Gly Leu Arg Leu Pro Val Ser Leu Val Phe
   2035 2040 2045
   gac cac ccg acc cct gcc aaa ctc gcc gta cac ctg cag aac caa ctg 6192
   Asp His Pro Thr Pro Ala Lys Leu Ala Val His Leu Gln Asn Gln Leu
   2050 2055 2060
   cgg ggc aca gca gcg gag tcg gct cct tca gcg gca gcc gtt acc gcc 6240
   Arg Gly Thr Ala Ala Glu Ser Ala Pro Ser Ala Ala Ala Val Thr Ala
   2065 2070 2075 2080
   gag gct tct gtc acc gag ccg atc gcc atc gtt ggc atg gcc tgt cgt 6288
   Glu Ala Ser Val Thr Glu Pro Ile Ala Ile Val Gly Met Ala Cys Arg
   2085 2090 2095
   ttc ccc ggc gga gtg acc tcg gcg gac gac ttc tgg gat ctg atc tcc 6336
   Phe Pro Gly Gly Val Thr Ser Ala Asp Asp Phe Trp Asp Leu Ile Ser
   2100 2105 2110
   tcc gag cag gac gcg atc ggc gga ttc ccc acc gac cgc ggc tgg gac     6384
   Ser Glu Gln Asp Ala Ile Gly Gly Phe Pro Thr Asp Arg Gly Trp Asp
           2115                2120                2125
   ctg gac acg ctc tac gac ccc gac ccc gac cac ccc ggc acc tgc tac     6432
   Leu Asp Thr Leu Tyr Asp Pro Asp Pro Asp His Pro Gly Thr Cys Tyr
       2130                2135                2140
   acc cga aac ggc gga ttc ctc tac gac gca ggc cac ttc gac gcc gaa     6480
   Thr Arg Asn Gly Gly Phe Leu Tyr Asp Ala Gly His Phe Asp Ala Glu
   2145                2150                2155                2160
   ttc ttc ggc atc agc ccc cgc gaa gcc ctc gcc atg gac ccc cag caa     6528
   Phe Phe Gly Ile Ser Pro Arg Glu Ala Leu Ala Met Asp Pro Gln Gln
                   2165                2170                2175
   cga ctc ctc ctc gaa acc gcc tgg gaa acc atc gaa cac gcc ggc atc     6576
   Arg Leu Leu Leu Glu Thr Ala Trp Glu Thr Ile Glu His Ala Gly Ile
               2180                2185                2190
   aac ccc cac acc ctc cac ggc acc ccc acc gga gtc ttc acc ggc acc     6624
   Asn Pro His Thr Leu His Gly Thr Pro Thr Gly Val Phe Thr Gly Thr
           2195                2200                2205
   aac gga cag gac tac gca ctt cgc gtg cac aac gcg ggc cag tca acc     6672
   Asn Gly Gln Asp Tyr Ala Leu Arg Val His Asn Ala Gly Gln Ser Thr
       2210                2215                2220
   gat ggt ttc gca ctg acc gga acc gcc ggc agc gtc atc tcc ggt cgt     6720
   Asp Gly Phe Ala Leu Thr Gly Thr Ala Gly Ser Val Ile Ser Gly Arg
   2225                2230                2235                2240
   atc tcg tac acg ttt ggt ttt gag ggt cct gcg gtg tcg gtg gac acg     6768
   Ile Ser Tyr Thr Phe Gly Phe Glu Gly Pro Ala Val Ser Val Asp Thr
                   2245                2250                2255
   gct tgt tcc tcg tcg ttg gtg gct ttg cat ctg gcc tgt cag gcg ttg     6816
   Ala Cys Ser Ser Ser Leu Val Ala Leu His Leu Ala Cys Gln Ala Leu
               2260                2265                2270
   cgt gcg ggt gag tgc tcg atg gcg ctt gcc ggg ggt gtg acg gtg atg     6864
   Arg Ala Gly Glu Cys Ser Met Ala Leu Ala Gly Gly Val Thr Val Met
           2275                2280                2285
   tcg tct ccg ggt gcc ttc gtg gag ttt tcg cgg cag cgg ggt ctg gcc     6912
   Ser Ser Pro Gly Ala Phe Val Glu Phe Ser Arg Gln Arg Gly Leu Ala
       2290                2295                2300
   gcg gac ggg cat tgc aag gcg ttc tcg gcg gcg gcg gac ggg acc ggc     6960
   Ala Asp Gly His Cys Lys Ala Phe Ser Ala Ala Ala Asp Gly Thr Gly
   2305                2310                2315                2320
   tgg ggt gag ggt gtg ggg atg ctg ctg gtg gag cgg ctc tcc gac gcc     7008
   Trp Gly Glu Gly Val Gly Met Leu Leu Val Glu Arg Leu Ser Asp Ala
                   2325                2330                2335
   cat cgc aac ggt cac cgt gtc ctg gcc gtg gtg cgt ggc agt gcg gtc 7056
   His Arg Asn Gly His Arg Val Leu Ala Val Val Arg Gly Ser Ala Val
   2340 2345 2350
   aac cag gac ggt gcg agc aac ggt ctg acc gcg ccc aac ggg ccg tcc 7104
   Asn Gln Asp Gly Ala Ser Asn Gly Leu Thr Ala Pro Asn Gly Pro Ser
   2355 2360 2365
   cag cag cgt gtc atc cgc cag gcc ctc gcc aac gcc ggc ttg tcg gcc 7152
   Gln Gln Arg Val Ile Arg Gln Ala Leu Ala Asn Ala Gly Leu Ser Ala
   2370 2375 2380
   ggt gat gtc gac gcg gtg gag gcc cac ggc acc ggc acc act ttg ggc 7200
   Gly Asp Val Asp Ala Val Glu Ala His Gly Thr Gly Thr Thr Leu Gly
   2385 2390 2395 2400
   gac ccg atc gag gcc cag gcc ctc ctc gcg acc tac gga cag gac cgt 7248
   Asp Pro Ile Glu Ala Gln Ala Leu Leu Ala Thr Tyr Gly Gln Asp Arg
   2405 2410 2415
   gcc ggc gag ggg ccg ctg tgg ctg ggc tcg gtc aag tcc aat gtc ggt 7296
   Ala Gly Glu Gly Pro Leu Trp Leu Gly Ser Val Lys Ser Asn Val Gly
   2420 2425 2430
   cac aca cag gct gcc gcg ggc gtc gcc ggg gtg atc aag atg gtg atg 7344
   His Thr Gln Ala Ala Ala Gly Val Ala Gly Val Ile Lys Met Val Met
   2435 2440 2445
   gcg ctg cgg cat ggt ctg ctg ccg cgg acg ttg cat gtg gat gag ccg 7392
   Ala Leu Arg His Gly Leu Leu Pro Arg Thr Leu His Val Asp Glu Pro
   2450 2455 2460
   tcg ccg cat gtg gac tgg tcc gcg ggt gcg gtg cag ctg ctg acg gag 7440
   Ser Pro His Val Asp Trp Ser Ala Gly Ala Val Gln Leu Leu Thr Glu
   2465 2470 2475 2480
   acg gtg ccc tgg ccc ggc ggg gag ggg cgg cta cgg cgg gca gga gtg 7488
   Thr Val Pro Trp Pro Gly Gly Glu Gly Arg Leu Arg Arg Ala Gly Val
   2485 2490 2495
   tca tca ttc ggc gtc agc ggc acc aac gcc cac gtc atc ctc gaa gaa 7536
   Ser Ser Phe Gly Val Ser Gly Thr Asn Ala His Val Ile Leu Glu Glu
   2500 2505 2510
   gca ccc gcc gac gac gtt ccg ggg gga cca ccc gcc ggc gag ggt gac 7584
   Ala Pro Ala Asp Asp Val Pro Gly Gly Pro Pro Ala Gly Glu Gly Asp
   2515 2520 2525
   gcg ggc agc gac gat gag gct gct gcc ggc agt cct ggg gtg tgg ccg 7632
   Ala Gly Ser Asp Asp Glu Ala Ala Ala Gly Ser Pro Gly Val Trp Pro
   2530 2535 2540
   tgg ctg gtg tcg gcc aag tcg cag ccg gcc ctg cgc gcc cag gcc cag 7680
   Trp Leu Val Ser Ala Lys Ser Gln Pro Ala Leu Arg Ala Gln Ala Gln
   2545 2550 2555 2560
   gcc ctg cac gcc cac ctc acc gac cac ccc ggc ctc gac ctc gcg gat 7728
   Ala Leu His Ala His Leu Thr Asp His Pro Gly Leu Asp Leu Ala Asp
                   2565                2570                2575
   gtc gga tac acc ctc gcc cac gcc cgc gcc gtg ttc gac cac cgc gcc    7776
   Val Gly Tyr Thr Leu Ala His Ala Arg Ala Val Phe Asp His Arg Ala
               2580                2585                2590
   acc ctc atc gcc gcg gac cgc gac acg ttc ctg caa gca ctc cag gca    7824
   Thr Leu Ile Ala Ala Asp Arg Asp Thr Phe Leu Gln Ala Leu Gln Ala
           2595                2600                2605
   ctc gcc gca ggc gag ccc cac ccc gcc gtc atc cac agc agc gcc ccg    7872
   Leu Ala Ala Gly Glu Pro His Pro Ala Val Ile His Ser Ser Ala Pro
       2610                2615                2620
   ggc ggg acc ggg acc ggg gag gcc gca gga aag acc gca ttc atc tgc    7920
   Gly Gly Thr Gly Thr Gly Glu Ala Ala Gly Lys Thr Ala Phe Ile Cys
   2625                2630                2635                2640
   tcc gga cag ggc acc caa cgc ccc ggc atg gcc cac ggc ctc tac cac    7968
   Ser Gly Gln Gly Thr Gln Arg Pro Gly Met Ala His Gly Leu Tyr His
                   2645                2650                2655
   acc cac ccc gtc ttc gcc gcc gca ctc aac gac atc tgc acc cac ctc    8016
   Thr His Pro Val Phe Ala Ala Ala Leu Asn Asp Ile Cys Thr His Leu
               2660                2665                2670
   gac ccc cac ctc gac cac ccc ctc ctc ccc ctc ctc acc caa aac gac    8064
   Asp Pro His Leu Asp His Pro Leu Leu Pro Leu Leu Thr Gln Asn Asp
           2675                2680                2685
   aac gac aac gag gac gcg gcc gca ctg ctc cag cag acc cgc tac gcc    8112
   Asn Asp Asn Glu Asp Ala Ala Ala Leu Leu Gln Gln Thr Arg Tyr Ala
       2690                2695                2700
   cag ccc gcc ctc ttc gcc ttc cag gtc gcc ctc cac cgc ctc ctc acc    8160
   Gln Pro Ala Leu Phe Ala Phe Gln Val Ala Leu His Arg Leu Leu Thr
   2705                2710                2715                2720
   gac ggc tac cac atc acc ccc cac tac tac gcc gga cac tcc ctc ggc    8208
   Asp Gly Tyr His Ile Thr Pro His Tyr Tyr Ala Gly His Ser Leu Gly
                   2725                2730                2735
   gaa atc acc gcc gcc cac ctc gcc ggc atc ctc acc ctc acc gac gcc    8256
   Glu Ile Thr Ala Ala His Leu Ala Gly Ile Leu Thr Leu Thr Asp Ala
               2740                2745                2750
   acc acc ctc atc acc caa cgc gcc acc ctc atg caa acc atg ccc ccc    8304
   Thr Thr Leu Ile Thr Gln Arg Ala Thr Leu Met Gln Thr Met Pro Pro
           2755                2760                2765
   ggc acc atg acc acc ctc cac acc acc ccc cac cac atc acc cac cac    8352
   Gly Thr Met Thr Thr Leu His Thr Thr Pro His His Ile Thr His His
       2770                2775                2780
   ctc acc gcc cac gaa aac gac ctc gcc atc gcc gcc atc aac acc ccc    8400
   Leu Thr Ala His Glu Asn Asp Leu Ala Ile Ala Ala Ile Asn Thr Pro
   2785                2790                2795                2800
   acc tcc ctc gtc atc agc ggc acc ccc cac acc gtc caa cac atc acc    8448
   Thr Ser Leu Val Ile Ser Gly Thr Pro His Thr Val Gln His Ile Thr
                   2805                2810                2815
   acc ctc tgc caa caa caa ggc atc aaa acc aaa acc ctc ccc acc aac    8496
   Thr Leu Cys Gln Gln Gln Gly Ile Lys Thr Lys Thr Leu Pro Thr Asn
               2820                2825                2830
   cac gcc ttc cac tcc ccc cac acc aac ccc atc ctc aac caa ctc cac    8544
   His Ala Phe His Ser Pro His Thr Asn Pro Ile Leu Asn Gln Leu His
           2835                2840                2845
   cag cac acc caa acc ctc acc tac cac cca ccc cac acc ccc ctc atc    8592
   Gln His Thr Gln Thr Leu Thr Tyr His Pro Pro His Thr Pro Leu Ile
       2850                2855                2860
   acc gcc aac acc cca ccc gac caa ctc ctc acc ccc cac tac tgg acc    8640
   Thr Ala Asn Thr Pro Pro Asp Gln Leu Leu Thr Pro His Tyr Trp Thr
   2865                2870                2875                2880
   caa caa gcc cgc aac acc gtc gac tac gcc acc acc acc caa acc ctc    8688
   Gln Gln Ala Arg Asn Thr Val Asp Tyr Ala Thr Thr Thr Gln Thr Leu
                   2885                2890                2895
   cac caa cac ggc gtc acc acc tac atc gaa ctc gga ccc gac aac acc    8736
   His Gln His Gly Val Thr Thr Tyr Ile Glu Leu Gly Pro Asp Asn Thr
               2900                2905                2910
   ctc acc acc ctc acc cac cac aac ctc ccc aac ccc ccc acc acc acc    8784
   Leu Thr Thr Leu Thr His His Asn Leu Pro Asn Pro Pro Thr Thr Thr
           2915                2920                2925
   ctc acc ctc acc cac ccc cac cac cac ccc caa acc cac ctc ctc acc    8832
   Leu Thr Leu Thr His Pro His His His Pro Gln Thr His Leu Leu Thr
       2930                2935                2940
   aac ctc gcc aaa acc acc acc acc tgg cac ccc cac cac tac acc cac    8880
   Asn Leu Ala Lys Thr Thr Thr Thr Trp His Pro His His Tyr Thr His
   2945                2950                2955                2960
   cac gac aac caa ccc cac acc cac acc cac ctc gac ctc ccc acc tac    8928
   His Asp Asn Gln Pro His Thr His Thr His Leu Asp Leu Pro Thr Tyr
                   2965                2970                2975
   ccc ttc caa cac cac cac tac tgg ctc gaa agc aca cag ccc ggt gcc    8976
   Pro Phe Gln His His His Tyr Trp Leu Glu Ser Thr Gln Pro Gly Ala
               2980                2985                2990
   ggc aac gtg tca gca gcc gga ctc gac ccc acc gaa cac ccc cta ctc    9024
   Gly Asn Val Ser Ala Ala Gly Leu Asp Pro Thr Glu His Pro Leu Leu
           2995                3000                3005
   ggc gcc aca ttg gaa ctg gcg act gac ggt gga gcg ctt ctt gca ggg    9072
   Gly Ala Thr Leu Glu Leu Ala Thr Asp Gly Gly Ala Leu Leu Ala Gly
       3010                3015                3020
   cgc ttg tct ttg agg tcg cat ccg tgg ctg gct gac cat gcc gtc ggc     9120
   Arg Leu Ser Leu Arg Ser His Pro Trp Leu Ala Asp His Ala Val Gly
   3025                3030                3035                3040
   ggc acg gtg ctg ctg tcg ggc gcc acc ttc ctc gaa ctc gcc ctt cat     9168
   Gly Thr Val Leu Leu Ser Gly Ala Thr Phe Leu Glu Leu Ala Leu His
                   3045                3050                3055
   gcg ggc aca tac gtg ggc tgc gac cga gtg gat gag ctg acg ctg cat     9216
   Ala Gly Thr Tyr Val Gly Cys Asp Arg Val Asp Glu Leu Thr Leu His
               3060                3065                3070
   gcg ccg ctg gtg gtt cct gtg gat ggg ggt gtg agt gtg cag gtt ggg     9264
   Ala Pro Leu Val Val Pro Val Asp Gly Gly Val Ser Val Gln Val Gly
           3075                3080                3085
   gtt gcg gct gcg gat ggg gag ggg cgg cgt ttg gtg agt gtg tat gcg     9312
   Val Ala Ala Ala Asp Gly Glu Gly Arg Arg Leu Val Ser Val Tyr Ala
       3090                3095                3100
   cgg ggt ggg agt gct tgt ggt ggg ggt ggt gcg tcg ggt ggg gtg tgg     9360
   Arg Gly Gly Ser Ala Cys Gly Gly Gly Gly Ala Ser Gly Gly Val Trp
   3105                3110                3115                3120
   acg tgt cat gcc tcg ggg gtg ctg gtt gag gct gct gct ggt ggt gtg     9408
   Thr Cys His Ala Ser Gly Val Leu Val Glu Ala Ala Ala Gly Gly Val
                   3125                3130                3135
   gtg gtg gat ggt ctg gcg ggg gtg tgg ccg ccg cgg ggt gcg gtg gcg     9456
   Val Val Asp Gly Leu Ala Gly Val Trp Pro Pro Arg Gly Ala Val Ala
               3140                3145                3150
   gtg gat gtc gat ggt gtc cgt gac cgt ttg gct ggg gct ggt tgt gtt     9504
   Val Asp Val Asp Gly Val Arg Asp Arg Leu Ala Gly Ala Gly Cys Val
           3155                3160                3165
   ttg ggg ccg gtg ttt tcg ggg ctg cgt gcg gtg tgg cgt gat ggg ggg     9552
   Leu Gly Pro Val Phe Ser Gly Leu Arg Ala Val Trp Arg Asp Gly Gly
       3170                3175                3180
   gat ttg ctg gct gag gtg tgt ctg ccg gag gag gcg tgg ggt gat gcg     9600
   Asp Leu Leu Ala Glu Val Cys Leu Pro Glu Glu Ala Trp Gly Asp Ala
   3185                3190                3195                3200
   gct ggt ttt ggg ctg cat ccg gcg ttg ctg gat ggt gtg gtc cag ccg     9648
   Ala Gly Phe Gly Leu His Pro Ala Leu Leu Asp Gly Val Val Gln Pro
                   3205                3210                3215
   ttg tcg gtg ttg ctt ccg ggt ggg acg ggg ttt ggg gag ggg gcg ggg     9696
   Leu Ser Val Leu Leu Pro Gly Gly Thr Gly Phe Gly Glu Gly Ala Gly
               3220                3225                3230
   ttc ggg gag ggt gtt cgg gtg ccg gct gtg tgg ggt ggt gtg tcg ctt     9744
   Phe Gly Glu Gly Val Arg Val Pro Ala Val Trp Gly Gly Val Ser Leu
           3235                3240                3245
   cac cgg gcg ggt gtg acc ggt gtg cgg gtg cgt gtg tcg gct gtc ggg 9792
   His Arg Ala Gly Val Thr Gly Val Arg Val Arg Val Ser Ala Val Gly
   3250 3255 3260
   cgg ggc ggc ggg cgt gag gcg gtg tcg gtc gtg gtc ggg gat gag gcg 9840
   Arg Gly Gly Gly Arg Glu Ala Val Ser Val Val Val Gly Asp Glu Ala
   3265 3270 3275 3280
   ggt gtg ccg gtg gcg tcg gtc gat cgt ctt gag ttg cgg cct gtg gat 9888
   Gly Val Pro Val Ala Ser Val Asp Arg Leu Glu Leu Arg Pro Val Asp
   3285 3290 3295
   atg ggt cag ttg cgt gct gtc tcg gtt tcg gcg ggg cgg cgg ggt tcg 9936
   Met Gly Gln Leu Arg Ala Val Ser Val Ser Ala Gly Arg Arg Gly Ser
   3300 3305 3310
   ctg tat gcg gtg cag tgg gct gag gtg ggt cct gtg ccg gtg tgt ggg 9984
   Leu Tyr Ala Val Gln Trp Ala Glu Val Gly Pro Val Pro Val Cys Gly
   3315 3320 3325
   cag gcg tgg gcg tgg cac gag gac gtg ggt gag agc ggt ggt ggg cct 10032
   Gln Ala Trp Ala Trp His Glu Asp Val Gly Glu Ser Gly Gly Gly Pro
   3330 3335 3340
   gtg ccg ggg gtg gtg gtg ttg cgg tgc ccg gat gcc ggt gcc ggt ggc 10080
   Val Pro Gly Val Val Val Leu Arg Cys Pro Asp Ala Gly Ala Gly Gly
   3345 3350 3355 3360
   ggt ggc ggt ggc ggt ggt ggc ggt ggt gtg ggt gag gtt gtt ggt ggg 10128
   Gly Gly Gly Gly Gly Gly Gly Gly Gly Val Gly Glu Val Val Gly Gly
   3365 3370 3375
   gtg ttg ggt gtg gtg cag ggg tgg ctg ggg ctg gag cgg ttt gcg ggt 10176
   Val Leu Gly Val Val Gln Gly Trp Leu Gly Leu Glu Arg Phe Ala Gly
   3380 3385 3390
   tcg cgg ctg gtg gtg gtg acc cgg ggt gcg gtg gtg gcc ggc ccg gag 10224
   Ser Arg Leu Val Val Val Thr Arg Gly Ala Val Val Ala Gly Pro Glu
   3395 3400 3405
   gac ggc ccg gtg gat gtg gtg ggt gcg tcg gtg tgg ggg ctg gtg cgt 10272
   Asp Gly Pro Val Asp Val Val Gly Ala Ser Val Trp Gly Leu Val Arg
   3410 3415 3420
   tcg gcg cag gct gag cat ccg gac cgg ttt gtc ctc ctc gac ctc gac 10320
   Ser Ala Gln Ala Glu His Pro Asp Arg Phe Val Leu Leu Asp Leu Asp
   3425 3430 3435 3440
   acc gac acc ggc acc gac ctc gac acc ggt gct ggt gct ggt tgg ggc 10368
   Thr Asp Thr Gly Thr Asp Leu Asp Thr Gly Ala Gly Ala Gly Trp Gly
   3445 3450 3455
   gtg gat ggt ggg cgt gtg gcg gcg gtg gtg gcg tgt ggt gag ccg cag 10416
   Val Asp Gly Gly Arg Val Ala Ala Val Val Ala Cys Gly Glu Pro Gln
   3460 3465 3470
   ttg gcg gtg cgt ggg gag cgg ttg ctg gcc gca cgc ctg aaa cga ctt 10464
   Leu Ala Val Arg Gly Glu Arg Leu Leu Ala Ala Arg Leu Lys Arg Leu
   3475 3480 3485
   gag tca tcc ggt gat gtt cca gcc cag cgg tcc ggt gac aca cga gcc 10512
   Glu Ser Ser Gly Asp Val Pro Ala Gln Arg Ser Gly Asp Thr Arg Ala
   3490 3495 3500
   cgg cgg tcc gac gtg cct gcc cag cgc tcc ggt ggc gtg cct gct cgg 10560
   Arg Arg Ser Asp Val Pro Ala Gln Arg Ser Gly Gly Val Pro Ala Arg
   3505 3510 3515 3520
   cgg tcg gtt gat gta tcg ggt cgg gag gtg ttg ccg tgg ttg tcg ggt 10608
   Arg Ser Val Asp Val Ser Gly Arg Glu Val Leu Pro Trp Leu Ser Gly
   3525 3530 3535
   ggg tcg gtg ttg gtg acg ggt ggg acg ggt gtg ctg ggt gcg gcg gtg 10656
   Gly Ser Val Leu Val Thr Gly Gly Thr Gly Val Leu Gly Ala Ala Val
   3540 3545 3550
   gcg cgg cat ctg gct ggt gtg tgt ggg gtg cgg gat ctg ctg ttg gtg 10704
   Ala Arg His Leu Ala Gly Val Cys Gly Val Arg Asp Leu Leu Leu Val
   3555 3560 3565
   agc cgg cgt ggt ccg gat gct ccg ggt gcg gag ggt ctg cgg gcg gag 10752
   Ser Arg Arg Gly Pro Asp Ala Pro Gly Ala Glu Gly Leu Arg Ala Glu
   3570 3575 3580
   ctg gcc gcg ttg ggg gcg gag gtg cgg att gtt gcg tgt gat gtg ggg 10800
   Leu Ala Ala Leu Gly Ala Glu Val Arg Ile Val Ala Cys Asp Val Gly
   3585 3590 3595 3600
   gag cgg cgg gag gtg gtc cgg ctg ctg gag ggt gtt cct gcc ggg tgt 10848
   Glu Arg Arg Glu Val Val Arg Leu Leu Glu Gly Val Pro Ala Gly Cys
   3605 3610 3615
   ccg ctg acg ggt gtc gtg cat gcg gct ggt gtg ctg gac gat gcg acg 10896
   Pro Leu Thr Gly Val Val His Ala Ala Gly Val Leu Asp Asp Ala Thr
   3620 3625 3630
   atc gcc tct ctc acg ccc gag cgg ctg ggc acg gtg ttc gcg gcc aag 10944
   Ile Ala Ser Leu Thr Pro Glu Arg Leu Gly Thr Val Phe Ala Ala Lys
   3635 3640 3645
   gtg gat gcc gct ctt ttg ctg gat gag ctg acg cgg ggt atg gag ctg 10992
   Val Asp Ala Ala Leu Leu Leu Asp Glu Leu Thr Arg Gly Met Glu Leu
   3650 3655 3660
   tcg gcg ttc gtg ctg ttc tcc tcg gcc gcg ggg atc ctg ggg tcg gcc 11040
   Ser Ala Phe Val Leu Phe Ser Ser Ala Ala Gly Ile Leu Gly Ser Ala
   3665 3670 3675 3680
   ggg cag ggc aac tac gcc gcg gcc aat gcc gct ctg gac gcg ctg gcg 11088
   Gly Gln Gly Asn Tyr Ala Ala Ala Asn Ala Ala Leu Asp Ala Leu Ala
   3685 3690 3695
   tac cgg cgg cgg gcg gcg ggt ctg ccg ggg gtg tcg ctg gcg tgg ggg 11136
   Tyr Arg Arg Arg Ala Ala Gly Leu Pro Gly Val Ser Leu Ala Trp Gly
                   3700                3705                3710
   ctg tgg gaa gag gcc agc ggg atg acc ggg cac ctg gcc ggc acc gac     11184
   Leu Trp Glu Glu Ala Ser Gly Met Thr Gly His Leu Ala Gly Thr Asp
           3715                3720                3725
   cac cgg cgc atc atc cgt tcc ggt ctg cat ccc atg tcg acc ccg gac     11232
   His Arg Arg Ile Ile Arg Ser Gly Leu His Pro Met Ser Thr Pro Asp
       3730                3735                3740
   gca ctg gcc ctc ttc gat gcg gcc ctg gct ctg gac cgg ccg gtc ctg     11280
   Ala Leu Ala Leu Phe Asp Ala Ala Leu Ala Leu Asp Arg Pro Val Leu
   3745                3750                3755                3760
   ctg ccc gcc gac ctg cgt ccc gcc ccg ccc ctg ccg ccc ctg ctg cag     11328
   Leu Pro Ala Asp Leu Arg Pro Ala Pro Pro Leu Pro Pro Leu Leu Gln
                   3765                3770                3775
   gac ctc ctg ccc gcc acc cgc cgc cgc acc acc cgc acc acc act acc     11376
   Asp Leu Leu Pro Ala Thr Arg Arg Arg Thr Thr Arg Thr Thr Thr Thr
               3780                3785                3790
   ggt ggt gcg gac aac ggc gcc cag ctg cac gcc cgg ctg gcc ggc cag     11424
   Gly Gly Ala Asp Asn Gly Ala Gln Leu His Ala Arg Leu Ala Gly Gln
           3795                3800                3805
   aca cac gaa caa cag cac acc acc ctc ctc gcc ctg gtc cgc tcc cac     11472
   Thr His Glu Gln Gln His Thr Thr Leu Leu Ala Leu Val Arg Ser His
       3810                3815                3820
   atc gcc acc gtc ctg ggc cac acc acc ccc gac acc atc ccc ccc gac     11520
   Ile Ala Thr Val Leu Gly His Thr Thr Pro Asp Thr Ile Pro Pro Asp
   3825                3830                3835                3840
   cgc gcg ttc cgc gac ctc ggc ttc gac tcc ctc acc gcc gtc gaa cta     11568
   Arg Ala Phe Arg Asp Leu Gly Phe Asp Ser Leu Thr Ala Val Glu Leu
                   3845                3850                3855
   cgc aac cgg ctc tcc cgc acc acc gga ctc cgc ctc ccc acc acc ctc     11616
   Arg Asn Arg Leu Ser Arg Thr Thr Gly Leu Arg Leu Pro Thr Thr Leu
               3860                3865                3870
   gcc ttc gac cac ccc aac ccc acc acc ctc acc cac cac ctc cac aca     11664
   Ala Phe Asp His Pro Asn Pro Thr Thr Leu Thr His His Leu His Thr
           3875                3880                3885
   caa ctc cag cca caa ccg gac aac gct gtc gcc ccc gtg ttg gcg gag     11712
   Gln Leu Gln Pro Gln Pro Asp Asn Ala Val Ala Pro Val Leu Ala Glu
       3890                3895                3900
   ctc gac aaa ctc gaa tcc gcc ctc tcc gcc ctc gac aaa acc gac agc     11760
   Leu Asp Lys Leu Glu Ser Ala Leu Ser Ala Leu Asp Lys Thr Asp Ser
   3905                3910                3915                3920
   gcc agc gaa aga gtc acc ctg cgg ctg aag tca ctc atg ttg agg tgg     11808
   Ala Ser Glu Arg Val Thr Leu Arg Leu Lys Ser Leu Met Leu Arg Trp
                   3925                3930                3935
   aac gca ccc cag cat ccg aca gcc gaa agc gct gat gac gac gag aag 11856
   Asn Ala Pro Gln His Pro Thr Ala Glu Ser Ala Asp Asp Asp Glu Lys
   3940 3945 3950
   ttc aca tcg gca aca gag gct gag att ttc aaa ttc att gac aac gac 11904
   Phe Thr Ser Ala Thr Glu Ala Glu Ile Phe Lys Phe Ile Asp Asn Asp
   3955 3960 3965
   ctc ggc ctg tcc 11916
   Leu Gly Leu Ser
<210> 4
   <211> 3972
   <212> PRT
   <213> Streptomyces avermitilis
<400> 4
   Val Gln Arg Met Asp Gly Gly Glu Glu Pro Arg Pro Ala Ala Gly Glu
   1 5 10 15
   Val Leu Gly Val Ala Asp Glu Ala Asp Gly Gly Val Val Phe Val Phe
   20 25 30
   Pro Gly Gln Gly Pro Gln Trp Pro Gly Met Gly Arg Glu Leu Leu Asp
   35 40 45
   Ala Ser Asp Val Phe Arg Glu Ser Val Arg Ala Cys Glu Ala Ala Phe
   50 55 60
   Ala Pro Tyr Val Asp Trp Ser Val Glu Gln Val Leu Arg Asp Ser Pro
   65 70 75 80
   Asp Ala Pro Gly Leu Asp Arg Val Asp Val Val Gln Pro Thr Leu Phe
   85 90 95
   Ala Val Met Ile Ser Leu Ala Ala Leu Trp Arg Ser Gln Gly Val Glu
   100 105 110
   Pro Cys Ala Val Leu Gly His Ser Leu Gly Glu Ile Ala Ala Ala His
   115 120 125
   Val Ser Gly Gly Leu Ser Leu Ala Asp Ala Ala Arg Val Val Thr Leu
   130 135 140
   Trp Ser Gln Ala Gln Thr Thr Leu Ala Gly Thr Gly Ala Leu Val Ser
   145 150 155 160
   Val Ala Ala Thr Pro Asp Glu Leu Leu Pro Arg Ile Ala Pro Trp Thr
   165 170 175
   Glu Asp Asn Pro Ala Arg Leu Ala Val Ala Ala Val Asn Gly Pro Arg
   180 185 190
   Ser Thr Val Val Ser Gly Ala Arg Glu Ala Val Ala Asp Leu Val Ala
   195 200 205
   Asp Leu Thr Ala Ala Gln Val Arg Thr Arg Met Ile Pro Val Asp Val
   210 215 220
   Pro Ala His Ser Pro Leu Met Tyr Ala Ile Glu Glu Arg Val Val Ser
   225 230 235 240
   Gly Leu Leu Pro Ile Thr Pro Arg Pro Ser Arg Ile Pro Phe His Ser
   245 250 255
   Ser Val Thr Gly Gly Arg Leu Asp Thr Arg Glu Leu Asp Ala Ala Tyr
   260 265 270
   Trp Tyr Arg Asn Met Ser Ser Thr Val Arg Phe Glu Pro Ala Ala Arg
   275 280 285
   Leu Leu Leu Gln Gln Gly Pro Lys Thr Phe Val Glu Met Ser Pro His
   290 295 300
   Pro Val Leu Thr Met Gly Leu Gln Glu Leu Ala Pro Asp Leu Gly Asp
   305 310 315 320
   Thr Thr Gly Thr Ala Asp Thr Val Ile Met Gly Thr Leu Arg Arg Gly
   325 330 335
   Gln Gly Thr Leu Asp His Phe Leu Thr Ser Leu Ala Gln Leu Arg Gly
   340 345 350
   His Gly Glu Thr Ser Ala Thr Thr Val Leu Ser Ala Arg Leu Thr Ala
   355 360 365
   Leu Ser Pro Thr Gln Gln Gln Ser Leu Leu Leu Asp Leu Val Arg Ala
   370 375 380
   His Thr Met Ala Val Leu Asn Asp Asp Gly Asn Glu Arg Thr Ala Ser
   385 390 395 400
   Asp Ala Gly Pro Ser Ala Ser Phe Ala His Leu Gly Phe Asp Ser Val
   405 410 415
   Met Gly Val Glu Leu Arg Asn Arg Leu Ser Lys Ala Thr Gly Leu Arg
   420 425 430
   Leu Pro Val Thr Leu Ile Phe Asp His Thr Thr Pro Ala Ala Val Ala
   435 440 445
   Ala Arg Leu Arg Thr Ala Ala Leu Gly His Leu Asp Glu Asp Thr Ala
   450 455 460
   Pro Val Pro Asp Ser Pro Ser Gly His Gly Gly Thr Ala Ala Ala Asp
   465 470 475 480
   Asp Pro Ile Ala Ile Ile Gly Met Ala Cys Arg Phe Pro Gly Gly Val
   485 490 495
   Arg Ser Pro Lys Asp Leu Trp Glu Leu Ala Ala Ser Gly Gly Asp Ala
   500 505 510
   Ile Gly Pro Phe Pro Thr Asp Arg Gly Trp Pro Thr Glu Gln Arg His
   515 520 525
   Ala Gln Asp Pro Thr Gln Pro Gly Thr Phe Tyr Pro Gln Gly Gly Gly
   530 535 540
   Phe Leu His Asp Ala Ala His Phe Asp Ala Gly Phe Phe Gly Ile Ser
   545 550 555 560
   Pro Arg Glu Ala Leu Ala Met Asp Pro Gln Gln Arg Leu Leu Leu Glu
   565 570 575
   Thr Ser Trp Glu Ala Phe Glu Arg Ala Gly Ile Asp Pro Leu Ser Val
   580 585 590
   Arg Gly Ser Arg Thr Gly Val Phe Ala Gly Ala Leu Ser Phe Asp Tyr
   595 600 605
   Gly Pro Arg Met Asp Thr Ala Ser Ser Glu Gly Ala Ala Asp Val Glu
   610 615 620
   Gly His Ile Leu Thr Gly Thr Thr Gly Ser Val Leu Ser Gly Arg Ile
   625 630 635 640
   Ala Tyr Ser Phe Gly Leu Glu Gly Pro Ala Ile Thr Val Asp Thr Gly
   645 650 655
   Cys Ser Ala Ser Leu Val Thr Leu His Leu Ala Cys Gln Ser Leu Arg
   660 665 670
   Ser Gly Glu Cys Thr Leu Ala Leu Ala Gly Gly Val Ser Val Met Ser
   675 680 685
   Thr Leu Gly Met Phe Ile Glu Phe Ser Arg Gln Arg Gly Leu Ser Val
   690 695 700
   Asp Gly Arg Cys Lys Ala Tyr Ser Ala Ala Ala Asp Gly Thr Gly Trp
   705 710 715 720
   Gly Glu Gly Val Gly Met Leu Leu Val Glu Arg Leu Ser Asp Ala Val
   725 730 735
   Arg Leu Gly His Arg Val Leu Ala Val Val Arg Gly Ser Ala Val Asn
   740 745 750
   Gln Asp Gly Ala Ser Asn Gly Leu Thr Ala Pro Asn Gly Pro Ala Gln
   755 760 765
   Glu Arg Val Ile Arg Gln Ala Leu Ala Asn Ala Gly Leu Ser Val Ala
   770 775 780
   Asp Val Asp Val Val Glu Gly His Gly Thr Gly Thr Thr Leu Gly Asp
   785 790 795 800
   Pro Ile Glu Ala Gln Ala Leu Leu Ala Thr Tyr Gly Gln Arg Ala Gly
   805 810 815
   Asp Arg Pro Leu Trp Leu Gly Ser Leu Lys Ser Asn Ile Gly His Thr
   820 825 830
   Met Ala Ala Ala Gly Val Gly Gly Val Ile Lys Met Val Met Ala Leu
   835 840 845
   Arg Glu Gly Val Leu Pro Arg Thr Leu His Val Asp Lys Pro Ser Pro
   850 855 860
   Gln Val Asp Trp Ser Ala Gly Ala Val Arg Leu Leu Thr Glu Ala Val
   865 870 875 880
   Pro Trp Pro Gly Asp Ala Ala Gly Arg Leu Arg Arg Ala Gly Val Ser
   885 890 895
   Ser Phe Gly Ile Gly Gly Thr Asn Ala His Val Ile Leu Glu Glu Ala
   900 905 910
   Pro Ala Ala Gly Gly Cys Val Ala Gly Gly Gly Val Leu Glu Gly Ala
   915 920 925
   Pro Gly Leu Ala Ile Ser Val Ala Glu Ser Val Ala Ala Pro Val Ala
   930 935 940
   Val Ser Ala Pro Val Ala Glu Ser Val Pro Val Pro Val Pro Val Pro
   945 950 955 960
   Val Pro Val Pro Val Ser Ala Arg Ser Glu Ala Gly Leu Arg Ala Gln
   965 970 975
   Ala Glu Ala Leu Arg Gln Tyr Val Ala Val Arg Pro Asp Val Ser Leu
   980 985 990
   Ala Asp Val Gly Ala Gly Leu Ala Cys Gly Arg Ala Val Leu Glu His
   995 1000 1005
   Arg Ala Val Val Leu Ala Ala Asp Arg Glu Glu Leu Val Gln Gly Leu
   1010 1015 1020
   Gly Ala Leu Ala Ala Gly Glu Pro Asp Arg Arg Val Thr Thr Gly His
   1025 1030 1035 1040
   Ala Pro Gly Gly Asp Arg Gly Gly Val Val Phe Val Phe Pro Gly Gln
   1045 1050 1055
   Gly Gly Gln Trp Ala Gly Met Gly Val Arg Leu Leu Ala Ser Ser Pro
   1060 1065 1070
   Val Phe Ala Arg Arg Met Gln Ala Cys Glu Glu Ala Leu Ala Pro Trp
   1075 1080 1085
   Val Asp Trp Ser Val Val Asp Ile Leu Arg Arg Asp Ala Gly Asp Ala
   1090 1095 1100
   Val Trp Glu Arg Ala Asp Val Val Gln Pro Val Leu Phe Ser Val Met
   1105 1110 1115 1120
   Val Ser Leu Ala Ala Leu Trp Arg Ser Tyr Gly Ile Glu Pro Asp Ala
   1125 1130 1135
   Val Leu Gly His Ser Gln Gly Glu Ile Ala Ala Ala His Val Cys Gly
   1140 1145 1150
   Ala Leu Ser Leu Lys Asp Ala Ala Lys Thr Val Ala Leu Arg Ser Arg
   1155 1160 1165
   Ala Leu Ala Ala Val Arg Gly Arg Gly Gly Met Ala Ser Val Pro Leu
   1170 1175 1180
   Pro Ala Gln Glu Val Glu Gln Leu Ile Gly Glu Arg Trp Ala Gly Arg
   1185 1190 1195 1200
   Leu Trp Val Ala Ala Val Asn Gly Pro Arg Ser Thr Ala Val Ser Gly
   1205 1210 1215
   Asp Ala Glu Ala Val Asp Glu Val Leu Ala Tyr Cys Ala Gly Thr Gly
   1220 1225 1230
   Val Arg Ala Arg Arg Ile Pro Val Asp Tyr Ala Ser His Cys Pro His
   1235 1240 1245
   Val Gln Pro Leu Arg Glu Glu Leu Leu Glu Leu Leu Gly Asp Ile Ser
   1250 1255 1260
   Pro Gln Pro Ser Gly Val Pro Phe Phe Ser Thr Val Glu Gly Thr Trp
   1265 1270 1275 1280
   Leu Asp Thr Thr Thr Leu Asp Ala Ala Tyr Trp Tyr Arg Asn Leu His
   1285 1290 1295
   Gln Pro Val Arg Phe Ser Asp Ala Val Gln Ala Leu Ala Asp Asp Gly
   1300 1305 1310
   His Arg Val Phe Val Glu Val Ser Pro His Pro Thr Leu Val Pro Ala
   1315 1320 1325
   Ile Glu Asp Thr Thr Glu Asp Thr Ala Glu Asp Val Thr Ala Ile Gly
   1330 1335 1340
   Ser Leu Arg Arg Gly Asp Asn Asp Thr Arg Arg Phe Leu Thr Ala Leu
   1345 1350 1355 1360
   Ala His Thr His Thr Thr Gly Ile Gly Thr Pro Thr Thr Trp His His
   1365 1370 1375
   His Tyr Thr His His His Thr His Pro His Pro His Thr His Leu Asp
   1380 1385 1390
   Leu Pro Thr Tyr Pro Phe Gln His Gln His Tyr Trp Leu Glu Ser Ser
   1395 1400 1405
   Gln Pro Gly Ala Gly Ser Gly Ser Gly Ala Gly Ala Gly Ser Gly Ala
   1410 1415 1420
   Gly Ser Gly Arg Ala Gly Thr Ala Gly Gly Thr Ala Glu Val Glu Ser
   1425 1430 1435 1440
   Arg Phe Trp Asp Ala Val Ala Arg Gln Asp Leu Glu Thr Val Ala Thr
   1445 1450 1455
   Thr Leu Ala Val Pro Pro Ser Ala Gly Leu Asp Thr Val Val Pro Ala
   1460 1465 1470
   Leu Ser Ala Trp His Arg His Gln His Asp Gln Ala Arg Ile Asn Thr
   1475 1480 1485
   Trp Thr Tyr Gln Glu Thr Trp Lys Pro Leu Thr Leu Pro Thr Thr His
   1490 1495 1500
   Gln Pro His Gln Thr Trp Leu Ile Ala Ile Pro Glu Thr Gln Thr His
   1505 1510 1515 1520
   His Pro His Ile Thr Asn Ile Leu Thr Asn Leu His His His Gly Ile
   1525 1530 1535
   Thr Pro Ile Pro Leu Thr Leu Asn His Thr His Thr Asn Pro Gln His
   1540 1545 1550
   Leu His His Thr Leu His His Thr Arg Gln Gln Ala Gln Asn His Thr
   1555 1560 1565
   Thr Gly Ala Ile Thr Gly Leu Leu Ser Leu Leu Ala Leu Asp Glu Thr
   1570 1575 1580
   Pro His Pro His His Pro His Thr Pro Thr Gly Thr Leu Leu Asn Leu
   1585 1590 1595 1600
   Thr Leu Thr Gln Thr His Thr Gln Thr His Pro Pro Thr Pro Leu Trp
   1605 1610 1615
   Tyr Ala Thr Thr Asn Ala Thr Thr Thr His Pro Asn Asp Pro Leu Thr
   1620 1625 1630
   His Pro Thr Gln Ala Gln Thr Trp Gly Leu Ala Arg Thr Thr Leu Leu
   1635 1640 1645
   Glu His Pro Thr His Thr Ala Gly Ile Ile Asp Leu Pro Thr Thr Pro
   1650 1655 1660
   Thr Pro His Thr Leu Gln His Leu Thr Gln Thr Leu Thr Gln Pro His
   1665 1670 1675 1680
   His Gln Thr Gln Leu Ala Ile Arg Thr Thr Gly Thr His Thr Arg Arg
   1685 1690 1695
   Leu Thr Pro Thr Thr Leu Thr Pro Thr His Gln Pro Pro Thr Pro Thr
   1700 1705 1710
   Pro His Gly Thr Thr Leu Ile Thr Gly Gly Thr Gly Ala Leu Ala Thr
   1715 1720 1725
   His Leu Thr His His Leu Thr Thr His Gln Pro Thr Gln His Leu Leu
   1730 1735 1740
   Leu Thr Ser Arg Thr Gly Pro His Thr Pro His Ala Gln His Leu Thr
   1745 1750 1755 1760
   Thr Gln Leu Gln Gln Lys Gly Ile His Leu Thr Ile Thr Thr Cys Asp
   1765 1770 1775
   Thr Ser Asn Pro Asp Gln Leu Gln Gln Leu Leu Asn Thr Ile Pro Pro
   1780 1785 1790
   Gln His Pro Leu Thr Thr Val Ile His Thr Ala Gly Ile Leu Asp Asp
   1795 1800 1805
   Ala Thr Leu Thr Asn Leu Thr Pro Thr Gln Leu Asn Asn Val Leu Arg
   1810 1815 1820
   Ala Lys Ala His Ser Ala His Leu Leu His Gln Leu Thr Gln His Thr
   1825 1830 1835 1840
   Pro Leu Thr Ala Phe Val Leu Tyr Ser Ser Ala Ala Ala Thr Phe Gly
   1845 1850 1855
   Ala Pro Gly Gln Ala Asn Tyr Ala Ala Ala Asn Ala Tyr Leu Asp Ala
   1860 1865 1870
   Leu Ala His His Arg His Thr His His Leu Pro Ala Thr Ser Ile Ala
   1875 1880 1885
   Trp Gly Thr Trp Gln Gly Asn Gly Leu Ala Asp Ser Asp Lys Ala Arg
   1890 1895 1900
   Ala Tyr Leu Asp Arg Arg Gly Phe Arg Pro Met Ser Pro Glu Leu Ala
   1905 1910 1915 1920
   Thr Ala Ala Val Thr Gln Ala Ile Ala Asp Thr Glu Arg Pro Tyr Val
   1925 1930 1935
   Val Ile Ala Asp Ile Asp Trp Ser Lys Ile Glu His Thr Ser Gln Thr
   1940 1945 1950
   Ser Asp Leu Val Ser Ala Ala Arg Glu Arg Glu Pro Ala Val Gln Arg
   1955 1960 1965
   Pro Thr Pro Pro Ala Glu Leu His Lys Thr Leu Ala His Gln Thr Ser
   1970 1975 1980
   Ala Asp Gln Arg Ala Ala Leu Leu Glu Leu Val Arg Asp His Val Ala
   1985 1990 1995 2000
   Ala Val Leu Arg His Ala Asp Pro Lys Ala Ile Ala Pro Asp Gln Ser
   2005 2010 2015
   Phe Arg Ala Leu Gly Phe Asp Ser Leu Thr Ala Val Glu Phe Arg Asn
   2020 2025 2030
   Leu Leu Ile Lys Ala Thr Gly Leu Arg Leu Pro Val Ser Leu Val Phe
   2035 2040 2045
   Asp His Pro Thr Pro Ala Lys Leu Ala Val His Leu Gln Asn Gln Leu
   2050 2055 2060
   Arg Gly Thr Ala Ala Glu Ser Ala Pro Ser Ala Ala Ala Val Thr Ala
   2065 2070 2075 2080
   Glu Ala Ser Val Thr Glu Pro Ile Ala Ile Val Gly Met Ala Cys Arg
   2085 2090 2095
   Phe Pro Gly Gly Val Thr Ser Ala Asp Asp Phe Trp Asp Leu Ile Ser
   2100 2105 2110
   Ser Glu Gln Asp Ala Ile Gly Gly Phe Pro Thr Asp Arg Gly Trp Asp
   2115 2120 2125
   Leu Asp Thr Leu Tyr Asp Pro Asp Pro Asp His Pro Gly Thr Cys Tyr
   2130 2135 2140
   Thr Arg Asn Gly Gly Phe Leu Tyr Asp Ala Gly His Phe Asp Ala Glu
   2145 2150 2155 2160
   Phe Phe Gly Ile Ser Pro Arg Glu Ala Leu Ala Met Asp Pro Gln Gln
   2165 2170 2175
   Arg Leu Leu Leu Glu Thr Ala Trp Glu Thr Ile Glu His Ala Gly Ile
   2180 2185 2190
   Asn Pro His Thr Leu His Gly Thr Pro Thr Gly Val Phe Thr Gly Thr
   2195 2200 2205
   Asn Gly Gln Asp Tyr Ala Leu Arg Val His Asn Ala Gly Gln Ser Thr
   2210 2215 2220
   Asp Gly Phe Ala Leu Thr Gly Thr Ala Gly Ser Val Ile Ser Gly Arg
   2225 2230 2235 2240
   Ile Ser Tyr Thr Phe Gly Phe Glu Gly Pro Ala Val Ser Val Asp Thr
   2245 2250 2255
   Ala Cys Ser Ser Ser Leu Val Ala Leu His Leu Ala Cys Gln Ala Leu
   2260 2265 2270
   Arg Ala Gly Glu Cys Ser Met Ala Leu Ala Gly Gly Val Thr Val Met
   2275 2280 2285
   Ser Ser Pro Gly Ala Phe Val Glu Phe Ser Arg Gln Arg Gly Leu Ala
   2290 2295 2300
   Ala Asp Gly His Cys Lys Ala Phe Ser Ala Ala Ala Asp Gly Thr Gly
   2305 2310 2315 2320
   Trp Gly Glu Gly Val Gly Met Leu Leu Val Glu Arg Leu Ser Asp Ala
   2325 2330 2335
   His Arg Asn Gly His Arg Val Leu Ala Val Val Arg Gly Ser Ala Val
   2340 2345 2350
   Asn Gln Asp Gly Ala Ser Asn Gly Leu Thr Ala Pro Asn Gly Pro Ser
   2355 2360 2365
   Gln Gln Arg Val Ile Arg Gln Ala Leu Ala Asn Ala Gly Leu Ser Ala
   2370 2375 2380
   Gly Asp Val Asp Ala Val Glu Ala His Gly Thr Gly Thr Thr Leu Gly
   2385 2390 2395 2400
   Asp Pro Ile Glu Ala Gln Ala Leu Leu Ala Thr Tyr Gly Gln Asp Arg
   2405 2410 2415
   Ala Gly Glu Gly Pro Leu Trp Leu Gly Ser Val Lys Ser Asn Val Gly
   2420 2425 2430
   His Thr Gln Ala Ala Ala Gly Val Ala Gly Val Ile Lys Met Val Met
   2435 2440 2445
   Ala Leu Arg His Gly Leu Leu Pro Arg Thr Leu His Val Asp Glu Pro
   2450 2455 2460
   Ser Pro His Val Asp Trp Ser Ala Gly Ala Val Gln Leu Leu Thr Glu
   2465 2470 2475 2480
   Thr Val Pro Trp Pro Gly Gly Glu Gly Arg Leu Arg Arg Ala Gly Val
   2485 2490 2495
   Ser Ser Phe Gly Val Ser Gly Thr Asn Ala His Val Ile Leu Glu Glu
   2500 2505 2510
   Ala Pro Ala Asp Asp Val Pro Gly Gly Pro Pro Ala Gly Glu Gly Asp
   2515 2520 2525
   Ala Gly Ser Asp Asp Glu Ala Ala Ala Gly Ser Pro Gly Val Trp Pro
   2530 2535 2540
   Trp Leu Val Ser Ala Lys Ser Gln Pro Ala Leu Arg Ala Gln Ala Gln
   2545 2550 2555 2560
   Ala Leu His Ala His Leu Thr Asp His Pro Gly Leu Asp Leu Ala Asp
   2565 2570 2575
   Val Gly Tyr Thr Leu Ala His Ala Arg Ala Val Phe Asp His Arg Ala
   2580 2585 2590
   Thr Leu Ile Ala Ala Asp Arg Asp Thr Phe Leu Gln Ala Leu Gln Ala
   2595 2600 2605
   Leu Ala Ala Gly Glu Pro His Pro Ala Val Ile His Ser Ser Ala Pro
   2610 2615 2620
   Gly Gly Thr Gly Thr Gly Glu Ala Ala Gly Lys Thr Ala Phe Ile Cys
   2625 2630 2635 2640
   Ser Gly Gln Gly Thr Gln Arg Pro Gly Met Ala His Gly Leu Tyr His
   2645 2650 2655
   Thr His Pro Val Phe Ala Ala Ala Leu Asn Asp Ile Cys Thr His Leu
   2660 2665 2670
   Asp Pro His Leu Asp His Pro Leu Leu Pro Leu Leu Thr Gln Asn Asp
   2675 2680 2685
   Asn Asp Asn Glu Asp Ala Ala Ala Leu Leu Gln Gln Thr Arg Tyr Ala
   2690 2695 2700
   Gln Pro Ala Leu Phe Ala Phe Gln Val Ala Leu His Arg Leu Leu Thr
   2705 2710 2715 2720
   Asp Gly Tyr His Ile Thr Pro His Tyr Tyr Ala Gly His Ser Leu Gly
   2725 2730 2735
   Glu Ile Thr Ala Ala His Leu Ala Gly Ile Leu Thr Leu Thr Asp Ala
   2740 2745 2750
   Thr Thr Leu Ile Thr Gln Arg Ala Thr Leu Met Gln Thr Met Pro Pro
   2755 2760 2765
   Gly Thr Met Thr Thr Leu His Thr Thr Pro His His Ile Thr His His
   2770 2775 2780
   Leu Thr Ala His Glu Asn Asp Leu Ala Ile Ala Ala Ile Asn Thr Pro
   2785 2790 2795 2800
   Thr Ser Leu Val Ile Ser Gly Thr Pro His Thr Val Gln His Ile Thr
   2805 2810 2815
   Thr Leu Cys Gln Gln Gln Gly Ile Lys Thr Lys Thr Leu Pro Thr Asn
   2820 2825 2830
   His Ala Phe His Ser Pro His Thr Asn Pro Ile Leu Asn Gln Leu His
   2835 2840 2845
   Gln His Thr Gln Thr Leu Thr Tyr His Pro Pro His Thr Pro Leu Ile
   2850 2855 2860
   Thr Ala Asn Thr Pro Pro Asp Gln Leu Leu Thr Pro His Tyr Trp Thr
   2865 2870 2875 2880
   Gln Gln Ala Arg Asn Thr Val Asp Tyr Ala Thr Thr Thr Gln Thr Leu
   2885 2890 2895
   His Gln His Gly Val Thr Thr Tyr Ile Glu Leu Gly Pro Asp Asn Thr
   2900 2905 2910
   Leu Thr Thr Leu Thr His His Asn Leu Pro Asn Pro Pro Thr Thr Thr
   2915 2920 2925
   Leu Thr Leu Thr His Pro His His His Pro Gln Thr His Leu Leu Thr
   2930 2935 2940
   Asn Leu Ala Lys Thr Thr Thr Thr Trp His Pro His His Tyr Thr His
   2945 2950 2955 2960
   His Asp Asn Gln Pro His Thr His Thr His Leu Asp Leu Pro Thr Tyr
   2965 2970 2975
   Pro Phe Gln His His His Tyr Trp Leu Glu Ser Thr Gln Pro Gly Ala
   2980 2985 2990
   Gly Asn Val Ser Ala Ala Gly Leu Asp Pro Thr Glu His Pro Leu Leu
   2995 3000 3005
   Gly Ala Thr Leu Glu Leu Ala Thr Asp Gly Gly Ala Leu Leu Ala Gly
   3010 3015 3020
   Arg Leu Ser Leu Arg Ser His Pro Trp Leu Ala Asp His Ala Val Gly
   3025 3030 3035 3040
   Gly Thr Val Leu Leu Ser Gly Ala Thr Phe Leu Glu Leu Ala Leu His
   3045 3050 3055
   Ala Gly Thr Tyr Val Gly Cys Asp Arg Val Asp Glu Leu Thr Leu His
   3060 3065 3070
   Ala Pro Leu Val Val Pro Val Asp Gly Gly Val Ser Val Gln Val Gly
   3075 3080 3085
   Val Ala Ala Ala Asp Gly Glu Gly Arg Arg Leu Val Ser Val Tyr Ala
   3090 3095 3100
   Arg Gly Gly Ser Ala Cys Gly Gly Gly Gly Ala Ser Gly Gly Val Trp
   3105 3110 3115 3120
   Thr Cys His Ala Ser Gly Val Leu Val Glu Ala Ala Ala Gly Gly Val
   3125 3130 3135
   Val Val Asp Gly Leu Ala Gly Val Trp Pro Pro Arg Gly Ala Val Ala
   3140 3145 3150
   Val Asp Val Asp Gly Val Arg Asp Arg Leu Ala Gly Ala Gly Cys Val
   3155 3160 3165
   Leu Gly Pro Val Phe Ser Gly Leu Arg Ala Val Trp Arg Asp Gly Gly
   3170 3175 3180
   Asp Leu Leu Ala Glu Val Cys Leu Pro Glu Glu Ala Trp Gly Asp Ala
   3185 3190 3195 3200
   Ala Gly Phe Gly Leu His Pro Ala Leu Leu Asp Gly Val Val Gln Pro
   3205 3210 3215
   Leu Ser Val Leu Leu Pro Gly Gly Thr Gly Phe Gly Glu Gly Ala Gly
   3220 3225 3230
   Phe Gly Glu Gly Val Arg Val Pro Ala Val Trp Gly Gly Val Ser Leu
   3235 3240 3245
   His Arg Ala Gly Val Thr Gly Val Arg Val Arg Val Ser Ala Val Gly
   3250 3255 3260
   Arg Gly Gly Gly Arg Glu Ala Val Ser Val Val Val Gly Asp Glu Ala
   3265 3270 3275 3280
   Gly Val Pro Val Ala Ser Val Asp Arg Leu Glu Leu Arg Pro Val Asp
   3285 3290 3295
   Met Gly Gln Leu Arg Ala Val Ser Val Ser Ala Gly Arg Arg Gly Ser
   3300 3305 3310
   Leu Tyr Ala Val Gln Trp Ala Glu Val Gly Pro Val Pro Val Cys Gly
   3315 3320 3325
   Gln Ala Trp Ala Trp His Glu Asp Val Gly Glu Ser Gly Gly Gly Pro
   3330 3335 3340
   Val Pro Gly Val Val Val Leu Arg Cys Pro Asp Ala Gly Ala Gly Gly
   3345 3350 3355 3360
   Gly Gly Gly Gly Gly Gly Gly Gly Gly Val Gly Glu Val Val Gly Gly
   3365 3370 3375
   Val Leu Gly Val Val Gln Gly Trp Leu Gly Leu Glu Arg Phe Ala Gly
   3380 3385 3390
   Ser Arg Leu Val Val Val Thr Arg Gly Ala Val Val Ala Gly Pro Glu
   3395 3400 3405
   Asp Gly Pro Val Asp Val Val Gly Ala Ser Val Trp Gly Leu Val Arg
   3410 3415 3420
   Ser Ala Gln Ala Glu His Pro Asp Arg Phe Val Leu Leu Asp Leu Asp
   3425 3430 3435 3440
   Thr Asp Thr Gly Thr Asp Leu Asp Thr Gly Ala Gly Ala Gly Trp Gly
   3445 3450 3455
   Val Asp Gly Gly Arg Val Ala Ala Val Val Ala Cys Gly Glu Pro Gln
   3460 3465 3470
   Leu Ala Val Arg Gly Glu Arg Leu Leu Ala Ala Arg Leu Lys Arg Leu
   3475 3480 3485
   Glu Ser Ser Gly Asp Val Pro Ala Gln Arg Ser Gly Asp Thr Arg Ala
   3490 3495 3500
   Arg Arg Ser Asp Val Pro Ala Gln Arg Ser Gly Gly Val Pro Ala Arg
   3505 3510 3515 3520
   Arg Ser Val Asp Val Ser Gly Arg Glu Val Leu Pro Trp Leu Ser Gly
   3525 3530 3535
   Gly Ser Val Leu Val Thr Gly Gly Thr Gly Val Leu Gly Ala Ala Val
   3540 3545 3550
   Ala Arg His Leu Ala Gly Val Cys Gly Val Arg Asp Leu Leu Leu Val
   3555 3560 3565
   Ser Arg Arg Gly Pro Asp Ala Pro Gly Ala Glu Gly Leu Arg Ala Glu
   3570 3575 3580
   Leu Ala Ala Leu Gly Ala Glu Val Arg Ile Val Ala Cys Asp Val Gly
   3585 3590 3595 3600
   Glu Arg Arg Glu Val Val Arg Leu Leu Glu Gly Val Pro Ala Gly Cys
   3605 3610 3615
   Pro Leu Thr Gly Val Val His Ala Ala Gly Val Leu Asp Asp Ala Thr
   3620 3625 3630
   Ile Ala Ser Leu Thr Pro Glu Arg Leu Gly Thr Val Phe Ala Ala Lys
   3635 3640 3645
   Val Asp Ala Ala Leu Leu Leu Asp Glu Leu Thr Arg Gly Met Glu Leu
   3650 3655 3660
   Ser Ala Phe Val Leu Phe Ser Ser Ala Ala Gly Ile Leu Gly Ser Ala
   3665 3670 3675 3680
   Gly Gln Gly Asn Tyr Ala Ala Ala Asn Ala Ala Leu Asp Ala Leu Ala
   3685 3690 3695
   Tyr Arg Arg Arg Ala Ala Gly Leu Pro Gly Val Ser Leu Ala Trp Gly
   3700 3705 3710
   Leu Trp Glu Glu Ala Ser Gly Met Thr Gly His Leu Ala Gly Thr Asp
   3715 3720 3725
   His Arg Arg Ile Ile Arg Ser Gly Leu His Pro Met Ser Thr Pro Asp
   3730 3735 3740
   Ala Leu Ala Leu Phe Asp Ala Ala Leu Ala Leu Asp Arg Pro Val Leu
   3745 3750 3755 3760
   Leu Pro Ala Asp Leu Arg Pro Ala Pro Pro Leu Pro Pro Leu Leu Gln
   3765 3770 3775
   Asp Leu Leu Pro Ala Thr Arg Arg Arg Thr Thr Arg Thr Thr Thr Thr
   3780 3785 3790
   Gly Gly Ala Asp Asn Gly Ala Gln Leu His Ala Arg Leu Ala Gly Gln
   3795 3800 3805
   Thr His Glu Gln Gln His Thr Thr Leu Leu Ala Leu Val Arg Ser His
   3810 3815 3820
   Ile Ala Thr Val Leu Gly His Thr Thr Pro Asp Thr Ile Pro Pro Asp
   3825 3830 3835 3840
   Arg Ala Phe Arg Asp Leu Gly Phe Asp Ser Leu Thr Ala Val Glu Leu
   3845 3850 3855
   Arg Asn Arg Leu Ser Arg Thr Thr Gly Leu Arg Leu Pro Thr Thr Leu
   3860 3865 3870
   Ala Phe Asp His Pro Asn Pro Thr Thr Leu Thr His His Leu His Thr
   3875 3880 3885
   Gln Leu Gln Pro Gln Pro Asp Asn Ala Val Ala Pro Val Leu Ala Glu
   3890 3895 3900
   Leu Asp Lys Leu Glu Ser Ala Leu Ser Ala Leu Asp Lys Thr Asp Ser
   3905 3910 3915 3920
   Ala Ser Glu Arg Val Thr Leu Arg Leu Lys Ser Leu Met Leu Arg Trp
   3925 3930 3935
   Asn Ala Pro Gln His Pro Thr Ala Glu Ser Ala Asp Asp Asp Glu Lys
   3940 3945 3950
   Phe Thr Ser Ala Thr Glu Ala Glu Ile Phe Lys Phe Ile Asp Asn Asp
   3955 3960 3965
   Leu Gly Leu Ser
   3970
<210> 5
   <211> 6239
   <212> PRT
   <213> Streptomyces avermitilis
<400> 5
   Met Gln Leu Ala Asn Glu Ala Lys Leu Leu Glu Tyr Leu Lys Arg Val
   1 5 10 15
   Thr Ala Asp Leu Asp Arg Thr Arg Arg Arg Leu Tyr Glu Val Val Glu
   20 25 30
   Arg Glu Gln Glu Pro Ile Ala Ile Val Gly Met Ala Cys Arg Tyr Pro
   35 40 45
   Gly Gly Ala Thr Ser Pro Thr Arg Leu Trp His Leu Val Lys Ser Gln
   50 55 60
   Thr Asp Ala Ile Gly Glu Phe Pro Thr Asp Arg Gly Trp Asn Leu Glu
   65 70 75 80
   Gln Leu Tyr Asp Pro Asp Pro Asp Arg Ser Gly Thr Ser Tyr Thr Arg
   85 90 95
   Ser Gly Gly Phe Leu Tyr Asp Ala Gly Asp Phe Asp Ala Ala Phe Phe
   100 105 110
   Glu Leu Ser Pro Arg Glu Ala Leu Ala Met Asp Pro Gln Gln Arg Leu
   115 120 125
   Leu Leu Glu Thr Thr Trp Glu Thr Phe Glu Gln Gly Gly Ile Asp Pro
   130 135 140
   Arg Ser Met Arg Gly Ser Arg Thr Gly Val Phe Val Gly Ile Asn Pro
   145 150 155 160
   Glu Asp Tyr Thr Thr Gly Tyr Thr His Gln Pro Ser Asn Ala Val Glu
   165 170 175
   Gly Tyr Leu Leu Thr Gly Ser Ala Ala Ser Ile Ala Ser Gly Arg Ile
   180 185 190
   Ser Tyr Asn Phe Gly Leu Glu Gly Pro Ala Ile Thr Ile Asp Thr Ala
   195 200 205
   Cys Ser Ser Ser Leu Val Ala Leu His Leu Ala Cys Gln Ala Leu Arg
   210 215 220
   Ser Gly Glu Cys Thr Met Ala Leu Ala Gly Gly Ala Ser Val Met Ala
   225 230 235 240
   Thr Pro Phe Val Phe Thr Glu Phe Ser Arg Gln Arg Gly Leu Ala Ala
   245 250 255
   Asp Gly Arg Cys Lys Ala Phe Ser Ala Ala Ala Asp Gly Thr Gly Trp
   260 265 270
   Ser Glu Gly Val Gly Met Leu Leu Val Glu Arg Leu Ser Asp Ala Arg
   275 280 285
   Arg Asn Gly His Arg Val Leu Ala Val Val Arg Gly Ser Ala Val Asn
   290 295 300
   Gln Asp Gly Ala Ser Asn Gly Leu Thr Ala Pro Asn Gly Arg Ser Gln
   305 310 315 320
   Val Lys Val Ile Arg Gln Ala Leu Ala Asn Ala His Leu Ser Pro Ala
   325 330 335
   Asp Val Asp Ala Val Glu Ala His Gly Thr Gly Thr Thr Leu Gly Asp
   340 345 350
   Pro Ile Glu Ala Gln Ala Leu Val Glu Ala Tyr Gly Gln Asp Arg Pro
   355 360 365
   Asn Gly Arg Pro Leu Trp Leu Gly Thr Leu Lys Ser Asn Ile Gly His
   370 375 380
   Ser Met Ala Ala Ala Gly Val Gly Gly Val Ile Lys Met Val Met Ala
   385 390 395 400
   Leu Arg Asn Gly Leu Leu Pro Arg Thr Leu His Val Asp Glu Pro Ser
   405 410 415
   Pro His Val Asp Trp Ser Ala Gly Ala Val Gln Leu Leu Thr Glu Thr
   420 425 430
   Val Pro Trp Pro Gly Gly Glu Gly Arg Leu Arg Arg Ala Gly Val Ser
   435 440 445
   Ser Phe Gly Val Ser Gly Thr Asn Ala His Val Ile Leu Glu Glu Ala
   450 455 460
   Pro Ala His Asn Ile Pro Ser Asp Thr Pro Ala Asp Asp Val Pro Gly
   465 470 475 480
   Glu Ser Ala Ala Asp Glu Asp Ala Gly Ser Gly Asp Glu Ala Ala Ala
   485 490 495
   Gly Ser Pro Gly Val Trp Pro Trp Leu Val Ser Ala Lys Ser Gln Pro
   500 505 510
   Ala Leu Arg Ala Gln Ala Gln Ala Leu His Ala His Leu Thr Asp His
   515 520 525
   Pro Gly Leu Asp Leu Ala Asp Val Gly Tyr Thr Leu Ala His Ala Arg
   530 535 540
   Ala Val Phe Asp His Arg Ala Thr Leu Ile Ala Ala Asp Arg Asp Thr
   545 550 555 560
   Phe Leu Gln Ala Leu Gln Ala Leu Ala Ala Gly Glu Pro His Pro Ala
   565 570 575
   Val Ile His Ser Ser Ala Pro Gly Gly Thr Gly Thr Gly Glu Ala Ala
   580 585 590
   Gly Lys Thr Ala Phe Ile Cys Ser Gly Gln Gly Thr Gln Arg Pro Gly
   595 600 605
   Met Ala His Gly Leu Tyr His Thr His Pro Val Phe Ala Ala Ala Leu
   610 615 620
   Asn Asp Ile Cys Thr His Leu Asp Pro His Leu Asp His Pro Leu Leu
   625 630 635 640
   Pro Leu Leu Thr Gln Asp Pro Asn Thr Gln Asp Thr Thr Thr Leu Glu
   645 650 655
   Glu Ala Ala Ala Leu Leu Gln Gln Thr Arg Tyr Ala Gln Pro Ala Leu
   660 665 670
   Phe Ala Phe Gln Val Ala Leu His Arg Leu Leu Thr Asp Gly Tyr His
   675 680 685
   Ile Thr Pro His Tyr Tyr Ala Gly His Ser Leu Gly Glu Ile Thr Ala
   690 695 700
   Ala His Leu Ala Gly Ile Leu Thr Leu Thr Asp Ala Thr Thr Leu Ile
   705 710 715 720
   Thr Gln Arg Ala Thr Leu Met Gln Thr Met Pro Pro Gly Thr Met Thr
   725 730 735
   Thr Leu His Thr Thr Pro His His Ile Thr His His Leu Thr Ala His
   740 745 750
   Glu Asn Asp Leu Ala Ile Ala Ala Ile Asn Thr Pro Thr Ser Leu Val
   755 760 765
   Ile Ser Gly Thr Pro His Thr Val Gln His Ile Thr Thr Leu Cys Gln
   770 775 780
   Gln Gln Gly Ile Lys Thr Lys Thr Leu Pro Thr Asn His Ala Phe His
   785 790 795 800
   Ser Pro His Thr Asn Pro Ile Leu Asn Gln Leu His Gln His Thr Gln
   805 810 815
   Thr Leu Thr Tyr His Pro Pro His Thr Pro Leu Ile Thr Ala Asn Thr
   820 825 830
   Pro Pro Asp Gln Leu Leu Thr Pro His Tyr Trp Thr Gln Gln Ala Arg
   835 840 845
   Asn Thr Val Asp Tyr Ala Thr Thr Thr Gln Thr Leu His Gln His Gly
   850 855 860
   Val Thr Thr Tyr Ile Glu Leu Gly Pro Asp Asn Thr Leu Thr Thr Leu
   865 870 875 880
   Thr His Asp Asn Leu Pro Asn Thr Pro Thr Thr Thr Leu Thr Leu Thr
   885 890 895
   His Pro His His His Pro Gln Thr His Leu Leu Thr Asn Leu Ala Lys
   900 905 910
   Thr Thr Thr Thr Trp His Pro His His Tyr Thr His His His Asn Gln
   915 920 925
   Pro His Thr His Thr His Leu Asp Leu Pro Thr Tyr Pro Phe Gln His
   930 935 940
   His His Tyr Trp Leu Gln Pro Pro Gly Lys Pro Ser Asp Pro Ser Pro
   945 950 955 960
   Ser Glu Gly Arg Glu Gln Ala Thr Thr Pro Ser Thr Pro Leu Arg Asp
   965 970 975
   Val Leu Val Gly Lys Ser Pro Gln Glu Arg Asp Glu Glu Leu Leu Arg
   980 985 990
   Leu Val Arg Thr His Ala Ala Ala Val Leu Gly His Ala Thr Pro Glu
   995 1000 1005
   Val Ile Val Pro Asn Lys Ala Phe Lys Glu Leu Gly Phe Asp Ser Leu
   1010 1015 1020
   Ala Ala Ile Gln Leu Arg Asn Arg Leu Leu Ala Asp Val Asp Leu Pro
   1025 1030 1035 1040
   Leu Pro Ala Thr Leu Ile Phe Asp Tyr Pro Thr Pro Met Ala Leu Cys
   1045 1050 1055
   Gln Phe Leu Arg Ala Ala Ile Val Gly Ala Asp Thr Gly Thr Thr Thr
   1060 1065 1070
   Arg Leu Pro Leu Thr Ala Val Pro Ala Asp Glu Pro Ile Ala Ile Val
   1075 1080 1085
   Gly Met Ala Cys Arg Tyr Pro Gly Asp Val Arg Thr Val Asp Asp Leu
   1090 1095 1100
   Trp Gln Val Val Ser Gly Gly His Asp Ala Ile Gly Gly Phe Pro Thr
   1105 1110 1115 1120
   Asn Arg Gly Trp Asp Leu Asp Thr Leu Tyr Asn Pro Asp Pro Asp His
   1125 1130 1135
   His Gly Thr Ser Tyr Thr Arg Ser Gly Gly Phe Leu Tyr Asp Ala Gly
   1140 1145 1150
   Asn Phe Asp Pro Asp Phe Phe Gly Ile Ser Pro Arg Glu Ala Leu Ala
   1155 1160 1165
   Met Asp Pro Gln Gln Arg Leu Leu Leu Glu Thr Ala Trp Glu Ser Ile
   1170 1175 1180
   Glu His Ala Cys Ile Asn Pro Asp Ser Leu Arg Gly Thr Pro Thr Gly
   1185 1190 1195 1200
   Val Phe Ala Gly Leu Thr Tyr His Asp Tyr Ala Ala Arg Phe Pro Thr
   1205 1210 1215
   Ala Pro Ala Gly Phe Glu Gly Tyr Leu Gly His Gly Ser Ala Gly Ser
   1220 1225 1230
   Ile Ala Ser Gly Arg Val Ala Tyr Ala Leu Gly Leu Glu Gly Pro Ala
   1235 1240 1245
   Leu Thr Val Asp Thr Ala Cys Ser Ser Ser Leu Val Ala Leu His Leu
   1250 1255 1260
   Ala Cys Gln Ala Leu Arg Ser Gly Glu Cys Ser Met Ala Leu Ala Gly
   1265 1270 1275 1280
   Gly Val Thr Val Met Ser Thr Pro Ala Gly Phe Val Glu Phe Ser Arg
   1285 1290 1295
   Gln Arg Gly Leu Ala Val Asp Gly Arg Cys Lys Ala Phe Ser Ala Ala
   1300 1305 1310
   Ala Asp Gly Thr Gly Trp Gly Glu Gly Val Gly Met Leu Leu Val Glu
   1315 1320 1325
   Arg Leu Ser Asp Ala Arg Arg Leu Gly His Arg Ile Leu Ala Val Val
   1330 1335 1340
   Arg Gly Ser Ala Val Asn Gln Asp Gly Ala Ser Asn Gly Leu Thr Ala
   1345 1350 1355 1360
   Pro Asn Gly Pro Ser Gln Glu Arg Val Ile Arg Leu Ala Leu Ala Asn
   1365 1370 1375
   Ala Asp Leu Thr Pro Ala Asp Val Asp Ala Val Glu Ala His Gly Thr
   1380 1385 1390
   Gly Thr Thr Leu Gly Asp Pro Ile Glu Ala Gln Ala Leu Leu Ala Thr
   1395 1400 1405
   Tyr Gly Gln Asp Arg Pro Gly Asn Glu Pro Leu Trp Leu Gly Ser Met
   1410 1415 1420
   Lys Ser Asn Ile Gly His Ala Gln Ala Ala Ala Gly Val Gly Gly Val
   1425 1430 1435 1440
   Ile Lys Met Val Met Ala Leu Arg Asn Gly Leu Leu Pro Arg Thr Leu
   1445 1450 1455
   His Val Asp Glu Pro Ser Pro His Val Asp Trp Ser Ala Gly Ala Val
   1460 1465 1470
   Gln Leu Leu Thr Glu Thr Val Pro Trp Pro Gly Gly Glu Gly Arg Leu
   1475 1480 1485
   Arg Arg Ala Gly Val Ser Ser Phe Gly Val Ser Gly Thr Asn Ala His
   1490 1495 1500
   Val Ile Leu Glu Glu Ala Pro Ala His Asn Ile Pro Ser Asp Thr Pro
   1505 1510 1515 1520
   Ala Asp Asp Ala Pro Gly Glu Ala Ala Ala Asp Asp Val Pro Gly Glu
   1525 1530 1535
   Ala Ala Gly Asp Asp Ala Gly Thr Gly Gly Glu Ala Thr Gly Pro Ala
   1540 1545 1550
   Ala Gly Ser Pro Gly Val Trp Pro Trp Leu Val Ser Ala Lys Ser Gln
   1555 1560 1565
   Pro Ala Leu Arg Ala Gln Ala Gln Ala Leu His Ala His Leu Thr Asp
   1570 1575 1580
   His Pro Gly Leu Asp Leu Ala Asp Val Gly Tyr Thr Leu Ala His Ala
   1585 1590 1595 1600
   Arg Ala Val Phe Asp His Arg Ala Thr Leu Ile Ala Ala Asp Arg Asp
   1605 1610 1615
   Thr Phe Leu Gln Ala Leu Gln Ala Leu Ala Ala Gly Glu Pro His Pro
   1620 1625 1630
   Ala Val Ile His Ser Ser Ala Pro Gly Gly Thr Gly Thr Gly Glu Ala
   1635 1640 1645
   Ala Gly Lys Thr Ala Phe Ile Cys Ser Gly Gln Gly Thr Gln Arg Pro
   1650 1655 1660
   Gly Met Ala His Gly Leu Tyr His Thr His Pro Val Phe Ala Ala Ala
   1665 1670 1675 1680
   Leu Asn Asp Ile Cys Thr His Leu Asp Pro His Leu Asp His Pro Leu
   1685 1690 1695
   Leu Pro Leu Leu Thr Gln Asp Pro Asn Thr Gln Asp Thr Thr Thr Leu
   1700 1705 1710
   Glu Glu Ala Ala Ala Leu Leu Gln Gln Thr Pro Tyr Ala Gln Pro Ala
   1715 1720 1725
   Leu Phe Ala Phe Gln Val Ala Leu His Arg Leu Leu Thr Asp Gly Tyr
   1730 1735 1740
   His Ile Thr Pro His Tyr Tyr Ala Gly His Ser Leu Gly Glu Ile Thr
   1745 1750 1755 1760
   Ala Ala His Leu Ala Gly Ile Leu Thr Leu Thr Asp Ala Thr Thr Leu
   1765 1770 1775
   Ile Thr Gln Arg Ala Thr Leu Met Gln Thr Met Pro Pro Gly Thr Met
   1780 1785 1790
   Thr Thr Leu His Thr Thr Pro His His Ile Thr His His Leu Thr Ala
   1795 1800 1805
   His Glu Asn Asp Leu Ala Ile Ala Ala Ile Asn Thr Pro Thr Ser Leu
   1810 1815 1820
   Val Ile Ser Gly Thr Pro His Thr Val Gln His Ile Thr Thr Leu Cys
   1825 1830 1835 1840
   Gln Gln Gln Gly Ile Lys Thr Lys Thr Leu Pro Thr Lys Asn Ala Phe
   1845 1850 1855
   His Ser Pro His Thr Asn Pro Ile Leu Asn Gln Leu His Gln His Thr
   1860 1865 1870
   Gln Thr Leu Thr Tyr His Pro Pro His Thr Pro Leu Ile Thr Ala Asn
   1875 1880 1885
   Thr Pro Pro Asp Gln Leu Leu Thr Pro His Tyr Trp Thr Gln Gln Ala
   1890 1895 1900
   Arg Asn Thr Val Asp Tyr Ala Thr Thr Thr Gln Thr Leu His Gln His
   1905 1910 1915 1920
   Gly Val Thr Thr Tyr Ile Glu Leu Gly Pro Asp Asn Thr Leu Thr Thr
   1925 1930 1935
   Leu Thr His His Asn Leu Pro Asn Thr Pro Thr Thr Thr Leu Thr Leu
   1940 1945 1950
   Thr His Pro His His His Pro Gln Thr His Leu Leu Thr Asn Leu Ala
   1955 1960 1965
   Lys Thr Thr Thr Thr Trp His Pro His His Tyr Thr His His His Asn
   1970 1975 1980
   Gln Pro His Thr His Thr His Leu Asp Leu Pro Thr Tyr Pro Phe Gln
   1985 1990 1995 2000
   His Gln His Tyr Trp Leu Glu Ser Thr Gln Pro Gly Ala Gly Ser Gly
   2005 2010 2015
   Ser Gly Ser Gly Ser Gly Arg Ala Gly Thr Ala Gly Gly Thr Ala Glu
   2020 2025 2030
   Val Glu Ser Arg Phe Trp Asp Ala Val Ala Arg Gln Asp Leu Glu Thr
   2035 2040 2045
   Val Ala Thr Thr Leu Ala Val Pro Pro Ser Ala Gly Leu Asp Thr Val
   2050 2055 2060
   Val Pro Ala Leu Ser Ala Trp His Arg His Gln His Asp Gln Ala Arg
   2065 2070 2075 2080
   Ile Asn Thr Trp Thr Tyr Gln Glu Thr Trp Lys Pro Leu Thr Leu Pro
   2085 2090 2095
   Thr Thr His Gln Pro His Gln Thr Trp Leu Ile Ala Ile Pro Glu Thr
   2100 2105 2110
   Gln Thr His His Pro His Ile Thr Asn Ile Leu Thr Asn Leu His His
   2115 2120 2125
   His Gly Ile Thr Pro Ile Pro Leu Thr Leu Asn His Thr His Thr Asn
   2130 2135 2140
   Pro Gln His Leu His His Thr Arg Gln Gln Ala Gln Asn His Thr Thr
   2145 2150 2155 2160
   Gly Pro Ile Thr Gly Leu Leu Ser Leu Leu Ala Leu Asp Glu Thr Pro
   2165 2170 2175
   His Pro His His Pro His Thr Pro Thr Gly Thr Leu Leu Asn Leu Thr
   2180 2185 2190
   Leu Thr Gln Thr His Thr Gln Thr His Pro Pro Thr Pro Leu Trp Tyr
   2195 2200 2205
   Ala Thr Thr Asn Ala Thr Thr Thr His Pro Asn Asp Pro Leu Thr His
   2210 2215 2220
   Pro Thr Gln Ala Gln Thr Trp Gly Leu Ala Arg Thr Thr Leu Leu Glu
   2225 2230 2235 2240
   His Pro Thr His Thr Ala Gly Ile Ile Asp Leu Pro Thr Thr Pro Thr
   2245 2250 2255
   Pro His Thr Leu His His Leu Thr Gln Thr Leu Thr Gln Pro His His
   2260 2265 2270
   Gln Thr Gln Leu Ala Ile Arg Thr Thr Gly Thr His Thr Arg Arg Leu
   2275 2280 2285
   Thr Pro Thr Thr Leu Thr Pro Thr His Gln Pro Pro Thr Pro Thr Pro
   2290 2295 2300
   His Gly Thr Thr Leu Ile Thr Gly Gly Thr Gly Ala Leu Ala Thr His
   2305 2310 2315 2320
   Leu Thr His His Leu Thr Thr His Gln Pro Thr Gln His Leu Leu Leu
   2325 2330 2335
   Thr Ser Arg Thr Gly Pro His Thr Pro His Ala Gln His Leu Thr Thr
   2340 2345 2350
   Gln Leu Gln Gln Lys Gly Ile His Leu Thr Ile Thr Thr Cys Asp Thr
   2355 2360 2365
   Ser Asn Pro Asp Gln Leu Gln Gln Leu Leu Asn Thr Ile Pro Pro Gln
   2370 2375 2380
   His Pro Leu Thr Thr Val Ile His Thr Ala Gly Ile Leu Asp Asp Ala
   2385 2390 2395 2400
   Thr Leu Thr Asn Leu Thr Pro Thr Gln Leu Asn Asn Val Leu Arg Ala
   2405 2410 2415
   Lys Ala His Ser Ala His Leu Leu His Gln Leu Thr Gln His Thr Pro
   2420 2425 2430
   Leu Asn Ala Phe Val Leu Tyr Ser Ser Ala Ala Ala Thr Phe Gly Ala
   2435 2440 2445
   Pro Gly Gln Ala Asn Tyr Ala Ala Ala Asn Ala Tyr Leu Asp Ala Leu
   2450 2455 2460
   Ala His His Arg His Thr His His Leu Pro Ala Thr Ser Ile Ala Trp
   2465 2470 2475 2480
   Gly Thr Trp Gln Gly Asn Gly Leu Ala Thr Gly Gln Val Ser Glu His
   2485 2490 2495
   Leu Arg Arg Arg Gly Met Phe Ala Met Pro Pro Glu Leu Ala Val Thr
   2500 2505 2510
   Ala Val Asp Gly Ala Ile Ala Ser Gly Arg Pro Ser Leu Leu Val Ala
   2515 2520 2525
   Asp Ile Asp Trp Lys Lys Leu Gly Pro Val Leu Ser Ser Lys Ser Ser
   2530 2535 2540
   Val Leu Leu Glu Asp Leu Pro Gln Ala Gln Gly Thr Glu Glu Ala Arg
   2545 2550 2555 2560
   Ser Thr Val Glu Gln Thr Glu Ser Thr Asn Leu Arg Gln Leu Leu Met
   2565 2570 2575
   Gly Arg Ser Arg Ser Glu Gln Glu Glu Glu Leu Leu Ser Leu Val Arg
   2580 2585 2590
   Ile His Ser Ala Ala Val Leu Gly Arg Asp Asp Ser Glu Ala Ile Pro
   2595 2600 2605
   Pro Gly Arg Leu Phe Arg Asp Leu Gly Phe Asp Ser Leu Ala Ala Val
   2610 2615 2620
   Glu Leu Arg Asn His Leu Ala Ala Gln Thr Glu Leu Ala Leu Pro Thr
   2625 2630 2635 2640
   Thr Leu Val Phe Asp Tyr Pro Ser Pro Thr Lys Leu Ala Gln Phe Leu
   2645 2650 2655
   Leu Ser Glu Ile Ala Glu Phe Gln Pro Asp Asn Ser Thr Pro Leu Pro
   2660 2665 2670
   Arg Pro Arg Ala Glu Leu Asp Glu Pro Ile Ala Ile Val Gly Met Ala
   2675 2680 2685
   Cys Arg Phe Pro Gly Gly Val Thr Ser Ala Asp Asp Phe Trp Asp Leu
   2690 2695 2700
   Ile Ser Ser Glu Gln Asp Ala Ile Gly Gly Phe Pro Thr Asp Arg Gly
   2705 2710 2715 2720
   Trp Asp Leu Asp Thr Leu Tyr Asp Pro Asp Pro Asp His Pro Gly Thr
   2725 2730 2735
   Cys Tyr Thr Arg Asn Gly Gly Phe Leu Tyr Asp Ala Gly His Phe Asp
   2740 2745 2750
   Ala Glu Phe Phe Gly Ile Ser Pro Arg Glu Ala Leu Ala Met Asp Pro
   2755 2760 2765
   Gln Gln Arg Leu Leu Leu Glu Thr Ala Trp Glu Thr Ile Glu His Ala
   2770 2775 2780
   Gly Ile Asn Pro His Thr Leu His Gly Thr Pro Thr Gly Val Phe Thr
   2785 2790 2795 2800
   Gly Thr Asn Gly Gln Asp His Ala Ala His Ile Arg Gln Ala Pro Ser
   2805 2810 2815
   Gly Thr Glu Gly Phe Val Leu Thr Gly Ala Ala Thr Ser Ile Ala Ser
   2820 2825 2830
   Gly Arg Ile Ser Tyr Ile Leu Gly Leu Glu Gly Pro Ala Val Thr Leu
   2835 2840 2845
   Asp Thr Ala Cys Ser Ser Ser Leu Val Ala Leu His Leu Ala Cys Gln
   2850 2855 2860
   Ser Leu Arg Ser Gly Glu Cys Thr Met Ala Leu Ala Gly Gly Ala Thr
   2865 2870 2875 2880
   Val Met Thr Thr Pro Ile Thr Phe Thr Glu Phe Ala Arg Gln Arg Gly
   2885 2890 2895
   Leu Ala Pro Asp Gly Arg Cys Lys Ala Phe Ser Ala Ala Ala Asp Gly
   2900 2905 2910
   Thr Gly Trp Gly Glu Gly Val Gly Met Leu Leu Val Glu Arg Leu Ser
   2915 2920 2925
   Asp Ala Arg Arg Asn Gly His Arg Val Leu Ala Val Val Arg Gly Ser
   2930 2935 2940
   Ala Val Asn Gln Asp Gly Ala Ser Asn Gly Leu Thr Ala Pro Asn Gly
   2945 2950 2955 2960
   Pro Ser Gln Gln Arg Val Ile Arg Gln Ala Leu Ala Asn Ala Asp Leu
   2965 2970 2975
   Thr Pro Ala Asp Val Asp Ala Val Glu Ala His Gly Thr Gly Thr Thr
   2980 2985 2990
   Leu Gly Asp Pro Ile Glu Ala Gln Ala Ile Leu Ala Thr Tyr Gly Gln
   2995 3000 3005
   Asp Arg Pro Gly Asn Gly Pro Leu Trp Leu Gly Ser Val Lys Ser Asn
   3010 3015 3020
   Val Gly His Thr Gln Ala Ala Ala Gly Val Ala Gly Val Ile Lys Met
   3025 3030 3035 3040
   Val Met Ala Leu Arg His Arg Thr Leu Pro Pro Thr Leu His Ala Asp
   3045 3050 3055
   Glu Pro Ser Pro His Val Asp Trp Ser Ala Gly Ala Val Gln Leu Leu
   3060 3065 3070
   Thr Glu Thr Val Pro Trp Pro Gly Gly Glu Gly Arg Pro Arg Arg Ala
   3075 3080 3085
   Gly Val Ser Ser Phe Gly Val Ser Gly Thr Asn Ala His Val Ile Leu
   3090 3095 3100
   Glu Glu Ala Pro Ala Asp Asp Val Pro Gly Gly Pro Pro Ala Asp Glu
   3105 3110 3115 3120
   Asp Ala Gly Ser Gly Glu Glu Ala Ala Ala Gly Ser Pro Gly Val Trp
   3125 3130 3135
   Pro Trp Leu Val Ser Ala Lys Ser Gln Pro Ala Leu Arg Ala Gln Ala
   3140 3145 3150
   Gln Ala Leu His Ala His Leu Thr Asp His Pro Gly Leu Asp Leu Ala
   3155 3160 3165
   Asp Val Gly Tyr Thr Leu Ala His Ala Arg Ala Val Phe Asp His Arg
   3170 3175 3180
   Ala Thr Leu Ile Ala Ala Asp Arg Asp Thr Phe Leu Gln Ala Leu Gln
   3185 3190 3195 3200
   Ala Leu Ala Ala Gly Glu Pro His Pro Ala Val Ile His Ser Ser Ala
   3205 3210 3215
   Pro Gly Gly Thr Gly Thr Gly Glu Ala Ala Gly Lys Thr Ala Phe Ile
   3220 3225 3230
   Cys Ser Gly Gln Gly Thr Gln Arg Pro Gly Met Ala His Gly Leu Tyr
   3235 3240 3245
   His Thr His Pro Val Phe Ala Ala Ala Leu Asn Asp Ile Cys Thr His
   3250 3255 3260
   Leu Asp Pro His Leu Asp His Pro Leu Leu Pro Leu Leu Thr Gln Asn
   3265 3270 3275 3280
   Asp Asn Asp Asn Asp Asn Glu Asp Ala Ala Ala Leu Leu Gln Gln Thr
   3285 3290 3295
   Pro Tyr Ala Gln Pro Ala Leu Phe Ala Phe Gln Val Ala Leu His Arg
   3300 3305 3310
   Leu Leu Thr Asp Gly Tyr His Ile Thr Pro His Tyr Tyr Ala Gly His
   3315 3320 3325
   Ser Leu Gly Glu Ile Thr Ala Ala His Leu Ala Gly Ile Leu Thr Leu
   3330 3335 3340
   Thr Asp Ala Thr Thr Leu Ile Thr Gln Arg Ala Thr Leu Met Gln Thr
   3345 3350 3355 3360
   Met Pro Pro Gly Thr Met Thr Thr Leu His Thr Thr Pro His His Ile
   3365 3370 3375
   Thr His His Leu Thr Ala His Glu Asn Asp Leu Ala Ile Ala Ala Ile
   3380 3385 3390
   Asn Thr Pro Thr Ser Leu Val Ile Ser Gly Thr Pro His Thr Val Gln
   3395 3400 3405
   His Ile Thr Thr Leu Cys Gln Gln Gln Gly Ile Lys Thr Lys Thr Leu
   3410 3415 3420
   Pro Thr Asn His Ala Phe His Ser Pro His Thr Asn Pro Ile Leu Asn
   3425 3430 3435 3440
   Gln Leu His Gln His Thr Gln Thr Leu Thr Tyr His Pro Pro His Thr
   3445 3450 3455
   Pro Leu Ile Thr Ala Asn Thr Pro Pro Asp Gln Leu Leu Thr Pro His
   3460 3465 3470
   Tyr Trp Thr Gln Gln Ala Arg Asn Thr Val Asp Tyr Ala Thr Thr Thr
   3475 3480 3485
   Gln Thr Leu His Gln His Gly Val Thr Thr Tyr Ile Glu Leu Gly Pro
   3490 3495 3500
   Asp Asn Thr Leu Thr Thr Leu Thr His His Asn Leu Pro Asn Thr Pro
   3505 3510 3515 3520
   Thr Thr Thr Leu Thr Leu Thr His Pro His His His Pro Gln Thr His
   3525 3530 3535
   Leu Leu Thr Asn Leu Ala Lys Thr Thr Thr Thr Trp His Pro His His
   3540 3545 3550
   Tyr Thr His His His Asn Gln Pro His Thr His Thr His Leu Asp Leu
   3555 3560 3565
   Pro Thr Tyr Pro Phe Gln His His His Tyr Trp Leu Glu Leu Pro Ser
   3570 3575 3580
   Ala Gln Thr Ser Pro Gly Gln Arg Arg Ser Arg Arg Ser Ala Pro Asp
   3585 3590 3595 3600
   Thr Ala Glu Ser Glu Phe Trp Asp Ala Val Asn Glu Glu Asp Leu Gln
   3605 3610 3615
   Ser Leu Ala Glu Thr Leu Asp Ile Asp Ala Ser Ala Leu Asp Thr Val
   3620 3625 3630
   Val Pro Ala Leu Ser Ala Trp His Arg His Gln His Asp Gln Ala Arg
   3635 3640 3645
   Ile Asn Thr Trp Thr Tyr Gln Glu Thr Trp Lys Pro Leu Thr Leu Pro
   3650 3655 3660
   Thr Thr His Gln Pro His Gln Thr Trp Leu Ile Ala Ile Pro Glu Thr
   3665 3670 3675 3680
   Gln Thr His His Pro His Ile Thr Asn Ile Leu Thr Asn Leu His His
   3685 3690 3695
   His Gly Ile Thr Pro Ile Pro Leu Thr Val Asn His Thr His Thr Asn
   3700 3705 3710
   Pro Gln His Leu His His Thr Leu His His Thr Arg Gln Gln Ala Gln
   3715 3720 3725
   Asn His Thr Thr Gly Pro Ile Thr Gly Leu Leu Ser Leu Leu Ala Leu
   3730 3735 3740
   Asp Glu Thr Pro His Pro His His Pro His Thr Pro Thr Gly Thr Leu
   3745 3750 3755 3760
   Leu Asn Leu Thr Leu Pro Gln Thr His Thr Gln Thr His Pro Pro Thr
   3765 3770 3775
   Pro Leu Trp Tyr Ala Thr Thr Asn Ala Thr Thr Thr His Pro Asn Asp
   3780 3785 3790
   Pro Leu Thr His Pro Thr Gln Ala Gln Thr Trp Gly Leu Ala Arg Thr
   3795 3800 3805
   Thr Leu Leu Glu His Pro Thr His Thr Ala Gly Ile Ile Asp Leu Pro
   3810 3815 3820
   Thr Thr Pro Thr Pro His Thr Leu His His Leu Thr Gln Thr Leu Thr
   3825 3830 3835 3840
   Gln Pro His His Gln Thr Gln Leu Ala Ile Arg Thr Thr Gly Thr His
   3845 3850 3855
   Thr Arg Arg Leu Thr Pro Thr Thr Leu Thr Pro Thr His Gln Pro Pro
   3860 3865 3870
   Thr Pro Thr Pro His Gly Thr Thr Leu Ile Thr Gly Gly Thr Gly Ala
   3875 3880 3885
   Leu Ala Thr His Leu Thr His His Leu Thr Thr His Gln Pro Thr Gln
   3890 3895 3900
   His Leu Leu Leu Thr Ser Arg Thr Gly Pro His Thr Pro His Ala Gln
   3905 3910 3915 3920
   His Leu Thr Thr Gln Leu Gln Gln Lys Gly Ile His Leu Thr Ile Thr
   3925 3930 3935
   Thr Cys Asp Thr Ser Asn Pro Asp Gln Leu Gln Gln Leu Leu Asn Thr
   3940 3945 3950
   Ile Pro Pro Gln His Pro Leu Thr Thr Val Ile His Thr Ala Gly Val
   3955 3960 3965
   Asn Leu Phe Ala Pro Val Ser Glu Thr Asp Ala Glu Ser Phe Ser Ser
   3970 3975 3980
   Val Thr Ala Ala Lys Ala Thr Gly Ala Ala Ile Leu His Glu Leu Leu
   3985 3990 3995 4000
   Leu Asp His Glu Thr Leu Glu His Phe Ile Leu Phe Ser Ser Gly Ala
   4005 4010 4015
   Gly Ala Trp Gly Ser Gly Asn Gln Cys Ala Tyr Ser Ala Ala Asn Ala
   4020 4025 4030
   Tyr Leu Asp Ala Leu Ala Thr His Arg Gln Thr His Gly Leu Pro Gly
   4035 4040 4045
   Ala Ser Ile Ala Trp Gly Pro Trp Ala Gly Lys Gly Met Ser Ala Gly
   4050 4055 4060
   Asp Ala Ala His Gly Tyr Leu Glu Lys Arg Gly Ile Leu Pro Met Glu
   4065 4070 4075 4080
   Pro Arg Met Ala Leu Ala Ala Phe His Arg Ala Arg Ala Gln Arg Pro
   4085 4090 4095
   Asn Ser Asn Leu Ile Ile Ala Asp Ile Asp Trp Glu Arg Phe Val Pro
   4100 4105 4110
   Ala Phe Thr Ala Arg Arg His Ser Pro Leu Ile Glu Asp Ile Pro Glu
   4115 4120 4125
   Val Arg Gln Ala Ala Gln Glu Leu Glu Ala Ala Ala Ser Thr Ala Lys
   4130 4135 4140
   Thr Thr Thr Ala Gln Pro Ile Ala Thr Ser Leu Arg Glu Arg Leu Ala
   4145 4150 4155 4160
   Arg Leu Thr Ser Ser Lys Gln Asn Gln Val Leu Leu Gly Leu Ile Arg
   4165 4170 4175
   Thr Gly Ile Cys Thr Val Leu Gly Leu Arg Asn Pro Glu Gly Ile Glu
   4180 4185 4190
   Asp Gln Arg Ala Phe Arg Asp Leu Gly Phe Asp Ser Leu Thr Ser Ala
   4195 4200 4205
   Gln Phe Ser Lys Glu Leu Ala Lys Glu Thr Gly Leu Pro Leu Pro Pro
   4210 4215 4220
   Ser Leu Val Phe Asp Tyr Pro Thr Pro Gln Glu Cys Ala Ala His Leu
   4225 4230 4235 4240
   Arg Thr Gln Leu Val Asp Leu Asp Asp Glu Glu Asp Ala Ala Leu Ser
   4245 4250 4255
   Asn Ala Leu Pro Gln Val Ala His Arg Arg Thr Val Glu Asp Glu Pro
   4260 4265 4270
   Ile Ala Ile Ile Gly Met Ala Cys Arg Phe Pro Gly Gly Val Arg Ser
   4275 4280 4285
   Ala Asp Asp Leu Trp Glu Leu Leu Ala Ser Gly Lys Asp Ala Ile Gly
   4290 4295 4300
   Val Phe Pro Thr Asp Arg Gly Trp Asp Leu Asp Thr Leu Tyr Asp Pro
   4305 4310 4315 4320
   Asp Pro Asp His Pro Gly Thr Cys Tyr Thr Arg Asn Gly Gly Phe Leu
   4325 4330 4335
   Tyr Gly Ala Gly His Phe Asp Ala Glu Phe Phe Gly Ile Ser Pro Arg
   4340 4345 4350
   Glu Ala Leu Ala Met Asp Pro Gln Gln Arg Leu Leu Leu Glu Thr Ala
   4355 4360 4365
   Trp Glu Thr Ile Glu His Ala Gly Ile Asn Pro His Thr Leu His Gly
   4370 4375 4380
   Thr Pro Thr Gly Val Phe Ala Gly Ile Asn Ala Gln Asp His Ala Ala
   4385 4390 4395 4400
   His Ile Arg Gln Ser Arg Asp Val Glu Thr Ile Glu Gly Tyr Ala Leu
   4405 4410 4415
   Thr Gly Ser Ser Gly Ser Val Ala Ser Gly Arg Val Ala Tyr Thr Leu
   4420 4425 4430
   Gly Leu Glu Gly Pro Ala Val Ser Val Asp Thr Ala Cys Ser Ser Ser
   4435 4440 4445
   Leu Val Ala Leu His Trp Ala Ala Gln Ala Leu Arg Ala Gly Glu Cys
   4450 4455 4460
   Ser Met Ala Leu Ala Gly Gly Val Thr Val Met Ser Ser Pro Gly Thr
   4465 4470 4475 4480
   Phe Val Glu Phe Ser Arg Gln Arg Gly Leu Ala Ala Asp Gly Arg Cys
   4485 4490 4495
   Lys Ala Tyr Ser Ala Ala Ala Asp Gly Thr Gly Trp Ala Glu Gly Val
   4500 4505 4510
   Gly Met Leu Leu Val Glu Arg Leu Ser Asp Ala Arg Arg Asn Gly His
   4515 4520 4525
   Arg Val Leu Ala Val Val Arg Gly Ser Ala Val Asn Gln Asp Gly Ala
   4530 4535 4540
   Ser Asn Gly Leu Thr Ala Pro Asn Gly Pro Ser Gln Gln Arg Val Ile
   4545 4550 4555 4560
   Arg Gln Ala Leu Ala Asn Ala Gly Leu Thr Pro Ala Asp Val Asp Ala
   4565 4570 4575
   Val Glu Gly His Gly Thr Gly Thr Thr Leu Gly Asp Pro Ile Glu Ala
   4580 4585 4590
   Gln Ala Leu Leu Ala Ala Tyr Gly Gln His Arg Pro His His Arg Pro
   4595 4600 4605
   Leu Trp Leu Gly Ser Leu Lys Ser Asn Ile Gly His Ala Gln Ala Ala
   4610 4615 4620
   Ala Gly Val Gly Gly Val Ile Lys Met Val Met Ala Leu Arg Asn Gly
   4625 4630 4635 4640
   Leu Leu Pro Gln Thr Leu His Val Asp Glu Pro Thr Pro Gln Val Asp
   4645 4650 4655
   Trp Ser Thr Gly Ala Val Gln Leu Leu Thr Gln Pro Val Pro Trp Pro
   4660 4665 4670
   Ala Asp Pro Ala Gly Arg Pro Arg His Ala Gly Val Ser Ser Phe Gly
   4675 4680 4685
   Val Ser Gly Thr Asn Ala His Ile Ile Leu Glu Glu Ala Pro Thr Pro
   4690 4695 4700
   Gln Asp Ser Asp Thr Asp Asp Glu Pro Pro Ala Asn Ala Pro Ala Leu
   4705 4710 4715 4720
   Pro His Pro Leu Pro Leu Pro Val Pro Val Ser Ala Arg Ser Glu Ala
   4725 4730 4735
   Gly Leu Arg Ala Gln Ala Gln Ala Leu Arg Gln Tyr Val Ala Ala Arg
   4740 4745 4750
   Pro Asp Met Ser Pro Ala Asp Ile Gly Ala Gly Leu Ala Arg Gly Arg
   4755 4760 4765
   Ala Val Leu Glu His Arg Ala Val Ile Leu Ala Ala Asp Arg Glu Glu
   4770 4775 4780
   Leu Ala Gln Ala Leu Thr Ala Leu Ala Ala Gly Glu Pro His Pro His
   4785 4790 4795 4800
   Ile Thr Thr Gly His Thr Arg Gly Gly Asp Arg Gly Gly Val Val Phe
   4805 4810 4815
   Val Phe Pro Gly Gln Gly Gly Gln Trp Ala Gly Met Gly Leu Thr Leu
   4820 4825 4830
   Leu Thr Ser Ser Pro Val Phe Ala Glu His Ile Asp Ala Cys Glu Lys
   4835 4840 4845
   Ala Leu Thr Pro Trp Val Pro Trp Ser Leu Thr Asp Ile Leu His Arg
   4850 4855 4860
   Asp Pro Asp Asp Pro Ala Trp Gln Gln Ala Asp Val Val Gln Pro Val
   4865 4870 4875 4880
   Leu Phe Ser Ile Met Val Ser Leu Ala Ala Leu Trp Arg Ser Tyr Gly
   4885 4890 4895
   Ile Glu Pro Asp Ala Val Leu Gly His Ser Gln Gly Glu Ile Ala Ala
   4900 4905 4910
   Ala His Ile Cys Gly Ala Leu Ser Leu Lys Asp Ala Ala Lys Thr Val
   4915 4920 4925
   Ala Leu Arg Ser Arg Ala Leu Ala Ala Val Arg Gly Arg Gly Ala Met
   4930 4935 4940
   Ala Ser Leu Pro Leu Pro Ala Gln Asp Val Gln Gln Leu Ile Ser Glu
   4945 4950 4955 4960
   Arg Trp Glu Gly Gln Leu Trp Val Ala Ala Leu Asn Gly Pro His Ser
   4965 4970 4975
   Thr Thr Val Ser Gly Asp Thr Lys Ala Val Asp Glu Val Leu Ala His
   4980 4985 4990
   Cys Thr Asp Thr Gly Leu Arg Ala Lys Arg Ile Pro Val Asp Tyr Ala
   4995 5000 5005
   Ser His Cys Pro His Val Gln Pro Leu His Asp Glu Leu Leu His Leu
   5010 5015 5020
   Leu Gly Asp Ile Thr Pro Gln Pro Ser Thr Val Pro Phe Phe Ser Thr
   5025 5030 5035 5040
   Val Glu Gly Thr Trp Leu Asp Thr Thr Thr Leu Asp Ala Ala Tyr Trp
   5045 5050 5055
   Tyr Arg Asn Leu His Gln Pro Val Arg Phe Ser His Ala Ile Gln Thr
   5060 5065 5070
   Leu Thr Asp Asp Gly His Arg Ala Phe Ile Glu Ile Ser Pro His Pro
   5075 5080 5085
   Thr Leu Val Pro Ala Ile Glu Asp Thr Thr Glu Asn Thr Thr Glu Asn
   5090 5095 5100
   Ile Thr Ala Thr Gly Ser Leu Arg Arg Gly Asp Asn Asp Thr His Arg
   5105 5110 5115 5120
   Phe Leu Thr Ala Leu Ala His Thr His Thr Thr Gly Ile Gly Thr Pro
   5125 5130 5135
   Thr Thr Trp His His His Tyr Thr Gln Thr His Pro His Pro Asn Pro
   5140 5145 5150
   His Thr His Leu Asp Leu Pro Thr Tyr Pro Phe Gln His Gln His Tyr
   5155 5160 5165
   Trp Leu Gln Pro Pro Thr Thr Thr Thr Asp Leu Thr Thr Thr Gly Leu
   5170 5175 5180
   Thr Pro Thr His His Pro Leu Leu Thr Ala Thr Leu Thr Leu Ala Asp
   5185 5190 5195 5200
   Asn Asn Thr Gln Leu Leu Thr Gly Arg Leu Ser Leu Arg Thr His Pro
   5205 5210 5215
   Trp Leu Thr Asp His Thr Val Ala Gly Met Val Leu Leu Pro Gly Thr
   5220 5225 5230
   Ala Leu Leu Glu Leu Ala Leu Gln Ala Gly Glu Arg Val Asp Cys Pro
   5235 5240 5245
   Arg Val Glu Glu Leu Thr Leu His Ala Pro Leu Val Ile Pro His Thr
   5250 5255 5260
   Glu Asp Val Thr Leu Gln Val Thr Val Arg Ala Ala Asp Glu Ser Gly
   5265 5270 5275 5280
   His Arg Ala Leu Ala Ile His Ser Tyr Ser Gly Thr Ala Ser Ser Ala
   5285 5290 5295
   Asp Arg Glu Trp Thr Arg His Ala Thr Gly Leu Leu Thr His His Ala
   5300 5305 5310
   Asp Thr Asp His Arg Ala Asp Thr His Thr Asp Ala Cys Leu Gly Gly
   5315 5320 5325
   Ser Trp Pro Pro Pro Gly Ala Gln Pro Ile Glu Leu Gly Asp Val Tyr
   5330 5335 5340
   Gly Arg Met Ala Ala Asp Ser Asp Ile Ala Tyr Gly Pro Val Phe Gln
   5345 5350 5355 5360
   Gly Leu His Ala Ala Trp Arg Phe Gly Asp Asp Val Leu Ala Glu Val
   5365 5370 5375
   Arg Leu Pro Glu Glu Ala Leu Arg Asp Ala Pro Ala Ala Ala Phe Gly
   5380 5385 5390
   Val His Pro Ala Leu Leu Asp Ala Ala Leu His Ala Thr Ala Leu Thr
   5395 5400 5405
   Pro Gln Asn Gly Asp Gly Ser Thr Glu Asn Val Ala Gln Glu Ser Met
   5410 5415 5420
   Pro Asp Arg Ala Ala His Gln Ala Arg Leu Pro Phe Ser Trp Ser Gly
   5425 5430 5435 5440
   Val Ser Leu His Thr Ala Gly Ser Ser Val Leu Arg Val Arg Leu Ser
   5445 5450 5455
   Arg Ser Pro Gln His Gly Asn Ala Val Ala Leu Thr Ala Ala Asp Glu
   5460 5465 5470
   Asp Gly Arg Pro Val Val Thr Ile Glu Ser Leu Ala Leu Arg Pro Val
   5475 5480 5485
   Ser Thr Glu Glu Leu Arg Ala Ala Ala Asp Arg Thr Pro Glu His Glu
   5490 5495 5500
   Ser Leu Phe Arg Leu Asp Trp Val Ser Val Pro Val Pro Ala Asn Ala
   5505 5510 5515 5520
   Pro Ser Pro Thr Ala Asp Arg Pro Trp Ala Val Ile Gly Ala Gly Leu
   5525 5530 5535
   Pro His Leu Pro Gly Leu Thr Glu His Glu His Val Thr Ala Tyr Asp
   5540 5545 5550
   Glu Pro Ala Asp Leu Leu Leu Ala Leu Asp Arg Gly Ala Pro Pro Pro
   5555 5560 5565
   Gly Val Leu Val Val Gly Gly Val Ala His Thr Glu Ala Arg Glu Tyr
   5570 5575 5580
   Ser Ala Glu Ala Pro Gly Glu Arg Gly Thr Glu Ala Cys Glu Ala Arg
   5585 5590 5595 5600
   Pro Asp Val Val His Val Gly Val Val His Thr Ala Ala Val His Ala
   5605 5610 5615
   Ala Ala Ala Gln Met Leu Ala Arg Leu Gln Ala Trp Leu Gly Asp Glu
   5620 5625 5630
   Arg Leu Ala Asp Ser Arg Leu Leu Val Leu Thr Cys Gly Ala Val Ala
   5635 5640 5645
   Arg Ala Ser Gly Asp Asp Ala Thr Asp Leu Pro Gly Ala Ala Val Trp
   5650 5655 5660
   Gly Leu Val Arg Ser Ala Gln Ser Glu His Pro Asp Arg Ile Thr Leu
   5665 5670 5675 5680
   Leu Asp Phe Glu Arg Gly Thr Glu Ala Glu Pro Gly Gln Leu Ala Thr
   5685 5690 5695
   Ala Leu Asn Cys Gly Glu Arg Gln Leu Ala Val Arg Pro Gly Gly Leu
   5700 5705 5710
   Phe Thr Pro Arg Leu Val Arg Ala Pro Arg Val Ala Asp Ala Val Pro
   5715 5720 5725
   Ala Val Pro Ala Val Ala Val Pro Ser Ala Gly His Ala Ala Val Pro
   5730 5735 5740
   Ala Ala Gly Pro Phe Leu Pro Gly Gly Thr Val Leu Ile Thr Gly Gly
   5745 5750 5755 5760
   Thr Gly Val Leu Gly Arg Leu Val Ala Arg His Leu Val Glu Ala His
   5765 5770 5775
   Gly Val Arg His Leu Leu Leu Ala Gly Arg Arg Gly Pro Asp Ala Glu
   5780 5785 5790
   Gly Ala Pro Glu Leu Arg Ala Glu Leu Gly Gly Leu Gly Ala Thr Val
   5795 5800 5805
   Glu Val Val Ala Cys Asp Ala Ala Asp Arg Gln Gln Leu Ala Asp Leu
   5810 5815 5820
   Leu Thr Arg Ile Pro Asp Asp Arg Pro Leu Thr Gly Val Val His Ser
   5825 5830 5835 5840
   Ala Gly Ile Leu Asp Asp Gly Val Ile Thr Ser Leu Ser Pro Glu Arg
   5845 5850 5855
   Leu Gly Ala Val Leu Arg Ala Lys Ala Asp Ala Ala Leu Leu Leu Asp
   5860 5865 5870
   Glu Leu Thr Arg Gly Ala Glu Leu Ser Ala Phe Val Met Phe Ser Ser
   5875 5880 5885
   Ala Ser Ala Val Val Gly Ser Pro Gly Gln Gly Asn Tyr Ala Ala Ala
   5890 5895 5900
   Asn Ala Val Leu Asp Phe Leu Ala His Arg Arg Arg Ala Glu Gly Leu
   5905 5910 5915 5920
   Pro Ala Val Ser Leu Ala Trp Gly Leu Trp Glu Glu Gly Thr Gly Met
   5925 5930 5935
   Thr Gly His Leu Asp Val Asp Asp His Ala Arg Ile Ser Arg Ala Gly
   5940 5945 5950
   Met Arg Pro Leu Pro Thr Ala Glu Ala Leu Ala Leu Phe Asp Ala Ala
   5955 5960 5965
   Leu Ala Asp Gly Glu Pro Phe Leu Met Pro Ala Arg Leu Asp Leu Thr
   5970 5975 5980
   Ala Val Arg Ser Gly Ala Ala Ser Ala Pro Val Pro Pro Leu Leu Gln
   5985 5990 5995 6000
   Gly Leu Leu Gln Leu Pro Arg Ser Arg Ser Ala Ala Ala Ala Pro Gly
   6005 6010 6015
   His Gly Ala Pro Ala Ala Asp Glu Ala Ala Ala Trp Arg Glu Arg Leu
   6020 6025 6030
   Ala Arg Gln Ser Ala Gly Glu Arg Arg Gln Ala Leu Leu Arg Leu Val
   6035 6040 6045
   Arg Ser His Val Ala Ala Val Leu Gly His Ser Gly Ala Asp Gly Ile
   6050 6055 6060
   Asp Ala Ser Arg Ala Phe Arg Glu Leu Gly Phe Asp Ser Leu Thr Ala
   6065 6070 6075 6080
   Val Glu Leu Arg Asn Arg Leu Thr Ala Ala Thr Gly Leu Arg Leu Arg
   6085 6090 6095
   Ala Thr Leu Ala Phe Asp Phe Pro Thr Pro Ala Ala Leu Ala Glu His
   6100 6105 6110
   Leu Gly Glu Arg Leu Leu Pro Asp Gln Glu Ala Thr Gly Glu Gln Ala
   6115 6120 6125
   Gly Asp Gln Leu Ser Gly Gly Ser Glu Glu Asp Val Arg Ser Leu Leu
   6130 6135 6140
   Thr Ser Ile Pro Ile Gly Arg Leu Arg Asp Ala Gly Leu Leu Gly Pro
   6145 6150 6155 6160
   Leu Leu Thr Leu Ala Asp Thr Gly Arg Gly Ala Ser Gly Ala Ala Ala
   6165 6170 6175
   Gly Pro Glu Asp Ala Pro Pro Ser Gly Gln Asp Thr Pro Ala Pro Val
   6180 6185 6190
   Ser Ile Asp Glu Met Asp Ile Asp Asp Leu Met Asp Leu Ala His Gly
   6195 6200 6205
   His Gly Thr Ala Pro Ala Arg Glu Pro Ala Asp Ala Glu Asp Ser Ser
   6210 6215 6220
   Ser Ser Arg Asn Arg Thr His His Thr His Glu Gly Glu Thr Ala
   6225 6230 6235
<210> 6
   <211> 4881
   <212> PRT
   <213> Streptomyces avermitilis
<400> 6
   Met Ala Asn Glu Glu Lys Leu Arg Asp Tyr Leu Lys Arg Val Thr Ala
   1 5 10 15
   Asp Leu Leu Asn Val Arg Arg Arg Leu Gln Gln Ile Glu Ser Gly Glu
   20 25 30
   Gln Glu Pro Ile Ala Ile Val Gly Met Ala Cys Arg Phe Pro Gly Gly
   35 40 45
   Val Glu Ser Ala Glu Asp Phe Trp Glu Leu Ile Ala Ser Gly Arg Asp
   50 55 60
   Ala Val Gly Glu Phe Pro Val Asp Arg Gly Trp Asp Val Glu Ala Phe
   65 70 75 80
   Tyr Asp Pro Glu Pro Gly Arg Ala Gly Ser Ser Tyr Thr Arg Arg Gly
   85 90 95
   Gly Phe Leu Glu Gly Ala Ala Glu Phe Asp Ala Gly Phe Phe Gly Ile
   100 105 110
   Ser Pro Arg Glu Ala Leu Ala Met Asp Pro Gln Gln Arg Leu Met Leu
   115 120 125
   Glu Val Ser Trp Glu Ala Leu Glu Arg Ala Gly Ile Asp Pro Ala Thr
   130 135 140
   Leu Arg Gly Ser Arg Thr Gly Val Phe Ala Gly Leu Met Ser Gln Asp
   145 150 155 160
   Tyr Ala Thr Arg Leu Leu Ser Val Pro Asp Asp Leu Ala Gly Tyr Leu
   165 170 175
   Gly Asn Gly Asn Ala Gly Ser Ile Leu Ser Gly Arg Val Ala Tyr Thr
   180 185 190
   Phe Gly Phe Glu Gly Pro Ala Val Thr Val Asp Thr Ala Cys Ser Ser
   195 200 205
   Ser Leu Val Ala Leu His Leu Ala Cys Gln Ser Leu Arg Thr Gly Glu
   210 215 220
   Ser Ser Phe Ala Leu Ala Gly Gly Val Thr Val Met Ser Thr Pro Gly
   225 230 235 240
   Met Phe Val Glu Phe Ser Arg Gln Arg Gly Leu Ser Pro Asp Gly Arg
   245 250 255
   Cys Lys Ala Tyr Ala Ser Ala Ala Asp Gly Thr Gly Met Ser Glu Gly
   260 265 270
   Val Gly Ile Leu Leu Leu Glu Arg Leu Ser Glu Ala Glu Arg Arg Gly
   275 280 285
   His Arg Val Leu Ala Val Val Arg Gly Ser Ala Val Asn Gln Asp Gly
   290 295 300
   Ala Ser Asn Gly Leu Thr Ala Pro Asn Gly Pro Ser Gln Gln Arg Val
   305 310 315 320
   Ile Arg Gln Ala Leu Ala Cys Ala Gly Leu Ser Val Ala Asp Val Asp
   325 330 335
   Val Val Glu Gly His Gly Thr Gly Thr Thr Leu Gly Asp Pro Ile Glu
   340 345 350
   Ala Gln Ala Leu Leu Ala Thr Tyr Gly Gln Arg Ala Gly Asp Thr Pro
   355 360 365
   Val Trp Leu Gly Ser Val Lys Ser Asn Ile Gly His Ala Gln Ala Ala
   370 375 380
   Ala Gly Val Ala Gly Val Ile Lys Met Val Met Ala Leu Arg Ala Gly
   385 390 395 400
   Val Leu Pro Arg Thr Leu His Val Asp Glu Pro Ser Ser Gln Val Asp
   405 410 415
   Trp Ser Ser Gly Ser Val Arg Val Leu Ala Asp Glu Val Glu Trp Pro
   420 425 430
   Gly Val Glu Gly Arg Leu Arg Arg Ala Gly Val Ser Ala Phe Gly Val
   435 440 445
   Ser Gly Thr Asn Ala His Val Ile Leu Glu Glu Ala Ser Gly Gly Ala
   450 455 460
   Gly Gly Gly Ala Gly Arg Leu Gln Glu Leu Gly Pro Gly Val Val Ser
   465 470 475 480
   Gly Ser Gly Val Val Pro Trp Val Val Ser Ala Arg Ser Glu Leu Ala
   485 490 495
   Leu Arg Gly Gln Ala Arg Arg Leu Arg Gly Val Val Ala Val Gly Gly
   500 505 510
   Gly Ala Asp Gly Val Gly Val Ser Pro Ala Gly Val Gly Arg Ala Leu
   515 520 525
   Val Ser Glu Arg Ser Val Phe Glu His Arg Ala Val Val Val Ala Glu
   530 535 540
   Asp Arg Asp Glu Phe Leu His Ala Leu Asp Ala Leu Ala Gly Gly Arg
   545 550 555 560
   Pro Val Pro Gly Val Val Glu Gly Arg Thr Thr Ser Gly Glu Leu Ala
   565 570 575
   Val Leu Phe Ala Gly Gln Gly Thr Gln Arg Ala Gly Met Gly Arg Glu
   580 585 590
   Leu Tyr Glu Ala Tyr Pro Val Phe Ala Gln Ala Ile Asp Glu Ile Cys
   595 600 605
   Ala Glu Ala Asp Thr Ala Arg Thr Asp Pro Gly Ala Pro Gly Leu Arg
   610 615 620
   Asp Val Leu Phe Ala Pro Gln Asp Ser Pro Glu Gly Arg Leu Ile Glu
   625 630 635 640
   Asp Thr Gly Phe Ala Gln Pro Ala Leu Phe Ala Phe Glu Val Ala Leu
   645 650 655
   Phe Arg Leu Leu Glu Thr Trp Gly Leu Thr Pro Asp Tyr Val Leu Gly
   660 665 670
   His Ser Val Gly Glu Leu Ala Ala Ala His Val Ala Gly Met Leu Cys
   675 680 685
   Leu Ala Asp Ala Val Ala Leu Val Val Ala Arg Gly Arg Leu Met Gln
   690 695 700
   Gly Leu Pro Ser Gly Gly Ala Met Val Ala Ile Glu Ala Ser Glu Asp
   705 710 715 720
   Glu Ile Leu Pro Leu Pro Asp Glu Tyr Ala Ser Arg Val Ala His Ala
   725 730 735
   Ala Val Asn Gly Pro Arg Ser Ile Val Leu Ser Gly Asp Glu Asp Ala
   740 745 750
   Val Leu Asp Leu Ala Gln Gln Trp Ala Ala Arg Gly Arg Arg Thr Arg
   755 760 765
   Arg Leu Arg Thr Ser His Ala Phe His Ser Pro His Met Asp Ala Met
   770 775 780
   Leu Gly Asp Phe Arg Arg Ala Ala Glu Gln Val Thr Phe Ser Ala Pro
   785 790 795 800
   Arg Ile Pro Val Val Ser Asn Val Thr Gly Ala Pro Leu Pro Ala Glu
   805 810 815
   Thr Met Cys Thr Pro Asp Tyr Trp Val Glu His Ala Arg Ser Thr Val
   820 825 830
   Arg Phe Ala Asp Gly Ile Ser Trp Leu Gln Glu Gln Gly Val Thr Thr
   835 840 845
   Cys Leu Glu Ile Gly Pro Asp Gly Thr Leu Ser Ala Leu Ala Gln Asp
   850 855 860
   Ser Leu Ser Ala Pro Ala Arg Ala Ile Pro Ala Leu Arg Pro Asp Gln
   865 870 875 880
   Pro Glu Ala Arg Ser Val Met Thr Ala Leu Ala Glu Leu Phe Val Ala
   885 890 895
   Gly Thr Ala Val Glu Trp Ala Gly Val Phe Glu Gly Thr Ala Arg Glu
   900 905 910
   Val Gly Asp Gly Cys Gly Val Glu Leu Pro Thr Tyr Ala Phe Glu Arg
   915 920 925
   Glu Arg Phe Trp Leu Asp Val Glu Glu Gly Ser Ala Gly Gly Ser Gly
   930 935 940
   Val Ser Gly Met Trp Gly Gly Pro Leu Trp Glu Ala Val Glu Cys Gly
   945 950 955 960
   Asp Ala Gly Val Val Ala Ser Leu Leu Gly Val Asp Glu Gly Ala Ser
   965 970 975
   Leu Gly Ala Val Val Ser Ala Leu Gly Glu Trp Gly Arg Val Arg His
   980 985 990
   Glu Arg Glu Val Val Asp Gly Trp Arg Tyr Arg Glu Val Trp Arg Pro
   995 1000 1005
   Val Ser Gly Gly Gly Val Gly Gly Leu Ser Gly Ala Trp Leu Val Val
   1010 1015 1020
   Ser Glu Gly Glu Ala Gly Pro Val Asp Val Val Ala Glu Gly Leu Glu
   1025 1030 1035 1040
   Arg Cys Gly Ala Arg Val Val Arg Val Glu Val Glu Ala Gly Cys Val
   1045 1050 1055
   Ser Arg Glu Val Leu Ala Gly His Leu Arg Glu Ala Val Asp Gly Glu
   1060 1065 1070
   Ala Val Gly Gly Val Val Ser Leu Val Gly Trp Gly Ser Gly Val Val
   1075 1080 1085
   Gln Ala Gly Val Ala Ser Val Gly Leu Val Gln Ala Leu Gly Asp Val
   1090 1095 1100
   Gly Val Gly Ala Arg Leu Trp Cys Val Thr Gly Gly Ala Val Ser Val
   1105 1110 1115 1120
   Gly Gly Arg Asp Ala Val Trp Gly Pro Ala Ser Gly Val Val Trp Gly
   1125 1130 1135
   Leu Gly Arg Val Val Gly Ala Glu Ala Pro Asp Arg Trp Gly Gly Leu
   1140 1145 1150
   Val Asp Val Pro Glu Leu Val Asp Glu Arg Val Val Asp Gly Leu Val
   1155 1160 1165
   Gly Val Leu Ala Gly Val Gly Gly Gly Gly Glu Ser Glu Phe Ala Val
   1170 1175 1180
   Arg Ser Ser Gly Ala Phe Val Arg Arg Leu Val Arg Ala Pro Leu Glu
   1185 1190 1195 1200
   Glu Ala Val Ala Glu Arg Glu Trp Arg Pro Arg Gly Thr Val Leu Val
   1205 1210 1215
   Thr Gly Gly Thr Gly Glu Leu Gly Ala His Val Ala Arg Trp Met Ala
   1220 1225 1230
   Arg Arg Gly Ala Glu His Leu Leu Leu Val Ser Arg Arg Gly Glu Ser
   1235 1240 1245
   Ala Gln Gly Val Glu Glu Leu Arg Ala Asp Leu Met Gly Leu Gly Ala
   1250 1255 1260
   Arg Val Ser Val Val Ala Cys Asp Ala Ala Asp Arg Glu Ala Leu Ala
   1265 1270 1275 1280
   Glu Val Leu Arg Ser Ala Val Pro Ala Glu Cys Pro Leu Gly Val Val
   1285 1290 1295
   Val His Ala Ala Gly Val Val Asp Asp Gly Val Leu Glu Gly Leu Ser
   1300 1305 1310
   Ser Glu Arg Val Thr Gly Val Leu Arg Ala Lys Ala Leu Ala Ala Trp
   1315 1320 1325
   Asn Leu His Glu Leu Thr Arg Gly Ala Asp Leu Ser Gly Phe Val Val
   1330 1335 1340
   Phe Ser Ser Ala Ala Ala Thr Phe Gly Pro Ala Gly Gln Gly Ser Tyr
   1345 1350 1355 1360
   Ala Ala Ala Asn Ala Tyr Val Glu Ala Ile Val Arg His Arg Arg Gly
   1365 1370 1375
   Glu Gly Leu Pro Gly Leu Ala Val Ala Trp Gly Pro Trp Ala Gly Gly
   1380 1385 1390
   Gly Met Ala Glu Gly Ala Val Gly Gln Met Arg Arg Arg Gly Leu Ala
   1395 1400 1405
   Ala Met Thr Pro Glu Thr Ala Leu Val Ala Leu Gly Gln Ala Leu Asp
   1410 1415 1420
   His Asp Glu Thr Cys Val Thr Val Ala Asp Ile Asp Trp Asp Arg Phe
   1425 1430 1435 1440
   Thr Ala Asn Ser Leu Pro Gly Ser Arg Leu Ser Pro Leu Ile Ser Asp
   1445 1450 1455
   Ile Pro Glu Ala Arg Leu Ala Arg Glu Thr Thr Gly Leu Asp Thr Ala
   1460 1465 1470
   Thr Ala Ser Pro Asp Ser Phe Ser Ala Arg Leu Lys Ala Met Asp Thr
   1475 1480 1485
   Ala Glu Gln Glu Arg Ala Leu Leu Asp Leu Val Arg Thr Tyr Ala Ala
   1490 1495 1500
   Thr Val Leu Gly His Ser Thr Pro Thr Ala Val Arg Pro Glu Arg Ala
   1505 1510 1515 1520
   Phe Arg Asp Leu Gly Phe Val Ser Val Ser Ala Val Glu Leu Arg Asn
   1525 1530 1535
   Arg Leu Asn Ala Val Thr Gly Leu Leu Leu Pro Thr Thr Leu Ile Phe
   1540 1545 1550
   Asp Tyr Pro Thr Pro Ser Ala Leu Ala Gly Tyr Leu Lys Glu Gln Leu
   1555 1560 1565
   Glu Glu Gly Ala Gly Gly Gln Arg Asp Ile Ala Pro Pro Val Pro Ala
   1570 1575 1580
   Ser Arg Val Asp Val Asp Glu Pro Ile Ala Ile Val Gly Met Ala Cys
   1585 1590 1595 1600
   Arg Phe Pro Gly Gly Val Glu Ser Ala Glu Asp Leu Trp Glu Leu Val
   1605 1610 1615
   Ala Ser Gly Arg Asp Ala Val Gly Glu Phe Pro Val Asp Arg Gly Trp
   1620 1625 1630
   Asp Val Glu Ala Phe Tyr Asp Pro Glu Pro Gly Arg Ala Gly Ser Ser
   1635 1640 1645
   Tyr Thr Arg Arg Gly Gly Phe Leu Glu Gly Ala Ala Glu Phe Asp Ala
   1650 1655 1660
   Gly Phe Phe Gly Ile Ser Pro Arg Glu Ala Leu Ala Met Asp Pro Gln
   1665 1670 1675 1680
   Gln Arg Leu Met Leu Glu Val Ser Trp Glu Ala Leu Glu Arg Ala Gly
   1685 1690 1695
   Ile Asp Pro Ala Thr Leu Arg Gly Ser Thr Thr Gly Val Phe Ala Gly
   1700 1705 1710
   Met Cys Ser Gln Asp Tyr Ala Asp Leu Val Arg Arg Ala Thr Glu Asp
   1715 1720 1725
   Leu Glu Gly Tyr Ala Met Thr Gly Leu Ser Ser Ser Val Thr Ser Gly
   1730 1735 1740
   Arg Val Ala Tyr Thr Leu Gly Leu Glu Gly Pro Ala Val Thr Val Asp
   1745 1750 1755 1760
   Thr Ala Cys Ser Ser Ser Leu Val Ala Leu His Leu Ala Cys Gln Ala
   1765 1770 1775
   Leu Arg Ser Gly Glu Cys Ser Leu Ala Leu Ala Gly Gly Val Thr Val
   1780 1785 1790
   Met Ser Thr Pro Gly Ala Phe Val Glu Phe Ser Arg Gln Arg Gly Leu
   1795 1800 1805
   Ser Pro Asp Gly Arg Cys Lys Ala Tyr Gly Ser Gly Ala Asp Gly Val
   1810 1815 1820
   Gly Trp Ala Glu Gly Val Gly Val Leu Leu Val Glu Arg Leu Ser Glu
   1825 1830 1835 1840
   Ala Glu Arg Arg Gly His Arg Val Leu Ala Val Val Arg Gly Ser Ala
   1845 1850 1855
   Val Asn Gln Asp Gly Ala Ser Asn Gly Leu Thr Ala Pro Asn Gly Pro
   1860 1865 1870
   Ser Gln Gln Arg Val Ile Arg Gln Ala Leu Ala Cys Ala Gly Leu Ser
   1875 1880 1885
   Val Ala Asp Val Asp Val Val Glu Gly His Gly Thr Gly Thr Thr Leu
   1890 1895 1900
   Gly Asp Pro Ile Glu Ala Gln Ala Leu Leu Ala Thr Tyr Gly Gln Gly
   1905 1910 1915 1920
   Arg Ser Gly Glu Arg Pro Val Trp Leu Gly Ser Val Lys Ser Asn Ile
   1925 1930 1935
   Gly His Ala Gln Ala Ala Ala Gly Val Ala Gly Val Ile Lys Met Val
   1940 1945 1950
   Met Ala Leu Arg Ala Gly Val Leu Pro Arg Thr Leu His Val Asp Glu
   1955 1960 1965
   Pro Ser Ser Gln Val Asp Trp Ser Ser Gly Ser Val Arg Val Leu Ala
   1970 1975 1980
   Asp Glu Val Glu Trp Pro Gly Val Glu Gly Arg Leu Arg Arg Ala Gly
   1985 1990 1995 2000
   Val Ser Ala Phe Gly Val Ser Gly Thr Asn Ala His Val Ile Leu Glu
   2005 2010 2015
   Glu Ala Ser Gly Gly Ala Asp Gly Gly Ala Gly Arg Leu Gln Glu Leu
   2020 2025 2030
   Gly Pro Gly Val Val Ser Gly Ser Gly Val Val Pro Trp Val Val Ser
   2035 2040 2045
   Ala Arg Ser Glu Leu Ala Leu Arg Gly Gln Ala Arg Arg Leu Arg Gly
   2050 2055 2060
   Val Val Ala Val Gly Gly Gly Ala Asp Gly Val Gly Val Ser Pro Ala
   2065 2070 2075 2080
   Gly Val Gly Arg Ala Leu Val Ser Glu Arg Ser Val Phe Glu His Arg
   2085 2090 2095
   Ala Val Val Val Ala Glu Asp Arg Asp Glu Phe Leu His Ala Leu Asp
   2100 2105 2110
   Ala Leu Ala Glu Gly Ala Pro Thr Ala Gly Val Val Gln Gly Val Ala
   2115 2120 2125
   Gly Pro Ala Ala Asp Gly Lys Ile Ala Met Leu Phe Gly Gly Gln Gly
   2130 2135 2140
   Thr His Trp Glu Gly Met Ala Gln Glu Leu Leu Gly Ser Ser Pro Val
   2145 2150 2155 2160
   Phe Ala Gln Gln Met Ser Asp Cys Ala Gln Ala Leu Glu Pro Tyr Leu
   2165 2170 2175
   Asp Trp Ser Leu Leu Asp Val Leu Arg Gly Ala Pro Asp Ala Pro Pro
   2180 2185 2190
   Leu Gln Arg Val Asp Val Val Gln Pro Val Leu Phe Ala Val Met Val
   2195 2200 2205
   Ser Leu Ala Ala Leu Trp Arg Ser Tyr Gly Val His Pro Asp Ala Val
   2210 2215 2220
   Ala Gly His Ser Gln Gly Glu Ile Ala Ala Ala Tyr Val Ala Gly Ala
   2225 2230 2235 2240
   Leu Ser Leu Asp Asp Ala Ala Arg Val Thr Ala Leu Arg Ser Gln Ala
   2245 2250 2255
   Leu Ala Ala Leu Ala Gly Gln Gly Ala Met Ala Ser Val Gly Leu Pro
   2260 2265 2270
   Val Glu Lys Leu Glu Pro Arg Leu Ala Thr Trp Gly Asp Arg Leu Val
   2275 2280 2285
   Ile Ala Ala Val Asn Gly Ala Arg Ser Ala Val Val Ser Gly Glu Pro
   2290 2295 2300
   Glu Ala Val Asp Ala Leu Val Glu Glu Leu Ser His Glu Asp Val Pro
   2305 2310 2315 2320
   Ala Arg Arg Leu Met Val Asp Trp Ala Ser His Ser Pro Gln Val Glu
   2325 2330 2335
   Ala Ile Gln Gly Arg Leu Leu Glu Leu Leu Ala Pro Ile Arg Ala Arg
   2340 2345 2350
   Thr Gly Asp Val Pro Phe Tyr Ser Thr Val Thr Gly Glu Arg Ile Asp
   2355 2360 2365
   Gly Thr Glu Leu Asp Ala Asp Tyr Trp Tyr Arg Asn Leu Arg Gln Val
   2370 2375 2380
   Val Arg Phe Arg Asp Ala Thr Gln Ala Leu Val Arg Ala Gly His Thr
   2385 2390 2395 2400
   Val Phe Ile Glu Ala Cys Pro His Pro Ala Val Ala Val Gly Val Gln
   2405 2410 2415
   Glu Thr Leu Asp Glu Met Gly Asp Leu Asp Ser Leu Val Val Gly Ser
   2420 2425 2430
   Leu Arg Arg Gly Glu Gly Gly Leu Arg Arg Phe Leu Met Ser Val Ala
   2435 2440 2445
   Glu Leu Phe Val Gly Gly Val Ala Val Glu Trp Ser Gly Val Phe Gly
   2450 2455 2460
   Ser Val Gly Arg Gly Val Ala Gly Gly Cys Gly Val Glu Leu Pro Thr
   2465 2470 2475 2480
   Tyr Ala Phe Glu Arg Glu Arg Phe Trp Leu Asp Val Glu Gly Ala Pro
   2485 2490 2495
   Arg Gly Ser Gly Val Ser Gly Gln Trp Gly Gly Gln Leu Ser Glu Ala
   2500 2505 2510
   Val Asp Thr Val Arg Gly Gly Met Leu Arg Asp Cys Leu Ala Gly Leu
   2515 2520 2525
   Asp Pro Ala Ala Gln Ala Glu Thr Val Leu Asp Leu Val Leu Thr His
   2530 2535 2540
   Ala Ala Ala Val Leu Gly His Gly Thr Ala Asp Ala Val Val Pro Glu
   2545 2550 2555 2560
   Arg Ala Phe Arg Asp Leu Gly Phe Asp Ser Leu Thr Ala Val Glu Leu
   2565 2570 2575
   Arg Asn Arg Leu Asn Thr Ala Thr Gly Leu Arg Phe Pro Arg Thr Leu
   2580 2585 2590
   Val Phe Asp His Pro Arg Pro Val Ala Leu Ala Ala His Ile His Glu
   2595 2600 2605
   Gln Leu Ser Gly Gly Ser Pro Thr Thr Gly Thr Ala Leu Ala Leu Ala
   2610 2615 2620
   Leu Arg Ala Pro Ala Pro Arg Val Asp Val Asp Glu Pro Ile Ala Ile
   2625 2630 2635 2640
   Val Gly Met Ala Cys Arg Phe Pro Gly Gly Val Glu Ser Ala Glu Asp
   2645 2650 2655
   Phe Trp Glu Leu Ile Ala Ser Gly Arg Asp Ala Val Gly Glu Phe Pro
   2660 2665 2670
   Val Asp Arg Gly Trp Asp Val Glu Ala Phe Tyr Asp Pro Glu Pro Gly
   2675 2680 2685
   Arg Ala Gly Thr Ser Tyr Thr Arg Cys Gly Gly Phe Leu Gln Gly Ala
   2690 2695 2700
   Ala Glu Phe Asp Ala Gly Phe Phe Gly Ile Ser Pro Arg Glu Ala Leu
   2705 2710 2715 2720
   Ala Met Asp Pro Gln Gln Arg Leu Met Leu Glu Val Ser Trp Glu Ala
   2725 2730 2735
   Leu Glu Arg Ala Gly Ile Asp Pro Ala Thr Leu His Gly Ser Thr Thr
   2740 2745 2750
   Gly Val Phe Ala Gly Val Ser Gln Gln Asp Tyr Ala Glu Leu Leu Arg
   2755 2760 2765
   Arg Gly Thr Gln Asp His Glu Gly Tyr Ala Leu Thr Gly Val Ser Asn
   2770 2775 2780
   Ser Val Val Ser Gly Arg Leu Ser Tyr Thr Phe Gly Phe Glu Gly Pro
   2785 2790 2795 2800
   Ala Val Thr Val Asp Thr Ala Cys Ser Ser Ser Leu Val Ala Leu His
   2805 2810 2815
   Leu Ala Cys Gln Ala Leu Arg Ser Gly Glu Cys Ser Leu Ala Leu Ala
   2820 2825 2830
   Gly Gly Val Thr Val Met Ser Thr Pro Gly Ala Phe Val Glu Phe Ser
   2835 2840 2845
   Arg Gln Arg Gly Leu Ser Pro Asp Gly Arg Cys Lys Ala Tyr Gly Ser
   2850 2855 2860
   Gly Ala Asp Gly Val Gly Trp Ala Glu Gly Val Gly Val Leu Leu Val
   2865 2870 2875 2880
   Glu Arg Leu Ser Glu Ala Glu Arg Arg Gly His Arg Val Leu Ala Val
   2885 2890 2895
   Val Arg Gly Ser Ala Val Asn Gln Asp Gly Ala Ser Asn Gly Leu Thr
   2900 2905 2910
   Ala Pro Asn Gly Pro Ser Gln Gln Arg Val Ile Arg Gln Ala Leu Ala
   2915 2920 2925
   Cys Ala Gly Leu Ser Val Ala Asp Val Asp Val Val Glu Gly His Gly
   2930 2935 2940
   Thr Gly Thr Thr Leu Gly Asp Pro Ile Glu Ala Gln Ala Leu Leu Ala
   2945 2950 2955 2960
   Thr Tyr Gly Gln Gly Arg Ser Gly Glu Arg Pro Val Trp Leu Gly Ser
   2965 2970 2975
   Val Lys Ser Asn Ile Gly His Ala Gln Ala Ala Ala Gly Val Ala Gly
   2980 2985 2990
   Val Ile Lys Met Val Met Ala Leu Asn His Glu Leu Leu Pro Thr Ser
   2995 3000 3005
   Leu His Ile Asp Glu Pro Ser Pro His Ile Asp Trp Ser Ser Gly Gly
   3010 3015 3020
   Val Arg Leu Leu Thr Glu Pro Val Pro Trp Gln Gln Asn Gly Arg Pro
   3025 3030 3035 3040
   Arg Arg Ala Gly Val Ser Ala Phe Gly Val Ser Gly Thr Asn Ala His
   3045 3050 3055
   Val Ile Ile Glu Gln Ala Pro Val Glu Ala His Val Ile Ser Glu Pro
   3060 3065 3070
   Val Pro Ala Glu Ala His Val Ile Val Glu Gln Ala Pro Val Glu Ala
   3075 3080 3085
   Pro His Val Val Asp Ala Thr Gly Pro Ala Asp Leu Thr Glu Pro Gln
   3090 3095 3100
   Glu Glu Ala Ala Glu Pro Glu Cys Val Ala Asp Ala Val Thr Glu Met
   3105 3110 3115 3120
   Ser Ala Glu Pro Glu Cys Val Ala Asp Ala Met Ser Glu Met Ser Ala
   3125 3130 3135
   Glu Cys Val Ala Glu Ala Val Ser Asp Lys Ser Ala Glu Pro Glu Cys
   3140 3145 3150
   Val Ala Asp Ala Met Ser Asp Lys Pro Ala Leu Leu Pro Ile Pro Trp
   3155 3160 3165
   Leu Leu Ser Ala Lys Ser Glu Arg Ala Leu Arg Gly Gln Ala Arg Arg
   3170 3175 3180
   Leu Arg Gln Phe Ala Ala Arg Ala Ser Asp Ala Arg Pro Ala Asp Val
   3185 3190 3195 3200
   Ala His Ala Leu Ala Ala Gln Arg Ser Val Phe Asp His Arg Ala Val
   3205 3210 3215
   Val Val Ala Glu Asp Arg Asp Gly Phe Leu Gln Ala Leu Asp Ala Leu
   3220 3225 3230
   Ala Glu Gly Arg Ser Ala Asp Gly Leu Ile Glu Gly Ser Val Gly Pro
   3235 3240 3245
   Arg Gly Gly His Ser Gly Arg Arg Arg Gly Lys Thr Ala Met Leu Phe
   3250 3255 3260
   Ala Gly Gln Gly Thr Gln Arg Val Gly Met Gly Arg Gln Leu Tyr Ala
   3265 3270 3275 3280
   Ala His Pro Ala Tyr Ala Asp Ala Leu Asp Gln Val Leu Ala Glu Leu
   3285 3290 3295
   Asp Gly His Leu Asp Gln Pro Leu Arg Pro Leu Ile His Ala Ser Ala
   3300 3305 3310
   Asp Leu Ala Asp Val Ala Asp Ala Ala Asp Val Leu Asp Arg Thr Arg
   3315 3320 3325
   Tyr Ala Gln Pro Ala Leu Phe Ala Val Gln Val Ala Leu Phe Arg His
   3330 3335 3340
   Leu Glu Arg Leu Gly Val Arg Ala Asp Phe Val Ala Gly His Ser Ile
   3345 3350 3355 3360
   Gly Glu Leu Ala Ala Ala His Val Ala Gly Val Leu Pro Leu Ala Ala
   3365 3370 3375
   Ala Cys Arg Leu Val Ala Ala Arg Gly Arg Leu Met Glu Gln Leu Ala
   3380 3385 3390
   Pro Gly Gly Ala Met Val Ala Val Arg Ala Ser Glu Ala Glu Ala Arg
   3395 3400 3405
   Gln Ala Leu Asp Gly Arg Glu Ala Arg Val Ser Val Ala Ala Val Asn
   3410 3415 3420
   Gly Pro Ala Ser Val Val Phe Ser Gly Ala Glu Asp Glu Val Gly Asn
   3425 3430 3435 3440
   Met Ala Asp Trp Phe Ala Glu Arg Gly Arg Arg Val Lys Arg Leu Arg
   3445 3450 3455
   Thr Gly His Ala Phe His Ser Pro Leu Met Asp Pro Met Leu Glu Glu
   3460 3465 3470
   Phe Gln Gln Val Ala Ala Ser Leu Thr Tyr Ser Glu Pro Ala Ile Pro
   3475 3480 3485
   Met Val Ser Thr Leu Thr Gly Asp Ile Val Ala Ala Gly Glu Leu Ser
   3490 3495 3500
   Asp Pro Glu Tyr Trp Val Arg Gln Val Arg Arg Thr Val Arg Phe Gly
   3505 3510 3515 3520
   Asp Ala Ile Ser Arg Leu His Thr Asp Gly Val Arg Thr Phe Met Glu
   3525 3530 3535
   Leu Gly Pro Asp Gly Thr Leu Ser Ala Leu Ala Glu Glu Cys Leu Glu
   3540 3545 3550
   Ala Thr Ala Asp Ser His Pro Ala Asp Asp Asp Thr Gly Thr Pro Gln
   3555 3560 3565
   Glu Asn Leu Leu Ile Pro Leu Leu Arg Pro Asp Ser Pro Glu Pro Gly
   3570 3575 3580
   Thr Leu Leu Thr Gly Leu Ala Arg Leu His Thr His Gly Ala Ala Ala
   3585 3590 3595 3600
   Val Asn Trp Pro Ala Ala Leu Pro Glu Arg Asp Arg Ala Arg His Leu
   3605 3610 3615
   Asp Leu Pro Thr Tyr Ala Phe Asp His His Arg Tyr Trp Val Asp Thr
   3620 3625 3630
   Ser Ala Gly His Pro Gly Asp Leu Ser Ala Ala Gly Leu Gly Thr Ala
   3635 3640 3645
   Gly His Pro Leu Leu Gly Ser Ala Val Ala Leu Ala Glu Ser Gln Glu
   3650 3655 3660
   Leu Leu Phe Thr Gly Arg Leu Ser Leu Arg Thr His Pro Trp Leu Ala
   3665 3670 3675 3680
   Asp His Ala Ile Phe Gly Thr Val Leu Leu Pro Gly Thr Ala Ile Leu
   3685 3690 3695
   Glu Leu Ala Val Arg Ala Gly Asp Glu Val Asp Cys Gly Thr Val Glu
   3700 3705 3710
   Glu Leu Thr Leu Arg Thr Pro Leu Val Leu Pro Glu Gln Gly Ser Val
   3715 3720 3725
   Ile Leu Gln Leu Ser Val Gly Ala Pro Gln Gly Pro Gln Thr Pro Glu
   3730 3735 3740
   Glu Pro Glu Arg Arg Thr Phe Ala Leu Tyr Ala Arg Glu Asp Asp Gly
   3745 3750 3755 3760
   Leu Ser Ser Ser Ser Ala Ala Ala Thr Gly Thr Glu Trp Thr Cys His
   3765 3770 3775
   Ala Thr Gly Val Leu Thr Gly Thr Ala Arg Pro Ala Glu Glu His Thr
   3780 3785 3790
   Gln Glu Pro Trp Pro Pro Ala Asp Ala Ala Pro Val Asp Leu Asp Gly
   3795 3800 3805
   Trp Tyr Glu Gln Leu Ala Gly Ala Gly Leu Gly Tyr Gly Pro Val Phe
   3810 3815 3820
   Gln Gly Leu Arg Glu Val Trp Arg Arg Gly Asp Glu Val Phe Ala Val
   3825 3830 3835 3840
   Val Thr Leu Pro Glu Ser Thr Glu Gly Gln Ala Ala Asp Ala Ala Arg
   3845 3850 3855
   Tyr Ala Leu His Pro Ala Leu Leu Asp Ala Ala Leu His Pro Val Val
   3860 3865 3870
   Leu Arg His Glu Gly Asp Ala Ala Ala Asp Gly His Gly Trp Leu Pro
   3875 3880 3885
   Phe Ser Trp Thr Gly Val Thr Val Ala Ala Ser Gly Ala Ser Thr Leu
   3890 3895 3900
   His Val Arg Leu Thr Val Arg Thr Asp Glu Asp Ala Val Gly Leu Leu
   3905 3910 3915 3920
   Ala Thr Asp Ala Ser Gly Arg Ile Val Ile Ser Ala Gly Ser Leu Ala
   3925 3930 3935
   Phe Arg Pro Val Ser Ala Glu Gln Leu Gln Ala Ala Arg Thr Gly Tyr
   3940 3945 3950
   His Asp His Leu Phe Arg Ile Glu Trp Arg Pro Leu His Leu Pro Thr
   3955 3960 3965
   Thr Pro Ala Arg Thr Ala Asp Trp Ala Leu Ile Gly Pro Gly Ala Arg
   3970 3975 3980
   Arg Thr Ala Ala Val Leu Glu Arg Asn Gly Ala Ser Trp Gln Ala Tyr
   3985 3990 3995 4000
   Pro Asp Pro Ala Ala Leu Ala Glu Ala Leu Ala Ala Gly Ala Pro Ala
   4005 4010 4015
   Pro Gly Met Val Val Ile Ser Cys Glu Pro Asp Gly Ala Ser Ala Pro
   4020 4025 4030
   Thr Asp Ser Ala Leu Thr Asp Ser Ala Leu Thr Asp Ser Ala Pro Ala
   4035 4040 4045
   Gly Ser Ala Pro Ala Asp Ser Thr Ala Leu Ala Asp Ala Thr Arg Gln
   4050 4055 4060
   Ala Thr Thr Arg Val Leu Ala Leu Leu Gln Glu Trp Val Ala Asp Glu
   4065 4070 4075 4080
   Arg Leu Ala Ala Cys Arg Leu Ala Leu Leu Thr His Gly Ser Val Thr
   4085 4090 4095
   Ala Thr Pro Asp Glu Pro Val Ser Asp Leu Ala His Ala Ala Val Trp
   4100 4105 4110
   Gly Leu Val Arg Ser Val Gln Thr Glu Asn Pro Asp Arg Phe Leu Leu
   4115 4120 4125
   Ala Asp Thr Asp Asp Thr Asp Ala Ser Arg Asn Ala Leu Pro Leu Leu
   4130 4135 4140
   Ala Gly Glu Pro Gln Ile Ala Leu Arg Asn Gly Ala Val Arg Ile Pro
   4145 4150 4155 4160
   Arg Met Thr Arg Val Pro Val Arg Gln Pro Gln Pro Ser Thr Thr Asp
   4165 4170 4175
   Ala Asp Trp Asp Pro Glu Ala Thr Val Leu Ile Thr Gly Gly Thr Gly
   4180 4185 4190
   Val Leu Gly Arg Leu Val Ala Arg His Leu Ala Thr Ala His Gly Val
   4195 4200 4205
   Arg His Leu Leu Leu Ala Thr Arg Arg Gly Thr Ala Ala Asp Gly Ala
   4210 4215 4220
   Ala Asp Leu Val Ala Glu Leu Ala Gly Leu Gly Ala Glu Ala Thr Val
   4225 4230 4235 4240
   Ala Ala Cys Asp Ile Gly Asp Arg Ala Ala Val Ala Ala Leu Leu Asp
   4245 4250 4255
   Gln Val Pro Ala Gln His Pro Leu Lys Ala Val Ile His Thr Ala Gly
   4260 4265 4270
   Val Val Asp Asp Gly Ile Leu Thr Ser Leu Thr Pro Glu Arg Met Glu
   4275 4280 4285
   Ala Val Leu His Ala Lys Ala Phe Gly Ala Ala His Leu His Asp Leu
   4290 4295 4300
   Thr Arg Asp Ala Gly Leu Thr Thr Phe Thr Val Phe Ser Ser Ala Ala
   4305 4310 4315 4320
   Ala Ser Phe Gly Ser Pro Gly Gln Gly Asn Tyr Thr Ala Ala Asn Ala
   4325 4330 4335
   Phe Leu Asp Ala Leu Met Gln His Arg His Thr Gln Ala Leu Pro Gly
   4340 4345 4350
   Arg Ser Leu Ala Trp Gly Leu Trp Gly Glu Ala Asp Gly Met Thr Arg
   4355 4360 4365
   Asn Leu Ala Gly Thr Asp Phe Ala Arg Met Ala Arg Gly Gly Leu Leu
   4370 4375 4380
   Pro Leu Ser Asn Ala Gln Gly Leu Ala Leu Leu Asp Thr Ala Asp Arg
   4385 4390 4395 4400
   Leu Gly Pro Phe Gly Asp Gly Leu Leu Leu Ala Thr Arg Leu Asp Ala
   4405 4410 4415
   Ala Thr Leu His Ala Gln Ala Thr Ala Gly Ala Leu Pro Arg Ile Leu
   4420 4425 4430
   His Gly Leu Ile Arg Ile Pro Ala Arg Arg Ser Ala Asp His Gly Ile
   4435 4440 4445
   Ala Thr Asp Thr Pro Ala Thr Leu Arg Glu Arg Leu Ala Gly Leu Thr
   4450 4455 4460
   Ile Pro Ala Gln Arg Thr Gly Leu Leu Leu Glu Leu Val Arg Thr His
   4465 4470 4475 4480
   Ala Ala Ala Val Leu Gly His Pro Thr Ser Ala Val Thr Ala Ala Asp
   4485 4490 4495
   Gly Ala Leu Pro Asp Asp Leu Val Pro Ala Asp Thr Glu Phe Arg Asp
   4500 4505 4510
   Leu Gly Phe Asp Ser Leu Thr Ala Val Glu Leu Arg Asn Arg Ile Asn
   4515 4520 4525
   Ala Val Thr Gly Leu Arg Leu Pro Ala Thr Leu Ile Phe Asp Gln Pro
   4530 4535 4540
   Ser Pro Ala Ala Leu Ala Asp His Leu Ala Thr Arg Leu Thr Ala Glu
   4545 4550 4555 4560
   Ala Gly Thr Pro Asp Glu Pro Ala Pro Ala Ala Ala Ala Ala Gly Ala
   4565 4570 4575
   Gly Ser Ala Gly Ser Ala Glu Thr Gly Gln Gln Arg Ser Thr Gly Ser
   4580 4585 4590
   Glu Lys Gln Gln Thr Arg Gly Gly Thr Ser Thr Glu Thr Val Glu Ser
   4595 4600 4605
   Leu Phe Trp Ile Gly His Asp Thr Arg Arg Ile Glu Glu Ser Met Ala
   4610 4615 4620
   Leu Leu Ser Ala Ala Ser Phe Phe Arg Pro Ala Phe Thr Asp Pro Ser
   4625 4630 4635 4640
   Asp Ile Pro Glu Pro Thr Phe Val Arg Leu Ala Gln Gly Glu Ala Arg
   4645 4650 4655
   Ala Gln Gly Glu Ala Leu Ala Arg Gly Glu Thr Arg Pro Ala Leu Ile
   4660 4665 4670
   Cys Leu Pro Thr Val Ala Ala Val Ser Ser Val Tyr Gln Tyr Ser Arg
   4675 4680 4685
   Phe Ala Ala Gly Leu Asn Gly His Arg Asp Val Trp Tyr Val Pro Ala
   4690 4695 4700
   Pro Gly Phe Leu Glu Gly Glu Pro Leu Pro Ser Gly Ile Gly Ala Val
   4705 4710 4715 4720
   Thr Arg Met Phe Ala Asp Ala Ile Val Arg Phe Thr Asp Gly Ala Pro
   4725 4730 4735
   Phe Ala Leu Ala Gly His Ser Ala Gly Gly Trp Phe Val Tyr Ala Val
   4740 4745 4750
   Thr Ser His Leu Glu Arg Leu Gly Val Arg Pro Glu Ala Val Val Thr
   4755 4760 4765
   Met Asp Ala Tyr Leu Pro Asp Asp Gly Ile Ala Pro Val Ala Ser Ala
   4770 4775 4780
   Leu Thr Ser Glu Ile Phe Asp Arg Val Thr Gln Phe Val Asp Val Asp
   4785 4790 4795 4800
   Tyr Thr Arg Leu Val Ala Met Gly Gly Tyr Phe Arg Ile Phe Ser Gly
   4805 4810 4815
   Trp Ser Pro Pro Asp Ile Thr Thr Pro Ala Leu Phe Leu Arg Gly Arg
   4820 4825 4830
   Asp Gly Glu Gln Met Pro Pro Pro Trp Gly Val Pro His Thr Val Leu
   4835 4840 4845
   Asp Ile Gln Gly Asn His Phe Thr Met Leu Glu Gln Phe Ala Asp Ser
   4850 4855 4860
   Thr Ala Arg His Val Asp Glu Trp Leu Thr Glu Ile Ala Ser Val Arg
   4865 4870 4875 4880
   Arg
<210> 7
   <211> 5532
   <212> PRT
   <213> Streptomyces avermitilis
<400> 7
   Met Asp Thr Ser Ser Glu Lys Leu Val Asp Ala Leu Arg Ala Ser Leu
   1 5 10 15
   Lys Ala Asn Gln Thr Leu Arg Ala Arg Asn Glu Gln Leu Ala Ala Ala
   20 25 30
   Met Glu Ala Ser Ser Glu Pro Ile Ala Ile Val Gly Met Ala Cys Arg
   35 40 45
   Phe Pro Gly Gly Val Cys Ser Pro Glu Glu Leu Trp Glu Leu Val Ala
   50 55 60
   Ser Gly Gly Asp Ala Ile Gly Glu Phe Pro Ala Gly Arg Gly Trp Asp
   65 70 75 80
   Leu Glu Gly Leu Phe Asp Ser Asp Pro Asp Arg Ser Gly Thr Ser Tyr
   85 90 95
   Ala Arg Tyr Gly Gly Phe Leu Tyr Glu Ala Gly Glu Phe Asp Ala Asp
   100 105 110
   Phe Phe Gly Ile Ser Pro Arg Glu Ala Leu Ala Met Asp Pro Gln Gln
   115 120 125
   Arg Leu Leu Leu Glu Thr Ser Trp Glu Ala Phe Glu Arg Ala Gly Ile
   130 135 140
   Asp Pro Leu Ser Met Arg Gly Ser Arg Thr Gly Val Phe Ala Gly Val
   145 150 155 160
   Met Tyr His Asp Tyr Gly Ser Arg Leu Gly Thr Ile Pro Glu Gly Phe
   165 170 175
   Glu Gly Tyr Ile Gly Asn Gly Ser Gly Gly Ala Val Ala Ser Gly Arg
   180 185 190
   Val Ala Tyr Thr Leu Gly Leu Glu Gly Pro Ala Val Ser Val Asp Thr
   195 200 205
   Ala Cys Ser Ser Ser Leu Val Ala Leu His Leu Ala Cys Gln Ser Leu
   210 215 220
   Arg Ser Gly Glu Cys Thr Leu Ala Leu Ala Gly Gly Val Thr Val Met
   225 230 235 240
   Ser Thr Pro His Leu Phe Val Glu Phe Ser Arg Gln Arg Gly Leu Ser
   245 250 255
   Val Asp Gly Arg Cys Lys Ser Phe Ala Gly Gly Ala Asp Gly Thr Gly
   260 265 270
   Met Gly Glu Gly Val Gly Met Leu Leu Val Glu Arg Leu Ser Asp Ala
   275 280 285
   Val Arg Leu Gly His Arg Val Leu Ala Val Leu Arg Gly Ser Ala Val
   290 295 300
   Asn Gln Asp Gly Ala Ser Asn Gly Leu Thr Ala Pro Asn Gly Pro Ala
   305 310 315 320
   Gln Glu Arg Val Ile Arg Gln Ala Leu Ala Asn Ala Gly Leu Ser Val
   325 330 335
   Ala Asp Val Asp Val Val Glu Gly His Gly Thr Gly Thr Thr Leu Gly
   340 345 350
   Asp Pro Ile Glu Ala Gln Ala Leu Leu Ala Thr Tyr Gly Gln Arg Ala
   355 360 365
   Gly Asn Arg Pro Leu Trp Leu Gly Ser Val Lys Ser Asn Ile Gly His
   370 375 380
   Ala Gln Ala Ala Ala Gly Val Gly Gly Val Ile Lys Met Val Met Ala
   385 390 395 400
   Leu Arg Glu Gly Val Leu Pro Arg Thr Leu His Val Asp Glu Pro Ser
   405 410 415
   Pro Gln Val Asp Trp Ser Ala Gly Ala Val Arg Leu Leu Thr Glu Ala
   420 425 430
   Val Pro Trp Pro Gly Asp Ala Ala Gly Arg Leu Arg Arg Ala Gly Val
   435 440 445
   Ser Ser Phe Gly Val Ser Gly Thr Asn Ala His Val Ile Leu Glu Glu
   450 455 460
   Ala Pro Ala Ala Gly Gly Cys Val Ala Gly Gly Gly Val Leu Glu Gly
   465 470 475 480
   Ala Pro Gly Leu Ala Ile Ser Val Ala Glu Ser Val Ala Ala Pro Val
   485 490 495
   Ala Val Ser Ala Pro Val Ala Glu Ser Val Pro Val Pro Val Pro Val
   500 505 510
   Pro Val Pro Val Pro Val Ser Ala Arg Ser Glu Ala Gly Leu Arg Ala
   515 520 525
   Gln Ala Glu Ala Leu Arg Gln Tyr Val Ala Val Arg Pro Asp Val Ser
   530 535 540
   Leu Ala Asp Val Gly Ala Gly Leu Ala Cys Gly Arg Ala Val Leu Glu
   545 550 555 560
   His Arg Ala Val Val Leu Ala Ala Asp Arg Glu Glu Leu Val Gln Gly
   565 570 575
   Leu Gly Ala Leu Ala Ala Gly Glu Pro Asp Arg Arg Val Thr Thr Gly
   580 585 590
   His Ala Pro Gly Gly Asp Arg Gly Gly Val Val Phe Val Phe Pro Gly
   595 600 605
   Gln Gly Gly Gln Trp Ala Gly Met Gly Val Arg Leu Leu Ala Ser Ser
   610 615 620
   Pro Val Phe Ala Arg Arg Met Gln Ala Cys Glu Glu Ala Leu Ala Pro
   625 630 635 640
   Trp Val Asp Trp Ser Val Val Asp Ile Leu Arg Arg Asp Ala Gly Asp
   645 650 655
   Ala Val Trp Glu Arg Ala Asp Val Val Gln Pro Val Leu Phe Ser Val
   660 665 670
   Met Val Ser Leu Ala Ala Leu Trp Arg Ser Tyr Gly Ile Glu Pro Asp
   675 680 685
   Ala Val Leu Gly His Ser Gln Gly Glu Ile Ala Ala Ala His Val Cys
   690 695 700
   Gly Ala Leu Ser Leu Lys Asp Ala Ala Lys Thr Val Ala Leu Arg Ser
   705 710 715 720
   Arg Ala Leu Ala Ala Val Arg Gly Arg Gly Gly Met Ala Ser Val Pro
   725 730 735
   Leu Pro Ala Gln Glu Val Glu Gln Leu Ile Gly Glu Arg Trp Ala Gly
   740 745 750
   Arg Leu Trp Val Ala Ala Val Asn Gly Pro Arg Ser Thr Ala Val Ser
   755 760 765
   Gly Asp Ala Glu Ala Val Asp Glu Val Leu Ala Tyr Cys Ala Gly Thr
   770 775 780
   Gly Val Arg Ala Arg Arg Ile Pro Val Asp Tyr Ala Ser His Cys Pro
   785 790 795 800
   His Val Gln Pro Leu Arg Glu Glu Leu Leu Glu Leu Leu Gly Asp Ile
   805 810 815
   Ser Pro Gln Pro Ser Gly Val Pro Phe Phe Ser Thr Val Glu Gly Thr
   820 825 830
   Trp Leu Asp Thr Thr Thr Leu Asp Ala Ala Tyr Trp Tyr Arg Asn Leu
   835 840 845
   His Gln Pro Val Arg Phe Ser Asp Ala Val Gln Ala Leu Ala Asp Asp
   850 855 860
   Gly His Arg Val Phe Val Glu Val Ser Pro His Pro Thr Leu Val Pro
   865 870 875 880
   Ala Ile Glu Asp Thr Thr Glu Asp Thr Ala Glu Asp Val Thr Ala Ile
   885 890 895
   Gly Ser Leu Arg Arg Gly Asp Asn Asp Thr Arg Arg Phe Leu Thr Ala
   900 905 910
   Leu Ala His Thr His Thr Thr Gly Ile Gly Thr Pro Thr Thr Trp His
   915 920 925
   His His Tyr Thr His His His Thr His Pro His Asn His His Leu Asp
   930 935 940
   Leu Pro Thr Tyr Pro Phe Gln Arg Gln His Tyr Trp Leu Asp Ala Pro
   945 950 955 960
   Thr Gly Ala Gly Asp Val Ala Ala Ala Gly Leu Glu Pro Ala Glu His
   965 970 975
   Pro Leu Leu Ala Ala Thr Val Gln Leu Ala Asp Thr Asp Gly Cys Leu
   980 985 990
   Leu Thr Gly Arg Leu Ser Leu Arg Ser His Pro Trp Leu Gly Asp Tyr
   995 1000 1005
   Glu Val Gly Gly Ala Val Leu Leu Ser Gly Ser Ala Phe Val Glu Leu
   1010 1015 1020
   Ala Val Gln Val Gly Glu Arg Val Gly Cys Thr Arg Ile Glu Gln Leu
   1025 1030 1035 1040
   Thr Val His Ala Pro Leu Val Val Pro Val Gly Gly Gly Val Ser Val
   1045 1050 1055
   Gln Val Gly Val Ala Ala Ala Asp Gly Glu Gly Arg Arg Leu Val Ser
   1060 1065 1070
   Val Tyr Ala Arg Gly Gly Ser Ala Cys Gly Gly Gly Gly Ala Ser Gly
   1075 1080 1085
   Gly Val Trp Thr Cys His Ala Ser Gly Val Leu Val Glu Ala Ala Ala
   1090 1095 1100
   Gly Gly Gly Val Val Val Asp Gly Leu Ala Gly Val Trp Pro Pro Arg
   1105 1110 1115 1120
   Gly Ala Val Ala Val Asp Val Asp Gly Val Arg Asp Arg Leu Ala Gly
   1125 1130 1135
   Ala Gly Cys Val Leu Gly Pro Val Phe Ser Gly Leu Arg Ala Val Trp
   1140 1145 1150
   Arg Asp Gly Gly Asp Leu Leu Ala Glu Val Cys Leu Pro Glu Glu Ala
   1155 1160 1165
   Trp Gly Asp Ala Ala Gly Phe Gly Leu His Pro Ala Leu Leu Asp Gly
   1170 1175 1180
   Val Val Gln Pro Leu Ser Val Leu Leu Pro Gly Gly Thr Gly Phe Gly
   1185 1190 1195 1200
   Glu Gly Ala Gly Phe Gly Glu Gly Val Arg Val Pro Ala Val Trp Gly
   1205 1210 1215
   Gly Val Ser Leu His Arg Ala Gly Val Thr Gly Val Arg Val Arg Val
   1220 1225 1230
   Trp Ala Val Gly Arg Gly Gly Gly Arg Glu Ala Val Ser Val Val Val
   1235 1240 1245
   Gly Asp Glu Ala Gly Val Pro Val Ala Ser Val Asp Arg Leu Glu Leu
   1250 1255 1260
   Arg Pro Val Asp Met Gly Gln Leu Arg Ala Val Ser Val Ser Ala Gly
   1265 1270 1275 1280
   Arg Arg Gly Ser Leu Tyr Ala Val Gln Trp Ala Glu Val Gly Pro Val
   1285 1290 1295
   Pro Val Cys Gly Gln Ala Trp Ala Trp His Glu Asp Val Gly Glu Ser
   1300 1305 1310
   Gly Gly Gly Pro Val Pro Gly Val Val Val Leu Arg Cys Pro Asp Ala
   1315 1320 1325
   Gly Ala Gly Gly Gly Gly Gly Gly Gly Val Gly Glu Val Val Gly Gly
   1330 1335 1340
   Val Leu Gly Val Val Gln Gly Trp Leu Gly Leu Glu Arg Phe Ala Gly
   1345 1350 1355 1360
   Ser Arg Leu Val Val Val Thr Arg Gly Ala Val Val Ala Gly Gln Glu
   1365 1370 1375
   Asp Gly Pro Val Asp Val Val Gly Ala Ala Val Trp Gly Leu Val Arg
   1380 1385 1390
   Ser Ala Gln Ala Glu His Pro Asp Arg Phe Val Leu Leu Asp Leu Asp
   1395 1400 1405
   Thr Asp Thr Asp Thr Gly Thr Asp Leu Asp Thr Gly Ala Gly Ala Gly
   1410 1415 1420
   Ala Gly Ala Gly Trp Gly Val Asp Gly Gly His Val Ala Ala Val Val
   1425 1430 1435 1440
   Ala Cys Gly Glu Pro Gln Leu Ala Val Arg Gly Glu Arg Val Leu Ala
   1445 1450 1455
   Ala Arg Leu Thr Arg Leu Glu Ser Ser Val Asp Val Pro Ala Gln Arg
   1460 1465 1470
   Ser Gly Asp Val Ala Gly Arg Glu Val Leu Pro Trp Leu Ser Gly Gly
   1475 1480 1485
   Ser Val Leu Val Thr Gly Gly Thr Gly Val Leu Gly Ala Ala Val Ala
   1490 1495 1500
   Arg His Leu Ala Gly Val Cys Gly Val Arg Asp Leu Leu Leu Val Ser
   1505 1510 1515 1520
   Arg Arg Gly Pro Asp Ala Pro Gly Ala Glu Gly Leu Arg Ala Glu Leu
   1525 1530 1535
   Ala Ala Leu Gly Ala Glu Val Arg Ile Val Ala Cys Asp Val Gly Glu
   1540 1545 1550
   Arg Arg Glu Val Val Arg Leu Leu Glu Gly Val Pro Ala Gly Cys Pro
   1555 1560 1565
   Leu Thr Gly Val Val His Ala Ala Gly Val Leu Asp Asp Ala Thr Ile
   1570 1575 1580
   Ala Ser Leu Thr Pro Glu Arg Leu Gly Thr Val Phe Ala Ala Lys Val
   1585 1590 1595 1600
   Asp Ala Ala Leu Leu Leu Asp Glu Leu Thr Arg Gly Met Glu Leu Ser
   1605 1610 1615
   Ala Phe Val Leu Phe Ser Ser Ala Ala Gly Ile Leu Gly Ser Ala Gly
   1620 1625 1630
   Gln Gly Asn Tyr Ala Ala Ala Asn Ala Ala Leu Asp Ala Leu Ala Tyr
   1635 1640 1645
   Arg Arg Arg Ala Ala Gly Leu Pro Gly Val Ser Leu Ala Trp Gly Leu
   1650 1655 1660
   Trp Glu Glu Ala Ser Gly Met Thr Gly His Leu Ala Gly Thr Asp His
   1665 1670 1675 1680
   Arg Arg Ile Ile Arg Ser Gly Leu His Pro Met Ser Thr Pro Asp Ala
   1685 1690 1695
   Leu Ala Leu Phe Asp Ala Ala Leu Ala Leu Asp Arg Pro Val Leu Leu
   1700 1705 1710
   Pro Ala Asp Leu Arg Pro Ala Pro Pro Leu Pro Pro Leu Leu Gln Asp
   1715 1720 1725
   Leu Leu Pro Ala Thr Arg Arg Arg Thr Thr Arg Thr Thr Thr Thr Gly
   1730 1735 1740
   Gly Ala Asp Asn Gly Ala Gln Leu His Ala Arg Leu Ala Gly Gln Thr
   1745 1750 1755 1760
   His Glu Gln Gln His Thr Thr Leu Leu Ala Leu Val Arg Ser His Ile
   1765 1770 1775
   Ala Thr Val Leu Gly His Thr Thr Pro Asp Thr Ile Pro Pro Asp Arg
   1780 1785 1790
   Ala Phe Arg Asp Leu Gly Phe Asp Ser Leu Thr Ala Val Glu Leu Arg
   1795 1800 1805
   Asn Arg Leu Ser Arg Thr Thr Gly Leu Arg Leu Pro Thr Thr Leu Ala
   1810 1815 1820
   Phe Asp His Pro Asn Pro Thr Thr Leu Thr His His Leu His Thr Gln
   1825 1830 1835 1840
   Leu Leu Gly Ser Asp Ser Thr Ala Ser Ile Pro Ala Pro Arg Ala Ala
   1845 1850 1855
   Ala Val Pro Ala Asp Gln Asp Glu Pro Val Ala Ile Ile Gly Met Ala
   1860 1865 1870
   Cys Arg Tyr Pro Gly Gly Val Thr Ser Ala Glu Glu Leu Trp Glu Leu
   1875 1880 1885
   Leu Ala Ser Gly Arg Asp Thr Val Gly Glu Phe Pro Thr Asp Arg Gly
   1890 1895 1900
   Trp Asp Leu Glu Ala Leu Phe Asp Pro Glu Pro Gly Arg Pro Gly Thr
   1905 1910 1915 1920
   Ser Tyr Thr Arg Cys Gly Ser Phe Leu Tyr Asp Ala Gly Glu Phe Asp
   1925 1930 1935
   Ala Gly Phe Phe Gly Ile Ser Pro Arg Glu Ala Leu Ala Met Asp Pro
   1940 1945 1950
   Gln Gln Arg Leu Leu Leu Glu Ala Ser Trp Glu Ala Met Glu Gln Ala
   1955 1960 1965
   Gly Ile Asp Pro Thr Thr Val Arg Gly Ser Gln Thr Gly Val Phe Ala
   1970 1975 1980
   Gly Leu Ile Pro Gln Ala Tyr Gly Pro Arg Leu His Glu Asn Ala Ala
   1985 1990 1995 2000
   Ala Asp Thr Glu Gly Tyr Val Leu Thr Gly Thr Ser Gly Ser Val Ala
   2005 2010 2015
   Ser Gly Arg Ile Ser Tyr Thr Phe Gly Phe Glu Gly Pro Ala Val Ser
   2020 2025 2030
   Val Asp Thr Ala Cys Ser Ser Ser Leu Val Ala Leu His Leu Ala Cys
   2035 2040 2045
   Gln Ala Leu Arg Ala Gly Glu Cys Ser Met Ala Leu Ala Gly Gly Val
   2050 2055 2060
   Thr Val Met Ser Ser Pro Gly Ala Phe Val Glu Phe Ser Arg Gln Arg
   2065 2070 2075 2080
   Gly Leu Ala Ala Asp Gly His Cys Lys Ala Phe Ser Ala Ala Ala Asp
   2085 2090 2095
   Gly Thr Gly Trp Gly Glu Gly Val Gly Met Leu Leu Val Glu Arg Leu
   2100 2105 2110
   Ser Asp Ala Arg Arg Asn Gly His Arg Val Leu Ala Val Val Arg Gly
   2115 2120 2125
   Ser Ala Val Asn Gln Asp Gly Ala Ser Asn Gly Leu Thr Ala Pro Asn
   2130 2135 2140
   Gly Pro Ser Gln Gln Arg Val Ile Arg Gln Ala Leu Ala Asn Ala Gly
   2145 2150 2155 2160
   Leu Ser Ala Gly Asp Val Asp Ala Val Glu Ala His Gly Thr Gly Thr
   2165 2170 2175
   Thr Leu Gly Asp Pro Ile Glu Ala Gln Ala Leu Leu Ala Thr Tyr Gly
   2180 2185 2190
   Gln Asp Arg Ala Gly Glu Gly Pro Leu Trp Leu Gly Ser Val Lys Ser
   2195 2200 2205
   Asn Val Gly His Thr Gln Ala Ala Ala Gly Val Ala Gly Val Ile Lys
   2210 2215 2220
   Met Val Met Ala Leu Arg Asn Gly Leu Leu Pro Arg Thr Leu His Val
   2225 2230 2235 2240
   Asp Glu Pro Ser Pro His Val Asp Trp Ser Ala Gly Ala Val Gln Leu
   2245 2250 2255
   Leu Thr Glu Thr Val Pro Trp Pro Gly Gly Glu Gly Arg Leu Arg Arg
   2260 2265 2270
   Ala Gly Val Ser Ser Phe Gly Val Ser Gly Thr Asn Ala His Val Ile
   2275 2280 2285
   Leu Glu Glu Ala Pro Ala His Asn Ile Pro Ser Asp Thr Pro Ala Asp
   2290 2295 2300
   Asp Val Pro Gly Gly Pro Pro Ala Gly Glu Asp Ala Gly Ser Gly Glu
   2305 2310 2315 2320
   Glu Ala Ala Ala Gly Ser Pro Gly Val Trp Pro Trp Leu Val Ser Ala
   2325 2330 2335
   Lys Ser Gln Pro Ala Leu Arg Ala Gln Ala Gln Ala Leu His Ala His
   2340 2345 2350
   Leu Thr Asp His Pro Gly Leu Asp Leu Ala Asp Val Gly Tyr Thr Leu
   2355 2360 2365
   Ala His Ala Arg Ala Val Phe Asp His Arg Ala Thr Leu Ile Ala Ala
   2370 2375 2380
   Asp Arg Asp Thr Phe Leu Gln Ala Leu Gln Ala Leu Ala Ala Gly Glu
   2385 2390 2395 2400
   Pro His Pro Ala Val Ile His Ser Ser Ala Pro Gly Gly Thr Gly Thr
   2405 2410 2415
   Gly Glu Ala Ala Gly Lys Thr Ala Phe Ile Cys Ser Gly Gln Gly Thr
   2420 2425 2430
   Gln Arg Pro Gly Met Ala His Gly Leu Tyr His Thr His Pro Val Phe
   2435 2440 2445
   Ala Ala Ala Leu Asn Asp Ile Cys Thr His Leu Asp Pro His Leu Asp
   2450 2455 2460
   His Pro Leu Leu Pro Leu Leu Thr Gln Asp Pro Asn Thr Gln Asp Thr
   2465 2470 2475 2480
   Thr Thr Leu Glu Glu Ala Ala Ala Leu Leu Gln Gln Thr Pro Tyr Ala
   2485 2490 2495
   Gln Pro Ala Leu Phe Ala Phe Gln Val Ala Leu His Arg Leu Leu Thr
   2500 2505 2510
   Asp Gly Tyr His Ile Thr Pro His Tyr Tyr Ala Gly His Ser Leu Gly
   2515 2520 2525
   Glu Ile Thr Ala Ala His Leu Ala Gly Ile Leu Thr Leu Thr Asp Ala
   2530 2535 2540
   Thr Thr Leu Ile Thr Gln Arg Ala Thr Leu Met Gln Thr Met Pro Pro
   2545 2550 2555 2560
   Gly Thr Met Thr Thr Leu His Thr Thr Pro His His Ile Thr His His
   2565 2570 2575
   Ile Thr Ala His Glu Asn Asp Leu Ala Ile Ala Ala Ile Asn Thr Pro
   2580 2585 2590
   Thr Ser Leu Val Ile Ser Gly Thr Pro His Thr Val Gln His Ile Thr
   2595 2600 2605
   Thr Leu Cys Gln Gln Gln Gly Ile Lys Thr Lys Thr Leu Pro Thr Asn
   2610 2615 2620
   His Ala Phe His Ser Pro His Thr Asn Pro Ile Leu Asn Gln Leu His
   2625 2630 2635 2640
   Gln His Thr Gln Thr Leu Thr Tyr His Pro Pro His Thr Pro Leu Ile
   2645 2650 2655
   Thr Ala Asn Thr Pro Pro Asp Gln Leu Leu Thr Pro His Tyr Trp Thr
   2660 2665 2670
   Gln Gln Ala Arg Asn Thr Val Asp Ile Ala Thr Thr Thr Gln Thr Leu
   2675 2680 2685
   His Gln His Gly Val Thr Thr Tyr Ile Glu Leu Gly Pro Asp Asn Thr
   2690 2695 2700
   Leu Thr Thr Leu Thr His His Asn Leu Pro Asn Thr Pro Thr Thr Thr
   2705 2710 2715 2720
   Leu Thr Leu Thr His Pro His His His Pro Gln Thr His Leu Leu Thr
   2725 2730 2735
   Asn Leu Ala Lys Thr Thr Thr Thr Trp His Pro His His Tyr Thr His
   2740 2745 2750
   His His Asn Gln Pro His Thr His Thr His Leu Asp Leu Pro Thr Tyr
   2755 2760 2765
   Pro Phe Gln His His His Tyr Trp Leu Glu Ser Thr Gln Pro Gly Ala
   2770 2775 2780
   Gly Asn Val Ser Ala Ala Gly Leu Asp Pro Thr Glu His Pro Leu Leu
   2785 2790 2795 2800
   Gly Ala Thr Leu Glu Leu Ala Glu Gly Asp Gly Cys Leu Leu Thr Gly
   2805 2810 2815
   Arg Leu Ser Leu Arg Thr His Pro Trp Leu Ala Gly His Ala Val Gly
   2820 2825 2830
   Gly Val Val Leu Leu Pro Gly Thr Ala Phe Ala Glu Leu Ala Leu His
   2835 2840 2845
   Ala Gly Glu Ser Val Gly Cys Asp His Val Asp Glu Leu Thr Leu His
   2850 2855 2860
   Thr Pro Leu Val Ile Pro Glu Val Gly Asp Val Thr Leu Gln Val Ala
   2865 2870 2875 2880
   Ile Ala Ala Pro Asp Glu Ser Gly Arg Arg Met Met Thr Ile His Ser
   2885 2890 2895
   Arg Gly Glu Gly Gly Ser Gly Gly Ala Asp Ala Ser Ala Ser Ala Trp
   2900 2905 2910
   Thr Arg His Ala Ala Gly Val Leu Ser Pro Ala Lys Asp Asp Asp Thr
   2915 2920 2925
   Ala Ser Tyr Glu Leu Leu Ala Gly Pro Trp Pro Pro Val Gly Ala Thr
   2930 2935 2940
   Pro Val Asp Leu Asn Thr Ala Tyr Asp Gln Met Ala Asp Ala Gly Phe
   2945 2950 2955 2960
   Ala Tyr Gly Leu Ala Phe Gln Gly Leu Arg Ala Ala Trp Arg Tyr Gly
   2965 2970 2975
   Asp Asp Ile Leu Val Glu Ala Arg Leu Pro Glu Glu Val Ser Gly Asp
   2980 2985 2990
   Ala Ala Ala Tyr Gly Leu His Pro Ala Leu Leu Asp Ala Ala Leu Gln
   2995 3000 3005
   Gly Thr Gly Leu Leu Ser Val Ala Gly Pro Gly Thr Pro Val Val Pro
   3010 3015 3020
   His Val Trp Asn Gly Leu Arg Phe Arg Thr His Gly Ala Val Ser Val
   3025 3030 3035 3040
   Arg Ala Cys Leu Ser Thr Leu Gly Ala Thr Gly Ala Ala Val Cys Val
   3045 3050 3055
   Arg Ile Thr Asp Asp Thr Gly Val Pro Val Ala Ser Val Asp Arg Leu
   3060 3065 3070
   Glu Leu Arg Pro Val Asp Met Gly Gln Leu Arg Ala Val Ser Val Ser
   3075 3080 3085
   Ala Gly Arg Arg Gly Ser Leu Tyr Ala Val Gln Trp Ala Glu Val Gly
   3090 3095 3100
   Pro Val Pro Val Cys Gly Gln Ala Trp Ala Trp His Glu Asp Val Gly
   3105 3110 3115 3120
   Glu Ser Gly Gly Gly Pro Val Pro Gly Val Val Val Leu Arg Cys Pro
   3125 3130 3135
   Asp Ala Gly Ala Asp Gly Gly Gly Gly Gly Gly Val Gly Glu Val Val
   3140 3145 3150
   Gly Gly Val Leu Gly Val Val Gln Gly Trp Leu Gly Leu Glu Arg Phe
   3155 3160 3165
   Ala Gly Ser Arg Leu Val Val Val Thr Arg Gly Ala Val Val Ala Gly
   3170 3175 3180
   Pro Glu Asp Gly Pro Val Asp Val Val Gly Ala Ala Val Trp Gly Leu
   3185 3190 3195 3200
   Val Arg Ser Ala Gln Ala Glu His Pro Asp Arg Phe Val Leu Leu Asp
   3205 3210 3215
   Leu Asp Thr Asp Leu Asp Ser Gly Ala Asp Ala Asp Ala Gly Asn Glu
   3220 3225 3230
   Ala Gly Met Gly Ser Gly Leu Asp Gly Gly Arg Val Ala Ala Val Val
   3235 3240 3245
   Ala Cys Gly Glu Pro Gln Leu Ala Val Arg Gly Glu Arg Val Leu Ala
   3250 3255 3260
   Ala Arg Leu Thr Arg Leu Glu Ser Pro Val Asp Val Ser Gly Arg Glu
   3265 3270 3275 3280
   Val Leu Pro Trp Leu Ser Gly Gly Ser Val Leu Val Thr Gly Gly Thr
   3285 3290 3295
   Gly Val Leu Gly Ala Ala Val Ala Arg His Leu Ala Gly Val Cys Gly
   3300 3305 3310
   Val Arg Asp Leu Leu Leu Val Ser Arg Arg Gly Pro Asp Ala Pro Gly
   3315 3320 3325
   Ala Glu Gly Leu Arg Ala Glu Leu Ala Ala Leu Gly Ala Glu Val Arg
   3330 3335 3340
   Ile Val Ala Cys Asp Val Gly Glu Arg Arg Glu Val Val Arg Leu Leu
   3345 3350 3355 3360
   Glu Gly Val Pro Ala Gly Cys Pro Leu Thr Gly Val Val His Ala Ala
   3365 3370 3375
   Gly Val Leu Asp Asp Ala Thr Ile Ala Ser Leu Thr Pro Glu Arg Leu
   3380 3385 3390
   Gly Thr Val Phe Ala Ala Lys Val Asp Ala Ala Leu Leu Leu Asp Glu
   3395 3400 3405
   Leu Thr Arg Gly Met Glu Leu Ser Ala Phe Val Leu Phe Ser Ser Ala
   3410 3415 3420
   Ala Gly Ile Leu Gly Ser Ala Gly Gln Gly Asn Tyr Ala Ala Ala Asn
   3425 3430 3435 3440
   Ala Ala Leu Asp Ala Leu Ala Tyr Arg Arg Arg Ala Ala Gly Leu Pro
   3445 3450 3455
   Gly Val Ser Leu Ala Trp Gly Leu Trp Glu Glu Ala Ser Gly Met Thr
   3460 3465 3470
   Gly His Leu Ala Gly Thr Asp His Arg Arg Ile Ile Arg Ser Gly Leu
   3475 3480 3485
   His Pro Met Ser Thr Pro Asp Ala Leu Ala Leu Phe Asp Ala Ala Leu
   3490 3495 3500
   Ala Leu Asp Arg Pro Val Leu Leu Pro Ala Asp Leu Arg Pro Ala Pro
   3505 3510 3515 3520
   Pro Leu Pro Pro Leu Leu Gln Asp Leu Leu Pro Ala Thr Arg Arg Arg
   3525 3530 3535
   Thr Thr Arg Thr Thr Thr Thr Gly Gly Ala Asp Asn Gly Ala Gln Leu
   3540 3545 3550
   His Ala Arg Leu Ala Gly Gln Thr His Glu Gln Gln His Thr Thr Leu
   3555 3560 3565
   Leu Ala Leu Val Arg Ser His Ile Ala Thr Val Leu Gly His Asn Ala
   3570 3575 3580
   Pro Glu Met Ile Pro Val Asp Ser Ala Phe Arg Asp Leu Gly Phe Asp
   3585 3590 3595 3600
   Ser Leu Thr Ala Val Glu Leu Arg Asn Arg Leu Gly Glu Ala Thr Gly
   3605 3610 3615
   Leu Arg Leu Pro Thr Ser Leu Val Phe Asp Gln Pro Asn Ala Ala Thr
   3620 3625 3630
   Leu Ala Arg His Leu Arg Arg Glu Leu Met Gly Asp Asp Ala Glu Gly
   3635 3640 3645
   Glu Thr Pro Ser Gln Val Ala Leu His Gln Val Ala Ala Asp Glu Pro
   3650 3655 3660
   Ile Ala Ile Val Gly Met Ala Cys Arg Phe Pro Gly Gly Val Cys Ser
   3665 3670 3675 3680
   Pro Glu Glu Leu Trp Glu Leu Val Ala Ser Gly Gly Asp Ala Ile Gly
   3685 3690 3695
   Glu Phe Pro Ala Gly Arg Gly Trp Asp Leu Glu Gly Leu Phe Asp Ser
   3700 3705 3710
   Asp Pro Asp Arg Ser Gly Thr Ser Tyr Ala Arg Tyr Gly Gly Phe Leu
   3715 3720 3725
   Tyr Glu Ala Gly Glu Phe Asp Ala Asp Phe Phe Gly Ile Ser Pro Arg
   3730 3735 3740
   Glu Ala Leu Ala Met Asp Pro Gln Gln Arg Leu Leu Leu Glu Thr Ser
   3745 3750 3755 3760
   Trp Glu Ala Phe Glu Arg Ala Gly Ile Asp Pro Leu Ser Met Arg Gly
   3765 3770 3775
   Ser Arg Thr Gly Val Phe Ala Gly Val Met Tyr His Asp Tyr Ala Ala
   3780 3785 3790
   Arg Leu His His Val Pro Glu Gly Phe Glu Gly Leu Ile Ala Asn Gly
   3795 3800 3805
   Ser Ala Gly Ser Val Ala Thr Gly Arg Val Ala Tyr Ser Phe Gly Leu
   3810 3815 3820
   Glu Gly Pro Ala Val Thr Val Asp Thr Ala Cys Ser Ser Ser Leu Val
   3825 3830 3835 3840
   Ala Leu His Trp Ala Ala Gln Ala Leu Arg Ala Gly Glu Cys Ser Met
   3845 3850 3855
   Ala Leu Ala Gly Gly Val Thr Val Met Ser Ser Pro Gly Thr Phe Val
   3860 3865 3870
   Glu Phe Ser Arg Gln Arg Gly Leu Ala Ala Asp Gly Arg Cys Lys Ala
   3875 3880 3885
   Tyr Ser Ala Ala Ala Asp Gly Thr Gly Trp Ala Glu Gly Val Gly Met
   3890 3895 3900
   Leu Leu Val Glu Arg Leu Ser Asp Ala Arg Arg Asn Gly His Arg Val
   3905 3910 3915 3920
   Leu Ala Val Val Arg Gly Ser Ala Val Asn Gln Asp Gly Ala Ser Asn
   3925 3930 3935
   Gly Leu Thr Ala Pro Asn Gly Pro Ser Gln Gln Arg Val Ile Arg Gln
   3940 3945 3950
   Ala Leu Ala Asn Ala Gly Leu Thr Pro Ala Asp Val Asp Ala Val Glu
   3955 3960 3965
   Gly His Gly Thr Gly Thr Thr Leu Gly Asp Pro Ile Glu Ala Gln Ala
   3970 3975 3980
   Leu Leu Ala Ala Tyr Gly Gln His Arg Pro His His Arg Pro Leu Trp
   3985 3990 3995 4000
   Leu Gly Ser Leu Lys Ser Asn Ile Gly His Ala Gln Ala Ala Ala Gly
   4005 4010 4015
   Val Gly Gly Val Ile Lys Met Val Met Ala Leu Arg Asn Gly Leu Leu
   4020 4025 4030
   Pro Gln Thr Leu His Val Asp Glu Pro Thr Pro Gln Val Asp Trp Ser
   4035 4040 4045
   Thr Gly Ala Val Gln Leu Leu Thr Gln Pro Val Pro Trp Pro Ala Asp
   4050 4055 4060
   Pro Ala Gly Arg Pro Arg His Ala Gly Val Ser Ser Phe Gly Val Ser
   4065 4070 4075 4080
   Gly Thr Asn Ala His Val Ile Leu Glu Glu Ala Pro Ala Ala Ala Gly
   4085 4090 4095
   Gly Ala Ala Gly Gly Gly Val Ser Val Gly Ala Pro Asn Pro Ala Leu
   4100 4105 4110
   Pro Val Ala Glu Ser Glu Pro Val Pro Val Pro Val Pro Val Ser Ala
   4115 4120 4125
   Arg Ser Glu Ala Gly Leu Arg Ala Gln Ala Gln Ala Leu Arg Gln Tyr
   4130 4135 4140
   Val Ala Ala Arg Pro Asp Met Ser Pro Ala Asp Ile Gly Ala Gly Leu
   4145 4150 4155 4160
   Ala Arg Gly Arg Ala Val Leu Glu His Arg Ala Val Ile Leu Ala Ala
   4165 4170 4175
   Asp Arg Glu Glu Leu Ala Gln Ala Leu Thr Ala Leu Ala Ala Gly Glu
   4180 4185 4190
   Pro His Pro His Ile Thr Thr Gly His Thr Arg Gly Ser Asp Arg Gly
   4195 4200 4205
   Gly Val Val Phe Val Phe Pro Gly Gln Gly Gly Gln Trp Ala Gly Met
   4210 4215 4220
   Gly Leu Thr Leu Leu Thr Ser Ser Pro Val Phe Ala Glu His Ile Asp
   4225 4230 4235 4240
   Ala Cys Glu Lys Ala Leu Thr Pro Trp Val Pro Trp Ser Leu Thr Asp
   4245 4250 4255
   Ile Leu His Arg Asp Pro Asp Asp Pro Ala Trp Gln Gln Ala Asp Val
   4260 4265 4270
   Val Gln Pro Val Leu Phe Ser Ile Met Val Ser Leu Ala Ala Leu Trp
   4275 4280 4285
   Arg Ser Tyr Gly Ile Glu Pro Asp Ala Val Leu Gly His Ser Gln Gly
   4290 4295 4300
   Glu Ile Ala Ala Ala His Ile Cys Gly Ala Leu Ser Leu Lys Asp Ala
   4305 4310 4315 4320
   Ala Lys Thr Val Ala Leu Arg Ser Gln Ala Leu Ala Ala Val Arg Gly
   4325 4330 4335
   Arg Gly Ala Met Val Ser Leu Pro Leu Pro Ala Gln Asp Val Gln Gln
   4340 4345 4350
   Leu Ile Ser Glu Arg Trp Glu Gly Gln Leu Trp Val Ala Ala Leu Asn
   4355 4360 4365
   Gly Pro His Ser Thr Thr Val Ser Gly Asp Thr Thr Ala Val Glu Glu
   4370 4375 4380
   Leu Leu Thr His Cys Ala Asp Thr Gly Leu Arg Ala Lys Arg Ile Pro
   4385 4390 4395 4400
   Val Asp Tyr Ala Ser His Cys Pro His Val Gln Pro Leu His Asp Glu
   4405 4410 4415
   Leu Leu His Leu Leu Gly Asp Ile Thr Pro Gln Pro Ser Thr Met Pro
   4420 4425 4430
   Phe Phe Ser Thr Val Val Gly His Leu Val Trp Tyr Thr Thr Thr Leu
   4435 4440 4445
   Asp Ala Ala Tyr Trp Tyr Arg Asn Leu His Gln Pro Val Arg Phe Ser
   4450 4455 4460
   His Ala Ile Gln Thr Leu Thr Asp Asp Gly His Arg Pro Phe Ile Glu
   4465 4470 4475 4480
   Ile Ser Pro His Pro Thr Leu Val Pro Ala Ile Glu Asp Thr Thr Glu
   4485 4490 4495
   Asn Thr Thr Glu Asn Ile Thr Ala Thr Gly Ser Leu Arg Arg Gly Asp
   4500 4505 4510
   Asn Asp Thr His Arg Phe Leu Thr Ala Leu Ala His Thr His Thr Thr
   4515 4520 4525
   Gly Ile Arg Thr Pro Thr Thr Trp His His His Tyr Thr Gln Thr His
   4530 4535 4540
   Pro His Pro His Asn His His Leu Asp Leu Pro Thr Tyr Pro Phe Gln
   4545 4550 4555 4560
   His Gln His Tyr Trp Leu Gln Pro Pro Thr Thr Thr Thr Asp Leu Thr
   4565 4570 4575
   Thr Thr Gly Leu Thr Pro Thr His His Pro Leu Leu Thr Ala Thr Leu
   4580 4585 4590
   Thr Leu Ala Asn Asn Asn Thr Gln Leu Leu Thr Gly Arg Leu Ser Leu
   4595 4600 4605
   Arg Thr His Pro Trp Leu Thr Asp His Thr Val Val Gly Thr Thr Leu
   4610 4615 4620
   Val Pro Gly Thr Ala Leu Leu Glu Leu Ala Leu Gln Ala Thr Thr Thr
   4625 4630 4635 4640
   Asp His Leu Glu Glu Leu Ala Leu His Thr Pro Leu Val Ile Pro Arg
   4645 4650 4655
   Glu Gly Ala Val Asp Val Gln Val His Ile Asn Pro Pro Asp Asp Thr
   4660 4665 4670
   Asp Thr Arg Ser Leu Thr Ile Tyr Ser Arg Ser Glu Asn Ala Pro Ala
   4675 4680 4685
   Ala Ala Pro Trp Arg His His Ala Thr Ala Val Leu Gly Thr Lys Thr
   4690 4695 4700
   Ser Arg Ile Glu Thr Gly Arg Ser His Asp Asp Leu Ser Met Trp Pro
   4705 4710 4715 4720
   Pro Ala Gly Ala Val Arg Cys Ala Asp Glu Glu Leu Ala Ala Leu Tyr
   4725 4730 4735
   Gly Asp Tyr Glu Ala Asn Gly Phe Val Tyr Gly Pro Ala Phe Arg Gly
   4740 4745 4750
   Leu Thr Ala Ala Trp Arg Leu Gly Asp Glu Val Phe Ala Glu Val Arg
   4755 4760 4765
   Leu Pro Glu Gln Val His Gly Glu Ala Ser Ala Tyr Asn Leu His Pro
   4770 4775 4780
   Ala Leu Leu Asp Ala Ala Leu His Ala Ala Ala Phe Ala Pro Ser Gly
   4785 4790 4795 4800
   Ser Leu Pro Gln Gly Ser Val Pro Phe Ser Phe Thr Gly Val Thr Leu
   4805 4810 4815
   His Ala Ala Asn Ala Ser Ser Leu Arg Val Arg Leu Ser Pro Ala Asp
   4820 4825 4830
   Pro Asn Ser Gly His Ala Ala Val Ser Val Leu Val Thr Asp Asp Thr
   4835 4840 4845
   Gly Thr Pro Val Ala Ser Val Glu Ala Leu Ala Val Arg Pro Leu Ala
   4850 4855 4860
   Ala Asp Glu Leu Arg Ala Ala Glu Arg Ala Val Gln Arg Ala Glu Leu
   4865 4870 4875 4880
   Phe Asp Met Lys Trp Val Glu Val Pro Ser Asp Val Leu Val Ser Gly
   4885 4890 4895
   Gly Ala Ser Val Val Val Leu Asp Gly Ala Asp Asp Leu Val Gly Leu
   4900 4905 4910
   Ala Ala Glu Glu Asp Gly Val Pro Gly Val Val Val Leu Arg Cys Pro
   4915 4920 4925
   Asp Ala Gly Ala Asp Gly Gly Gly Gly Gly Gly Gly Val Gly Glu Val
   4930 4935 4940
   Val Gly Gly Val Leu Gly Val Val Gln Gly Trp Leu Gly Leu Glu Arg
   4945 4950 4955 4960
   Phe Ala Gly Ser Arg Leu Val Val Val Thr Arg Gly Ala Val Val Ala
   4965 4970 4975
   Gly Pro Glu Asp Gly Pro Val Asp Gly Pro Val Asp Val Val Gly Ala
   4980 4985 4990
   Ala Val Trp Gly Leu Val Arg Ser Ala Gln Ala Glu His Pro Asp Arg
   4995 5000 5005
   Phe Val Leu Leu Asp Leu Asp Thr Asp Leu Asp Ser Gly Ala Asp Arg
   5010 5015 5020
   Asp Ala Gly Asn Glu Ala Gly Met Gly Ser Gly Leu Asp Gly Gly Arg
   5025 5030 5035 5040
   Val Ala Ala Val Val Ala Cys Gly Glu Pro Gln Leu Ala Val Arg Gly
   5045 5050 5055
   Glu Arg Val Leu Ala Ala Arg Leu Thr Arg Leu Glu Ser Pro Val Asp
   5060 5065 5070
   Val Ser Gly Arg Glu Val Leu Pro Trp Leu Ser Gly Gly Ser Val Leu
   5075 5080 5085
   Val Thr Gly Gly Thr Gly Val Leu Gly Ala Ala Val Ala Arg His Leu
   5090 5095 5100
   Ala Gly Val Cys Gly Val Arg Asp Leu Leu Leu Val Ser Arg Arg Gly
   5105 5110 5115 5120
   Pro Asp Ala Pro Gly Ala Glu Gly Leu Arg Ala Glu Leu Ala Ala Leu
   5125 5130 5135
   Gly Ala Glu Val Arg Ile Val Ala Cys Asp Val Gly Glu Arg Arg Glu
   5140 5145 5150
   Val Val Arg Leu Leu Glu Gly Val Pro Ala Gly Cys Pro Leu Thr Gly
   5155 5160 5165
   Val Val His Ala Ala Gly Val Leu Asp Asp Ala Thr Ile Ala Ser Leu
   5170 5175 5180
   Thr Pro Glu Arg Leu Gly Thr Val Phe Ala Ala Lys Val Asp Ala Ala
   5185 5190 5195 5200
   Leu Leu Leu Asp Glu Leu Thr Arg Gly Met Glu Leu Ser Ala Phe Val
   5205 5210 5215
   Leu Phe Ser Ser Ala Ala Gly Ile Leu Gly Ser Ala Gly Gln Gly Asn
   5220 5225 5230
   Tyr Ala Ala Ala Asn Ala Ala Leu Asp Ala Leu Ala Tyr Arg Arg Arg
   5235 5240 5245
   Ala Ala Gly Leu Pro Gly Val Ser Leu Ala Trp Gly Leu Trp Glu Glu
   5250 5255 5260
   Ala Ser Gly Met Thr Gly His Leu Ala Gly Thr Asp His Arg Arg Ile
   5265 5270 5275 5280
   Ile Arg Ser Gly Leu His Pro Met Ser Thr Pro Asp Ala Leu Ala Leu
   5285 5290 5295
   Phe Asp Ala Ala Leu Ala Leu Asp Arg Pro Val Leu Leu Pro Ala Asp
   5300 5305 5310
   Leu Arg Pro Ala Pro Pro Leu Pro Pro Leu Leu Gln Asp Leu Leu Pro
   5315 5320 5325
   Ala Thr Arg Arg Arg Thr Thr Arg Thr Thr Thr Thr Gly Gly Ala Asp
   5330 5335 5340
   Asn Gly Ala Gln Leu His Gly Arg Leu Ala Gly Gln Thr His Glu Gln
   5345 5350 5355 5360
   Gln His Thr Thr Leu Leu Ala Leu Val Arg Ser His Ile Ala Thr Val
   5365 5370 5375
   Leu Gly His Thr Thr Pro Asp Thr Ile Pro Pro Asp Arg Ala Phe Arg
   5380 5385 5390
   Asp Leu Gly Phe Asp Ser Leu Thr Ala Val Glu Leu Arg Asn Arg Leu
   5395 5400 5405
   Ser His Thr Thr Gly Leu Arg Leu Pro Thr Thr Leu Ala Phe Asp His
   5410 5415 5420
   Pro Asn Pro Thr Thr Leu Thr His His Leu His Thr Gln Leu Val Ser
   5425 5430 5435 5440
   Lys Gly Leu Thr Ala Ala Ala Glu Pro Asp Ala Ala Thr Thr Pro Pro
   5445 5450 5455
   Gly Leu Pro Ser Leu Leu Ser Glu Leu Glu Arg Leu Glu Ala Val Val
   5460 5465 5470
   Leu Ser Ser Thr Thr Ser Ser Ala Ala Pro Leu Asp Asp Gly Ala Arg
   5475 5480 5485
   Thr Arg Leu Ala Ser Arg Leu His Ser Leu Ala Gln Lys Leu Asn Gly
   5490 5495 5500
   Asp Asp Thr Ala Pro Asp Leu Ala Glu Thr Ser Asp Glu Glu Met Phe
   5505 5510 5515 5520
   Ala Leu Ile Asp Arg Glu Val Gly Phe Glu Ser Gln
   5525 5530
<210> 8
   <211> 3972
   <212> PRT
   <213> Artificial Sequence
   <223> Description of Artificial Sequence; Protein one amino acid is sustituted
<400> 8
   Val Gln Arg Met Asp Gly Gly Glu Glu Pro Arg Pro Ala Ala Gly Glu
   1               5                   10                  15
   Val Leu Gly Val Ala Asp Glu Ala Asp Gly Gly Val Val Phe Val Phe
               20                  25                  30
   Pro Gly Gln Gly Pro Gln Trp Pro Gly Met Gly Arg Glu Leu Leu Asp
           35                  40                  45
   Ala Ser Asp Val Phe Arg Glu Ser Val Arg Ala Cys Glu Ala Ala Phe
       50                  55                  60
   Ala Pro Tyr Val Asp Trp Ser Val Glu Gln Val Leu Arg Asp Ser Pro
   65                  70                  75                  80
   Asp Ala Pro Gly Leu Asp Arg Val Asp Val Val Gln Pro Thr Leu Phe
                   85                  90                  95
   Ala Val Met Ile Ser Leu Ala Ala Leu Trp Arg Ser Gln Gly Val Glu
               100                 105                 110
   Pro Cys Ala Val Leu Gly His Ser Leu Gly Glu Ile Ala Ala Ala His
           115                 120                 125
   Val Ser Gly Gly Leu Ser Leu Ala Asp Ala Ala Arg Val Val Thr Leu
       130                 135                 140
   Trp Ser Gln Ala Gln Thr Thr Leu Ala Gly Thr Gly Ala Leu Val Ser
   145                 150                 155                 160
   Val Ala Ala Thr Pro Asp Glu Leu Leu Pro Arg Ile Ala Pro Trp Thr
                   165                 170                 175
   Glu Asp Asn Pro Ala Arg Leu Ala Val Ala Ala Val Asn Gly Pro Arg
               180                 185                 190
   Ser Thr Val Val Ser Gly Ala Arg Glu Ala Val Ala Asp Leu Val Ala
           195                 200                 205
   Asp Leu Thr Ala Ala Gln Val Arg Thr Arg Met Ile Pro Val Asp Val
       210                 215                 220
   Pro Ala His Ser Pro Leu Met Tyr Ala Ile Glu Glu Arg Val Val Ser
   225                 230                 235                 240
   Gly Leu Leu Pro Ile Thr Pro Arg Pro Ser Arg Ile Pro Phe His Ser
                   245                 250                 255
   Ser Val Thr Gly Gly Arg Leu Asp Thr Arg Glu Leu Asp Ala Ala Tyr
               260                 265                 270
   Trp Tyr Arg Asn Met Ser Ser Thr Val Arg Phe Glu Pro Ala Ala Arg
           275                 280                 285
   Leu Leu Leu Gln Gln Gly Pro Lys Thr Phe Val Glu Met Ser Pro His
       290                 295                 300
   Pro Val Leu Thr Met Gly Leu Gln Glu Leu Ala Pro Asp Leu Gly Asp
   305                 310                 315                 320
   Thr Thr Gly Thr Ala Asp Thr Val Ile Met Gly Thr Leu Arg Arg Gly
                   325                 330                 335
   Gln Gly Thr Leu Asp His Phe Leu Thr Ser Leu Ala Gln Leu Arg Gly
               340                 345                 350
   His Gly Glu Thr Ser Ala Thr Thr Val Leu Ser Ala Arg Leu Thr Ala
           355                 360                 365
   Leu Ser Pro Thr Gln Gln Gln Ser Leu Leu Leu Asp Leu Val Arg Ala
       370                 375                 380
   His Thr Met Ala Val Leu Asn Asp Asp Gly Asn Glu Arg Thr Ala Ser
   385 390 395 400
   Asp Ala Gly Pro Ser Ala Ser Phe Ala His Leu Gly Phe Asp Ser Val
   405 410 415
   Met Gly Val Glu Leu Arg Asn Arg Leu Ser Lys Ala Thr Gly Leu Arg
   420 425 430
   Leu Pro Val Thr Leu Ile Phe Asp His Thr Thr Pro Ala Ala Val Ala
   435 440 445
   Ala Arg Leu Arg Thr Ala Ala Leu Gly His Leu Asp Glu Asp Thr Ala
   450 455 460
   Pro Val Pro Asp Ser Pro Ser Gly His Gly Gly Thr Ala Ala Ala Asp
   465 470 475 480
   Asp Pro Ile Ala Ile Ile Gly Met Ala Cys Arg Phe Pro Gly Gly Val
   485 490 495
   Arg Ser Pro Lys Asp Leu Trp Glu Leu Ala Ala Ser Gly Gly Asp Ala
   500 505 510
   Ile Gly Pro Phe Pro Thr Asp Arg Gly Trp Pro Thr Glu Gln Arg His
   515 520 525
   Ala Gln Asp Pro Thr Gln Pro Gly Thr Phe Tyr Pro Gln Gly Gly Gly
   530 535 540
   Phe Leu His Asp Ala Ala His Phe Asp Ala Gly Phe Phe Gly Ile Ser
   545 550 555 560
   Pro Arg Glu Ala Leu Ala Met Asp Pro Gln Gln Arg Leu Leu Leu Glu
   565 570 575
   Thr Ser Trp Glu Ala Phe Glu Arg Ala Gly Ile Asp Pro Leu Ser Val
   580 585 590
   Arg Gly Ser Arg Thr Gly Val Phe Ala Gly Ala Leu Ser Phe Asp Tyr
   595 600 605
   Gly Pro Arg Met Asp Thr Ala Ser Ser Glu Gly Ala Ala Asp Val Glu
   610 615 620
   Gly His Ile Leu Thr Gly Thr Thr Gly Ser Val Leu Ser Gly Arg Ile
   625 630 635 640
   Ala Tyr Ser Phe Gly Leu Glu Gly Pro Ala Ile Thr Val Asp Thr Gly
   645 650 655
   Gly Ser Ala Ser Leu Val Thr Leu His Leu Ala Cys Gln Ser Leu Arg
   660 665 670
   Ser Gly Glu Cys Thr Leu Ala Leu Ala Gly Gly Val Ser Val Met Ser
   675 680 685
   Thr Leu Gly Met Phe Ile Glu Phe Ser Arg Gln Arg Gly Leu Ser Val
   690 695 700
   Asp Gly Arg Cys Lys Ala Tyr Ser Ala Ala Ala Asp Gly Thr Gly Trp
   705 710 715 720
   Gly Glu Gly Val Gly Met Leu Leu Val Glu Arg Leu Ser Asp Ala Val
   725 730 735
   Arg Leu Gly His Arg Val Leu Ala Val Val Arg Gly Ser Ala Val Asn
   740 745 750
   Gln Asp Gly Ala Ser Asn Gly Leu Thr Ala Pro Asn Gly Pro Ala Gln
   755 760 765
   Glu Arg Val Ile Arg Gln Ala Leu Ala Asn Ala Gly Leu Ser Val Ala
   770 775 780
   Asp Val Asp Val Val Glu Gly His Gly Thr Gly Thr Thr Leu Gly Asp
   785 790 795 800
   Pro Ile Glu Ala Gln Ala Leu Leu Ala Thr Tyr Gly Gln Arg Ala Gly
   805 810 815
   Asp Arg Pro Leu Trp Leu Gly Ser Leu Lys Ser Asn Ile Gly His Thr
   820 825 830
   Met Ala Ala Ala Gly Val Gly Gly Val Ile Lys Met Val Met Ala Leu
   835 840 845
   Arg Glu Gly Val Leu Pro Arg Thr Leu His Val Asp Lys Pro Ser Pro
   850 855 860
   Gln Val Asp Trp Ser Ala Gly Ala Val Arg Leu Leu Thr Glu Ala Val
   865 870 875 880
   Pro Trp Pro Gly Asp Ala Ala Gly Arg Leu Arg Arg Ala Gly Val Ser
   885 890 895
   Ser Phe Gly Ile Gly Gly Thr Asn Ala His Val Ile Leu Glu Glu Ala
   900 905 910
   Pro Ala Ala Gly Gly Cys Val Ala Gly Gly Gly Val Leu Glu Gly Ala
   915 920 925
   Pro Gly Leu Ala Ile Ser Val Ala Glu Ser Val Ala Ala Pro Val Ala
   930 935 940
   Val Ser Ala Pro Val Ala Glu Ser Val Pro Val Pro Val Pro Val Pro
   945 950 955 960
   Val Pro Val Pro Val Ser Ala Arg Ser Glu Ala Gly Leu Arg Ala Gln
   965 970 975
   Ala Glu Ala Leu Arg Gln Tyr Val Ala Val Arg Pro Asp Val Ser Leu
   980 985 990
   Ala Asp Val Gly Ala Gly Leu Ala Cys Gly Arg Ala Val Leu Glu His
   995 1000 1005
   Arg Ala Val Val Leu Ala Ala Asp Arg Glu Glu Leu Val Gln Gly Leu
   1010 1015 1020
   Gly Ala Leu Ala Ala Gly Glu Pro Asp Arg Arg Val Thr Thr Gly His
   1025 1030 1035 1040
   Ala Pro Gly Gly Asp Arg Gly Gly Val Val Phe Val Phe Pro Gly Gln
   1045 1050 1055
   Gly Gly Gln Trp Ala Gly Met Gly Val Arg Leu Leu Ala Ser Ser Pro
   1060 1065 1070
   Val Phe Ala Arg Arg Met Gln Ala Cys Glu Glu Ala Leu Ala Pro Trp
   1075 1080 1085
   Val Asp Trp Ser Val Val Asp Ile Leu Arg Arg Asp Ala Gly Asp Ala
   1090 1095 1100
   Val Trp Glu Arg Ala Asp Val Val Gln Pro Val Leu Phe Ser Val Met
   1105 1110 1115 1120
   Val Ser Leu Ala Ala Leu Trp Arg Ser Tyr Gly Ile Glu Pro Asp Ala
   1125 1130 1135
   Val Leu Gly His Ser Gln Gly Glu Ile Ala Ala Ala His Val Cys Gly
   1140 1145 1150
   Ala Leu Ser Leu Lys Asp Ala Ala Lys Thr Val Ala Leu Arg Ser Arg
   1155 1160 1165
   Ala Leu Ala Ala Val Arg Gly Arg Gly Gly Met Ala Ser Val Pro Leu
   1170 1175 1180
   Pro Ala Gln Glu Val Glu Gln Leu Ile Gly Glu Arg Trp Ala Gly Arg
   1185 1190 1195 1200
   Leu Trp Val Ala Ala Val Asn Gly Pro Arg Ser Thr Ala Val Ser Gly
   1205 1210 1215
   Asp Ala Glu Ala Val Asp Glu Val Leu Ala Tyr Cys Ala Gly Thr Gly
   1220 1225 1230
   Val Arg Ala Arg Arg Ile Pro Val Asp Tyr Ala Ser His Cys Pro His
   1235 1240 1245
   Val Gln Pro Leu Arg Glu Glu Leu Leu Glu Leu Leu Gly Asp Ile Ser
   1250 1255 1260
   Pro Gln Pro Ser Gly Val Pro Phe Phe Ser Thr Val Glu Gly Thr Trp
   1265 1270 1275 1280
   Leu Asp Thr Thr Thr Leu Asp Ala Ala Tyr Trp Tyr Arg Asn Leu His
   1285 1290 1295
   Gln Pro Val Arg Phe Ser Asp Ala Val Gln Ala Leu Ala Asp Asp Gly
   1300 1305 1310
   His Arg Val Phe Val Glu Val Ser Pro His Pro Thr Leu Val Pro Ala
   1315 1320 1325
   Ile Glu Asp Thr Thr Glu Asp Thr Ala Glu Asp Val Thr Ala Ile Gly
   1330 1335 1340
   Ser Leu Arg Arg Gly Asp Asn Asp Thr Arg Arg Phe Leu Thr Ala Leu
   1345 1350 1355 1360
   Ala His Thr His Thr Thr Gly Ile Gly Thr Pro Thr Thr Trp His His
   1365 1370 1375
   His Tyr Thr His His His Thr His Pro His Pro His Thr His Leu Asp
   1380 1385 1390
   Leu Pro Thr Tyr Pro Phe Gln His Gln His Tyr Trp Leu Glu Ser Ser
   1395 1400 1405
   Gln Pro Gly Ala Gly Ser Gly Ser Gly Ala Gly Ala Gly Ser Gly Ala
   1410 1415 1420
   Gly Ser Gly Arg Ala Gly Thr Ala Gly Gly Thr Ala Glu Val Glu Ser
   1425 1430 1435 1440
   Arg Phe Trp Asp Ala Val Ala Arg Gln Asp Leu Glu Thr Val Ala Thr
   1445 1450 1455
   Thr Leu Ala Val Pro Pro Ser Ala Gly Leu Asp Thr Val Val Pro Ala
   1460 1465 1470
   Leu Ser Ala Trp His Arg His Gln His Asp Gln Ala Arg Ile Asn Thr
   1475 1480 1485
   Trp Thr Tyr Gln Glu Thr Trp Lys Pro Leu Thr Leu Pro Thr Thr His
   1490 1495 1500
   Gln Pro His Gln Thr Trp Leu Ile Ala Ile Pro Glu Thr Gln Thr His
   1505 1510 1515 1520
   His Pro His Ile Thr Asn Ile Leu Thr Asn Leu His His His Gly Ile
   1525 1530 1535
   Thr Pro Ile Pro Leu Thr Leu Asn His Thr His Thr Asn Pro Gln His
   1540 1545 1550
   Leu His His Thr Leu His His Thr Arg Gln Gln Ala Gln Asn His Thr
   1555 1560 1565
   Thr Gly Ala Ile Thr Gly Leu Leu Ser Leu Leu Ala Leu Asp Glu Thr
   1570 1575 1580
   Pro His Pro His His Pro His Thr Pro Thr Gly Thr Leu Leu Asn Leu
   1585 1590 1595 1600
   Thr Leu Thr Gln Thr His Thr Gln Thr His Pro Pro Thr Pro Leu Trp
   1605 1610 1615
   Tyr Ala Thr Thr Asn Ala Thr Thr Thr His Pro Asn Asp Pro Leu Thr
   1620 1625 1630
   His Pro Thr Gln Ala Gln Thr Trp Gly Leu Ala Arg Thr Thr Leu Leu
   1635 1640 1645
   Glu His Pro Thr His Thr Ala Gly Ile Ile Asp Leu Pro Thr Thr Pro
   1650 1655 1660
   Thr Pro His Thr Leu Gln His Leu Thr Gln Thr Leu Thr Gln Pro His
   1665 1670 1675 1680
   His Gln Thr Gln Leu Ala Ile Arg Thr Thr Gly Thr His Thr Arg Arg
   1685 1690 1695
   Leu Thr Pro Thr Thr Leu Thr Pro Thr His Gln Pro Pro Thr Pro Thr
   1700 1705 1710
   Pro His Gly Thr Thr Leu Ile Thr Gly Gly Thr Gly Ala Leu Ala Thr
   1715 1720 1725
   His Leu Thr His His Leu Thr Thr His Gln Pro Thr Gln His Leu Leu
   1730 1735 1740
   Leu Thr Ser Arg Thr Gly Pro His Thr Pro His Ala Gln His Leu Thr
   1745 1750 1755 1760
   Thr Gln Leu Gln Gln Lys Gly Ile His Leu Thr Ile Thr Thr Cys Asp
   1765 1770 1775
   Thr Ser Asn Pro Asp Gln Leu Gln Gln Leu Leu Asn Thr Ile Pro Pro
   1780 1785 1790
   Gln His Pro Leu Thr Thr Val Ile His Thr Ala Gly Ile Leu Asp Asp
   1795 1800 1805
   Ala Thr Leu Thr Asn Leu Thr Pro Thr Gln Leu Asn Asn Val Leu Arg
   1810 1815 1820
   Ala Lys Ala His Ser Ala His Leu Leu His Gln Leu Thr Gln His Thr
   1825 1830 1835 1840
   Pro Leu Thr Ala Phe Val Leu Tyr Ser Ser Ala Ala Ala Thr Phe Gly
   1845 1850 1855
   Ala Pro Gly Gln Ala Asn Tyr Ala Ala Ala Asn Ala Tyr Leu Asp Ala
   1860 1865 1870
   Leu Ala His His Arg His Thr His His Leu Pro Ala Thr Ser Ile Ala
   1875 1880 1885
   Trp Gly Thr Trp Gln Gly Asn Gly Leu Ala Asp Ser Asp Lys Ala Arg
   1890 1895 1900
   Ala Tyr Leu Asp Arg Arg Gly Phe Arg Pro Met Ser Pro Glu Leu Ala
   1905 1910 1915 1920
   Thr Ala Ala Val Thr Gln Ala Ile Ala Asp Thr Glu Arg Pro Tyr Val
   1925 1930 1935
   Val Ile Ala Asp Ile Asp Trp Ser Lys Ile Glu His Thr Ser Gln Thr
   1940 1945 1950
   Ser Asp Leu Val Ser Ala Ala Arg Glu Arg Glu Pro Ala Val Gln Arg
   1955 1960 1965
   Pro Thr Pro Pro Ala Glu Leu His Lys Thr Leu Ala His Gln Thr Ser
   1970 1975 1980
   Ala Asp Gln Arg Ala Ala Leu Leu Glu Leu Val Arg Asp His Val Ala
   1985 1990 1995 2000
   Ala Val Leu Arg His Ala Asp Pro Lys Ala Ile Ala Pro Asp Gln Ser
   2005 2010 2015
   Phe Arg Ala Leu Gly Phe Asp Ser Leu Thr Ala Val Glu Phe Arg Asn
   2020 2025 2030
   Leu Leu Ile Lys Ala Thr Gly Leu Arg Leu Pro Val Ser Leu Val Phe
   2035 2040 2045
   Asp His Pro Thr Pro Ala Lys Leu Ala Val His Leu Gln Asn Gln Leu
   2050 2055 2060
   Arg Gly Thr Ala Ala Glu Ser Ala Pro Ser Ala Ala Ala Val Thr Ala
   2065 2070 2075 2080
   Glu Ala Ser Val Thr Glu Pro Ile Ala Ile Val Gly Met Ala Cys Arg
   2085 2090 2095
   Phe Pro Gly Gly Val Thr Ser Ala Asp Asp Phe Trp Asp Leu Ile Ser
   2100 2105 2110
   Ser Glu Gln Asp Ala Ile Gly Gly Phe Pro Thr Asp Arg Gly Trp Asp
   2115 2120 2125
   Leu Asp Thr Leu Tyr Asp Pro Asp Pro Asp His Pro Gly Thr Cys Tyr
   2130 2135 2140
   Thr Arg Asn Gly Gly Phe Leu Tyr Asp Ala Gly His Phe Asp Ala Glu
   2145 2150 2155 2160
   Phe Phe Gly Ile Ser Pro Arg Glu Ala Leu Ala Met Asp Pro Gln Gln
   2165 2170 2175
   Arg Leu Leu Leu Glu Thr Ala Trp Glu Thr Ile Glu His Ala Gly Ile
   2180 2185 2190
   Asn Pro His Thr Leu His Gly Thr Pro Thr Gly Val Phe Thr Gly Thr
   2195 2200 2205
   Asn Gly Gln Asp Tyr Ala Leu Arg Val His Asn Ala Gly Gln Ser Thr
   2210 2215 2220
   Asp Gly Phe Ala Leu Thr Gly Thr Ala Gly Ser Val Ile Ser Gly Arg
   2225 2230 2235 2240
   Ile Ser Tyr Thr Phe Gly Phe Glu Gly Pro Ala Val Ser Val Asp Thr
   2245 2250 2255
   Ala Cys Ser Ser Ser Leu Val Ala Leu His Leu Ala Cys Gln Ala Leu
   2260 2265 2270
   Arg Ala Gly Glu Cys Ser Met Ala Leu Ala Gly Gly Val Thr Val Met
   2275 2280 2285
   Ser Ser Pro Gly Ala Phe Val Glu Phe Ser Arg Gln Arg Gly Leu Ala
   2290 2295 2300
   Ala Asp Gly His Cys Lys Ala Phe Ser Ala Ala Ala Asp Gly Thr Gly
   2305 2310 2315 2320
   Trp Gly Glu Gly Val Gly Met Leu Leu Val Glu Arg Leu Ser Asp Ala
   2325 2330 2335
   His Arg Asn Gly His Arg Val Leu Ala Val Val Arg Gly Ser Ala Val
   2340 2345 2350
   Asn Gln Asp Gly Ala Ser Asn Gly Leu Thr Ala Pro Asn Gly Pro Ser
   2355 2360 2365
   Gln Gln Arg Val Ile Arg Gln Ala Leu Ala Asn Ala Gly Leu Ser Ala
   2370 2375 2380
   Gly Asp Val Asp Ala Val Glu Ala His Gly Thr Gly Thr Thr Leu Gly
   2385 2390 2395 2400
   Asp Pro Ile Glu Ala Gln Ala Leu Leu Ala Thr Tyr Gly Gln Asp Arg
   2405 2410 2415
   Ala Gly Glu Gly Pro Leu Trp Leu Gly Ser Val Lys Ser Asn Val Gly
   2420 2425 2430
   His Thr Gln Ala Ala Ala Gly Val Ala Gly Val Ile Lys Met Val Met
   2435 2440 2445
   Ala Leu Arg His Gly Leu Leu Pro Arg Thr Leu His Val Asp Glu Pro
   2450 2455 2460
   Ser Pro His Val Asp Trp Ser Ala Gly Ala Val Gln Leu Leu Thr Glu
   2465 2470 2475 2480
   Thr Val Pro Trp Pro Gly Gly Glu Gly Arg Leu Arg Arg Ala Gly Val
   2485 2490 2495
   Ser Ser Phe Gly Val Ser Gly Thr Asn Ala His Val Ile Leu Glu Glu
   2500 2505 2510
   Ala Pro Ala Asp Asp Val Pro Gly Gly Pro Pro Ala Gly Glu Gly Asp
   2515 2520 2525
   Ala Gly Ser Asp Asp Glu Ala Ala Ala Gly Ser Pro Gly Val Trp Pro
   2530 2535 2540
   Trp Leu Val Ser Ala Lys Ser Gln Pro Ala Leu Arg Ala Gln Ala Gln
   2545 2550 2555 2560
   Ala Leu His Ala His Leu Thr Asp His Pro Gly Leu Asp Leu Ala Asp
   2565 2570 2575
   Val Gly Tyr Thr Leu Ala His Ala Arg Ala Val Phe Asp His Arg Ala
   2580 2585 2590
   Thr Leu Ile Ala Ala Asp Arg Asp Thr Phe Leu Gln Ala Leu Gln Ala
   2595 2600 2605
   Leu Ala Ala Gly Glu Pro His Pro Ala Val Ile His Ser Ser Ala Pro
   2610 2615 2620
   Gly Gly Thr Gly Thr Gly Glu Ala Ala Gly Lys Thr Ala Phe Ile Cys
   2625 2630 2635 2640
   Ser Gly Gln Gly Thr Gln Arg Pro Gly Met Ala His Gly Leu Tyr His
   2645 2650 2655
   Thr His Pro Val Phe Ala Ala Ala Leu Asn Asp Ile Cys Thr His Leu
   2660 2665 2670
   Asp Pro His Leu Asp His Pro Leu Leu Pro Leu Leu Thr Gln Asn Asp
   2675 2680 2685
   Asn Asp Asn Glu Asp Ala Ala Ala Leu Leu Gln Gln Thr Arg Tyr Ala
   2690 2695 2700
   Gln Pro Ala Leu Phe Ala Phe Gln Val Ala Leu His Arg Leu Leu Thr
   2705 2710 2715 2720
   Asp Gly Tyr His Ile Thr Pro His Tyr Tyr Ala Gly His Ser Leu Gly
   2725 2730 2735
   Glu Ile Thr Ala Ala His Leu Ala Gly Ile Leu Thr Leu Thr Asp Ala
   2740 2745 2750
   Thr Thr Leu Ile Thr Gln Arg Ala Thr Leu Met Gln Thr Met Pro Pro
   2755 2760 2765
   Gly Thr Met Thr Thr Leu His Thr Thr Pro His His Ile Thr His His
   2770 2775 2780
   Leu Thr Ala His Glu Asn Asp Leu Ala Ile Ala Ala Ile Asn Thr Pro
   2785 2790 2795 2800
   Thr Ser Leu Val Ile Ser Gly Thr Pro His Thr Val Gln His Ile Thr
   2805 2810 2815
   Thr Leu Cys Gln Gln Gln Gly Ile Lys Thr Lys Thr Leu Pro Thr Asn
   2820 2825 2830
   His Ala Phe His Ser Pro His Thr Asn Pro Ile Leu Asn Gln Leu His
   2835 2840 2845
   Gln His Thr Gln Thr Leu Thr Tyr His Pro Pro His Thr Pro Leu Ile
   2850 2855 2860
   Thr Ala Asn Thr Pro Pro Asp Gln Leu Leu Thr Pro His Tyr Trp Thr
   2865 2870 2875 2880
   Gln Gln Ala Arg Asn Thr Val Asp Tyr Ala Thr Thr Thr Gln Thr Leu
   2885 2890 2895
   His Gln His Gly Val Thr Thr Tyr Ile Glu Leu Gly Pro Asp Asn Thr
   2900 2905 2910
   Leu Thr Thr Leu Thr His His Asn Leu Pro Asn Pro Pro Thr Thr Thr
   2915 2920 2925
   Leu Thr Leu Thr His Pro His His His Pro Gln Thr His Leu Leu Thr
   2930 2935 2940
   Asn Leu Ala Lys Thr Thr Thr Thr Trp His Pro His His Tyr Thr His
   2945 2950 2955 2960
   His Asp Asn Gln Pro His Thr His Thr His Leu Asp Leu Pro Thr Tyr
   2965 2970 2975
   Pro Phe Gln His His His Tyr Trp Leu Glu Ser Thr Gln Pro Gly Ala
   2980 2985 2990
   Gly Asn Val Ser Ala Ala Gly Leu Asp Pro Thr Glu His Pro Leu Leu
   2995 3000 3005
   Gly Ala Thr Leu Glu Leu Ala Thr Asp Gly Gly Ala Leu Leu Ala Gly
   3010 3015 3020
   Arg Leu Ser Leu Arg Ser His Pro Trp Leu Ala Asp His Ala Val Gly
   3025 3030 3035 3040
   Gly Thr Val Leu Leu Ser Gly Ala Thr Phe Leu Glu Leu Ala Leu His
   3045 3050 3055
   Ala Gly Thr Tyr Val Gly Cys Asp Arg Val Asp Glu Leu Thr Leu His
   3060 3065 3070
   Ala Pro Leu Val Val Pro Val Asp Gly Gly Val Ser Val Gln Val Gly
   3075 3080 3085
   Val Ala Ala Ala Asp Gly Glu Gly Arg Arg Leu Val Ser Val Tyr Ala
   3090 3095 3100
   Arg Gly Gly Ser Ala Cys Gly Gly Gly Gly Ala Ser Gly Gly Val Trp
   3105 3110 3115 3120
   Thr Cys His Ala Ser Gly Val Leu Val Glu Ala Ala Ala Gly Gly Val
   3125 3130 3135
   Val Val Asp Gly Leu Ala Gly Val Trp Pro Pro Arg Gly Ala Val Ala
   3140 3145 3150
   Val Asp Val Asp Gly Val Arg Asp Arg Leu Ala Gly Ala Gly Cys Val
   3155 3160 3165
   Leu Gly Pro Val Phe Ser Gly Leu Arg Ala Val Trp Arg Asp Gly Gly
   3170 3175 3180
   Asp Leu Leu Ala Glu Val Cys Leu Pro Glu Glu Ala Trp Gly Asp Ala
   3185 3190 3195 3200
   Ala Gly Phe Gly Leu His Pro Ala Leu Leu Asp Gly Val Val Gln Pro
   3205 3210 3215
   Leu Ser Val Leu Leu Pro Gly Gly Thr Gly Phe Gly Glu Gly Ala Gly
   3220 3225 3230
   Phe Gly Glu Gly Val Arg Val Pro Ala Val Trp Gly Gly Val Ser Leu
   3235 3240 3245
   His Arg Ala Gly Val Thr Gly Val Arg Val Arg Val Ser Ala Val Gly
   3250 3255 3260
   Arg Gly Gly Gly Arg Glu Ala Val Ser Val Val Val Gly Asp Glu Ala
   3265 3270 3275 3280
   Gly Val Pro Val Ala Ser Val Asp Arg Leu Glu Leu Arg Pro Val Asp
   3285 3290 3295
   Met Gly Gln Leu Arg Ala Val Ser Val Ser Ala Gly Arg Arg Gly Ser
   3300 3305 3310
   Leu Tyr Ala Val Gln Trp Ala Glu Val Gly Pro Val Pro Val Cys Gly
   3315 3320 3325
   Gln Ala Trp Ala Trp His Glu Asp Val Gly Glu Ser Gly Gly Gly Pro
   3330 3335 3340
   Val Pro Gly Val Val Val Leu Arg Cys Pro Asp Ala Gly Ala Gly Gly
   3345 3350 3355 3360
   Gly Gly Gly Gly Gly Gly Gly Gly Gly Val Gly Glu Val Val Gly Gly
   3365 3370 3375
   Val Leu Gly Val Val Gln Gly Trp Leu Gly Leu Glu Arg Phe Ala Gly
   3380 3385 3390
   Ser Arg Leu Val Val Val Thr Arg Gly Ala Val Val Ala Gly Pro Glu
   3395 3400 3405
   Asp Gly Pro Val Asp Val Val Gly Ala Ser Val Trp Gly Leu Val Arg
   3410 3415 3420
   Ser Ala Gln Ala Glu His Pro Asp Arg Phe Val Leu Leu Asp Leu Asp
   3425 3430 3435 3440
   Thr Asp Thr Gly Thr Asp Leu Asp Thr Gly Ala Gly Ala Gly Trp Gly
   3445 3450 3455
   Val Asp Gly Gly Arg Val Ala Ala Val Val Ala Cys Gly Glu Pro Gln
   3460 3465 3470
   Leu Ala Val Arg Gly Glu Arg Leu Leu Ala Ala Arg Leu Lys Arg Leu
   3475 3480 3485
   Glu Ser Ser Gly Asp Val Pro Ala Gln Arg Ser Gly Asp Thr Arg Ala
   3490 3495 3500
   Arg Arg Ser Asp Val Pro Ala Gln Arg Ser Gly Gly Val Pro Ala Arg
   3505 3510 3515 3520
   Arg Ser Val Asp Val Ser Gly Arg Glu Val Leu Pro Trp Leu Ser Gly
   3525 3530 3535
   Gly Ser Val Leu Val Thr Gly Gly Thr Gly Val Leu Gly Ala Ala Val
   3540 3545 3550
   Ala Arg His Leu Ala Gly Val Cys Gly Val Arg Asp Leu Leu Leu Val
   3555 3560 3565
   Ser Arg Arg Gly Pro Asp Ala Pro Gly Ala Glu Gly Leu Arg Ala Glu
   3570 3575 3580
   Leu Ala Ala Leu Gly Ala Glu Val Arg Ile Val Ala Cys Asp Val Gly
   3585 3590 3595 3600
   Glu Arg Arg Glu Val Val Arg Leu Leu Glu Gly Val Pro Ala Gly Cys
   3605 3610 3615
   Pro Leu Thr Gly Val Val His Ala Ala Gly Val Leu Asp Asp Ala Thr
   3620 3625 3630
   Ile Ala Ser Leu Thr Pro Glu Arg Leu Gly Thr Val Phe Ala Ala Lys
   3635 3640 3645
   Val Asp Ala Ala Leu Leu Leu Asp Glu Leu Thr Arg Gly Met Glu Leu
   3650 3655 3660
   Ser Ala Phe Val Leu Phe Ser Ser Ala Ala Gly Ile Leu Gly Ser Ala
   3665 3670 3675 3680
   Gly Gln Gly Asn Tyr Ala Ala Ala Asn Ala Ala Leu Asp Ala Leu Ala
   3685 3690 3695
   Tyr Arg Arg Arg Ala Ala Gly Leu Pro Gly Val Ser Leu Ala Trp Gly
   3700 3705 3710
   Leu Trp Glu Glu Ala Ser Gly Met Thr Gly His Leu Ala Gly Thr Asp
   3715 3720 3725
   His Arg Arg Ile Ile Arg Ser Gly Leu His Pro Met Ser Thr Pro Asp
   3730 3735 3740
   Ala Leu Ala Leu Phe Asp Ala Ala Leu Ala Leu Asp Arg Pro Val Leu
   3745 3750 3755 3760
   Leu Pro Ala Asp Leu Arg Pro Ala Pro Pro Leu Pro Pro Leu Leu Gln
   3765 3770 3775
   Asp Leu Leu Pro Ala Thr Arg Arg Arg Thr Thr Arg Thr Thr Thr Thr
   3780 3785 3790
   Gly Gly Ala Asp Asn Gly Ala Gln Leu His Ala Arg Leu Ala Gly Gln
   3795 3800 3805
   Thr His Glu Gln Gln His Thr Thr Leu Leu Ala Leu Val Arg Ser His
   3810 3815 3820
   Ile Ala Thr Val Leu Gly His Thr Thr Pro Asp Thr Ile Pro Pro Asp
   3825 3830 3835 3840
   Arg Ala Phe Arg Asp Leu Gly Phe Asp Ser Leu Thr Ala Val Glu Leu
   3845 3850 3855
   Arg Asn Arg Leu Ser Arg Thr Thr Gly Leu Arg Leu Pro Thr Thr Leu
   3860 3865 3870
   Ala Phe Asp His Pro Asn Pro Thr Thr Leu Thr His His Leu His Thr
   3875 3880 3885
   Gln Leu Gln Pro Gln Pro Asp Asn Ala Val Ala Pro Val Leu Ala Glu
   3890 3895 3900
   Leu Asp Lys Leu Glu Ser Ala Leu Ser Ala Leu Asp Lys Thr Asp Ser
   3905 3910 3915 3920
   Ala Ser Glu Arg Val Thr Leu Arg Leu Lys Ser Leu Met Leu Arg Trp
   3925 3930 3935
   Asn Ala Pro Gln His Pro Thr Ala Glu Ser Ala Asp Asp Asp Glu Lys
   3940 3945 3950
   Phe Thr Ser Ala Thr Glu Ala Glu Ile Phe Lys Phe Ile Asp Asn Asp
   3955 3960 3965
   Leu Gly Leu Ser
   3970
<210> 9
   <211> 32
   <212> DNA
   <213> Artificial Sequence
   <223> This is a primer based on the sequence between 1954 and 1985 of SEQ ID NO: 1.
<400> 9
   accgtggaca cggggggctc ggcatcgctc gt 32
<210> 10
   <211> 28
   <212> DNA
   <213> Artificial Sequence
   <223> This is an antisense primer based on the sequence between 1758 and 1776 of SEQ ID N0:1.
<400> 10
   ataagcttaa tcgatccgct gtccggta 28
<210> 11
   <211> 30
   <212> DNA
   <213> Artificial Sequence
   <223> This is an antisense primer based on the sequence between 2710 and 2729 of SEQ ID NO:1.
<400> 11
   atgaattccc tccaaaatca catgcgcatt 30